# EUROPEAN PATENT APPLICATION

(11) **EP 4 745 117 A1**
(43) Date of publication of application: **20.05.2026**
(21) Application number: 24913692.0
(22) Date of filing: 27.12.2024
(51) Int. Cl.: C07C 49/697, C07C 49/753, C07D 213/50, C07D 295/116, A61K 31/122, A61K 31/4412, A61P 25/28, A23L 33/10

(54) **NOVEL INDENONE DERIVATIVE AND USE THEREOF**

(30) Priority: 28.12.2023 KR 20230193884
(71) Applicant: Amyloid Solution Inc., Seongnam-si, Gyeonggi-do 13486 (KR)
(72) Inventor: PARK, Seong Jin, Seoul 03024 (KR); KIM, Seong Muk, Seoul 07651 (KR); CHANG, Hye Kyung, Seongnam-si, Gyeonggi-do 13486 (KR)
(74) Representative: Vossius & Partner Patentanwälte Rechtsanwälte mbB
(86) International application number: PCT/KR2024/021306
(87) International publication number: WO 2025/143888

(57) **Abstract**

One aspect provides novel indenone derivatives or a salt thereof, and uses thereof. According to one aspect, the novel indenone derivatives exhibit excellent efficacy in inhibiting aggregation of an amyloid-beta protein and/or disaggregating an aggregate of an amyloid-beta protein, inhibiting aggregation of a tau protein and/or disaggregating an aggregate of a tau protein, and/or inhibiting phosphorylation of a tau protein, and thus can be usefully utilized for the prevention or treatment of a neurodegenerative disease.

## Description

### Technical Field

The present invention relates to novel indenone derivatives or a salt thereof; a use thereof for (i) inhibiting aggregation of an amyloid-beta protein and/or disaggregating an aggregate of an amyloid-beta protein, (ii) inhibiting aggregation of a tau protein, disaggregating an aggregate of a tau protein, and/or inhibiting phosphorylation of a tau protein; and a use for preventing or treating a neurodegenerative brain disease.

### Background Art

A neurodegenerative disease is a disease that causes various symptoms, such as impairment of motor and sensory functions and inhibition of higher-order cognitive functions such as memory, learning, calculation, and reasoning, as degenerative changes appear in neurons of the central nervous system. Representative diseases include Alzheimer's disease, Parkinson's disease, and memory disorders. In neurodegenerative diseases, death of neurons occurs due to rapidly or slowly progressing necrosis or apoptosis. Therefore, an understanding of the mechanism of neuronal death is required for the development of methods for preventing, controlling, and treating central nervous system diseases.

As causative substances that induce neurodegenerative diseases, two types of proteins, namely amyloid-beta and Tau protein, have attracted attention.

Human amyloid-beta is a peptide molecule containing about 36-43 amino acids, and its self-assembly into oligomers or aggregates is known to be involved in the pathogenesis of neurodegenerative diseases such as Alzheimer's disease. Specifically, amyloid-beta peptide molecules are obtained by cleaving amyloid precursor protein (APP; UniProtKB P05067) with beta secretase and gamma secretase, and these amyloid-beta peptide molecules cause neurodegenerative diseases by aggregating to form neurotoxic oligomers.

In addition, the Tau protein is composed of four parts: an N-terminal projection domain, a proline-rich domain, a microtubule-binding domain, and a C-terminus, and it is known to cause neurodegenerative diseases such as Parkinson's disease and tauopathy when it is abnormally hyperphosphorylated or aggregated in the neurons of the central nervous system.

Therefore, a substance that inhibits the aggregation of amyloid-beta protein, disaggregates aggregates of amyloid-beta protein, inhibits the aggregation of tau protein, disaggregates aggregates of tau protein, or inhibits the phosphorylation of tau protein can be proposed as a therapeutic agent for neurodegenerative diseases.

### Disclosure of of Invention

### Technical Problem

One aspect provides a compound of Formula 1 below or a salt thereof:

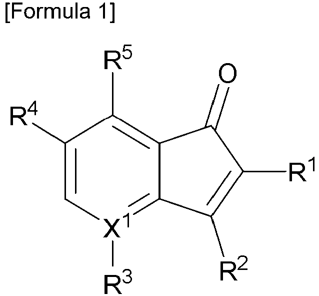

In the Formula 1,
R¹ and R² are each independently hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X¹ is carbon or nitrogen;
wherein when the X¹ is carbon, R³ is hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl, and when the X¹ is nitrogen, R³ is absent;
R⁴ and R⁵ are each independently hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
R^{a}, R^{b}, R^{c}, and R^{d} are each independently hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X^{a} and X^{b} are each independently a direct bond, oxygen, nitrogen, or sulfur; and
p and q are each independently an integer selected from 0 to 2.

Another aspect provides a composition including the compound of Formula 1, or a salt thereof.

Another aspect provides a pharmaceutical composition including the compound of Formula 1, or a pharmaceutically acceptable salt thereof.

Another aspect provides a use of the compound of Formula 1, or a pharmaceutically acceptable salt thereof.

Another aspect provides a use of the compound of Formula 1, or a pharmaceutically acceptable salt thereof, for the manufacture of a medicament.

Another aspect provides a pharmaceutical composition for preventing or treating a neurodegenerative brain disease, the pharmaceutical composition including the compound of Formula 1, or a pharmaceutically acceptable salt thereof.

Another aspect provides a use of the compound of Formula 1, or a pharmaceutically acceptable salt thereof, for preventing or treating a neurodegenerative brain disease.

Another aspect provides a use of the compound of Formula 1, or a pharmaceutically acceptable salt thereof, for the manufacture of a medicament for preventing or treating a neurodegenerative brain disease.

Another aspect provides a method of preventing or treating a neurodegenerative brain disease, including administering the compound of Formula 1, or a pharmaceutically acceptable salt thereof, to a subject in need thereof.

Another aspect provides a pharmaceutical composition for inhibiting aggregation of an amyloid-beta protein or disaggregating an aggregate of an amyloid-beta protein, the pharmaceutical composition including the compound of Formula 1, or a pharmaceutically acceptable salt thereof.

Another aspect provides a use of the compound of Formula 1, or a pharmaceutically acceptable salt thereof, for inhibiting aggregation of an amyloid-beta protein or disaggregating an aggregate of an amyloid-beta protein.

Another aspect provides a use of the compound of Formula 1, or a pharmaceutically acceptable salt thereof, for the manufacture of a medicament for inhibiting aggregation of an amyloid-beta protein or disaggregating an aggregate of an amyloid-beta protein.

Another aspect provides a method of inhibiting aggregation of an amyloid-beta protein or disaggregating an aggregate of an amyloid-beta protein, the method including administering the compound of Formula 1, or a pharmaceutically acceptable salt thereof, to a subject in need thereof.

Another aspect provides a pharmaceutical composition for inhibiting aggregation of a tau protein, disaggregating an aggregate of a tau protein, or inhibiting phosphorylation of a tau protein, the pharmaceutical composition including the compound of Formula 1, or a pharmaceutically acceptable salt thereof.

Another aspect provides a use of the compound of Formula 1, or a pharmaceutically acceptable salt thereof, for inhibiting aggregation of a tau protein, disaggregating an aggregate of a tau protein, or inhibiting phosphorylation of a tau protein.

Another aspect provides a use of the compound of Formula 1, or a pharmaceutically acceptable salt thereof, for the manufacture of a medicament for inhibiting aggregation of a tau protein, disaggregating an aggregate of a tau protein, or inhibiting phosphorylation of a tau protein.

Another aspect provides a method of inhibiting aggregation of a tau protein, disaggregating an aggregate of a tau protein, or inhibiting phosphorylation of a tau protein, the method including administering the compound of Formula 1, or a pharmaceutically acceptable salt thereof, to a subject in need thereof.

Another aspect provides a health functional food including the compound of Formula 1, or a food acceptable salt thereof.

Another aspect provides a health functional food for preventing or ameliorating a neurodegenerative brain disease, the health functional food including the compound of Formula 1, or a food acceptable salt thereof.

Another aspect provides a health functional food for inhibiting aggregation of an amyloid-beta protein or disaggregating an aggregate of an amyloid-beta protein, the health functional food including the compound of Formula 1, or a food acceptable salt thereof.

Another aspect provides a health functional food for inhibiting aggregation of a tau protein, disaggregating an aggregate of a tau protein, or inhibiting phosphorylation of a tau protein, the health functional food including the compound of Formula 1, or a food acceptable salt thereof.

### Solution of Problem

One aspect provides a compound of Formula 1 below, or a salt thereof:

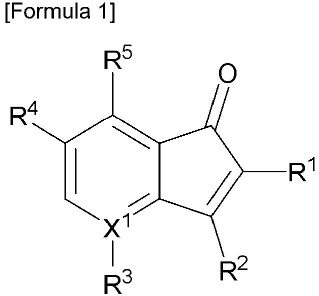

In the Formula 1,
R¹ and R² are each independently hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X¹ is carbon or nitrogen;
wherein when the X¹ is carbon, R³ is hydrogen, halogen, CN, OR^{a}, SR^{a}, CO₂R^{a}, CH₂CO₂R^{a}, CONR^{b}R^{c}, NRbRc, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl, and when the X¹ is nitrogen, R³ is absent;
R⁴ and R⁵ are each independently hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
R^{a}, R^{b}, R^{c}, and R^{d} are each independently hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X^{a} and X^{b} are each independently a direct bond, oxygen, nitrogen, or sulfur; and
p and q are each independently an integer selected from 0 to 2.

The compound or a salt thereof may have one or more chiral carbon centers, and thus may exist as R or S isomers, racemates, mixtures of diastereomers, and individual diastereomers.

In addition, the compound of Formula 1 may include hydrates or solvates of the compound. The hydrates and solvates may be prepared using known methods, and are preferably nontoxic and water-soluble.

The term "salt" refers to a salt prepared from a specific compound according to one aspect using relatively nontoxic acids or bases.

The term "Cₘ₋ₙ" (wherein m and n are each independently an integer of 1 or more) means having m to n carbon atoms.

The term "halogen" refers to an element belonging to Group 17 of the periodic table. For example, it includes fluorine (F), chlorine (CI), bromine (Br), iodine (I), and the like.

The term "alkyl" refers to a straight- or branched-chain saturated hydrocarbon group. For example, "C₁₋₆ alkyl" refers to a straight- or branched-chain saturated hydrocarbon group having 1, 2, 3, 4, 5, or 6 carbon atoms. Specifically, C₁₋₆ alkyl includes methyl, ethyl, n-propyl, isopropyl, n-butyl, isobutyl, tert-butyl, sec-butyl, n-pentyl, isopentyl, neopentyl, tert-pentyl, 1-methylbutyl, n-hexyl, 1-methylpentyl, 2-methylpentyl, 4-methyl-2-pentyl, 3,3-dimethylbutyl, 2-ethylbutyl, and the like. In addition, the alkyl may be unsubstituted or substituted with one or more identical or different substituents. For example, the alkyl may be substituted with substituted or unsubstituted 3- to 7-membered cycloalkyl, substituted or unsubstituted 3- to 7-membered heterocycloalkyl, substituted or unsubstituted 5- to 10-membered aryl, substituted or unsubstituted 5- to 10-membered heteroaryl, and the like.

The term "cycloalkyl" refers to a monocyclic or polycyclic saturated hydrocarbon ring. The polycyclic ring structure may include multiple ring structures such as spiro, bridged, and fused ring structures. For example, a "3- to 7-membered cycloalkyl" may be cyclopropyl, cyclobutyl, cyclopentyl, cyclohexyl, or cycloheptyl. In addition, the cycloalkyl may be unsubstituted or substituted with one or more identical or different substituents. For example, the cycloalkyl may be substituted with substituted or unsubstituted C₁₋₆ alkyl, and the like.

The term "heterocycloalkyl" refers to a monocyclic or polycyclic ring in which at least one carbon atom forming the ring of a saturated hydrocarbon ring is replaced by a heteroatom. The heteroatom may be nitrogen (N), oxygen (O), or sulfur (S). In this case, the heteroatom(s) included in the ring of the heterocycloalkyl may be of one type or two or more types; one or more heteroatoms of a single type may be included, or at least one of each of two or more types of heteroatoms may also be included. The heterocycloalkyl may be connected to the parent structure through a carbon atom while containing a heteroatom within the ring, or it may be connected through a heteroatom (in this case, if there are two or more heteroatoms, the ring also contains a heteroatom). For example, the heterocycloalkyl may include azetidinyl, azepanyl, oxiranyl, oxetanyl, morpholinyl, pyrrolidinyl, piperidinyl, piperazinyl, tetrahydrofuranyl, tetrahydrothiophenyl, tetrahydropyranyl, tetrahydrothiopyranyl, and the like. In addition, the heterocycloalkyl may be unsubstituted or substituted with one or more identical or different substituents. For example, it may be substituted with substituted or unsubstituted C₁₋₆ alkyl, substituted or unsubstituted 5- to 10-membered aryl, substituted or unsubstituted 5- to 10-membered heteroaryl, and the like.

The term "aryl" refers to a monocyclic or polycyclic aromatic hydrocarbon ring. For example, the aryl may include phenyl, naphthyl, biaryl, and the like. In addition, the aryl may be unsubstituted or substituted with one or more identical or different substituents. For example, the aryl may be substituted with substituted or unsubstituted C₁₋₆ alkyl, substituted or unsubstituted 5- to 10-membered aryl, substituted or unsubstituted 5- to 10-membered heteroaryl, substituted or unsubstituted C₁₋₃ alkoxy, -CONH(C₁₋₃ alkyl), and the like.

The term "heteroaryl" refers to a monocyclic or polycyclic ring in which at least one carbon atom of the aforementioned aryl is replaced by a heteroatom such as nitrogen (N), oxygen (O), or sulfur (S). In this case, the heteroatom(s) included in the ring of the heteroaryl may be of one type or two or more types; one or more heteroatoms of a single type may be included, or at least one of each of two or more types of heteroatoms may also be included. In addition, the heteroaryl may be connected to the parent structure through a carbon atom while containing a heteroatom within the ring, or it may be connected through a heteroatom (in this case, if there are two or more heteroatoms, the ring also contains a heteroatom). For example, the heteroaryl may include pyridinyl, thiophenyl, triazolyl, tetrazolyl, benzothiazolyl, benzothiophenyl, quinolinyl, indolyl, isoindolyl, benzofuranyl, benzopyrrolyl, furanyl, pyrrolyl, thiazolyl, isothiazolyl, imidazolyl, pyrazolyl, oxazolyl, oxadiazolyl, isooxazolyl, pyrazinyl, pyridazinyl, pyrimidinyl, isoquinolinyl, benzoxazolyl, benzimidazolyl, dihydrobenzothiophenyl, purinyl, indolizinyl, chromenyl, pyrrolopyridinyl, pyrazolopyridinyl, thiadiazolopyridinyl, triazinyl, triazolopyrimidinyl, triazolopyridinyl, triazolopyridazinyl, indazolyl, imidazopyridinyl, imidazopyridazinyl, oxadiazolopyridinyl, benzothiadiazolyl, benzotriazolyl, and the like. The heteroaryl may be unsubstituted or substituted with one or more identical or different substituents. For example, the heteroaryl may be substituted with substituted or unsubstituted C₁₋₆ alkyl, substituted or unsubstituted 5- to 10-membered aryl, substituted or unsubstituted 5- to 10-membered heteroaryl, substituted or unsubstituted C₁₋₃ alkoxy, - CONH(C₁₋₃ alkyl), and the like.

The term "C₁₋₃ alcohol" refers to the formula -C₁₋₃ alkyl-OH, and includes methyl alcohol, ethyl alcohol, n-propyl alcohol, isopropyl alcohol, and the like. The C₁₋₃ alcohol may be unsubstituted or substituted with one or more identical or different substituents. For example, the C₁₋₃ alcohol may be substituted with substituted or unsubstituted C₁₋₆ alkyl, and the like.

The term "C₁₋₃ alkoxy" refers to the formula -O-C₁₋₃ alkyl, and for example, includes methoxy, ethoxy, n-propoxy, isopropoxy, and the like. The C₁₋₃ alkoxy may be unsubstituted or substituted with one or more identical or different substituents. For example, the C₁₋₃ alkoxy may be substituted with substituted or unsubstituted C₁₋₆ alkyl, and the like.

The term "substitution" refers to the replacement of a hydrogen atom in a molecular structure with a substituent, such that the substitution results in a chemically stable compound without exceeding the valence of the specified atom. For example, the phrase "group A is substituted with substituent B" may mean that a hydrogen atom bonded to an atom, such as carbon, that constitutes the backbone of group A is replaced by substituent B, thereby forming a covalent bond between group A and substituent B.

In an embodiment, in the Formula 1 above, the R¹ and R² may each independently be hydrogen, halogen, CN, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the X¹ may be carbon or nitrogen;
wherein when the X¹ is carbon, the R³ may be hydrogen, halogen, OR^{a}, or substituted or unsubstituted C₁₋₆ alkyl, and when the X¹ is nitrogen, the R³ may be absent;
the R⁴ and R⁵ may each independently be hydrogen, halogen, OR^{a}, SR^{a}, O(CO)R^{a}, NHR^{a}, NH(CO)R^{a}, NR^{b}R^{c}, X^{a}(CH₂)ₚX^{b}(CH₂)_{q}R^{d}, substituted or unsubstituted C₁₋₆ alkyl, substituted or unsubstituted 3- to 7-membered cycloalkyl, or substituted or unsubstituted 3-to 7-membered heterocycloalkyl; and
the R^{a}, R^{b}, R^{c}, and R^{d} may each independently be hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl.

In an embodiment, in the Formula 1 above, the R¹ and R² may each independently be hydrogen, halogen, CN, substituted or unsubstituted 5- to 7-membered cycloalkyl, substituted or unsubstituted 5- to 7-membered heterocycloalkyl, substituted or unsubstituted 5- to 6-membered aryl, or substituted or unsubstituted 5- to 6-membered heteroaryl;
the X¹ may be carbon or nitrogen;
wherein when the X¹ is carbon, the R³ may be hydrogen, halogen, OR^{a}, or substituted or unsubstituted C₁₋₃ alkyl, and when the X¹ is nitrogen, the R³ may be absent;
the R⁴ may be hydrogen, halogen, OR^{a}, SR^{a}, O(CO)R^{a}, NHR^{a}, NH(CO)R^{a}, NR^{b}R^{c}, X^{a}(CH₂)ₚX^{b}(CH₂)_{q}R^{d}, substituted or unsubstituted C₁₋₄ alkyl, substituted or unsubstituted 5-to 7-membered cycloalkyl, or substituted or unsubstituted 5- to 7-membered heterocycloalkyl;
the R^{a}, R^{b}, R^{c}, and R^{d} may each independently be hydrogen, halogen, substituted or unsubstituted C₁₋₄ alkyl, substituted or unsubstituted 3- to 7-membered cycloalkyl, substituted or unsubstituted 3- to 7-membered heterocycloalkyl, substituted or unsubstituted 5- to 6-membered aryl, or substituted or unsubstituted 5- to 6-membered heteroaryl; and
the R⁵ may be hydrogen or halogen.

In an embodiment, in the Formula 1 above, the R¹ and R² may each independently be hydrogen, halogen, CN, substituted or unsubstituted 5- to 7-membered cycloalkyl, substituted or unsubstituted 5- to 7-membered heterocycloalkyl, substituted or unsubstituted 5- to 6-membered aryl, or substituted or unsubstituted 5- to 6-membered heteroaryl;
wherein the substituted 5- to 7-membered cycloalkyl, substituted 5- to 7-membered heterocycloalkyl, substituted 5- to 6-membered aryl, and substituted 5- to 6-membered heteroaryl in R¹ and R² may each independently be substituted with one or more selected from the group consisting of halogen, C₁₋₄ alkyl, 5- to 6-membered heterocycloalkyl, (C₁₋₆ alkyl)-(5- to 6-membered heterocycloalkyl), (5- to 6-membered heterocycloalkyl)-(C₁₋₆ alkyl), -O-(C₁₋₆ alkyl), (C₁₋₆ alkyl)-(5- to 6-membered heterocycloalkyl)-(C₁₋₆ alkyl), and -(CO)HN(C₁₋₆ alkyl);
the X¹ may be carbon or nitrogen;
wherein when the X¹ is carbon, the R³ may be hydrogen, halogen, OH, -O-(C₁₋₃ alkyl), or unsubstituted C₁₋₃ alkyl, and when the X¹ is nitrogen, the R³ may be absent;
the R⁴ may be hydrogen, halogen, OR^{a}, SR^{a}, O(CO)R^{a}, NHR^{a}, NH(CO)R^{a}, NR^{b}R^{c}, X^{a}(CH₂)ₚX^{b}(CH₂)_{q}R^{d}, substituted or unsubstituted C₁₋₄ alkyl, substituted or unsubstituted 5-to 7-membered cycloalkyl, or substituted or unsubstituted 5- to 7-membered heterocycloalkyl,
wherein the substituted C₁₋₄ alkyl, substituted 5- to 7-membered cycloalkyl, and substituted 5- to 7-membered heterocycloalkyl in R⁴ may each independently be substituted with one or more selected from the group consisting of halogen, C₁₋₄ alkyl, C₁₋₄ alcohol, (C₁₋₄ alkyl)-(5- to 6-membered aryl), (5- to 6-membered heterocycloalkyl)-(5- to 6-membered aryl), and (5- to 6-membered heteroaryl)-(5- to 6-membered aryl);
the R^{a}, R^{b}, R^{c}, and R^{d} may each independently be hydrogen, halogen, substituted or unsubstituted C₁₋₄ alkyl, substituted or unsubstituted 3- to 7-membered cycloalkyl, substituted or unsubstituted 3- to 7-membered heterocycloalkyl, substituted or unsubstituted 5- to 6-membered aryl, or substituted or unsubstituted 5- to 6-membered heteroaryl; and
wherein the substituted C₁₋₄ alkyl, substituted 3- to 7-membered cycloalkyl, substituted 3- to 7-membered heterocycloalkyl, substituted 5- to 6-membered aryl, and substituted 5- to 6-membered heteroaryl in R^{a}, R^{b}, R^{c}, and R^{d} may each independently be substituted with one or more selected from the group consisting of halogen, OH, 5- to 6-membered heterocycloalkyl, 5- to 6-membered aryl, and 5- to 6-membered heteroaryl.

In an embodiment, in the Formula 1 above, R¹ and R² may each independently be hydrogen, halogen, CN, 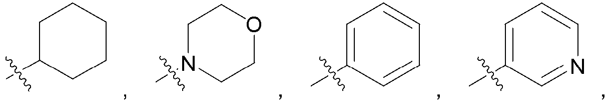 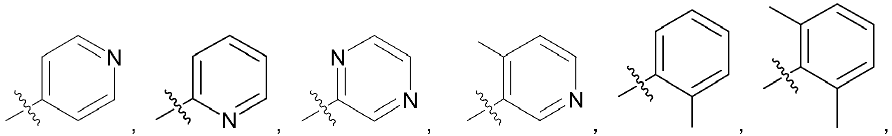 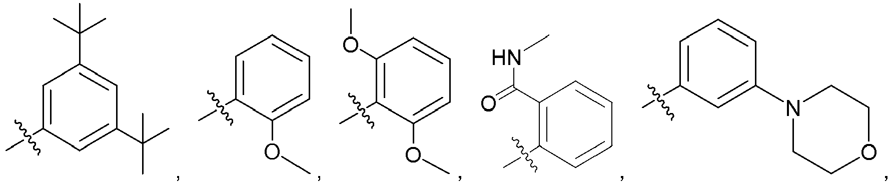 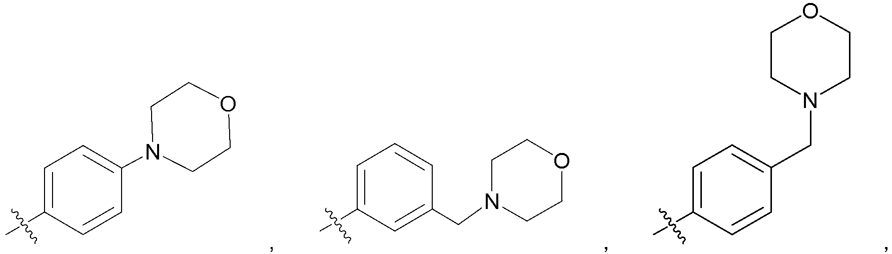 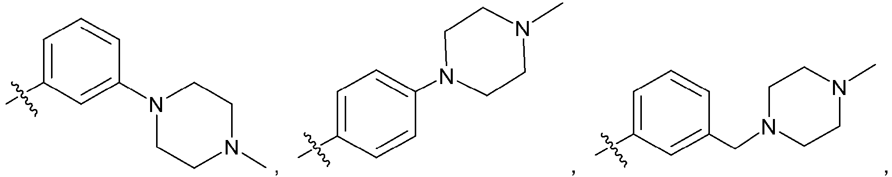 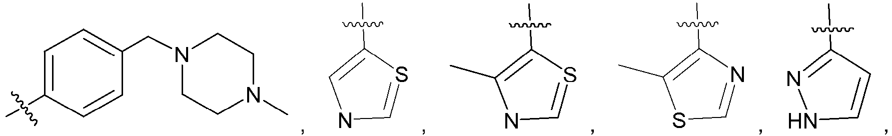 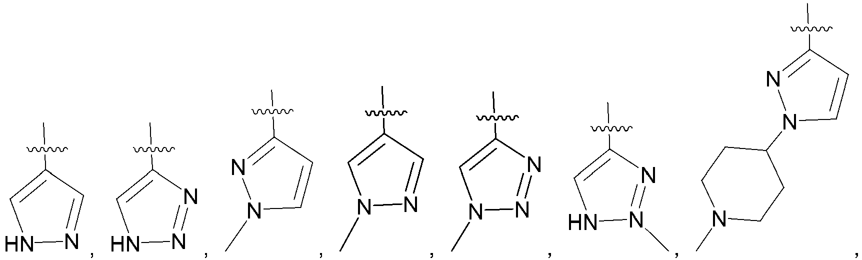 or 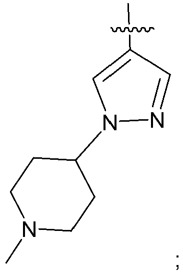
the X¹ may be carbon or nitrogen;
wherein when the X¹ is carbon, the R³ may be hydrogen, halogen, -CH₃, -OH, or 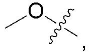 and when the X¹ is nitrogen, the R³ may be absent; and
the R⁴ may be hydrogen, halogen, -OH, -NH₂,

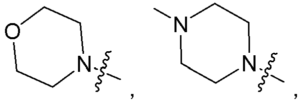

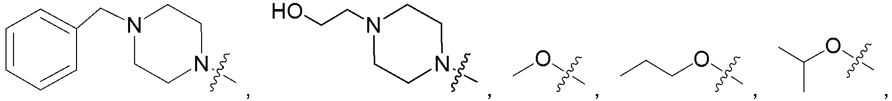

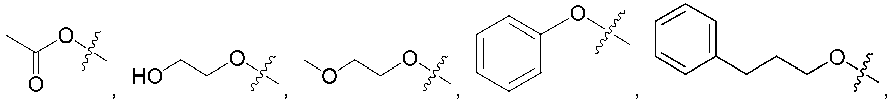

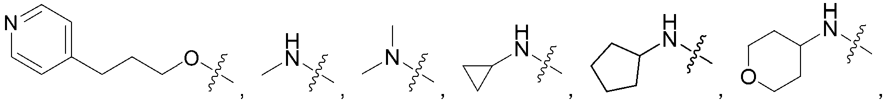

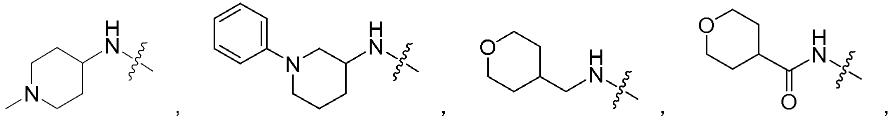

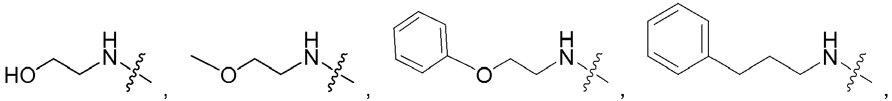

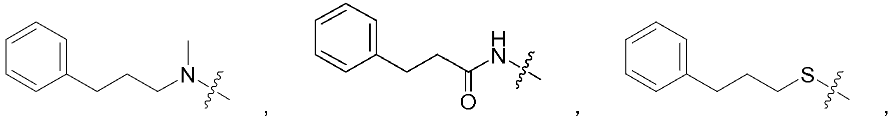

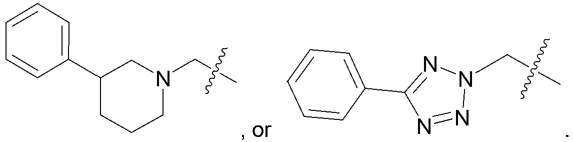

In an embodiment, in Formula 1 below,

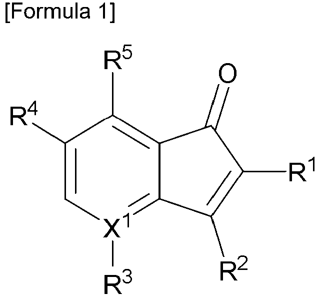

the R¹ may be hydrogen, CN, OR^{a}, SR^{a}, CO₂R^{a}, CH₂CO₂R^{a}, CONR^{b}R^{c}, NR^{b}R^{c}, substituted or unsubstituted 3- to 7-membered cycloalkyl, substituted or unsubstituted 3- to 7-membered heterocycloalkyl, 5- to 10-membered aryl substituted with substituted or unsubstituted 6-membered heterocycloalkyl, or 5- to 10-membered heteroaryl substituted with substituted or unsubstituted 6-membered heterocycloalkyl;
the R² may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the X¹ may be carbon or nitrogen;
wherein when the X¹ is carbon, the R³ may be hydrogen, halogen, CN, OR^{a}, SR^{a}, CO₂R^{a}, CH₂CO₂R^{a}, CONR^{b}R^{c}, NRbRc, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl, and when the X¹ is nitrogen, the R³ may be absent;
the R⁴ may be hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R⁵ may be hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R^{a}, R^{b}, R^{c}, and R^{d} may each independently be hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X^{a} and X^{b} may each independently be a direct bond, oxygen, nitrogen, or sulfur; and
p and q may each independently be an integer selected from 0 to 2.

In an embodiment, in Formula 1 below,

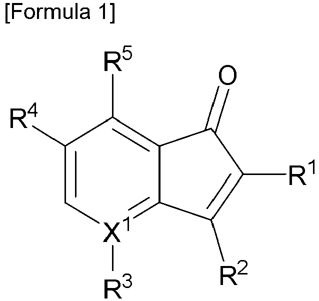

the R¹ may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R² may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, or substituted or unsubstituted 5- to 10-membered aryl;
the X¹ may be carbon or nitrogen;
wherein when the X¹ is carbon, the R³ may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl, and when the X¹ is nitrogen, the R³ may be absent;
the R⁴ may be hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R⁵ may be hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R^{a}, R^{b}, R^{c}, and R^{d} may each independently be hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X^{a} and X^{b} may each independently be a direct bond, oxygen, nitrogen, or sulfur; and
p and q may each independently be an integer selected from 0 to 2.

In an embodiment, in Formula 1 below,

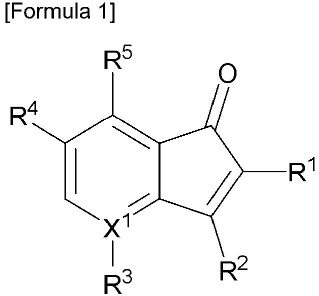

the R¹ may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R² may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the X¹ may be carbon or nitrogen;
wherein when the X¹ is carbon, the R³ may be halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl, and when the X¹ is nitrogen, the R³ may be absent;
the R⁴ may be hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R⁵ may be hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R^{a}, R^{b}, R^{c}, and R^{d} may each independently be hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X^{a} and X^{b} may each independently be a direct bond, oxygen, nitrogen, or sulfur; and
p and q may each independently be an integer selected from 0 to 2.

In an embodiment, in Formula 1 below,

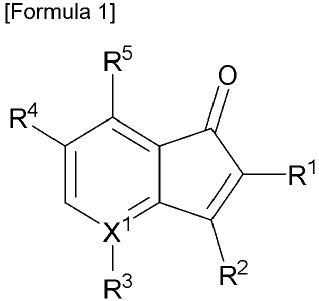

the R¹ may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R² may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the X¹ may be carbon or nitrogen,
wherein when the X¹ is carbon, the R³ may be hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl, and when the X¹ is nitrogen, the R³ may be absent;
the R⁴ may be hydrogen, halogen, CN, -O-(C₁₋₃ alkyl), OH, -O-(C₁₋₃ alcohol), -O-(C₁₋₃ alkyl)-(C₁₋₃ alkoxy), -O-(substituted or unsubstituted 5- to 6-membered aryl), N(CH₂)ₚX^{b}(CH₂)_{q}R^{d}, O(CO)R^{a}, CO₂R^{a}, NHR^{a}, NH(CO)R^{a}, CH₂CO₂R^{a}, CONR^{b}R^{c}, NR^{b}R^{c}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R⁵ may be hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
the R^{a}, R^{b}, R^{c}, and R^{d} may each independently be hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X^{a} and X^{b} may each independently be a direct bond, oxygen, nitrogen, or sulfur; and
p and q may each independently be an integer selected from 0 to 2.

In an embodiment, the compound or a salt thereof may be one selected from the group consisting of the following (1) to (101):
(1) 2,3-diphenylinden-1-one,
(2) 6-methoxy-2,3-diphenyl-1H-inden-1-one,
(3) 2,3-diphenyl-6-propoxy-1H-inden-1-one,
(4) isopropoxy-2,3-diphenyl-1H-inden-1-one,
(5) 2-phenyl-3-(o-tolyl)-1H-inden-1-one,
(6) 3-phenyl-2-(o-tolyl)-1H-inden-1-one,
(7) 3-phenyl-2-(pyridin-3-yl)-1H-inden-1-one,
(8) 2-morpholino-3-phenyl-1H-inden-1-one,
(9) 2-phenyl-3-(pyridin-3-yl)-1H-inden-1-one,
(10) 3-cyclohexyl-2-phenyl-1H-inden-1-one,
(11) 2-cyclohexyl-3-phenyl-1H-inden-1-one,
(12) 6-morpholino-2,3-diphenyl-1H-inden-1-one,
(13) 6-(4-methylpiperazin-1-yl)-2,3-diphenyl-1H-inden-1-one,
(14) 3-(4-morpholinophenyl)-2-phenyl-1H-inden-1-one,
(15) 3-(3-morpholinophenyl)-2-phenyl-1H-inden-1-one,
(16) 3-(4-(morpholinomethyl)phenyl)-2-phenyl-1H-inden-1-one,
(17) 2-(4-morpholinophenyl)-3-phenyl-1H-inden-1-one,
(18) 2-(3-morpholinophenyl)-3-phenyl-1H-inden-1-one,
(19) 3-(4-methylthiazol-5-yl)-2-(pyridin-3-yl)-1H-inden-1-one,
(20) 3-(4-methylthiazol-5-yl)-6-((3-phenylpropyl)amino)-2-(pyridin-3-yl)-1H-inden-1-one,
(21) 2-(4-methylpyridin-3-yl)-3-(4-methylthiazol-5-yl)-6-(3-phenylpropoxy)-1H-inden-1-one,
(22) 6-methoxy-2-(4-methylpyridin-3-yl)-3-(4-methylthiazol-5-yl)-1H-inden-1-one,
(23) 3-(5-methylthiazol-4-yl)-2-(pyridin-3-yl)-6-(3-(pyridin-4-yl)propoxy)-1H-inden-1-one,
(24) 2-bromo-3-(1-methyl-1H-pyrazol-3-yl)-1-oxo-1H-inden-6-yl acetate,
(25) 3-(4-methylthiazol-5-yl)-6-(3-phenylpropoxy)-2-(1H-pyrazol-3-yl)-1H-inden-1-one,
(26) 2-(1-methyl-1H-pyrazol-3-yl)-3-(4-methylthiazol-5-yl)-6-(3-phenylpropoxy)-1H-inden-1-one,
(27) 3-(4-methylthiazol-5-yl)-6-(3-phenylpropoxy)-2-(1H-pyrazol-4-yl)-1H-inden-1-one,
(28) 2-(1-methyl-1H-pyrazol-4-yl)-3-(4-methylthiazol-5-yl)-6-(3-phenylpropoxy)-1H-inden-1-one,
(29) 2-(1-(1-methylpiperidin-4-yl)-1H-pyrazol-3-yl)-3-(4-methylthiazol-5-yl)-6-(3-phenylpropoxy)-1H-inden-1-one,
(30) 2-(1-(1-methylpiperidin-4-yl)-1H-pyrazol-4-yl)-3-(4-methylthiazol-5-yl)-6-(3-phenylpropoxy)-1H-inden-1-one,
(31) 3-(4-methylthiazol-5-yl)-6-(3-phenylpropoxy)-2-(1H-1,2,3-triazol-4-yl)-1H-inden-1-one,
(32) 2-(1-methyl-1H-1,2,3-triazol-4-yl)-3-(4-methylthiazol-5-yl)-6-(3-phenylpropoxy)-1H-inden-1-one,
(33) 2-(2-methyl-2H-1,2,3-triazol-4-yl)-3-(4-methylthiazol-5-yl)-6-(3-phenylpropoxy)-1H-inden-1-one,
(34) 3-(4-methylthiazol-5-yl)-1-oxo-6-(3-phenylpropoxy)-1H-indene-2-carbonitrile,
(35) 6-hydroxy-3-(4-methylthiazol-5-yl)-2-(pyridin-3-yl)-1H-inden-1-one,
(36) 6-(2-hydroxyethoxy)-3-(4-methylthiazol-5-yl)-2-(pyridin-3-yl)-1H-inden-1-one,
(37) 6-(2-methoxyethoxy)-3-(4-methylthiazol-5-yl)-2-(pyridin-3-yl)-1H-inden-1-one,
(38) 6-(methyl(3-phenylpropyl)amino)-3-(4-methylthiazol-5-yl)-2-(pyridin-3-yl)-1H-inden-1-one,
(39) 3-(4-methylthiazol-5-yl)-6-(3-phenylpropoxy)-2-(pyridin-4-yl)-1H-inden-1-one,
(40) 3-(4-methylthiazol-5-yl)-6-(3-phenylpropoxy)-2-(pyridin-2-yl)-1H-inden-1-one,
(41) 3-(4-methylthiazol-5-yl)-6-(3-phenylpropoxy)-2-(pyrazin-2-yl)-1H-inden-1-one,
(42) 3-(1-methyl-1H-pyrazol-4-yl)-6-(3-phenylpropoxy)-2-(pyridin-3-yl)-1H-inden-1-one,
(43) 6-(3-phenylpropoxy)-3-(1H-pyrazol-4-yl)-2-(pyridin-3-yl)-1H-inden-1-one,
(44) 3-(2-methoxyphenyl)-6-(3-phenylpropoxy)-2-(pyridin-3-yl)-1H-inden-1-one,
(45) 3-(2,6-dimethoxyphenyl)-6-(3-phenylpropoxy)-2-(pyridin-3-yl)-1H-inden-1-one,
(46) 4-methyl-3-(4-methylthiazol-5-yl)-6-(3-phenylpropoxy)-2-(pyridin-3-yl)-1H-inden-1-one,
(47) 4-methoxy-3-(4-methylthiazol-5-yl)-6-(3-phenylpropoxy)-2-(pyridin-3-yl)-1H-inden-1-one,
(48) 4-hydroxy-3-(4-methylthiazol-5-yl)-6-(3-phenylpropoxy)-2-(pyridin-3-yl)-1H-inden-1-one,
(49) 6-(cyclopentylamino)-3-(4-methylthiazol-5-yl)-2-(pyridin-3-yl)-1H-inden-1-one,
(50) 6-(methylamino)-3-(4-methylthiazol-5-yl)-2-(pyridin-3-yl)-1H-inden-1-one,
(51) 6-((1-methylpiperidin-4-yl)amino)-3-(4-methylthiazol-5-yl)-2-(pyridin-3-yl)-1H-inden-1-one,
(52) 3-(4-methylthiazol-5-yl)-2-(pyridin-3-yl)-6-(((tetrahydro-2H-pyran-4-yl)methyl)amino)-1H-inden-1-one,
(53) 6-((2-hydroxyethyl)amino)-3-(4-methylthiazol-5-yl)-2-(pyridin-3-yl)-1H-inden-1-one,
(54) 6-((2-methoxyethyl)amino)-3-(4-methylthiazol-5-yl)-2-(pyridin-3-yl)-1H-inden-1-one,
(55) 3-(4-methylthiazol-5-yl)-6-((2-phenoxyethyl)amino)-2-(pyridin-3-yl)-1H-inden-1-one,
(56) 3-(4-methylthiazol-5-yl)-2-(pyridin-3-yl)-6-((tetrahydro-2H-pyran-4-yl)amino)-1H-inden-1-one,
(57) 3-(4-methylthiazol-5-yl)-6-((3-phenylpropyl)thio)-2-(pyridin-3-yl)-1H-inden-1-one,
(58) 3-(4-methylthiazol-5-yl)-6-((1-phenylpiperidin-3-yl)amino)-2-(pyridin-3-yl)-1H-inden-1-one,
(59) 6-amino-3-(4-methylthiazol-5-yl)-2-(pyridin-3-yl)-1H-inden-1-one,
(60) N-(3-(4-methylthiazol-5-yl)-1-oxo-2-(pyridin-3-yl)-1H-inden-6-yl)-3-phenylpropanamide,
(61) N-(3-(4-methylthiazol-5-yl)-1-oxo-2-(pyridin-3-yl)-1H-inden-6-yl)tetrahydro-2H-pyran-4-carboxamide,
(62) 6-(dimethylamino)-3-(4-methylthiazol-5-yl)-2-(pyridin-3-yl)-1H-inden-1-one,
(63) 6-(cyclopropylamino)-3-(4-methylthiazol-5-yl)-2-(pyridin-3-yl)-1H-inden-1-one,
(64) 6-(4-methylpiperazin-1-yl)-3-(4-methylthiazol-5-yl)-2-(pyridin-3-yl)-1H-inden-1-one,
(65) 6-(methyl(3-phenylpropyl)amino)-3-(4-methylthiazol-5-yl)-2-(pyridin-3-yl)-1H-inden-1-one,
(66) 3-(4-methylthiazol-5-yl)-6-phenoxy-2-(pyridin-3-yl)-1H-inden-1-one,
(67) 6-(methylamino)-2,3-diphenyl-1H-inden-1-one,
(68) 6-amino-2,3-diphenyl-1H-inden-1-one,
(69) 6-(dimethylamino)-2,3-diphenyl-1H-inden-1-one,
(70) 6-(4-(2-hydroxyethyl)piperazin-1-yl)-2,3-diphenyl-1H-inden-1-one,
(71) 4-methyl-6-(4-methylpiperazin-1-yl)-2,3-diphenyl-1H-inden-1-one,
(72) 3-(2,6-dimethylphenyl)-6-(4-methylpiperazin-1-yl)-2-phenyl-1H-inden-1-one,
(73) 6-(4-methylpiperazin-1-yl)-2-(3-morpholinophenyl)-3-phenyl-1H-inden-1-one,
(74) 6-(4-(2-hydroxyethyl)piperazin-1-yl)-2-(4-(4-methylpiperazin-1-yl)phenyl)-3-(4-methylthiazol-5-yl)-1H-inden-1-one,
(75) 6-(4-methylpiperazin-1-yl)-2-(4-(4-methylpiperazin-1-yl)phenyl)-3-(4-methylthiazol-5-yl)-1H-inden-1-one,
(76) 2-(4-(4-methylpiperazin-1-yl)phenyl)-3-(4-methylthiazol-5-yl)-6-(3-phenylpropoxy)-1H-inden-1-one,
(77) 2,3-bis(4-methylthiazol-5-yl)-6-(3-phenylpropoxy)-1H-inden-1-one,
(78) 2,3-diphenyl-6-(3-phenylpropoxy)-1H-inden-1-one,
(79) 6-(4-methylpiperazin-1-yl)-2,3-bis(4-methylthiazol-5-yl)-1H-inden-1-one,
(80) 6-(4-methylpiperazin-1-yl)-2,3-bis(3-morpholinophenyl)-1H-inden-1-one,
(81) 6-(4-(2-hydroxyethyl)piperazin-1-yl)-2,3-bis(4-methylthiazol-5-yl)-1H-inden-1-one,
(82) 6-hydroxy-2,3-di-o-tolyl-1H-inden-1-one,
(83) 6-(3-phenylpropoxy)-2,3-di(thiazol-5-yl)-1H-inden-1-one,
(84) 3-(2,6-dimethylphenyl)-6-hydroxy-2-(o-tolyl)-1H-inden-1-one,
(85) 2-(4-(4-methylpiperazin-1-yl)phenyl)-3-(4-methylthiazol-5-yl)-1H-inden-1-one,
(86) 2-(4-((4-methylpiperazin-1-yl)methyl)phenyl)-3-(4-methylthiazol-5-yl)-1H-inden-1-one,
(87) 3-(4-methylthiazol-5-yl)-2-(4-(morpholinomethyl)phenyl)-1H-inden-1-one,
(88) 2-(3-((4-methylpiperazin-1-yl)methyl)phenyl)-3-(4-methylthiazol-5-yl)-1H-inden-1-one,
(89) 3-(4-methylthiazol-5-yl)-2-(3-(morpholinomethyl)phenyl)-1H-inden-1-one,
(90) 2-(4-(4-methylpiperazin-1-yl)phenyl)-3-phenyl-1H-inden-1-one,
(91) 2-(3-(4-methylpiperazin-1-yl)phenyl)-3-phenyl-1H-inden-1-one,
(92) 3-(4-((4-methylpiperazin-1-yl)methyl)phenyl)-2-(4-methylthiazol-5-yl)-1H-inden-1-one,
(93) 3-(3-((4-methylpiperazin-1-yl)methyl)phenyl)-2-(4-methylthiazol-5-yl)-1H-inden-1-one,
(94) 3-(4-(4-methylpiperazin-1-yl)phenyl)-2-phenyl-1H-inden-1-one,
(95) 3-(3-(4-methylpiperazin-1-yl)phenyl)-2-phenyl-1H-inden-1-one,
(96) 3-(3,5-di-tert-butylphenyl)-2-phenyl-1H-inden-1-one,
(97) 4,6-dimethoxy-2,3-diphenyl-1H-inden-1-one,
(98) 6,7-diphenyl-5H-cyclopenta[b]pyridin-5-one,
(99) N-methyl-2-(1-oxo-3-(o-tolyl)-1H-inden-2-yl)benzamide,
(100) 6-(4-benzylpiperazin-1-yl)-3-(4-methylthiazol-5-yl)-2-(pyridin-3-yl)-1H-inden-1-one, and
(101) 3-(4-methylthiazol-5-yl)-6-((3-phenylpiperidin-1-yl)methyl)-2-(pyridin-3-yl)-1H-inden-1-one.

In an embodiment, it was confirmed that when the Compounds 1 to 99 (comp ounds of Formulas 2 to 100) and their intermediates (Compounds 5-4, 6-2, 12-3, 23-10, 39-3, 40-4, 41-4, 42-6, 42-7, 44-8, 45-8, 72-7, 73-7, 74-7, 76-1, 83-5, 83-6, 99-7, and 99-8) were administered, an amyloid-beta disaggregation and/or tau disaggregati on effect was exhibited (see Example 1).

In addition, when the Compound 13 (compound of Formula 14), Compound 30 (compound of Formula 31), Compound 63 (compound of Formula 64), Compound 65 (compound of Formula 66), Compound 70 (compound of Formula 71), Compound 72 (compound of Formula 73), Compound 74 (compound of Formula 75), Compound 74 -7 (intermediate compound of Compound 74), Compound 75 (compound of Formula 7 6), Compound 76-1 (intermediate compound of Compound 76), Compound and Comp ound 90 (compound of Formula 91) were each administered to amyloid-beta oligomer -expressing cells, it was confirmed that the compounds exhibited an amyloid-beta olig omer reduction effect through the disaggregation of amyloid-beta oligomer aggregates (see Example 2).

Accordingly, it was confirmed that the compounds of Formula 1 exhibit an exc ellent effect of disaggregating amyloid-beta protein aggregates and/or tau protein aggr egates in brain cells or tissues, and thus can be effectively utilized for the prevention, amelioration, or treatment of neurodegenerative diseases.

A compound or a salt thereof according to an aspect may be used for inhibiti ng aggregation of an amyloid-beta protein and/or disaggregating an aggregate of an amyloid-beta protein, inhibiting aggregation of a tau protein and/or disaggregating an aggregate of a tau protein, and/or inhibiting phosphorylation of a tau protein, and ma y be effectively utilized for the prevention, amelioration, or treatment of neurodegener ative diseases.

Another aspect provides a composition including the compound of Formula 1, or a salt thereof.

The terms "halogen", "alkyl", "heterocycloalkyl", "cycloalkyl", "aryl", "heteroaryl", etc., may be as defined above.

The composition including a compound or a salt thereof according to an aspe ct may be used for inhibiting aggregation of an amyloid-beta protein and/or disaggreg ating an aggregate of an amyloid-beta protein, inhibiting aggregation of a tau protein and/or disaggregating an aggregate of a tau protein, and/or inhibiting phosphorylation of a tau protein, and may be effectively utilized for the prevention, amelioration, or tr eatment of a neurodegenerative brain disease.

Another aspect provides a pharmaceutical composition including the compound of Formula 1, or a pharmaceutically acceptable salt thereof.

The terms "halogen", "alkyl", "heterocycloalkyl", "cycloalkyl", "aryl", "heteroaryl", etc., may be as defined above.

The term "pharmaceutically acceptable" refers to a characteristic of being non-toxic to cells or humans exposed to a specific compound according to one aspect.

When the compound includes a relatively acidic functional group, a base addition salt can be obtained by bringing a sufficient amount of a base into contact with the neutral form of such a compound in a pure solution or a suitable inert solvent. Pharmaceutically acceptable base addition salts include salts of sodium, potassium, calcium, ammonium, organic amine, or magnesium, or similar salts. When the compound includes a relatively basic functional group, an acid addition salt can be obtained by bringing a sufficient amount of an acid into contact with the neutral form of such a compound in a pure solution or a suitable inert solvent. Examples of pharmaceutically acceptable acid addition salts may include salts of inorganic acids such as hydrochloric acid, hydrobromic acid, nitric acid, carbonic acid, bicarbonate, phosphoric acid, monohydrogen phosphate, dihydrogen phosphate, sulfuric acid, bisulfate, hydroiodic acid, or phosphorous acid, and salts of organic acids such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; furthermore, salts of amino acids (for example, arginine, etc.) and salts of organic acids such as glucuronic acid are also included.

A pharmaceutical composition including a compound according to one aspect or a pharmaceutically acceptable salt thereof may be used for inhibiting aggregation of an amyloid-beta protein and/or disaggregating an aggregate of an amyloid-beta pro tein, inhibiting aggregation of a tau protein and/or disaggregating an aggregate of a t au protein, and/or inhibiting phosphorylation of a tau protein, and may be effectively utilized for the prevention, amelioration, or treatment of a neurodegenerative brain dis ease.

Another aspect provides a use of the compound of Formula 1, or a pharmaceutically acceptable salt thereof.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to one a spect may be used for inhibiting aggregation of an amyloid-beta protein and/or disagg regating an aggregate of an amyloid-beta protein, inhibiting aggregation of a tau prot ein and/or disaggregating an aggregate of a tau protein, and/or inhibiting phosphorylat ion of a tau protein, and may be effectively utilized for preventing, ameliorating, or tr eating a neurodegenerative brain disease.

Another aspect provides a use of the compound of Formula 1, or a pharmace utically acceptable salt thereof, for the manufacture of a medicament.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to an as pect may be used for the manufacture of a medicament for inhibiting aggregation of an amyloid-beta protein and/or disaggregating an aggregate of an amyloid-beta protei n, inhibiting aggregation of a tau protein and/or disaggregating an aggregate of a tau protein, and/or inhibiting phosphorylation of a tau protein, and may be effectively utiliz ed for the manufacture of a medicament for preventing, ameliorating, or treating a ne urodegenerative brain disease.

Another aspect provides a pharmaceutical composition for preventing or treating a neurodegenerative brain disease, the pharmaceutical composition including the compound of Formula 1, or a pharmaceutically acceptable salt thereof.

The terms "halogen", "alkyl", "heterocycloalkyl", "cycloalkyl", "aryl", "heteroaryl", "pharmaceutically acceptable salt", etc., may be as defined above.

The term "prevention" may refer to any act of inhibiting or delaying a neurodegenerative brain disease in a subject by administering the pharmaceutical composition according to one aspect.

The term "treatment" may refer to any act by which symptoms of a neurodegenerative brain disease in a subject are ameliorated or beneficially changed by administering the pharmaceutical composition according to one aspect.

In addition, the pharmaceutical composition may be provided as a pharmaceutical composition including one or more pharmaceutically acceptable carriers, excipients, or diluents.

Specifically, the carrier may be, for example, a colloidal suspension, powder, saline, lipid, liposome, microspheres, or nanospheres. These may form a complex with or be associated with a delivery vehicle, and may be delivered in vivo using a delivery system known in the art, such as lipids, liposomes, microparticles, gold, nanoparticles, polymers, condensation agents, polysaccharides, polyamino acids, dendrimers, saponins, absorption-enhancing substances, or fatty acids.

When the pharmaceutical composition is formulated, it may be prepared using diluents or excipients that are commonly used, such as lubricants, sweeteners, flavoring agents, emulsifying agents, suspending agents, preservatives, fillers, extenders, binders, wetting agents, disintegrating agents, and surfactants. Solid formulations for oral administration may include tablets, pills, powders, granules, capsules, and the like, and such solid formulations may be prepared by mixing at least one excipient, for example, starch, calcium carbonate, sucrose or lactose, gelatin, etc., with the composition. In addition to simple excipients, lubricants such as magnesium stearate and talc may also be used. Liquid formulations for oral administration include suspensions, solutions for internal use, emulsions, syrups, and the like, and in addition to commonly used simple diluents such as water and liquid paraffin, various excipients, for example, wetting agents, sweeteners, fragrances, preservatives, and the like, may be included. Formulations for parenteral administration may include sterilized aqueous solutions, non-aqueous solvents, suspensions, emulsions, lyophilized preparations, and suppositories. As non-aqueous solvents and suspending agents, propylene glycol, polyethylene glycol, vegetable oils such as olive oil, and injectable esters such as ethyl oleate may be used. As a base for suppositories, witepsol, macrogol, tween 61, cacao butter, laurin butter, glycerogelatin, and the like may be used, and when prepared in the form of an eye drop, known diluents or excipients may be used.

In an embodiment, the pharmaceutical composition for preventing or treating a neurodegenerative brain disease may be for administration to a subject selected from: (1) a subject in whom the level of amyloid-beta aggregation is higher than that of a normal individual without a neurodegenerative brain disease, or who is at risk thereof;
(2) a subject in whom the level of tau protein aggregation is higher than that of a normal individual without a neurodegenerative brain disease, or who is at risk thereof;
(3) a subject in whom the level of tau protein phosphorylation is higher than that of a normal individual without a neurodegenerative brain disease, or who is at risk thereof; and
(4) a subject corresponding to one or more of the (1) to (3) above.

The term "neurodegenerative brain disease" refers to any disease related to degenerative changes in the brain, particularly any disease that may be caused by one or more factors selected from the group consisting of aggregation of amyloid-beta protein, aggregation of tau protein, and phosphorylation of tau protein in the brain and/or brain neurons.

The aggregation level of the amyloid-beta or tau protein refers to the amount (concentration) of aggregates of the amyloid-beta protein or aggregates of the tau protein, or the ratio of aggregates of the amyloid-beta protein or aggregates of the tau protein to the total amyloid-beta protein or total tau protein.

The phosphorylation level of the tau protein refers to the amount (concentration) of phosphorylated tau protein or the ratio of phosphorylated tau protein to the total tau protein.

The "normal" may be a subject of the same species as the subject of application (patient) of the pharmaceutical composition who does not have the "neurodegenerative brain disease" as defined above, or a brain tissue or brain cell (neuron) isolated and/or cultured from the subject.

In an embodiment, the neurodegenerative brain disease may be one or more selected from the group consisting of dementia, Alzheimer's disease, preclinical Alzheimer's disease, Parkinson's disease, Huntington's disease, mild cognitive impairment, cerebral amyloid angiopathy, Down syndrome, amyloid-related stroke, systemic amyloidosis, Dutch-type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

The pharmaceutical composition is administered in a pharmaceutically effective amount. The term "pharmaceutically effective amount" refers to an amount sufficient to treat a disease at a reasonable benefit/risk ratio applicable to medical treatment, and the effective dose level may be determined according to factors including the type and severity of the patient's disease, the activity of the drug, the patient's sensitivity to the drug, the time of administration, the route of administration, the rate of excretion, the duration of treatment, and concurrently used drugs, as well as other factors well known in the medical field. The administration may be once a day or divided into several doses. For example, the administration may be every other day or once a week.

In an embodiment, the compound or a pharmaceutically acceptable salt thereof may be administered at a dose of 0.1 mg/kg to 100 mg/kg. Specifically, the compound or a pharmaceutically acceptable salt thereof may be administered at a dose of 1 mg/kg to 50 mg/kg, and more specifically, may be administered at a dose of 1 mg/kg to 10 mg/kg.

The term "administration" refers to the introduction of a predetermined substance to a subject by a suitable method, and "subject" refers to any organism that may have a neurodegenerative brain disease, including humans, as well as animals such as rats, mice, pigs, horses, cattle, and livestock. As a specific example, the subject may be a mammal, including a human.

The pharmaceutical composition may be administered orally or parenterally, and in the case of parenteral administration, the route of administration may be selected from topical application to the skin or intraperitoneal injection, subcutaneous injection, intravenous injection, intramuscular injection, intra-arterial injection, intramedullary injection, intracardiac injection, intradural injection, transdermal injection, intranasal injection, enteral injection, local injection, sublingual injection, rectal injection, or intrathoracic injection.

In one aspect, the pharmaceutical composition may further include another therapeutic agent for a neurodegenerative brain disease in addition to the compound or a pharmaceutically acceptable salt thereof.

The other therapeutic agent for a neurodegenerative brain disease may be included in the pharmaceutical composition in a minimum amount that can achieve the maximum effect without side effects, and this amount can be easily determined by a person skilled in the art.

In addition, in one aspect, the pharmaceutical composition may be administered alone or in combination with another therapeutic agent for a neurodegenerative brain disease. That is, the pharmaceutical composition may be administered in parallel with another therapeutic agent for a neurodegenerative brain disease, may be administered simultaneously, separately, or sequentially, and may be administered in a single or multiple doses.

When the other therapeutic agent for a neurodegenerative brain disease is administered in combination with the pharmaceutical composition, it may be administered in an amount or ratio that can achieve the maximum effect without side effects, and this amount or ratio can be easily determined by a person skilled in the art.

The other therapeutic agent for a neurodegenerative brain disease may be a conventionally known therapeutic agent for a neurodegenerative brain disease or a newly developed therapeutic agent for a neurodegenerative brain disease.

The pharmaceutical composition including a compound or a pharmaceutically a cceptable salt thereof according to one aspect exhibits an excellent effect of disaggre gating amyloid-beta protein aggregates and/or tau protein aggregates in brain cells or tissues, and may be effectively utilized for the prevention, amelioration, or treatment o f a neurodegenerative brain disease.

Another aspect provides a use of the compound of Formula 1, or a pharmaceutically acceptable salt thereof, for preventing or treating a neurodegenerative brain disease.

The terms "halogen", "alkyl", "heterocycloalkyl", "cycloalkyl", "aryl", "heteroaryl", "pharmaceutically acceptable salt", "prevention", "treatment", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be effectively utilized f or the prevention, amelioration, or treatment of a neurodegenerative brain disease.

Another aspect provides a use of the compound of Formula 1, or a pharmaceutically acceptable salt thereof, for the manufacture of a medicament for preventing or treating a neurodegenerative brain disease.

The terms "halogen", "alkyl", "heterocycloalkyl", "cycloalkyl", "aryl", "heteroaryl", "pharmaceutically acceptable salt", "prevention", "treatment", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be effectively utilized f or the manufacture of a medicament for preventing, ameliorating, or treating a neurod egenerative brain disease.

Another aspect provides a method of preventing or treating a neurodegenerative brain disease, the method including administering the compound of Formula 1, or a pharmaceutically acceptable salt thereof, to a subject in need thereof.

The terms "halogen", "alkyl", "heterocycloalkyl", "cycloalkyl", "aryl", "heteroaryl", "pharmaceutically acceptable salt", "administration", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be effectively utilized f or the prevention, amelioration, or treatment of a neurodegenerative brain disease.

Another aspect provides a pharmaceutical composition for inhibiting aggregation of an amyloid-beta protein or disaggregating an aggregate of an amyloid-beta protein, the pharmaceutical composition including the compound of Formula 1, or a pharmaceutically acceptable salt thereof.

The terms "halogen", "alkyl", "heterocycloalkyl", "cycloalkyl", "aryl", "heteroaryl", "pharmaceutically acceptable salt", etc., may be as defined above.

The term "amyloid beta (Aβ)" refers to a polypeptide derived from amyloid precursor protein (APP) or a fragment thereof, and may include a polynucleotide sequence encoding it or a fragment thereof.

The term "inhibition" refers to inhibiting any step among gene transcription, mRNA processing, translation, translocation, and maturation, or inhibiting protein-protein binding, protein activation, or signaling therethrough.

The term "aggregate of a protein" may include a polymer, fibril precursor, fibril, plaque, and protein monomers and polymers bound to a homologous protein. The fibril may include an oligomer of an amyloid-beta protein. For example, the aggregate may be a soluble aggregate and/or an insoluble aggregate of an amyloid-beta protein, an amyloid-beta oligomer, an amyloid-beta protofibril, an amyloid-beta fibril, and an amyloid-beta plaque.

The term "disaggregation" refers to the process of breaking down formed aggregates into a state of lower aggregation, i.e., lower-order aggregates. For example, disaggregation refers to the process of converting amyloid-beta fibrils to amyloid-beta oligomers, and amyloid-beta oligomers to amyloid-beta monomers.

The pharmaceutical composition including a compound or a pharmaceutically a cceptable salt thereof according to one aspect exhibits an excellent effect of disaggre gating amyloid-beta protein aggregates and/or tau protein aggregates in brain cells or tissues, and may be utilized for inhibiting aggregation of an amyloid-beta protein and/ or disaggregating an aggregate of an amyloid-beta protein.

Another aspect provides a use of the compound of Formula 1, or a pharmaceutically acceptable salt thereof, for inhibiting aggregation of an amyloid-beta protein or disaggregating an aggregate of an amyloid-beta protein.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be used for inhibiting aggregation of an amyloid-beta protein and/or disaggregating an aggregate of an amy loid-beta protein.

Another aspect provides a use of the compound of Formula 1, or a pharmaceutically acceptable salt thereof, for the manufacture of a medicament for inhibiting aggregation of an amyloid-beta protein or disaggregating an aggregate of an amyloid-beta protein.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be used for the manu facture of a medicament for inhibiting aggregation of an amyloid-beta protein and/or d isaggregating an aggregate of an amyloid-beta protein.

Another aspect provides a method of inhibiting aggregation of an amyloid-beta protein or disaggregating an aggregate of an amyloid-beta protein, the method including administering the compound of Formula 1, or a pharmaceutically acceptable salt thereof, to a subject in need thereof.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be used for inhibiting aggregation of an amyloid-beta protein and/or disaggregating an aggregate of an amy loid-beta protein.

Another aspect provides a pharmaceutical composition for inhibiting aggregation of a tau protein, disaggregating an aggregate of a tau protein, or inhibiting phosphorylation of a tau protein, the pharmaceutical composition including the compound of Formula 1, or a pharmaceutically acceptable salt thereof.

The terms "halogen", "alkyl", "heterocycloalkyl", "cycloalkyl", "aryl", "heteroaryl", "pharmaceutically acceptable salt", etc., may be as defined above.

The pharmaceutical composition including a compound or a pharmaceutically a cceptable salt thereof according to one aspect exhibits an excellent effect of disaggre gating amyloid-beta protein aggregates and/or tau protein aggregates in brain cells or tissues, and may be used for inhibiting aggregation of a tau protein and/or disaggreg ating an aggregate of a tau protein, and/or inhibiting phosphorylation of a tau protein.

Another aspect provides a use of the compound of Formula 1, or a pharmaceutically acceptable salt thereof, for inhibiting aggregation of a tau protein, disaggregating an aggregate of a tau protein, or inhibiting phosphorylation of a tau protein.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be used for inhibiting aggregation of a tau protein and/or disaggregating an aggregate of a tau protein, and /or inhibiting phosphorylation of a tau protein.

Another aspect provides a use of the compound of Formula 1, or a pharmaceutically acceptable salt thereof, for the manufacture of a medicament for inhibiting aggregation of a tau protein, disaggregating an aggregate of a tau protein, or inhibiting phosphorylation of a tau protein.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be used for the manu facture of a medicament for inhibiting aggregation of a tau protein and/or disaggregati ng an aggregate of a tau protein, and/or inhibiting phosphorylation of a tau protein.

Another aspect provides a method of inhibiting aggregation of a tau protein, disaggregating an aggregate of a tau protein, or inhibiting phosphorylation of a tau protein, the method including administering the compound of Formula 1, or a pharmaceutically acceptable salt thereof, to a subject in need thereof.

The terms "halogen", "alkyl", "heterocycloalkyl", "cycloalkyl", "aryl", "heteroaryl", "pharmaceutically acceptable salt", etc., may be as defined above.

A compound or a pharmaceutically acceptable salt thereof according to one a spect exhibits an excellent effect of disaggregating amyloid-beta protein aggregates a nd/or tau protein aggregates in brain cells or tissues, and may be used for inhibiting aggregation of a tau protein and/or disaggregating an aggregate of a tau protein, and /or inhibiting phosphorylation of a tau protein.

Another aspect provides a health functional food including the compound of Formula 1, or a food acceptable salt thereof.

The terms "halogen", "alkyl", "heterocycloalkyl", "cycloalkyl", "aryl", "heteroaryl", etc., may be as defined above.

The term "amelioration" may refer to any act of at least reducing a parameter related to the condition being treated, for example, the severity of a symptom. In this case, the health functional food may be used for the prevention or amelioration of a neurodegenerative brain disease before or after the onset of the disease, simultaneously with or separately from a therapeutic agent.

The "health functional food" includes health functional foods, health foods, and health supplement foods.

The term "health functional food" is synonymous with Food for Special Health Use (FoSHU) and refers to a food with high medical and healthcare effects, processed to efficiently exhibit bioregulatory functions in addition to nutritional supply. Here, "function(al)" refers to obtaining a useful effect for health purposes, such as regulating nutrients for the structure and function of the human body or physiological actions.

In addition, "health food" refers to a food that has a more active effect on maintaining or promoting health compared to general foods, and "health supplement food" refers to a food for the purpose of health supplementation.

In the health functional food, the compound or a food acceptable salt thereof may be added to food as is, or may be used in combination with other foods or food ingredients, and may be used appropriately according to conventional methods. The blended amount of the compound or a food acceptable salt thereof may be suitably determined according to its purpose of use (for preventing or ameliorating a neurodegenerative brain disease). Generally, when manufacturing a food or beverage, the compound or a food acceptable salt thereof may be added in an amount of about 15 % by weight or less, and more specifically, about 10 % by weight or less, with respect to the raw materials. However, in the case of long-term intake for the purpose of health and hygiene or for health control, the amount may be below the above range.

The health functional food may be formulated into one selected from the group consisting of tablets, pills, powders, granules, powders, capsules, and liquid formulations by further including one or more of carriers, diluents, excipients, and additives. Foods to which the compound according to one aspect can be added include various food types, granules, tablets, capsules, syrups, beverages, gums, teas, vitamin complexes, health functional foods, and the like.

Specific examples of the carriers, excipients, diluents, and additives may be one or more selected from the group consisting of lactose, dextrose, sucrose, sorbitol, mannitol, erythritol, starch, acacia gum, alginate, gelatin, calcium phosphate, calcium silicate, microcrystalline cellulose, cellulose, polyvinylpyrrolidone, methylcellulose, water, sugar syrup, methyl hydroxybenzoate, propyl hydroxybenzoate, talc, magnesium stearate, and mineral oil.

In addition to containing the compound or a food acceptable salt thereof, the health functional food may contain other ingredients as essential components without particular limitation. For example, like conventional beverages, the health functional food may contain various flavoring agents or natural carbohydrates as additional ingredients. Examples of the aforementioned natural carbohydrates may be conventional sugars (such as monosaccharides, e.g., glucose and fructose; disaccharides, e.g., maltose and sucrose; and polysaccharides, e.g., dextrin and cyclodextrin) and sugar alcohols such as xylitol, sorbitol, and erythritol. As flavoring agents other than those mentioned above, natural flavoring agents (thaumatin, stevia extract (for example, rebaudioside A, glycyrrhizin, etc.)) and synthetic flavoring agents (saccharin, aspartame, etc.) may be advantageously used. The proportion of the natural carbohydrates may be appropriately determined by the choice of a person skilled in the art.

In addition to the above, the health functional food according to one aspect may contain various nutrients, vitamins, minerals (electrolytes), flavoring agents such as synthetic flavoring agents and natural flavoring agents, coloring agents and bulking agents (cheese, chocolate, etc.), pectic acid and its salts, alginic acid and its salts, organic acids, protective colloidal thickeners, pH adjusting agents, stabilizers, preservatives, glycerin, alcohol, carbonating agents used in carbonated beverages, and the like. These ingredients may be used independently or in combination, and the proportion of these additives may also be appropriately selected by a person skilled in the art.

In an embodiment, the compound or a food acceptable salt thereof may be ingested at a dose of 0.1 mg/kg to 100 mg/kg. Specifically, it may be ingested at a dose of 1 mg/kg to 50 mg/kg, and more specifically, it may be ingested at a dose of 1 mg/kg to 10 mg/kg.

In an embodiment, the health functional food may further include another health functional food for preventing or ameliorating a neurodegenerative brain disease in addition to the compound or a food acceptable salt thereof.

The other health functional food for preventing or ameliorating a neurodegenerative brain disease may be included in the health functional food in a minimum amount that can achieve the maximum effect without side effects, and this can be easily determined by a person skilled in the art.

In addition, in an embodiment, the health functional food may be ingested alone or in combination with the health functional food for preventing or ameliorating a neurodegenerative brain disease. That is, the health functional food may be ingested in parallel with another health functional food for preventing or ameliorating a neurodegenerative brain disease, may be ingested simultaneously, separately, or sequentially, and may be ingested in a single or multiple doses.

When ingested in combination with the other health functional food for preventing or ameliorating a neurodegenerative brain disease, the health functional food may be ingested in an amount or ratio that can achieve the maximum effect without side effects, and this amount can be easily determined by a person skilled in the art.

The other health functional food for preventing or ameliorating a neurodegenerative brain disease may be a conventionally known health functional food for preventing or ameliorating a neurodegenerative brain disease or a newly developed health functional food for preventing or ameliorating a neurodegenerative brain disease.

The health functional food including a compound or a food acceptable salt the reof according to one aspect may be used for inhibiting aggregation of an amyloid-be ta protein and/or disaggregating an aggregate of an amyloid-beta protein, inhibiting ag gregation of a tau protein and/or disaggregating an aggregate of a tau protein, and/or inhibiting phosphorylation of a tau protein, and may be effectively utilized for the pre vention, amelioration, or treatment of a neurodegenerative brain disease.

Another aspect provides a health functional food for preventing or ameliorating a neurodegenerative brain disease, the health functional food including the compound of Formula 1, or a food acceptable salt thereof.

The terms "halogen", "alkyl", "heterocycloalkyl", "cycloalkyl", "aryl", "heteroaryl", etc., may be as defined above.

The health functional food including a compound or a food acceptable salt the reof according to one aspect exhibits an excellent effect of disaggregating amyloid-bet a protein aggregates and/or tau protein aggregates in brain cells or tissues, and may be effectively utilized for the prevention or amelioration of a neurodegenerative brain disease.

Another aspect provides a health functional food for inhibiting aggregation of an amyloid-beta protein or disaggregating an aggregate of an amyloid-beta protein, the health functional food including the compound of Formula 1, or a food acceptable salt thereof.

The terms "halogen", "alkyl", "heterocycloalkyl", "cycloalkyl", "aryl", "heteroaryl", etc., may be as defined above.

The health functional food including a compound or a food acceptable salt the reof according to one aspect exhibits an excellent effect of disaggregating amyloid-bet a protein aggregates and/or tau protein aggregates in brain cells or tissues, and may be utilized for inhibiting aggregation of an amyloid-beta protein and/or disaggregating an aggregate of an amyloid-beta protein.

Another aspect provides a health functional food for inhibiting aggregation of a tau protein, disaggregating an aggregate of a tau protein, or inhibiting phosphorylation of a tau protein, the health functional food including the compound of Formula 1, or a food acceptable salt thereof.

The terms "halogen", "alkyl", "heterocycloalkyl", "cycloalkyl", "aryl", "heteroaryl", etc., may be as defined above.

The health functional food including a compound or a food acceptable salt the reof according to one aspect exhibits an excellent effect of disaggregating amyloid-bet a protein aggregates and/or tau protein aggregates in brain cells or tissues, and may be used for inhibiting aggregation of a tau protein and/or disaggregating an aggregat e of a tau protein, and/or inhibiting phosphorylation of a tau protein.

### Advantageous Effects of Invention

According to one aspect, the novel indenone derivatives exhibit excellent efficacy in inhibiting aggregation of an amyloid-beta protein and/or disaggregating an aggregate of an amyloid-beta protein, inhibiting aggregation of a tau protein and/or disaggregating an aggregate of a tau protein, and/or inhibiting phosphorylation of a tau protein, and thus can be usefully utilized for preventing or treating a neurodegenerative brain disease.

### Brief Description of Drawings

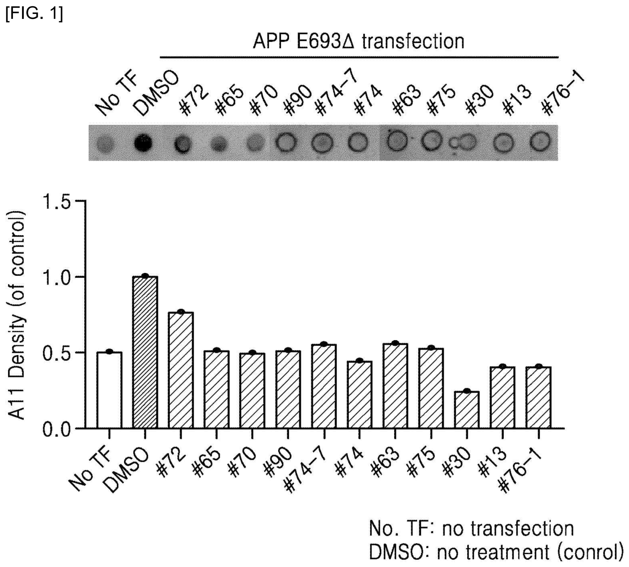

FIG. 1 is a graph showing the relative change in amyloid-beta oligomers by each compound and each compound intermediate, with transfected HEK293 cells not treated with a compound as the control group:
# : Compound and each compound intermediate number

### Modes of the Invention

Hereinafter, the present invention will be described in more detail through Examples. However, these Examples are for illustrating the present invention by way of example, and the scope of the present invention is not limited to these Examples.

### Preparation Examples

Compounds 1 to 101 shown in Table 1 below were prepared according to Pre paration Examples 1 to 101.

**[Table 1]**

| Compound No. | Formula No. | Compound Structure | Chemical Name |
|---|---|---|---|
| 1 | 2 | 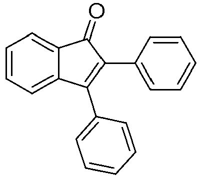 | 2,3-diphenylinden-1-one |
| 2 | 3 | 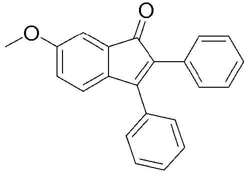 | 6-methoxy-2,3-diphenyl-1H-inden-1-one |
| 3 | 4 | 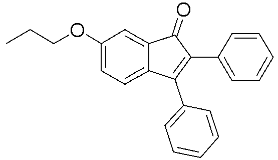 | 2,3-diphenyl-6-propoxy-1H-inden-1-one |
| 4 | 5 | 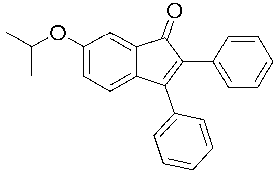 | isopropoxy-2,3-diphenyl-1H-inden-1-one |
| 5 | 6 | 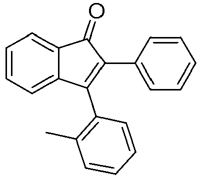 | 2-phenyl-3-(o-tolyl)-1H-inden-1-one |
| 6 | 7 | 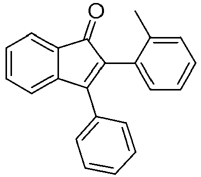 | 3-phenyl-2-(o-tolyl)-1H-inden-1-one |
| 7 | 8 | 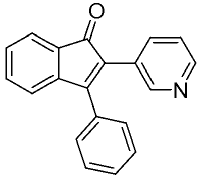 | 3-phenyl-2-(pyridin-3-yl)-1H-inden-1-one |
| 8 | 9 | 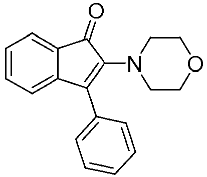 | 2-morpholino-3-phenyl-1H-inden-1-one |
| 9 | 10 | 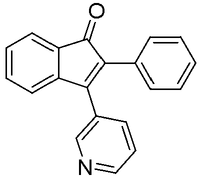 | 2-phenyl-3-(pyridin-3-yl)-1H-inden-1-one |
| 10 | 11 | 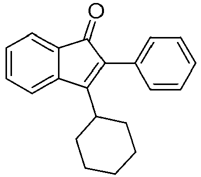 | 3-cyclohexyl-2-phenyl-1H-inden-1-one |
| 11 | 12 | 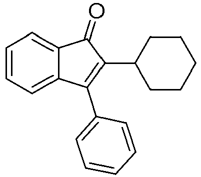 | 2-cyclohexyl-3-phenyl-1H-inden-1-one |
| 12 | 13 | 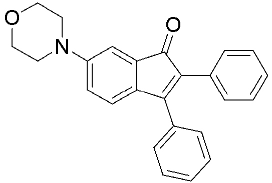 | 6-morpholino-2,3-diphenyl-1H-inden-1-one |
| 13 | 14 | 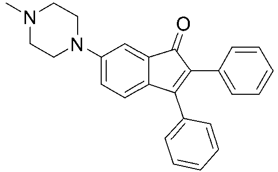 | 6-(4-methylpiperazin-1-yl)-2,3-diphenyl-1H-inden-1-one |
| 14 | 15 | 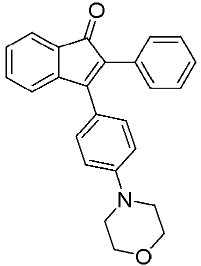 | 3-(4-morpholinophenyl)-2-phenyl-1H-inden-1-one |
| 15 | 16 | 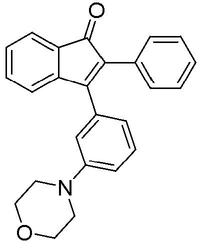 | 3-(3-morpholinophenyl)-2-phenyl-1H-inden-1-one |
| 16 | 17 | 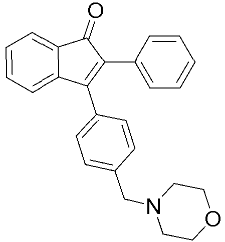 | 3-(4-(morpholinomethyl)phenyl )-2-phenyl-1H-inden-1-one |
| 17 | 18 | 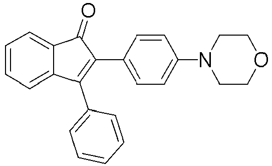 | 2-(4-morpholinophenyl)-3-phenyl-1H-inden-1-one |
| 18 | 19 | 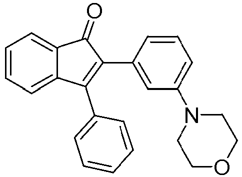 | 2-(3-morpholinophenyl)-3-phenyl-1H-inden-1-one |
| 19 | 20 | 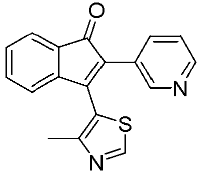 | 3-(4-methylthiazol-5-yl)-2-(pyridin-3-yl)-1H-inden-1-one |
| 20 | 21 | 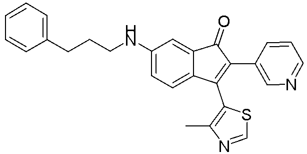 | 3-(4-methylthiazol-5-yl)-6-((3-phenylpropyl)amino)-2-(pyridin-3-yl)-1H-inden-1-one |
| 21 | 22 | 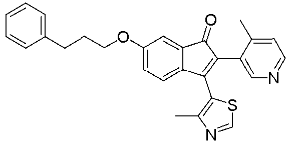 | 2-(4-methylpyridin-3-yl)-3-(4-methylthiazol-5-yl)-6-(3-phenylpropoxy)-1H-inden-1-one |
| 22 | 23 | 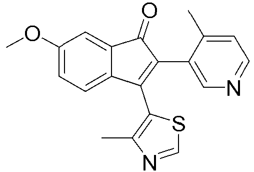 | 6-methoxy-2-(4-methylpyridin-3-yl)-3-(4-methylthiazol-5-yl)-1H-inden-1-one |
| 23 | 24 | 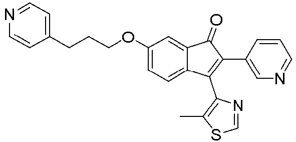 | 3-(5-methylthiazol-4-yl)-2-(pyridin-3-yl)-6-(3-(pyridin-4-yl)propoxy)-1H-inden-1-one |
| 24 | 25 | 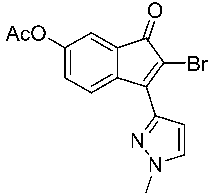 | 2-bromo-3-(1-methyl-1H-pyrazol-3-yl)-1-oxo-1H-inden-6-yl acetate, |
| 25 | 26 | 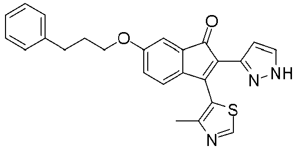 | 3-(4-methylthiazol-5-yl)-6-(3-phenylpropoxy)-2-(1H-pyrazol-3-yl)-1H-inden-1-one |
| 26 | 27 | 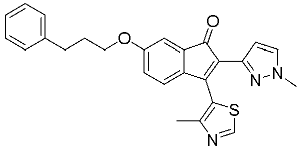 | 2-(1-methyl-1 H-pyrazol-3-yl)-3-(4-methylthiazol-5-yl)-6-(3-phenylpropoxy)-1H-inden-1-one |
| 27 | 28 | 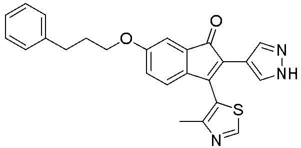 | 3-(4-methylthiazol-5-yl)-6-(3-phenylpropoxy)-2-(1H-pyrazol-4-yl)-1H-inden-1-one |
| 28 | 29 | 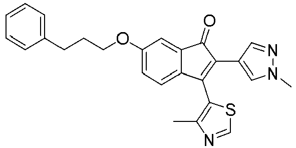 | 2-(1-methyl-1 H-pyrazol-4-yl)-3-(4-methylthiazol-5-yl)-6-(3-phenylpropoxy)-1H-inden-1-one |
| 29 | 30 | 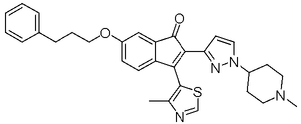 | 2-(1-(1-methylpiperidin-4-yl)-1H-pyrazol-3-yl)-3-(4-methylthiazol-5-yl)-6-(3-phenylpropoxy)-1H-inden-1-one |
| 30 | 31 | 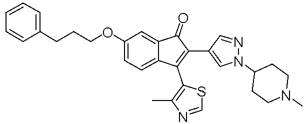 | 2-(1-(1-methylpiperidin-4-yl)-1H-pyrazol-4-yl)-3-(4-methylthiazol-5-yl)-6-(3-phenylpropoxy)-1H-inden-1-one |
| 31 | 32 | 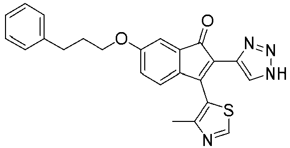 | 3-(4-methylthiazol-5-yl)-6-(3-phenylpropoxy)-2-(1H-1,2,3-triazol-4-yl)-1H-inden-1-one |
| 32 | 33 | 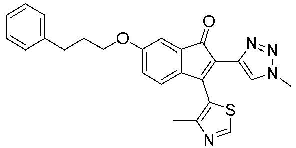 | 2-(1-methyl-1H-1,2,3-triazol-4-yl)-3-(4-methylthiazol-5-yl)-6-(3-phenylpropoxy)-1H-inden-1-one |
| 33 | 34 | 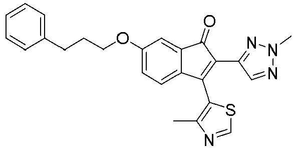 | 2-(2-methyl-2H-1,2,3-triazol-4-yl)-3-(4-methylthiazol-5-yl)-6-(3-phenylpropoxy)-1H-inden-1-one |
| 34 | 35 | 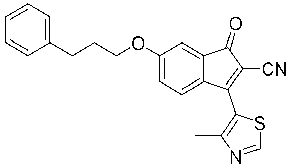 | 3-(4-methylthiazol-5-yl)-1-oxo-6-(3-phenylpropoxy)-1H-indene-2-carbonitrile, |
| 35 | 36 | 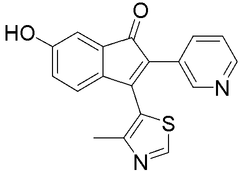 | 6-hydroxy-3-(4-methylthiazol-5-yl)-2-(pyridin-3-yl)-1H-inden-1-one |
| 36 | 37 | 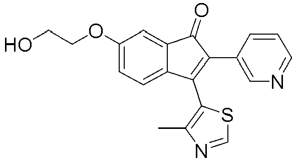 | 6-(2-hydroxyethoxy)-3-(4-methylthiazol-5-yl)-2-(pyridin-3-yl)-1H-inden-1-one |
| 37 | 38 | 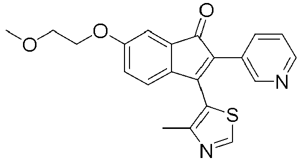 | 6-(2-methoxyethoxy)-3-(4-methylthiazol-5-yl)-2-(pyridin-3-yl)-1H-inden-1-one |
| 38 | 39 | 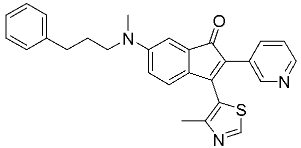 | 6-(methyl(3-phenylpropyl)amino)-3-(4-methylthiazol-5-yl)-2-(pyridin-3-yl)-1H-inden-1-one |
| 39 | 40 | 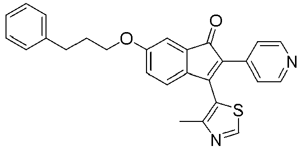 | 3-(4-methylthiazol-5-yl)-6-(3-phenylpropoxy)-2-(pyridin-4-yl)-1H-inden-1-one |
| 40 | 41 | 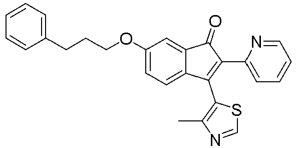 | 3-(4-methylthiazol-5-yl)-6-(3-phenylpropoxy)-2-(pyridin-2-yl)-1H-inden-1-one |
| 41 | 42 | 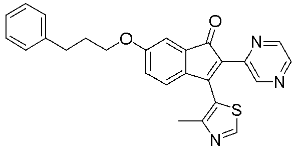 | 3-(4-methylthiazol-5-yl)-6-(3-phenylpropoxy)-2-(pyrazin-2-yl)-1H-inden-1-one |
| 42 | 43 | 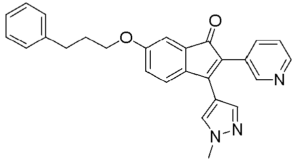 | 3-(1-methyl-1 H-pyrazol-4-yl)-6-(3-phenylpropoxy)-2-(pyridin-3-yl)-1H-inden-1-one |
| 43 | 44 | 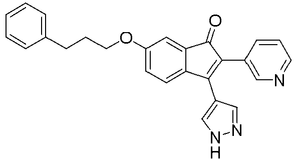 | 6-(3-phenylpropoxy)-3-(1H-pyrazol-4-yl)-2-(pyridin-3-yl)-1H-inden-1-one |
| 44 | 45 | 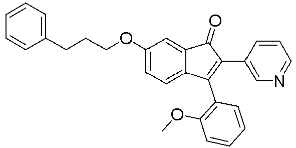 | 3-(2-methoxyphenyl)-6-(3-phenylpropoxy)-2-(pyridin-3-yl)-1H-inden-1-one |
| 45 | 46 | 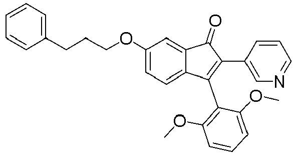 | 3-(2,6-dimethoxyphenyl)-6-(3-phenylpropoxy)-2-(pyridin-3-yl)-1H-inden-1-one |
| 46 | 47 | 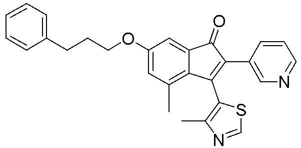 | 4-methyl-3-(4-methylthiazol-5-yl)-6-(3-phenylpropoxy)-2-(pyridin-3-yl)-1H-inden-1-one |
| 47 | 48 | 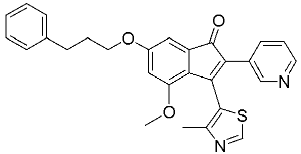 | 4-methoxy-3-(4-methylthiazol-5-yl)-6-(3-phenylpropoxy)-2-(pyridin-3-yl)-1H-inden-1-one |
| 48 | 49 | 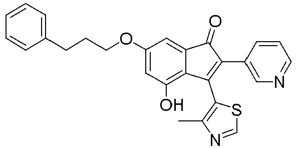 | 4-hydroxy-3-(4-methylthiazol-5-yl)-6-(3-phenylpropoxy)-2-(pyridin-3-yl)-1H-inden-1-one |
| 49 | 50 | 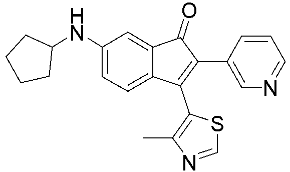 | 6-(cyclopentylamino)-3-(4-methylthiazol-5-yl)-2-(pyridin-3-yl)-1H-inden-1-one |
| 50 | 51 | 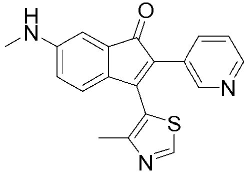 | 6-(methylamino)-3-(4-methylthiazol-5-yl)-2-(pyridin-3-yl)-1H-inden-1-one |
| 51 | 52 | 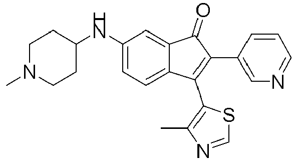 | 6-((1-methylpiperidin-4-yl)amino)-3-(4-methylthiazol-5-yl)-2-(pyridin-3-yl)-1H-inden-1-one |
| 52 | 53 | 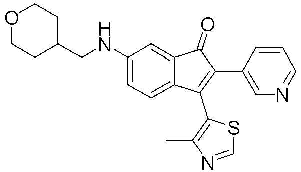 | 3-(4-methylthiazol-5-yl)-2-(pyridin-3-yl)-6-(((tetrahydro-2H-pyran-4-yl)methyl)amino)-1H-inden-1-one |
| 53 | 54 | 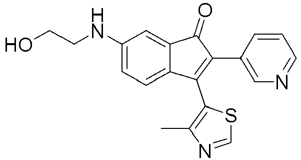 | 6-((2-hydroxyethyl)amino)-3-(4-methylthiazol-5-yl)-2-(pyridin-3-yl)-1H-inden-1-one |
| 54 | 55 | 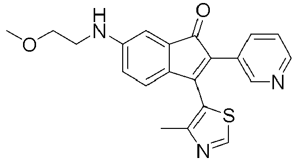 | 6-((2-methoxyethyl)amino)-3-(4-methylthiazol-5-yl)-2-(pyridin-3-yl)-1H-inden-1-one |
| 55 | 56 | 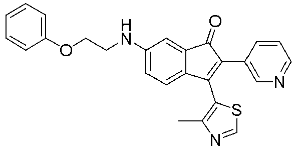 | 3-(4-methylthiazol-5-yl)-6-((2-phenoxyethyl)amino)-2-(pyridin-3-yl)-1H-inden-1-one |
| 56 | 57 | 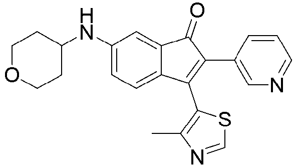 | 3-(4-methylthiazol-5-yl)-2-(pyridin-3-yl)-6-((tetrahydro-2H-pyran-4-yl)amino)-1H-inden-1-one |
| 57 | 58 | 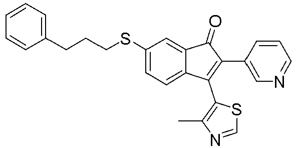 | 3-(4-methylthiazol-5-yl)-6-((3-phenylpropyl)thio)-2-(pyridin-3-yl)-1H-inden-1-one |
| 58 | 59 | 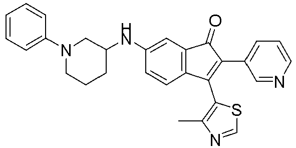 | 3-(4-methylthiazol-5-yl)-6-((1-phenylpiperidin-3-yl)amino)-2-(pyridin-3-yl)-1H-inden-1-one |
| 59 | 60 | 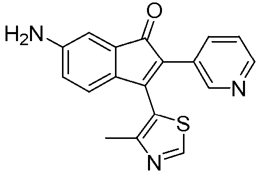 | 6-amino-3-(4-methylthiazol-5-yl)-2-(pyridin-3-yl)-1H-inden-1-one |
| 60 | 61 | 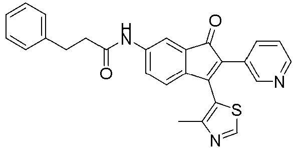 | N-(3-(4-methylthiazol-5-yl)-1-oxo-2-(pyridin-3-yl)-1H-inden-6-yl)-3-phenylpropanamide |
| 61 | 62 | 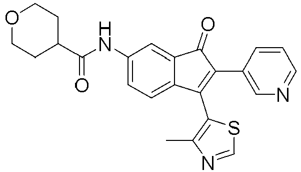 | N-(3-(4-methylthiazol-5-yl)-1-oxo-2-(pyridin-3-yl)-1H-inden-6-yl)tetrahydro-2H-pyran-4-carboxamide |
| 62 | 63 | 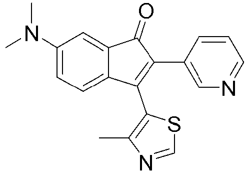 | 6-(dimethylamino)-3-(4-methylthiazol-5-yl)-2-(pyridin-3-yl)-1H-inden-1-one |
| 63 | 64 | 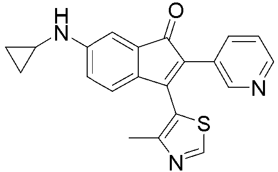 | 6-(cyclopropylamino)-3-(4-methylthiazol-5-yl)-2-(pyridin-3-yl)-1H-inden-1-one |
| 64 | 65 | 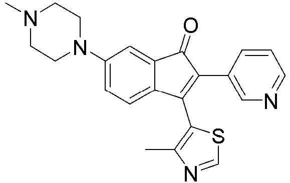 | 6-(4-methylpiperazin-1-yl)-3-(4-methylthiazol-5-yl)-2-(pyridin-3-yl)-1H-inden-1-one |
| 65 | 66 | 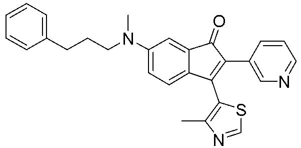 | 6-(methyl(3-phenylpropyl)amino)-3-(4-methylthiazol-5-yl)-2-(pyridin-3-yl)-1H-inden-1-one |
| 66 | 67 | 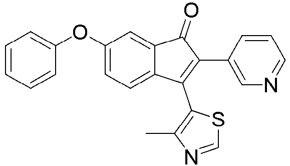 | 3-(4-methylthiazol-5-yl)-6-phenoxy-2-(pyridin-3-yl)-1H-inden-1-one |
| 67 | 68 | 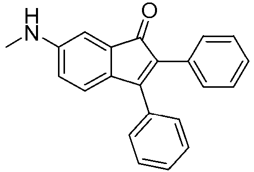 | 6-(methylamino)-2,3-diphenyl-1H-inden-1-one |
| 68 | 69 | 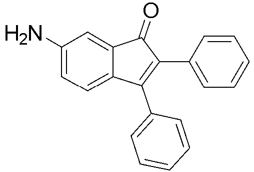 | 6-amino-2,3-diphenyl-1H-inden-1-one |
| 69 | 70 | 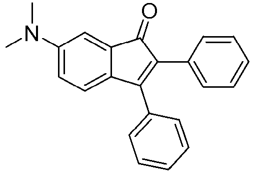 | 6-(dimethylamino)-2,3-diphenyl-1H-inden-1-one |
| 70 | 71 | 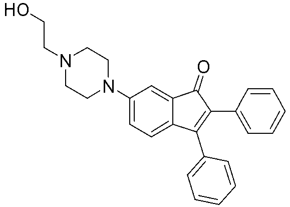 | 6-(4-(2-hydroxyethyl)piperazin-1-yl)-2,3-diphenyl-1H-inden-1-one |
| 71 | 72 | 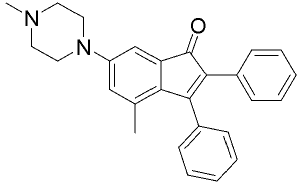 | 4-methyl-6-(4-methylpiperazin-1-yl)-2,3-diphenyl-1H-inden-1-one |
| 72 | 73 | 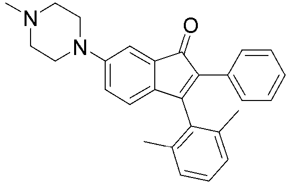 | 3-(2,6-dimethylphenyl)-6-(4-methylpiperazin-1-yl)-2-phenyl-1H-inden-1-one |
| 73 | 74 | 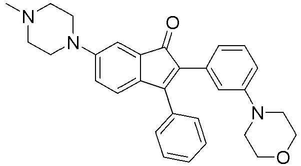 | 6-(4-methylpiperazin-1-yl)-2-(3-morpholinophenyl)-3-phenyl-1H-inden-1-one |
| 74 | 75 | 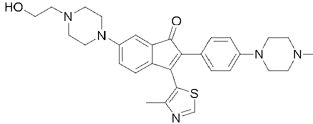 | 6-(4-(2-hydroxyethyl)piperazin-1-yl)-2-(4-(4-methylpiperazin-1-yl)phenyl)-3-(4-methylthiazol-5-yl)-1H-inden-1-one |
| 75 | 76 | 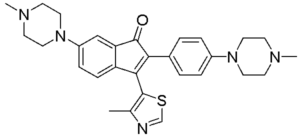 | 6-(4-methylpiperazin-1-yl)-2-(4-(4-methylpiperazin-1-yl)phenyl)-3-(4-methylthiazol-5-yl)-1H-inden-1-one |
| 76 | 77 | 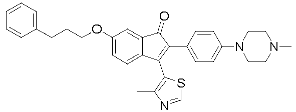 | 2-(4-(4-methylpiperazin-1-yl)phenyl)-3-(4-methylthiazol-5-yl)-6-(3-phenylpropoxy)-1H-inden-1-one |
| 77 | 78 | 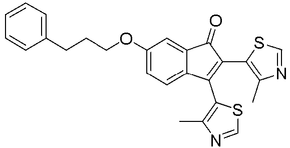 | 2,3-bis(4-methylthiazol-5-yl)-6-(3-phenylpropoxy)-1H-inden-1-one |
| 78 | 79 | 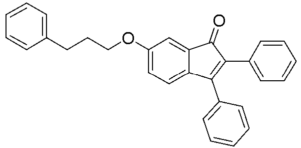 | 2,3-diphenyl-6-(3-phenylpropoxy)-1H-inden-1-one |
| 79 | 80 | 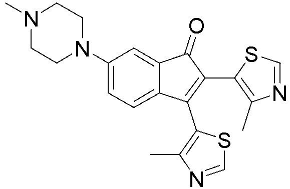 | 6-(4-methylpiperazin-1-yl)-2,3-bis(4-methylthiazol-5-yl)-1H-inden-1-one |
| 80 | 81 | 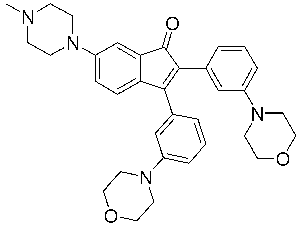 | 6-(4-methylpiperazin-1-yl)-2,3-bis(3-morpholinophenyl)-1H-inden-1-one |
| 81 | 82 | 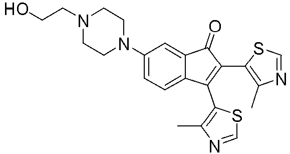 | 6-(4-(2-hydroxyethyl)piperazin-1-yl)-2,3-bis(4-methylthiazol-5-yl)-1H-inden-1-one |
| 82 | 83 | 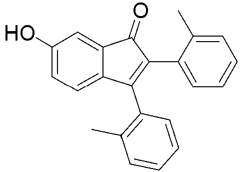 | 6-hydroxy-2,3-di-o-tolyl-1H-inden-1-one |
| 83 | 84 | 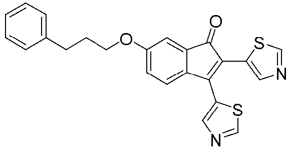 | 6-(3-phenylpropoxy)-2,3-di(thiazol-5-yl)-1H-inden-1-one |
| 84 | 85 | 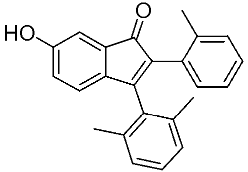 | 3-(2,6-dimethylphenyl)-6-hydroxy-2-(o-tolyl)-1H-inden-1-one |
| 85 | 86 | 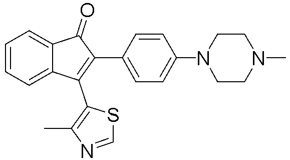 | 2-(4-(4-methylpiperazin-1-yl)phenyl)-3-(4-methylthiazol-5-yl)-1H-inden-1-one |
| 86 | 87 | 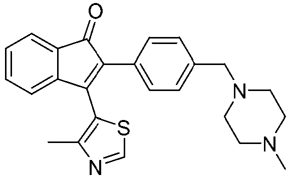 | 2-(4-((4-methylpiperazin-1-yl)methyl)phenyl)-3-(4-methylthiazol-5-yl)-1H-inden-1-one |
| 87 | 88 | 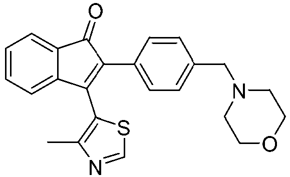 | 3-(4-methylthiazol-5-yl)-2-(4-(morpholinomethyl)phenyl )-1H-inden-1-one |
| 88 | 89 | 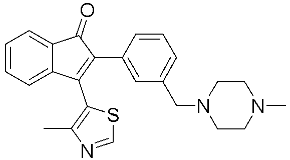 | 2-(3-((4-methylpiperazin-1-yl)methyl)phenyl)-3-(4-methylthiazol-5-yl)-1H-inden-1-one |
| 89 | 90 | 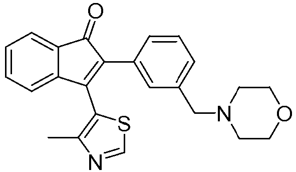 | 3-(4-methylthiazol-5-yl)-2-(3-(morpholinomethyl)phenyl )-1H-inden-1-one |
| 90 | 91 | 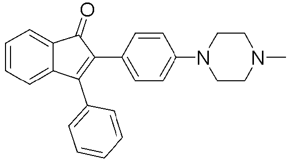 | 2-(4-(4-methylpiperazin-1-yl)phenyl)-3-phenyl-1H-inden-1-one |
| 91 | 92 | 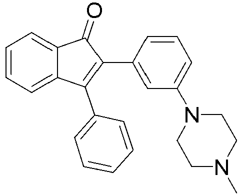 | 2-(3-(4-methylpiperazin-1-yl)phenyl)-3-phenyl-1H-inden-1-one |
| 92 | 93 | 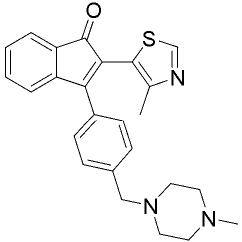 | 3-(4-((4-methylpiperazin-1-yl)methyl)phenyl)-2-(4-methylthiazol-5-yl)-1H-inden-1-one |
| 93 | 94 | 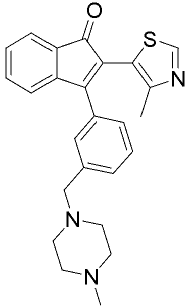 | 3-(3-((4-methylpiperazin-1-yl)methyl)phenyl)-2-(4-methylthiazol-5-yl)-1H-inden-1-one |
| 94 | 95 | 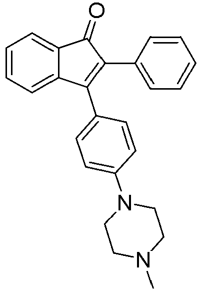 | 3-(4-(4-methylpiperazin-1-yl)phenyl)-2-phenyl-1H-inden-1-one |
| 95 | 96 | 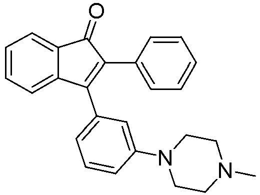 | 3-(3-(4-methylpiperazin-1-yl)phenyl)-2-phenyl-1H-inden-1-one |
| 96 | 97 | 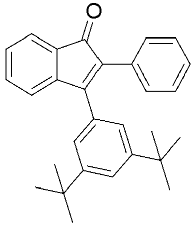 | 3-(3,5-di-tert-butylphenyl)-2-phenyl-1H-inden-1-one |
| 97 | 98 | 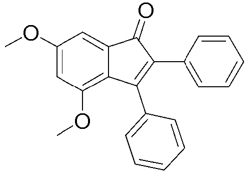 | 4,6-dimethoxy-2,3-diphenyl-1H-inden-1-one |
| 98 | 99 | 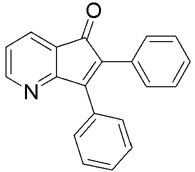 | 6,7-diphenyl-5H-cyclopenta[b]pyridin-5-one |
| 99 | 100 | 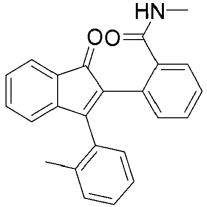 | N-methyl-2-(1-oxo-3-(o-tolyl)-1H-inden-2-yl)benzamide |
| 100 | 101 | 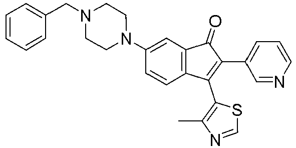 | 6-(4-benzylpiperazin-1-yl)-3-(4-methylthiazol-5-yl)-2-(pyridin-3-yl)-1H-inden-1-one |
| 101 | 102 | 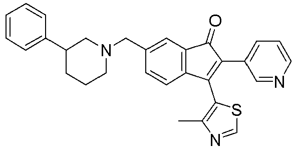 | 3-(4-methylthiazol-5-yl)-6-((3-phenylpiperidin-1-yl)methyl)-2-(pyridin-3-yl)-1H-inden-1-one |

### Preparation Example 1. 2,3-Diphenylinden-1-one (Compound 1)

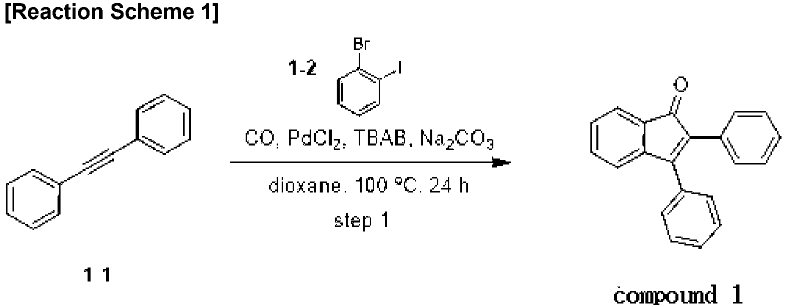

To a solution of (2-phenylethynyl)benzene (200 mg, 1.12 mmol) in 1,4-dioxane (5 mL) were sequentially added tetrabutylammonium bromide (361.76 mg, 1.12 mmol), Na₂CO₃ (237.88 mg, 2.24 mmol), 1-bromo-2-iodobenzene (634.95 mg, 2.24 mmol), and PdCl₂ (19.90 mg, 0.11 mmol) under a CO atmosphere. The reaction mixture was stirred at 100 °C for 24 hours under a CO atmosphere. The resulting mixture was treated with water (15 mL) and then extracted with EtOAc (3 x 15 mL). The organic layer was washed with brine, dried with sodium sulfate, and concentrated. The residue was then purified by silica gel column chromatography (0 % - 4 % EtOAc/PE) to obtain 2,3-diphenylinden-1-one (191.4 mg, 0.67 mmol, 59.9 %) as a red solid.

The obtained Compound 1 (Formula 2 below) was confirmed by MS(ESI) and¹H NMR, which yielded the following data:

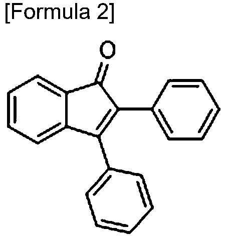

MS(ESI): C₂₁H₁₄O 282.1 m/z, found 282.7 [M+1]⁺.

¹H NMR (400 MHz, DMSO-d₆) δ 7.57 - 7.55 (m, 1H), 7.53 - 7.45 (m, 4H), 7.43 - 7.36 (m, 3H), 7.30 - 7.28 (m, 3H), 7.20 - 7.15 (m, 3H).

### Preparation Example 2. 6-methoxy-2,3-diphenyl-1H-inden-1-one (Compound 2)

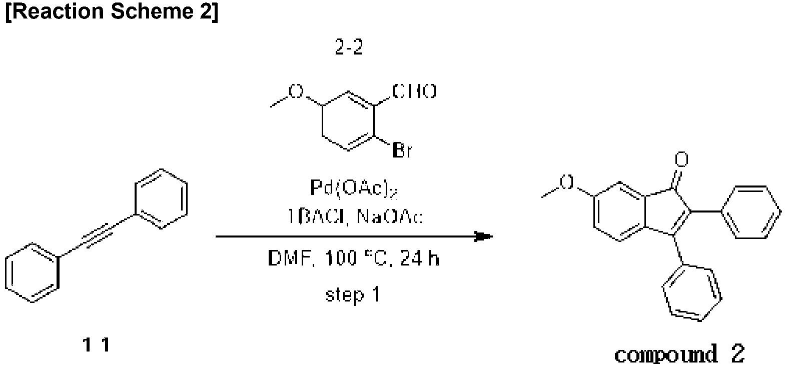

A mixture of (2-phenylethynyl)benzene (3.56 g, 0.02 mol), 2-bromo-5-methoxybenzaldehyde (4.30 g, 0.02 mol), Pd(OAc)₂ (0.23 g, 0.001 mol), TBACI (5.56 g, 0.02 mol), NaOAc (6.56 g, 0.08 mol), and DMF (100 mL) in a round-bottom flask was stirred in an oil bath heated to 100 °C for 24 hours. To the resulting mixture was added H₂O (500 mL), and the mixture was extracted with EtOAc (3 x 300 mL). The resulting organic layer was washed with brine, dried with sodium sulfate, and then concentrated under reduced pressure. The residue was purified by silica gel column chromatography (10 % EtOAc/PE) to obtain 6-methoxy-2,3-diphenyl-1H-inden-1-one (3.60 g, 11.54 mmol, 57.7 % yield) as a red solid.

The obtained Compound 2 (Formula 3 below) was confirmed by MS(ESI) and¹H NMR, which yielded the following data:

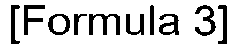

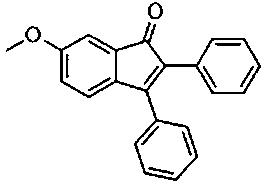

MS(ESI): C₂₂H₁₆O₂ 312.1 m/z, found 312.8 [M+1]⁺.

¹H NMR (400 MHz, DMSO-d₆) δ 7.47 - 7.46 (m, 3H), 7.39 - 7.37 (m, 2H), 7.31 - 7.23 (m, 3H), 7.17 - 7.14 (m, 3H), 7.07 (d, J = 8.1 Hz, 1H), 6.97 (dd, J = 8.1, 2.4 Hz, 1H), 3.83 (s, 3H).

### Preparation Example 3. 2,3-diphenyl-6-propoxy-1H-inden-1-one (Compound 3)

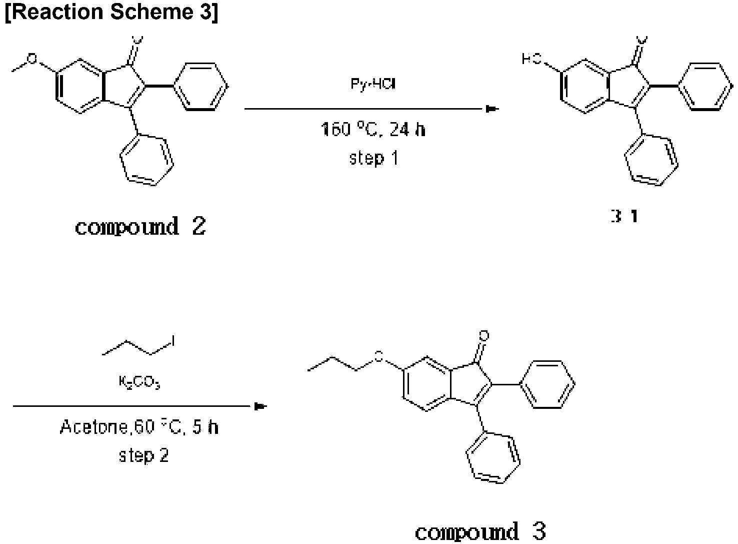

### Step 1: Synthesis of 6-hydroxy-2,3-diphenyl-1H-inden-1-one

A mixture of 6-methoxy-2,3-diphenyl-1H-inden-1-one (100 mg, 0.3201 mmol) and Py·HCl (147.96 mg, 1.2804 mmol) was stirred at 160 °C for 24 hours under N₂. After cooling to room temperature, the residue was diluted with water and extracted with ethyl acetate (3 x 20 mL). The combined organic layers were washed with water and brine, dried with sodium sulfate, and then concentrated under reduced pressure. The residue was purified by silica gel column chromatography (5 % MeOH/DCM) to obtain 6-hydroxy-2,3-diphenyl-1H-inden-1-one (60 mg, 0.1911 mmol, 59.70 %) as a white solid.

MS (ESI): C₂₁H₁₄O₂ 298.1 m/z, found 299.1 [M+1]⁺.

¹H NMR (400 MHz, DMSO-d₆) δ 10.13 (s, 1H), 7.48 - 7.42 (m, 3H), 7.39 - 7.33 (m, 2H), 7.29 - 7.22 (m, 3H), 7.14 (d, J = 6.9 Hz, 2H), 6.96 (d, J = 7.6 Hz, 2H), 6.78 (dd, J = 7.7, 1.9 Hz, 1H).

### Step 2: Synthesis of 2,3-diphenyl-6-propoxy-1H-inden-1-one (Compound 3)

A mixture of 6-hydroxy-2,3-diphenyl-1H-inden-1-one (50 mg, 0.1676 mmol), 1-iodopropane (42.74 mg, 0.2514 mmol), K₂CO₃ (69.49 mg, 0.5028 mmol), and acetone (4 mL) was stirred at 60 °C for 5 hours. The mixture was filtered through Celite, and the filtrate was concentrated in vacuo. The residue was purified by silica gel column chromatography (10 % EA/PE) to obtain 2,3-diphenyl-6-propoxy-1H-inden-1-one (30 mg, 0.0837 mmol, 49.94 % yield).

The obtained Compound 3 (Formula 4 below) was confirmed by MS(ESI) and¹H NMR, which yielded the following data:

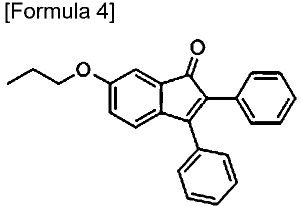

MS (ESI): C₂₄H₂₀O₂ 340.1 m/z, found 341.1 [M+1]⁺.

¹H NMR (400 MHz, DMSO-d₆) δ 7.49 - 7.43 (m, 3H), 7.38 (dt, J = 6.0, 3.2 Hz, 2H), 7.31 - 7.23 (m, 3H), 7.16 (dd, J = 7.6, 1.7 Hz, 2H), 7.11 (d, J = 2.3 Hz, 1H), 7.05 (d, J = 8.1 Hz, 1H), 6.96 (dd, J = 8.1, 2.4 Hz, 1H), 4.01 (t, J = 6.5 Hz, 2H), 1.81 - 1.65 (m, 2H), 0.98 (t, J = 7.4 Hz, 3H).

### Preparation Example 4. 6-isopropoxy-2,3-diphenyl-1H-inden-1-one (Compound 4)

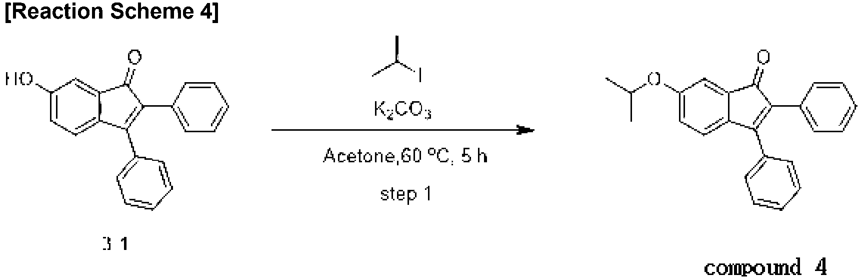

A mixture of 6-hydroxy-2,3-diphenyl-1H-inden-1-one (50 mg, 0.1676 mmol), 2-iodopropane (42.74 mg, 0.2514 mmol), and K₂CO₃ (69.49 mg, 0.5028 mmol) in acetone (4 mL) was reacted at 60 °C for 5 hours. The reaction mixture was filtered through Celite, and the filtrate was concentrated under reduced pressure. The residue was purified by silica gel column chromatography (10 % EA/PE) to obtain 6-isopropoxy-2,3-diphenyl-1H-inden-1-one (30 mg, 0.0837 mmol, 49.94 % yield).

The obtained Compound 4 (Formula 5 below) was confirmed by MS(ESI) and¹H NMR, which yielded the following data:

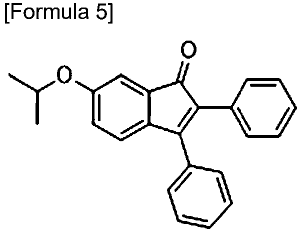

MS (ESI): C₂₄H₂₀O₂ 340.1 m/z, found 340.8 [M+1]⁺.

¹H NMR (400 MHz, DMSO-d₆) δ 7.49 - 7.42 (m, 3H), 7.38 (dd, J = 6.6, 3.0 Hz, 2H), 7.31 - 7.23 (m, 3H), 7.19 - 7.12 (m, 2H), 7.10 (d, J = 2.3 Hz, 1H), 7.04 (d, J = 8.1 Hz, 1H), 6.95 (dd, J = 8.1, 2.3 Hz, 1H), 4.72 (dt, J = 12.0, 6.0 Hz, 1H), 1.28 (d, J = 6.0 Hz, 6H).

### Preparation Example 5. 2-phenyl-3-(o-tolyl)-1H-inden-1-one (Compound 5)

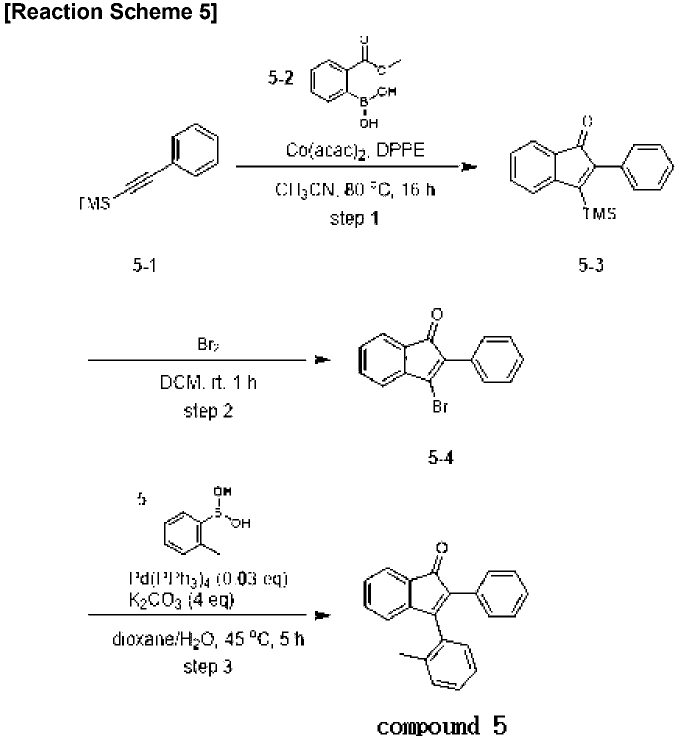

### Step 1: Synthesis of 2-phenyl-3-(trimethylsilyl)-1H-inden-1-one

To a mixture of trimethyl(2-phenylethynyl)silane (10 g, 57 mmol), methyl 2-(dihydroxyboranyl)benzoate (15.4 g, 85 mmol), and 1,2-bis(diphenylphosphino)ethane (2.3 g, 5 mmol) was added ACN (200 mL), and then Co(acac)₂ (2 g, 5 mmol) was added. The reaction mixture was stirred at 80 °C for 16 hours under N₂. After cooling to room temperature, the mixture was filtered through Celite, and the filtrate was concentrated in vacuo. The residue was diluted with water, and then extracted with EtOAc (3 x 20 mL). The organic layer was washed with water and brine, dried with sodium sulfate, and concentrated in vacuo. The residue was purified by silica gel column chromatography (10 % EtOAc/PE) to obtain 2-phenyl-3-(trimethylsilyl)-1H-inden-1-one (12 g, 43 mmol, 75.44 %) as a white solid.

MS (ESI): mass calcd. C₁₈H₁₈OSi 278.1 m/z, found 279.1 [M+1]+.

### Step 2: Synthesis of 3-bromo-2-phenyl-1H-inden-1-one

To a solution of 2-phenyl-3-(trimethylsilyl)-1H-inden-1-one (6.0 g, 21.6 mmol) in DCM (100 mL) was added Br₂ (4.14 g, 25.9 mmol). The reaction mixture was stirred at room temperature for 2 hours and then extracted with DCM (3 x 100 mL). The combined organic layers were washed with water and brine, dried with sodium sulfate, and concentrated in vacuo. The residue was then purified by silica gel column chromatography (15 % EtOAc/PE) to obtain 3-bromo-2-phenyl-1H-inden-1-one (4.1 g, 14.3 mmol, 66.60 % yield) as a yellow solid.

MS (ESI): C₁₅H₉BrO 284.0 m/z, found 285.0 [M+1]⁺.

¹H NMR (400 MHz, DMSO-d₆) δ 7.70 - 7.56 (m, 3H), 7.55 - 7.41 (m, 5H), 7.35 - 7.33 (m, 1H).

### Step 3: Synthesis of 2-phenyl-3-(o-tolyl)-1H-inden-1-one (Compound 5)

To a mixture of 3-bromo-2-phenyl-1H-inden-1-one (200 mg, 0.7014 mmol), (2-methylphenyl)boranediol (143.04 mg, 1.0521 mmol), and tetrakis(triphenylphosphine)palladium (40.53 mg, 0.035 mmol) were added dioxane/water (5 mL; 4:1), and then K₂CO₃ (387.76 mg, 2.8056 mmol) was added. The reaction mixture was stirred at 45 °C for 5 hours. The mixture was extracted with EtOAc (3 x 30 mL). The organic layer was washed with water and brine, dried with sodium sulfate, and then concentrated in vacuo. The resulting residue was purified by prep-HPLC (Gemini 5 µm C18 column, 150 x 21.2 mm, 30 % - 90 % ACN/H₂O w/0.1 % FA) to obtain 2-phenyl-3-(o-tolyl)-1H-inden-1-one (70 mg, 0.2364 mmol, 33.72 %) as a white solid.

The obtained Compound 5 (Formula 6 below) was confirmed by MS(ESI) and¹H NMR, which yielded the following data:

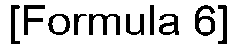

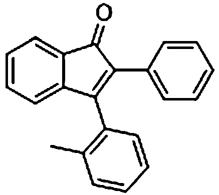

MS (ESI): C₂₂H₁₆O 296.1 m/z, found 297.1 [M+1]⁺.

¹H NMR (400 MHz, DMSO-d₆) δ 7.56 (d, J = 6.8 Hz, 1H), 7.47 (t, J = 7.2 Hz, 1H), 7.42- 7.23 (m, 8H), 7.19 (dd, J = 7.2, 2.4 Hz, 2H), 6.79 (d, J = 7.2 Hz, 1H), 2.02 (s, 3H).

### Preparation Example 6. 3-phenyl-2-(o-tolyl)-1H-inden-1-one (Compound 6)

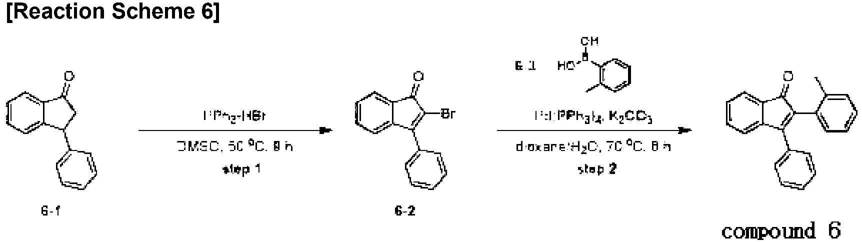

### Step 1: Synthesis of 2-bromo-3-phenyl-1H-inden-1-one

A mixture of 3-phenyl-2,3-dihydro-1H-inden-1-one (500 mg, 2.40 mmol), PPh₃·HBr (1647.98 mg, 4.80 mmol), and DMSO (10 mL) was stirred at 50 °C for 9 hours. After cooling to room temperature, the mixture was diluted with water and extracted with EA (3 x 20 mL). The combined organic layers were washed with water and brine, dried with sodium sulfate, and then concentrated in vacuo. The residue was purified by silica gel column chromatography (10 % EA/PE) to obtain 2-bromo-3-phenyl-1H-inden-1-one (500 mg, 1.6659 mmol, 69.39 % yield).

MS (ESI): C₁₅H₉BrO 284.0 m/z, found 284.6 [M+1]⁺.

¹H NMR (400 MHz, DMSO-d₆) δ 7.74 - 7.55 (m, 6H), 7.50 (t, J = 8.0 Hz, 1H), 7.38 (t, J = 7.6 Hz, 1H), 7.20 (d, J = 7.6 Hz, 1H).

### Step 2: Synthesis of 3-phenyl-2-(o-tolyl)-1H-inden-1-one (Compound 6)

A mixture of 2-bromo-3-phenyl-1H-inden-1-one (100 mg, 0.3507 mmol), o-tolylboronic acid (71.52 mg, 0.5260 mmol), Pd(PPh₃)₄ (40.53 mg, 0.0350 mmol), K₂CO₃ (96.94 mg, 0.7014 mmol), and 1,4-dioxane/water (5 mL/1 mL) was stirred at 70 °C for 6 hours. The mixture was filtered through Celite, and the filtrate was concentrated in vacuo. The residue was purified by silica gel column chromatography (10 % EA/PE) to obtain 3-phenyl-2-(o-tolyl)-1H-inden-1-one (40 mg, 0.1282 mmol, 36.56 %).

The obtained Compound 6 (Formula 7 below) was confirmed by MS(ESI) and¹H NMR, which yielded the following data:

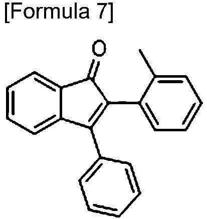

MS (ESI): C₂₂H₁₆O 296.1 m/z, found 296.7 [M+1]⁺.

¹H NMR (400 MHz, DMSO-d₆) δ 7.54 (dd, J = 14.8, 7.2 Hz, 2H), 7.41 (t, J = 5.7 Hz, 4H), 7.34 (dd, J = 6.0, 2.6 Hz, 2H), 7.30 (d, J = 7.4 Hz, 1H), 7.19 (qd, J = 14.8, 7.3 Hz, 3H), 7.05 (d, J = 7.5 Hz, 1H), 1.97 (s, 3H).

### Preparation Example 7. 3-phenyl-2-(pyridin-3-yl)-1H-inden-1-one (Compound 7)

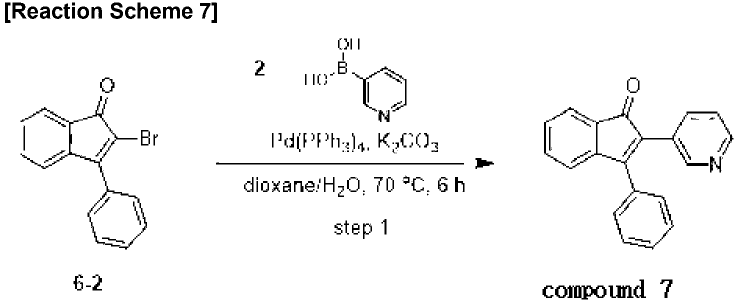

A mixture of 2-bromo-3-phenyl-1H-inden-1-one (100 mg, 0.3507 mmol), pyridin-3-ylboronic acid (64.66 mg, 0.5260 mmol), Pd(PPh₃)₄ (40.53 mg, 0.0350 mmol), K₂CO₃ (96.94 mg, 0.7014 mmol), and 1,4-dioxane/water (5 mL/1 mL) was stirred at 70 °C for 6 hours. The mixture was filtered through Celite, and the filtrate was concentrated in vacuo. The residue was purified by silica gel column chromatography (10 % EA/PE) to obtain 3-phenyl-2-(pyridin-3-yl)-1H-inden-1-one (40 mg, 0.1341 mmol, 38.24 %).

The obtained Compound 7 (Formula 8 below) was confirmed by MS(ESI) and¹H NMR, which yielded the following data:

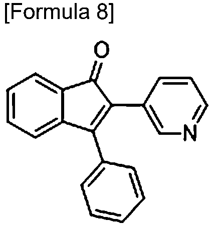

MS (ESI): C₂₀H₁₃NO 283.1 m/z, found 283.7 [M+1]⁺.

¹H NMR (400 MHz, DMSO-d₆) δ 8.46 (dd, J = 4.8, 1.6 Hz, 1H), 8.34 (d, J = 1.6 Hz, 1H), 7.64 - 7.59 (m, 2H), 7.53 (ddd, J = 10.0, 7.1, 1.7 Hz, 4H), 7.47 - 7.40 (m, 3H), 7.37 (dd, J = 7.9, 4.8 Hz, 1H), 7.21 (d, J = 7.3 Hz, 1H).

### Preparation Example 8. 2-morpholino-3-phenyl-1H-inden-1-one (Compound 8)

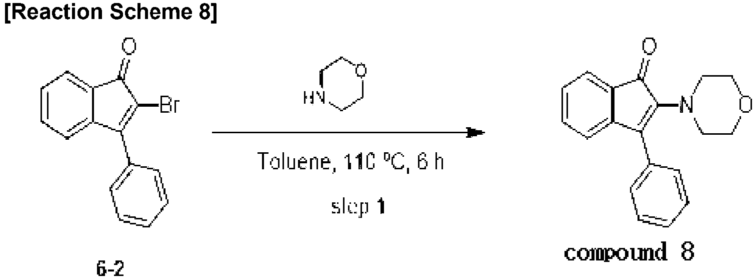

A mixture of 2-bromo-3-phenyl-1H-inden-1-one (50 mg, 0.1754 mmol), morpholine (76.4 mg, 0.877 mmol), and toluene (3 mL) was stirred at 110 °C for 6 hours. The mixture was filtered through Celite, and the filtrate was concentrated in vacuo. The residue was purified by silica gel column chromatography (10 % EA/PE) to obtain 2-morpholino-3-phenyl-1H-inden-1-one (40 mg, 0.1304 mmol, 74.34 % yield).

The obtained Compound 8 (Formula 9 below) was confirmed by MS(ESI) and¹H NMR, which yielded the following data:

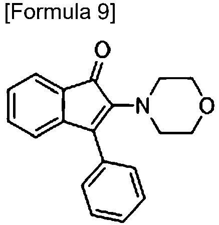

MS (ESI): C₁₉H₁₇NO₂ 291.1 m/z, found 292.0 [M+1]⁺.

¹H NMR (400 MHz, DMSO-d₆) δ 7.54 - 7.46 (m, 2H), 7.41 (dd, J = 14.9, 7.1 Hz, 3H), 7.24 (t, J = 7.5 Hz, 2H), 7.02 (t, J = 7.3 Hz, 1H), 6.65 (d, J = 7.2 Hz, 1H), 3.59 - 3.42 (m, 4H), 3.11 - 2.97 (m, 4H).

### Preparation Example 9. 2-phenyl-3-(pyridin-3-yl)-1H-inden-1-one (Compound 9)

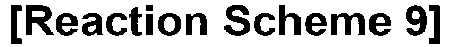

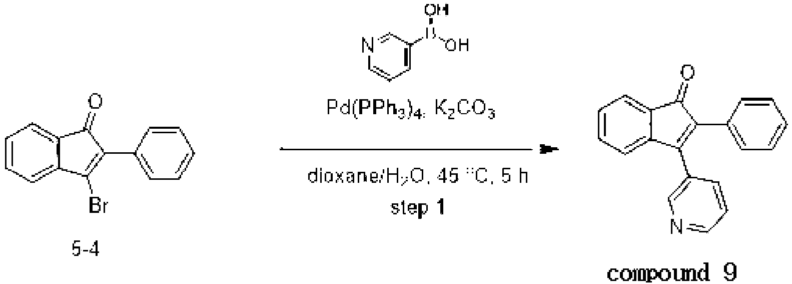

To a mixture of 3-bromo-2-phenyl-1-inden-1-one (200 mg, 0.7014 mmol), pyridin-3-ylboranediol (129.32 mg, 1.0521 mmol), and [1,1'-bis(diphenylphosphino)ferrocene]palladium(II) dichloride (51.32 mg, 0.0701 mmol) in dioxane/water (5 mL; 4:1) was added potassium carbonate (290.82 mg, 2.1042 mmol). The reaction mixture was stirred at 45 °C for 5 hours. The mixture was extracted with EtOAc (3 x 25 mL), and the organic layer was washed with water and brine, dried with sodium sulfate, and then concentrated under reduced pressure. The residue was purified by prep-HPLC (Gemini 5 µm C18 column, 150 x 21.2 mm, 40 % - 90 % ACN/H₂O with 0.1 % FA) to obtain 2-phenyl-3-(pyridin-3-yl)-1H-inden-1-one (26 mg, 0.0919 mmol, 13.10 %) as a yellow solid.

The obtained Compound 9 (Formula 10 below) was confirmed by MS(ESI) and¹H NMR, which yielded the following data:

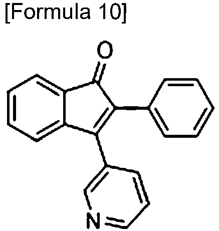

MS (ESI): C₂₀H₁₃NO 283.1 m/z, found 284.1 [M+1]⁺.

¹H NMR (400 MHz, DMSO-d₆) δ 8.64 (dd, J = 4.8, 1.6 Hz, 1H), 8.54 (d, J = 1.6 Hz, 1H), 7.89 (dt, J = 8.0, 2.0 Hz, 1H), 7.59 (d, J = 6.8 Hz, 1H), 7.53 (m, 2H), 7.42 (t, J = 7.2 Hz, 1H), 7.32 (m, 3H), 7.24 - 7.14 (m, 3H).

### Preparation Example 10. 3-cyclohexyl-2-phenyl-1H-inden-1-one (Compound 10)

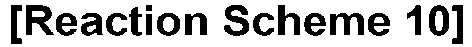

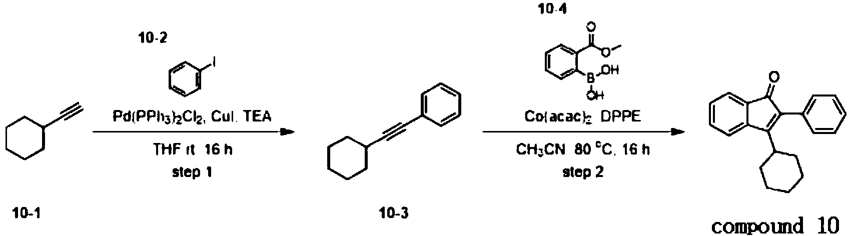

### Step 1: Synthesis of (cyclohexylethynyl)benzene

To a mixture of ethynylcyclohexane (1 g, 9.244 mmol), iodobenzene (2.07 g, 10.168 mmol), copper(I) iodide (175 mg, 0.924 mmol), and Pd(PPh₃)₂Cl₂ (645 mg, 0.924 mmol) were added THF (50 mL) and TEA (1.87 g, 18.488 mmol). The reaction mixture was stirred at room temperature for 16 hours. The mixture was filtered through Celite, and the filtrate was concentrated in vacuo. The residue was diluted with water and extracted with EtOAc (3 x 100 mL). The combined organic phase was washed with water and brine, dried with sodium sulfate, concentrated in vacuo, and then purified by silica gel column chromatography (1 % PE/EtOAc) to obtain (cyclohexylethynyl)benzene (800 mg, 4.348 mmol, 47.03 %) as a white oil.

### Step 2: Synthesis of 3-cyclohexyl-2-phenyl-1H-inden-1-one (Compound 10)

To a solution of 1,2-bis(diphenylphosphino)ethane (108.11 mg, 0.2713 mmol) in ACN (10 mL) were added (2-cyclohexylethynyl)benzene (250 mg, 1.3567 mmol), methyl 2-(dihydroxyboranyl)benzoate (366.24 mg, 2.035 mmol), and Co(acac)₂ (48.3 mg, 0.1356 mmol). The reaction mixture was stirred at 80 °C for 16 hours. The mixture was extracted with EtOAc (3 x 30 mL). The organic layer was washed with water and brine, dried with sodium sulfate, concentrated under reduced pressure, and then purified by prep-HPLC (Gemini 5 µm C18 column, 150 x 21.2 mm, 60 % - 90 % ACN/H₂O with 0.1 % FA) to obtain 3-cyclohexyl-2-phenyl-1H-inden-1-one (100 mg, 0.3472 mmol, 25.59 %).

The obtained Compound 10 (Formula 11 below) was confirmed by MS(ESI) and¹H NMR, which yielded the following data:

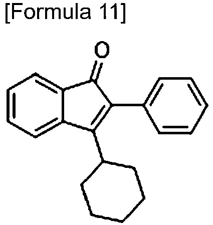

MS (ESI): C₂₁H₂₀O 288.2 m/z, found 289.2 [M+1]⁺.

¹H NMR (400 MHz, DMSO-d₆) δ 7.61 (d, J = 7.2 Hz, 1H), 7.57 - 7.43 (m, 4H), 7.39 (d, J = 7.2 Hz, 1H), 7.32 (t, J = 7.6 Hz, 1H), 7.29 - 7.21 (m, 2H), 3.15 - 2.77 (m, 1H), 2.02-1.59 (m, 7H), 1.52 - 1.13 (m, 3H).

### Preparation Example 11. 2-cyclohexyl-3-phenyl-1H-inden-1-one (Compound 11)

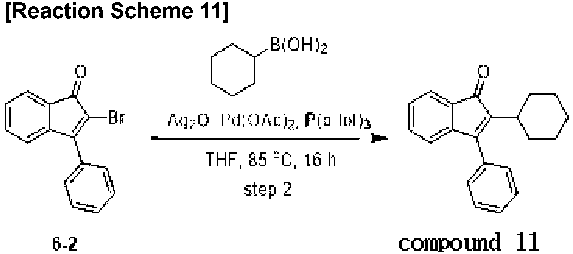

To a mixture of 2-bromo-3-phenyl-1H-inden-1-one (300 mg, 1.052 mmol), cyclohexylboranediol (269.3 mg, 2.104 mmol), and Pd(OAc)₂ (23.6 mg, 0.105 mmol) were sequentially added THF (20 mL), P(o-tol)₃ (64.2 mg, 0.210 mmol), and Ag₂O (1219.1 mg, 5.26 mmol). The reaction mixture was stirred at 85 °C for 16 hours. The mixture was extracted with DCM (3 x 60 mL). The resulting organic layer was washed with water and brine, dried with sodium sulfate, and concentrated under reduced pressure. The residue was purified by prep-HPLC (Gemini 5 µm C18 column, 150 x 21.2 mm, 60 - 90 % ACN/H₂O with 0.1 % FA) to obtain 2-cyclohexyl-3-phenyl-1H-inden-1-one (23 mg, 0.0799 mmol, 7.81 %) as a white solid.

The obtained Compound 11 (Formula 12 below) was confirmed by MS(ESI) and¹H NMR, which yielded the following data:

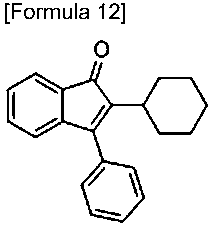

MS (ESI): C₂₁H₂₀O 288.2 m/z, found 289.2 [M+1]+.

¹H NMR (400 MHz, CDCl₃) δ 7.58 - 7.41 (m, 4H), 7.41 - 7.35 (m, 2H), 7.30 - 7.23 (m, 1H), 7.18 (t, J = 7.2 Hz, 1H), 6.89 (d, J = 7.2 Hz, 1H), 2.47 (m, 1H), 1.85 (m, 2H), 1.74 (d, J = 12.8 Hz, 2H), 1.69 - 1.51 (m, 3H), 1.35 - 1.01 (m, 3H).

### Preparation Example 12. 6-morpholino-2,3-diphenyl-1H-inden-1-one (Compound 12)

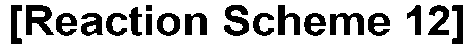

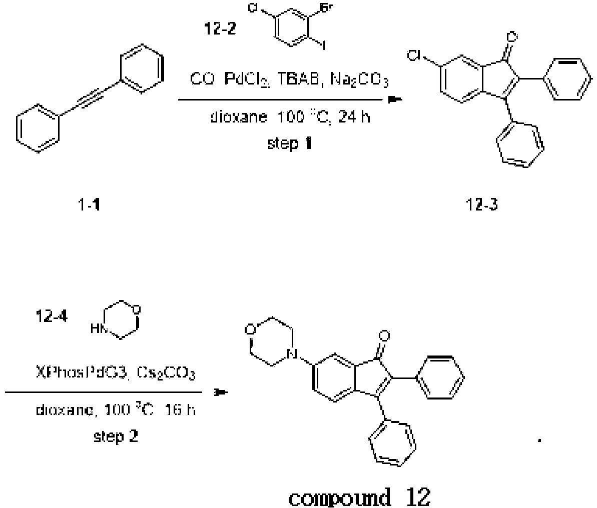

### Step 1: Synthesis of 6-chloro-2,3-diphenyl-1H-inden-1-one

To a mixture of (2-phenylethynyl)benzene (1 g, 0.0056 mol), 2-bromo-4-chloro-1-iodobenzene (3.55 g, 0.0112 mmol), and PdCl₂ (0.1 g, 0.0005 mol) were added dioxane (60 mL), sodium carbonate (1.78 g, 0.0168 mol), and tetrabutylammonium bromide (1.81 g, 0.0056 mol). The reaction mixture was stirred at 100 °C under a CO atmosphere. The mixture was cooled to room temperature and then filtered through Celite, and the filtrate was concentrated under reduced pressure. Water was added to the residue, and the mixture was extracted with EtOAc (3 x 100 mL). The organic layer was washed with water and brine, dried with sodium sulfate, and concentrated under reduced pressure. The residue was purified by silica gel column chromatography (30 % PE/EtOAc) to obtain 6-chloro-2,3-diphenyl-1H-inden-1-one (320 mg, 1.0126 mmol, 18.08 %) as a white solid.

### Step 2: Synthesis of 6-morpholino-2,3-diphenyl-1H-inden-1-one (Compound 12)

To a mixture of 6-chloro-2,3-diphenyl-1H-inden-1-one (150 mg, 0.4735 mmol), morpholine (82.5 mg, 0.947 mmol), and xphosPdG3 (40.06 mg, 0.0473 mmol) were added dioxane (6 mL) and Cs₂CO₃ (308.55 mg, 0.947 mmol). The reaction mixture was stirred at 100 °C for 16 hours. The mixture was extracted with EtOAc (3 x 20 mL). The organic layer was washed with water and brine, dried with sodium sulfate, and concentrated under reduced pressure. The residue was purified by prep-HPLC (Gemini 5 µm C₁₈ column, 150 x 21.2 mm, 30 - 90 % ACN/H₂O with 0.1 % TFA) to obtain 6-morpholino-2,3-diphenyl-1 H-inden-1-one (41 mg, 0.1117 mmol, 23.59 %) as a yellow solid.

The obtained Compound 12 (Formula 13 below) was confirmed by MS(ESI) and¹H NMR, which yielded the following data:

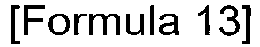

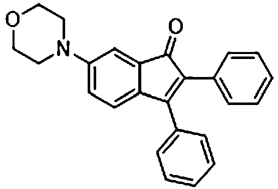

MS (ESI): C₂₃H₂₁NO₂ 367.2 m/z, found 368.2 [M+1]⁺.

¹H NMR (400 MHz, DMSO-d₆) δ 7.50 - 7.43 (m, 3H), 7.36 (m, 2H), 7.30 - 7.21 (m, 4H), 7.15 (dd, J = 7.6, 1.6 Hz, 2H), 6.98 (d, J = 8.0 Hz, 1H), 6.86 (dd, J = 8.4, 2.4 Hz, 1H), 3.77 - 3.69 (m, 4H), 3.28 - 3.06 (m, 4H).

### Preparation Example 13. 6-(4-methylpiperazin-1-yl)-2,3-diphenyl-1H-inden-1-one (Compound 13)

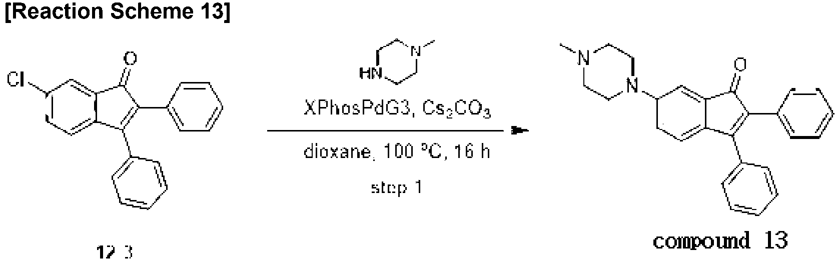

To a mixture of 6-chloro-2,3-diphenylinden-1-one (150 mg, 0.4735 mmol), 1-methylpiperazine (94.7 mg, 0.947 mmol), and xphosPdG3 (40.06 mg, 0.0473 mmol) were added dioxane (6 mL) and Cs₂CO₃ (308.55 mg, 0.947 mmol). The reaction mixture was stirred at 100 °C for 16 hours. The mixture was extracted with EtOAc (3 x 30 mL), and the organic layer was washed with water and brine, dried with sodium sulfate, concentrated under reduced pressure, and then purified by prep-HPLC (Gemini 5 µm C₁₈ column, 150 x 21.2 mm, 30 - 90 % ACN/H₂O with 0.1 % FA) to obtain 6-(4-methylpiperazin-1-yl)-2,3-diphenyl-1H-inden-1-one (35 mg, 0.0921 mmol, 19.45 %) as a yellow solid.

The obtained Compound 13 (Formula 14 below) was confirmed by MS(ESI) and¹H NMR, which yielded the following data:

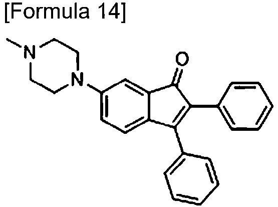

MS (ESI): C₂₆H₂₄N₂O 380.2 m/z, found 381.2 [M+1]⁺.

¹H NMR (400 MHz, DMSO-d₆) δ 7.49 - 7.42 (m, 3H), 7.36 (m, 2H), 7.31 - 7.20 (m, 4H), 7.14 (m, 2H), 6.96 (d, J = 8.0 Hz, 1H), 6.85 (dd, J = 8.0, 2.0 Hz, 1H), 3.34 - 3.16 (m, 4H), 2.48 - 2.35 (m, 4H), 2.22 (s, 3H).

### Preparation Example 14. 3-(4-morpholinophenyl)-2-phenyl-1H-inden-1-one (Compound 14)

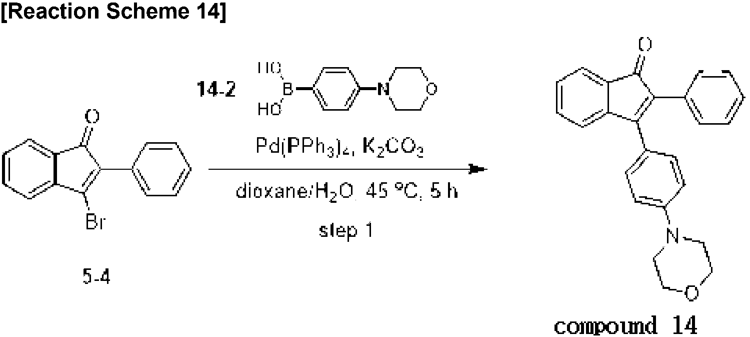

To a mixture of 3-bromo-2-phenyl-1H-inden-1-one (200 mg, 0.7014 mmol), [4-(morpholin-4-yl)phenyl]boranediol (290.43 mg, 1.4028 mmol), and tetrakis(triphenylphosphine)palladium(0) (81.05 mg, 0.0701 mmol) were added dioxane/water (5 mL; 4:1) and potassium carbonate (290.82 mg, 2.1042 mmol). The reaction mixture was stirred at 45 °C for 5 hours. The mixture was extracted with EtOAc (3 x 30 mL), and the organic layer was washed with water and brine, dried with sodium sulfate, and then concentrated under reduced pressure. The residue was then purified by prep-HPLC (Gemini 5 µm C18 column, 150 x 21.2 mm, 40 - 90 % ACN/H₂O with 0.1 % FA) to obtain 3-(4-morpholinophenyl)-2-phenyl-1H-inden-1-one (70 mg, 0.1907 mmol, 27.19 % yield).

The obtained Compound 14 (Formula 15 below) was confirmed by MS(ESI) and¹H NMR, which yielded the following data:

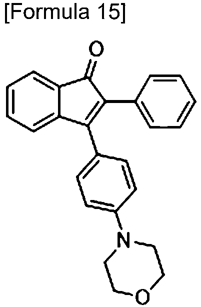

MS (ESI): mass calcd. C₂₅H₂₁NO₂ 367.2 m/z, found 368.2 [M+1]+.

¹H NMR (400 MHz, DMSO-d₆) δ 7.51 (m, 2H), 7.39 (d, J = 7.2 Hz, 1H), 7.29 (m, 6H), 7.21 (d, J = 7.6 Hz, 2H), 6.98 (d, J = 8.4 Hz, 2H), 3.95 - 3.63 (m, 4H), 3.27 - 3.17 (m, 4H).

### Preparation Example 15. 3-(3-morpholinophenyl)-2-phenyl-1H-inden-1-one (Compound 15)

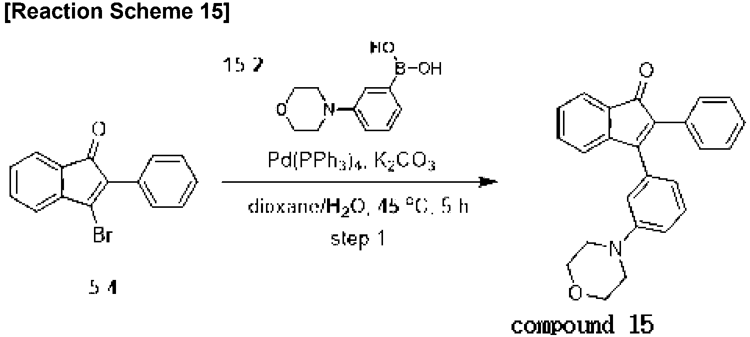

To a mixture of 3-bromo-2-phenyl-1H-inden-1-one (200 mg, 0.7014 mmol), [3-(morpholin-4-yl)phenyl]boranediol (290.43 mg, 1.4028 mmol), and tetrakis(triphenylphosphine)palladium(0) (81.05 mg, 0.0701 mmol) were added dioxane/water (5 mL; 4:1) and potassium carbonate (290.82 mg, 2.1042 mmol). The reaction mixture was stirred at 45 °C for 5 hours. The mixture was extracted with EtOAc (3 x 30 mL). The organic layer was washed with water and brine, dried with sodium sulfate, and concentrated under reduced pressure. The residue was then purified by prep-HPLC (Gemini 5 µm C18 column, 150 x 21.2 mm, 40 % - 90 % ACN/H₂O with 0.1 % FA) to obtain 3-(3-morpholinophenyl)-2-phenyl-1H-inden-1-one (135 mg, 0.3678 mmol, 52.44 %).

The obtained Compound 15 (Formula 16 below) was confirmed by MS(ESI) and¹H NMR, which yielded the following data:

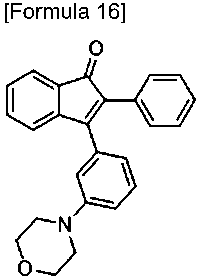

MS (ESI): C₂₅H₂₁NO₂ 367.2 m/z, found 368.2 [M+1]+.

¹H NMR (400 MHz, DMSO-d₆) δ 7.58 - 7.45 (m, 2H), 7.38 (t, J = 7.2 Hz, 1H), 7.34 - 7.25 (m, 4H), 7.24 - 7.17 (m, 3H), 7.04 (d, J = 8.4 Hz, 1H), 6.90 (s, 1H), 6.80 (d, J = 7.2 Hz, 1H), 4.01 - 3.54 (m, 4H), 3.09 - 2.90 (m, 4H).

### Preparation Example 16. 3-(4-(morpholinomethyl)phenyl)-2-phenyl-1H-inden-1-one (Compound 16)

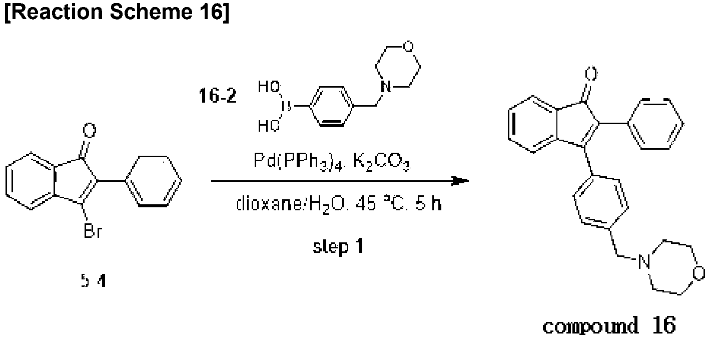

3-bromo-2-phenyl-1H-inden-1-one (200 mg, 0.7014 mmol), [4-(morpholin-4-ylmethyl)phenyl]boranediol (310.10 mg, 1.4028 mmol), and tetrakis(triphenylphosphine)palladium(0) (81.05 mg, 0.0701 mmol) were added to potassium carbonate (290.82 mg, 2.1042 mmol) in dioxane/water (5 mL; 4:1). The reaction mixture was stirred at 45 °C for 5 hours. The mixture was extracted with EtOAc (3 x 30 mL). The combined organic phase was washed with water and brine, dried with sodium sulfate, and concentrated in vacuo. The residue was then purified by prep-HPLC (Gemini 5 µm C18 column, 150 x 21.2 mm, from 40 % to 90 % ACN/H₂O containing 0.1 % FA) to obtain 3-(4-(morpholinomethyl)phenyl)-2-phenyl-1H-inden-1-one (100 mg, 0.2625 mmol, 37.42 % yield).

The obtained Compound 16 (Formula 17 below) was confirmed by MS(ESI) and¹H NMR, which yielded the following data:

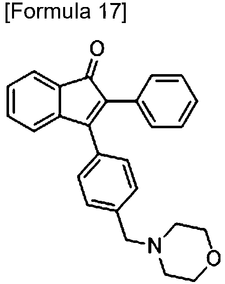

MS (ESI): C₂₆H₂₃NO₂ 381.2 m/z, found 382.2 [M+1]+.

¹H NMR (400 MHz, DMSO-d6) δ 7.55 (d, J = 6.8 Hz, 1H), 7.51 (m, 1H), 7.43 - 7.33 (m, 5H), 7.33 - 7.26 (m, 3H), 7.23 - 7.15 (m, 3H), 3.64 - 3.55 (m, 4H), 3.51 (s, 2H), 2.37 (s, 4H).

### Preparation Example 17. 2-(4-morpholinophenyl)-3-phenyl-1H-inden-1-one (Compound 17)

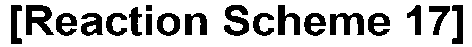

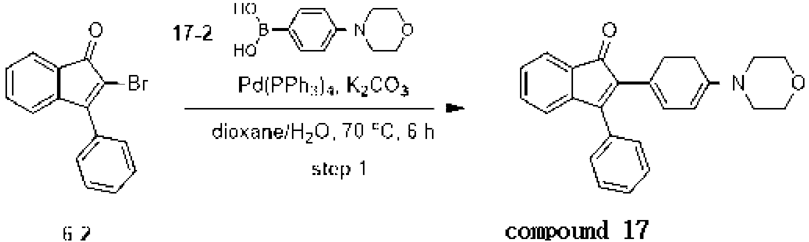

A mixture of 2-bromo-3-phenyl-1H-inden-1-one (100 mg, 0.3507 mmol), (4-morpholinophenyl)boronic acid (108.91 mg, 0.5260 mmol), Pd(PPh₃)₄ (40.53 mg, 0.0350 mmol), K₂CO₃ (96.94 mg, 0.7014 mmol), and 1,4-dioxane/water (5 mL/1 mL) was stirred at 70 °C for 6 hours. The mixture was filtered through Celite, and the filtrate was concentrated under reduced pressure. The residue was purified by silica gel column chromatography (20 % EA/PE) to obtain 2-(4-morpholinophenyl)-3-phenyl-1H-inden-1-one (60 mg, 0.1551 mmol, 44.23 % yield).

The obtained Compound 17 (Formula 18 below) was confirmed by MS(ESI) and¹H NMR, which yielded the following data:

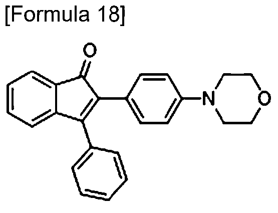

MS (ESI): C₂₅H₂₁NO₂ 367.2 m/z, found 368.1 [M+1]+

¹H NMR (400 MHz, DMSO-d₆) δ 7.53 - 7.44 (m, 5H), 7.41 (dd, *J* = 7.7, 1.6 Hz, 2H), 7.33 (t, *J = 7.2* Hz, 1H), 7.09 (dd, *J* = 11.5, 8.1 Hz, 3H), 6.84 (d, *J* = 8.9 Hz, 2H), 3.76 - 3.61 (m, 4H), 3.16 - 3.06 (m, 4H).

### Preparation Example 18. 2-(3-morpholinophenyl)-3-phenyl-1H-inden-1-one (Compound 18)

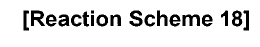

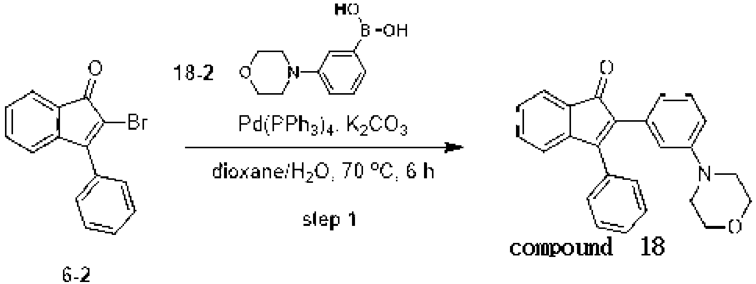

A mixture of 2-bromo-3-phenyl-1H-inden-1-one (100 mg, 0.3507 mmol), (3-morpholinophenyl)boronic acid (108.91 mg, 0.5260 mmol), Pd(PPh₃)₄ (40.53 mg, 0.0350 mmol), K₂CO₃ (96.94 mg, 0.7014 mmol), and 1,4-dioxane/water (5 mL/1 mL) was stirred at 70 °C for 6 hours. The mixture was filtered through Celite, and the filtrate was concentrated under reduced pressure. The residue was purified by silica gel column chromatography (20 % EA/PE) to obtain 2-(3-morpholinophenyl)-3-phenyl-1H-inden-1-one (50 mg, 0.1293 mmol, 36.87 %).

The obtained Compound 18 (Formula 19 below) was confirmed by MS(ESI) and¹H NMR, which yielded the following data:

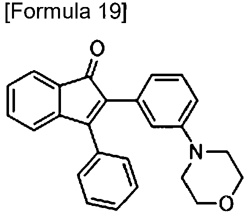

MS (ESI): C₂₅H₂₁NO₂ 367.2 m/z, found 367.7 [M+1]+.

¹H NMR (400 MHz, DMSO-d₆) δ 7.54 (d, *J* = 6.9 Hz, 1H), 7.48 (dt, *J* = 8.0, 5.0 Hz, 4H), 7.43 - 7.35 (m, 3H), 7.15 (dd, J = 9.7, 7.6 Hz, 2H), 6.87 (dd, *J* = 8.2, 2.2 Hz, 1H), 6.72 (s, 1H), 6.67 (d, *J* = 7.6 Hz, 1H), 3.72 - 3.57 (m, 4H), 2.96 - 2.84 (m, 4H).

### Preparation Example 19. 3-(4-methylthiazol-5-yl)-2-(pyridin-3-yl)-1H-inden-1-one (Compound 19)

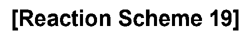

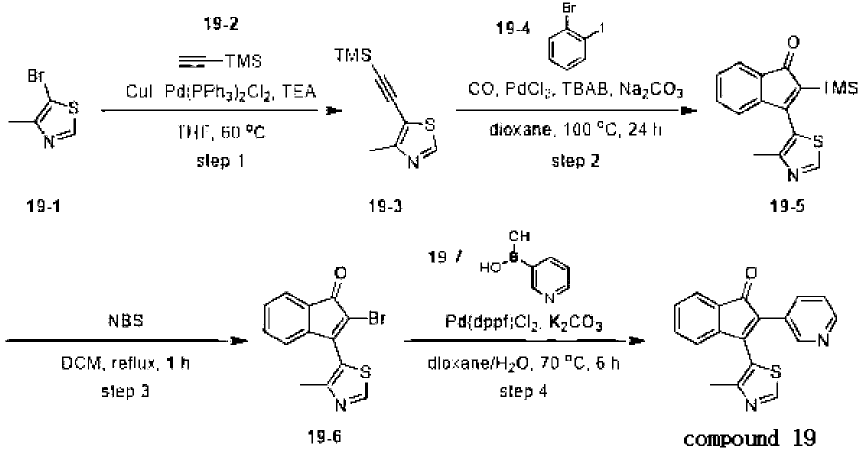

### Step 1: Synthesis of 4-methyl-5-((trimethylsilyl)ethynyl)thiazole

To a mixture of 5-bromo-4-methyl-1,3-thiazole (1 g, 0.0056 mol), ethynyltrimethylsilane (1.1 g, 0.0112 mmol), and copper(I) iodide (0.11 g, 0.0005 mol) were added TEA (20 mL) and [1,1'-bis(diphenylphosphino)ferrocene]palladium(II) dichloride (0.41 g, 0.0005 mol). The reaction mixture was stirred at 60 °C for 12 hours under N₂. The reaction mixture was cooled to room temperature and then passed through Celite, and the filtrate was concentrated under reduced pressure. The residue was diluted with water and extracted with EtOAc (3 x 60 mL). The organic layer was washed with water and brine, dried with sodium sulfate, and concentrated under reduced pressure. The residue was then purified by silica gel column chromatography (10 % EtOAc/PE) to obtain 4-methyl-5-((trimethylsilyl)ethynyl)thiazole (800 mg, 4.1026 mmol, 73.26 % yield) as a red oil.

MS (ESI): C₉H₁₃NSSi 195.1 m/z, found 196.1 [M+1]+.

### Step 2: Synthesis of 3-(4-methylthiazol-5-yl)-2-(trimethylsilyl)-1H-inden-1-one

To a mixture of 4-methyl-5-[2-(trimethylsilyl)ethynyl]-1,3-thiazole (500 mg, 2.559 mmol), 1-bromo-2-iodobenzene (1447.9 mg, 5.118 mmol), and PdCl₂ (45.4 mg, 0.255 mmol) were added dioxane (40 mL), sodium carbonate (813.7 mg, 7.677 mmol), and tetrabutylammonium bromide (824.9 mg, 2.559 mmol). The reaction mixture was stirred at 100 °C under a CO atmosphere. The reaction mixture was cooled to room temperature and then extracted with DCM (3 x 100 mL). The organic layer was washed with water and brine, dried with sodium sulfate, and concentrated under reduced pressure. The residue was then purified by silica gel column chromatography (15 % EtOAc/PE) to obtain 3-(4-methylthiazol-5-yl)-2-(trimethylsilyl)-1H-inden-1-one (200 mg, 0.6667 mmol, 26.05 %) as a yellow solid.

### Step 3: Synthesis of 2-bromo-3-(4-methylthiazol-5-yl)-1H-inden-1-one

To a mixture of 3-(4-methylthiazol-5-yl)-2-(trimethylsilyl)inden-1-one (200 mg, 0.668 mmol) in DCM (5 mL) was added N-bromosuccinimide (237.8 mg, 1.336 mmol). The reaction mixture was stirred at 40 °C for 1 hour. The mixture was extracted with DCM (3 x 25 mL). The organic layer was washed with water and brine, dried with sodium sulfate, and concentrated under reduced pressure. The residue was purified by silica gel column chromatography (35 % EtOAc/PE) to obtain 2-bromo-3-(4-methylthiazol-5-yl)-1H-inden-1-one (160 mg, 0.5246 mol, 78.53 %) as a yellow solid.

### Step 4: Synthesis of 3-(4-methylthiazol-5-yl)-2-(pyridin-3-yl)-1H-inden-1-one (Compound 19)

To a mixture of 2-bromo-3-(4-methyl-thiazol-5-yl)-1H-inden-1-one (160 mg, 0.5232 mmol), pyridin-3-ylboranediol (128.64 mg, 1.0464 mmol), and [1,1'-bis(diphenylphosphino)ferrocene]palladium(II) dichloride (38.24 mg, 0.0512 mmol) were added dioxane/water (8 mL; 4:1) and potassium carbonate (216.96 mg, 1.5696 mmol). The reaction mixture was stirred at 70 °C for 6 hours. The mixture was extracted with EtOAc (3 x 40 mL). The organic layer was washed with water and brine, dried with sodium sulfate, and concentrated under reduced pressure. The residue was purified by prep-HPLC (Gemini 5 µm C18 column, 150 x 21.2 mm, 30 % - 90 % ACN/H₂O with 0.1% FA) to obtain 3-(4-methylthiazol-5-yl)-2-(pyridin-3-yl)-1H-inden-1-one (30 mg, 0.0986 mmol, 18.86 %) as a yellow solid.

The obtained Compound 19 (Formula 20 below) was confirmed by MS(ESI) and¹H NMR, which yielded the following data:

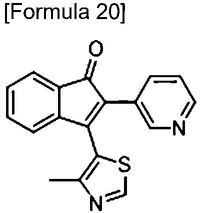

MS (ESI): C₁₃H₁₂N₂OS 304.1 m/z, found 305.1 [M+1]+.

¹H NMR (400 MHz, DMSO) δ 9.32 (s, 1H), 8.51 (dd, *J* = 4.8, 1.6 Hz, 1H), 8.38 (d, *J* = 1.6 Hz, 1H), 7.72 - 7.55 (m, 3H), 7.52 - 7.39 (m, 2H), 7.25 (d, *J* = 7.6 Hz, 1H), 1.97 (s, 3H).

### Preparation Example 20. 3-(4-methylthiazol-5-yl)-6-((3-phenylpropyl)amino)-2-(pyridin-3-yl)-1H-inden-1-one (Compound 20)

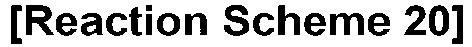

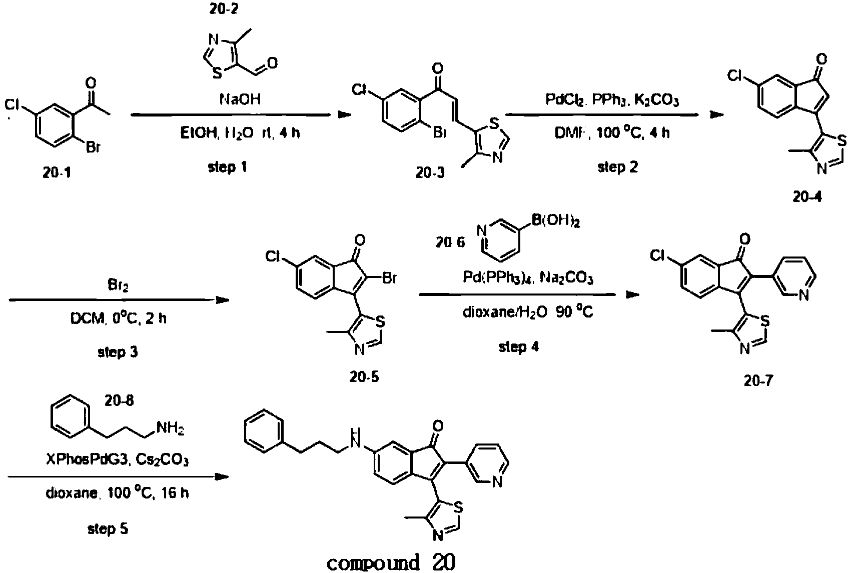

### Step 1: Synthesis of (E)-1-(2-bromo-5-chlorophenyl)-3-(4-methylthiazol-5-yl)prop-2-en-1-one

A mixture of 5-methyl-1,3-thiazole-4-carbaldehyde (2 g, 0.0086 mol) and 1-(3-hydroxyphenyl)ethanone (1.2 g, 0.0094 mol) in EtOH (30 mL) was stirred at 20-25 °C for 10 minutes. After the resulting solution was cooled to 0-5 °C, H₂O (5 mL) and NaOH (516 mg, 0.0129 mol) were added. Then, the mixture was stirred at room temperature for 4 hours, adjusted to pH = 7 with 1N HCl, and the resulting solid was collected by filtration to obtain (E)-1-(2-bromo-5-chlorophenyl)-3-(4-methylthiazol-5-yl)prop-2-en-1-one (2.1 g, 0.0061 mmol, 70.93 % yield).

### Step 2: Synthesis of 6-chloro-3-(4-methylthiazol-5-yl)-1H-inden-1-one

To a mixture of (E)-1-(2-bromo-5-chlorophenyl)-3-(4-methylthiazol-5-yl)prop-2-en-1-one (2 g, 5.8372 mmol), PdCl₂ (103.52 mg, 0.2536 mmol), and PPh₃ (306.2 mg, 1.1672 mmol) were added DMF (50 mL) and potassium carbonate (1.6 g, 11.6744 mmol). The reaction mixture was stirred at 100 °C for 4 hours. The mixture was filtered through Celite, and the filtrate was concentrated under reduced pressure. Water was added to the residue, and the mixture was extracted with DCM (3 x 120 mL). The organic layer was washed with water and brine, dried with sodium sulfate, and concentrated under reduced pressure. The residue was purified by silica gel column chromatography (2 % MeOH/DCM) to obtain 6-chloro-3-(4-methylthiazol-5-yl)-1H-inden-1-one (550 mg, 2.1072 mmol, 36.33 %) as a yellow solid.

### Step 3: Synthesis of 2-bromo-6-chloro-3-(4-methylthiazol-5-yl)-1H-inden-1-one

To a solution of 6-chloro-3-(4-methylthiazol-5-yl)-1H-inden-1-one (400 mg, 1.5283 mmol) in DCM (5 mL) was added Br₂ (268.66 mg, 1.6811 mmol). The reaction mixture was stirred at 0 °C for 2 hours. The reaction mixture was treated with water and extracted with DCM (3 x 20 mL). The organic layer was washed with water and brine, dried with sodium sulfate, and concentrated under reduced pressure. The residue was then purified by silica gel column chromatography (2 % MeOH/DCM) to obtain 2-bromo-6-chloro-3-(4-methylthiazol-5-yl)-1H-inden-1-one (350 mg, 1.0294 mmol, 67.37 % yield) as a yellow solid.

### Step 4: Synthesis of 6-chloro-3-(4-methylthiazol-5-yl)-2-(pyridin-3-yl)-1H-inden-1-one

To a mixture of 2-bromo-6-chloro-3-(4-methylthiazol-5-yl)-1H-inden-1-one (200 mg, 0.587 mmol), pyridin-3-ylboranediol (108.2 mg, 0.88 mmol), and tetrakis(triphenylphosphine)palladium(0) (67.8 mg, 0.058 mmol) were added dioxane/water (10 mL; 4:1) and Na₂CO₃ (124.4 mg, 1.174 mmol). The reaction mixture was stirred at 90 °C for 5 hours. The mixture was filtered through Celite, and the filtrate was concentrated under reduced pressure. The residue was diluted with water and extracted with DCM (3 x 90 mL). The organic layer was washed with water and brine, dried with sodium sulfate, concentrated in vacuo, and then purified by silica gel column chromatography (10 % MeOH/DCM) to obtain 6-chloro-3-(4-methylthiazol-5-yl)-2-(pyridin-3-yl)-1H-inden-1-one (170 mg, 0.5060 mmol, 86.20 % yield).

### Step 5: Synthesis of 3-(4-methylthiazol-5-yl)-6-((3-phenylpropyl)amino)-2-(pyridin-3-yl)-1H-inden-1-one (Compound 20)

To a mixture of 6-chloro-3-(4-methylthiazol-5-yl)-2-(pyridin-3-yl)-1H-inden-1-one (100 mg, 0.2951 mmol), 2-phenylethanamine (71.52 mg, 0.5902 mmol), and xphosPdG3 (24.97 mg, 0.0295 mmol) were added dioxane (7 mL) and Cs₂CO₃ (288.45 mg, 0.8853 mmol). The reaction mixture was stirred at 100 °C for 16 hours under N₂. The mixture was filtered through Celite, and the filtrate was concentrated under in vacuo. The residue was diluted with water and extracted with EtOAc (3 x 40 mL). The organic layer was washed with water and brine, dried with sodium sulfate, concentrated in vacuo, and then purified by prep-HPLC (Gemini 5 µm C18 column, 150 x 21.2 mm, 30 % - 90 % ACN/H₂O with 0.1% FA) to obtain 3-(4-methylthiazol-5-yl)-6-((3-phenylpropyl)amino)-2-(pyridin-3-yl)-1H-inden-1-one (40 mg, 0.0915 mmol, 31.01 %) as a red solid.

The obtained Compound 20 (Formula 21 below) was confirmed by MS (ESI) and¹H NMR, which yielded the following data:

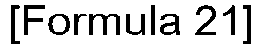

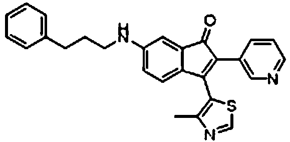

MS (ESI): C₂₇H₂₃N₃OS 437.2 m/z, found 438.2 [M+1]+.

¹H NMR (400 MHz, DMSO-d₆) δ 9.28 (s, 1H), 8.43 (dd, *J* = 4.8, 1.2 Hz, 1H), 8.31 (d, *J* = 1.2 Hz, 1H), 7.55 (d, *J* = 8.0 Hz, 1H), 7.42 - 7.13 (m, 6H), 7.01 - 6.86 (m, 2H), 6.62 (t, *J* = 5.2 Hz, 1H), 6.44 (dd, *J* = 8.4, 2.0 Hz, 1H), 3.10 (dd, *J* = 12.8, 6.8 Hz, 2H), 2.75 - 2.60 (m, 2H), 1.94 (s, 3H), 1.86 (m, 2H).

### Preparation Example 21. 2-(4-methylpyridin-3-yl)-3-(4-methylthiazol-5-yl)-6-(3-phenylpropoxy)-1H-inden-1-one (Compound 21)

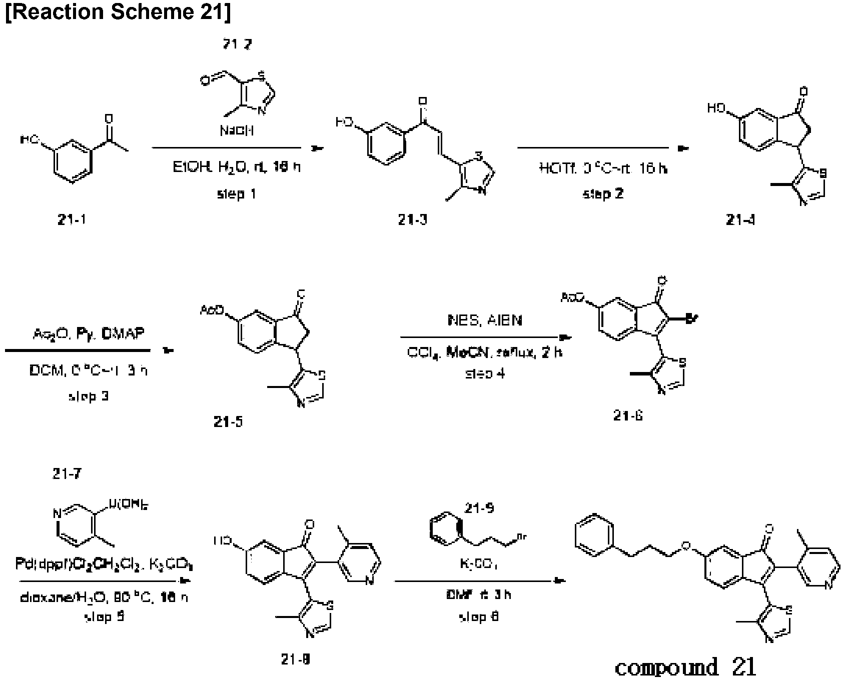

### Step 1: Synthesis of (E)-1-(3-hydroxyphenyl)-3-(4-methylthiazol-5-yl)prop-2-en-1-one

To a solution of 1-(3-hydroxyphenyl)ethanone (4.50 g, 33.1 mmol) and 4-methyl-1,3-thiazole-5-carbaldehyde (6.31 g, 49.6 mmol) in ethanol (100 mL) was added a solution of NaOH (2.65 g, 66.2 mmol, in 20 mL of H₂O) at 0 °C. The reaction mixture was stirred at 25 °C for 16 hours. The reaction mixture was concentrated, dissolved in ethyl acetate, and then adjusted to pH = 3 with a 1N HCl solution. Then, the mixture was extracted with EtOAc (3 x 200 mL), and the organic layer was washed with brine, dried with Na₂SO₄, and concentrated to obtain (E)-1-(3-hydroxyphenyl)-3-(4-methylthiazol-5-yl)prop-2-en-1-one (2.8 g, 90 % purity, 10.3 mmol, 31.1 % yield) as a yellow solid.

MS (ESI): C₁₃H₁₁NO₂S 245.1 m/z, found 246.1 [M+1]+.

### Step 2: Synthesis of 6-hydroxy-3-(4-methylthiazol-5-yl)-2,3-dihydro-1H-inden-1-one

To HOTf (25 mL) was added (E)-1-(3-hydroxyphenyl)-3-(4-methylthiazol-5-yl)prop-2-en-1-one (2.2 g, 9.00 mmol) portion-wise at 0 - 5 °C. The obtained mixture was stirred at 25 °C for 16 hours and then concentrated. The residue was then dissolved in DCM/water (50 mL/50 mL) and extracted with DCM (2 x 50 mL). Drying and concentration under reduced pressure yielded 6-hydroxy-3-(4-methylthiazol-5-yl)-2,3-dihydro-1 H-inden-1-one (2.2 g, 95 % purity, 8.53 mmol, 94.4 % yield) as a yellow solid.

MS (ESI): C₁₃H₁₁NO₂S 245.1 m/z, found 246.1 [M+1]+.

### Step 3: Synthesis of 1-(4-methylthiazol-5-yl)-3-oxo-2,3-dihydro-1H-inden-5-yl acetate

To a solution of 6-hydroxy-3-(4-methylthiazol-5-yl)-2,3-dihydro-1H-inden-1-one (2.2 g, 8.98 mmol) and pyridine (2.14 g, 26.9 mmol) in DCM (30 mL) was added Ac2O (2.76 g, 26.9 mmol) at 0 °C. The reaction mixture was stirred at 25 °C for 3 hours, and then treated with water (50 mL). Afterward, the mixture was extracted with DCM (3 x 50 mL), and the organic layer was washed with brine, dried with Na₂SO₄, concentrated, and then purified by silica gel column chromatography (6 % MeOH/DCM) to obtain 1-(4-methylthiazol-5-yl)-3-oxo-2,3-dihydro-1H-inden-5-yl acetate (2.4 g, 95 % purity, 7.94 mmol, 88.4 % yield) as a yellow solid.

MS (ESI): C₁₅H₁₃NO₃S 287.1 m/z, found 288.1 [M+1]+.

### Step 4: Synthesis of 2-bromo-3-(4-methylthiazol-5-yl)-1-oxo-1H-inden-6-yl acetate

A mixture of 1-(4-methylthiazol-5-yl)-3-oxo-2,3-dihydro-1H-inden-5-yl acetate (1.22 g, 4.25 mmol), NBS (1.64 g, 9.24 mmol), AIBN (0.07 g, 0.42 mmol), CCl₄ (20 mL), and ACN (4 mL) was stirred at 70 °C for 2 hours. The mixture was concentrated under reduced pressure and purified by silica gel column chromatography (15 % EtOAc/PE) to obtain 2-bromo-3-(4-methylthiazol-5-yl)-1-oxo-1H-inden-6-yl acetate (1.42 g, 80 % purity, 3.12 mmol, 73.4 %) as a red oil.

MS (ESI): C₁₅H₁₀BrNO₃S 363.0 m/z, found 364.1 [M+1]+.

### Step 5: Synthesis of 6-hydroxy-2-(4-methylpyridin-3-yl)-3-(4-methylthiazol-5-yl)-1H-inden-1-one

To a mixture of 2-bromo-3-(4-methylthiazol-5-yl)-1-oxo-1H-inden-6-yl acetate (1.42 g, 3.90 mmol), (4-methylpyridin-3-yl)boranediol (641 mg, 4.68 mmol), Pd(dppf)Cl2CH2Cl2 (159 mg, 0.195 mmol), and K₂CO₃ (1.62 g, 11.7 mmol) were added dioxane (15 mL) and water (3 mL), and the resulting mixture was stirred at 90 °C for 16 hours. Water (30 mL) was added to the resulting mixture, and the mixture was extracted with EtOAc (3 x 30 mL). The organic layer was washed with brine, dried with Na₂SO₄, concentrated, and then purified by silica gel column chromatography (6 % MeOH/DCM) to obtain 6-hydroxy-2-(4-methylpyridin-3-yl)-3-(4-methylthiazol-5-yl)-1H-inden-1-one (21-8) (600 mg, 70 % purity, 1.26 mmol, 32.2 %) as a yellow solid. The compound (260 mg, 70 % purity) was further purified by prep-HPLC (Gemini 5 µm C18 column, 150 x 21.2 mm, 22 - 55 % ACN/H₂O with 0.1 % FA) to obtain the title compound (20 mg, 98 % purity).

MS (ESI): C₁₉H₁₄N₂O₂S 334.1 m/z, found 349.1 [M+1]+.

¹H NMR (400 MHz, DMSO-d₆) δ 10.37 (br, 1H), 9.23 (s, 1H), 8.36 (d, *J* = 4.8Hz, 1H), 8.12 (s, 1H), 7.27 - 7.26 (m, 1H), 7.11 - 7.09 (m, 1H), 6.99 - 6.98 (m, 1H), 6.85 - 6.83 (m, 1H), 2.03 (s, 3H), 1.95 (s, 3H).

### Step 6: Synthesis of 2-(4-methylpyridin-3-yl)-3-(4-methylthiazol-5-yl)-6-(3-phenylpropoxy)-1H-inden-1-one (Compound 21)

A mixture of 6-hydroxy-2-(4-methylpyridin-3-yl)-3-(4-methylthiazol-5-yl)-1H-inden-1-one (21-8) (240 mg, 0.718 mmol), (3-bromopropyl)benzene (286 mg, 1.44 mmol), K₂CO₃ (297 mg, 2.15 mmol), and DMF (8 mL) was stirred at 25 °C for 3 hours. The resulting mixture was treated with water (20 mL) and then extracted with EtOAc (3 x 20 mL). The combined organic phase was washed with brine, dried with Na₂SO₄, concentrated, and then purified by prep-HPLC (Gemini 5 µm C18 column, 150 x 21.2 mm, 65 - 95 % ACN/H₂O with 0.1 % NH₃H₂O) to obtain 2-(4-methylpyridin-3-yl)-3-(4-methylthiazol-5-yl)-6-(3-phenylpropoxy)-1H-inden-1-one (Compound 21) (60 mg, 95.6 % purity, 0.126 mmol, 17.5 % yield).

The obtained Compound 21 (Formula 22 below) was confirmed by MS (ESI) and 1H NMR, which yielded the following data:

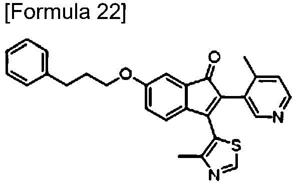

MS (ESI): C₂₈H₂₄N₂O₂S 452.2 m/z, found 453.2 [M+1]+.

¹H NMR (400 MHz, DMSO-d₆) δ 9.24 (s, 1H), 8.36 (d, *J =* 4.8Hz, 1H), 8.14 (s, 1H), 7.30 - 7.17 (m, 8H), 7.04 - 7.01 (m, 1H), 4.06 (t, *J* = 6.4Hz, 2H), 2.75 (t, *J* = 7.6Hz, 2H), 2.07 - 2.01 (m, 5H), 1.96 (s, 3H).

### Preparation Example 22. 6-methoxy-2-(4-methylpyridin-3-yl)-3-(4-methylthiazol-5-yl)-1H-inden-1-one (Compound 22)

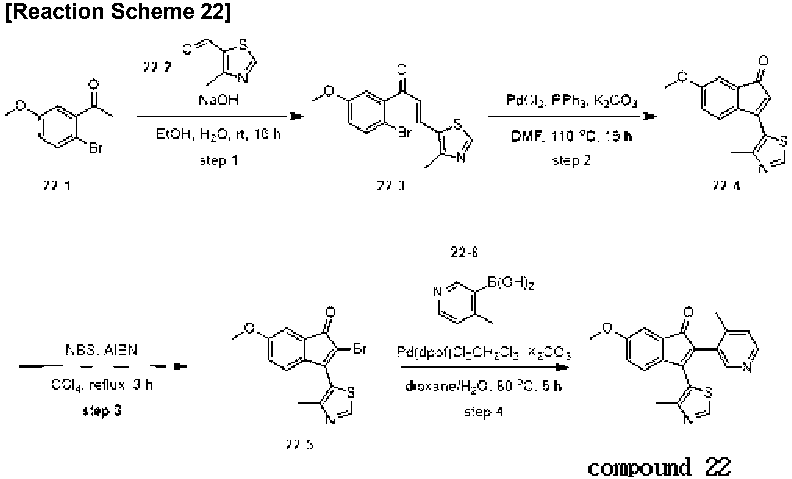

### Step 1: Synthesis of (E)-1-(2-bromo-5-methoxyphenyl)-3-(4-methylthiazol-5-yl)prop-2-en-1-one

To a mixture of 1-(2-bromo-5-methoxyphenyl)ethanone (4.56 g, 0.02 mol) and 4-methyl-1,3-thiazole-5-carbaldehyde (3.81 g, 0.03 mol) in ethanol (50 mL) was added a solution of NaOH (2.39 g, 0.06 mol in 10 mL H₂O) at 0 °C. The resulting mixture was stirred at 25 °C for 3 hours, and then water (100 mL) was added. Then, the mixture was extracted with DCM (100 mL x 3), and after the organic layer was washed with brine and dried with Na₂SO₄, it was concentrated, and the residue was purified by silica gel column chromatography (5 - 10 % MeOH/DCM) to obtain (E)-1-(2-bromo-5-methoxyphenyl)-3-(4-methylthiazol-5-yl)prop-2-en-1-one (3) (6.6 g, 80 % purity, 0.157 mol, 78.1 % yield) as a yellow solid.

MS (ESI): C₁₄H₁₂BrNO₂ 337.0 m/z, found 338.1 [M+1]+.

### Step 2: Synthesis of 6-methoxy-3-(4-methylthiazol-5-yl)-1H-inden-1-one

A mixture of (E)-1-(2-bromo-5-methoxyphenyl)-3-(4-methylthiazol-5-yl)prop-2-en-1-one (6.00 g, 17.7 mmol), PdCl₂ (160 mg, 0.885 mmol), PPh₃ (460 mg, 1.77 mmol), and K₂CO₃ (6.12 g, 44.3 mmol) in DMF (60 mL) was stirred at 110 °C for 16 hours. After the resulting mixture was cooled to room temperature, water (200 mL) was added, and the mixture was extracted with EtOAc (3 x 150 mL). The organic layer was washed with brine, dried with Na₂SO₄, and concentrated. The residue was then purified by silica gel column chromatography (15 % EtOAc/PE) to obtain 6-methoxy-3-(4-methylthiazol-5-yl)-1H-inden-1-one (22-4) (600 mg, 95 % purity, 2.22 mmol, 12.5 % yield) as a yellow solid.

MS (ESI): C₁₄H₁₁NO₂S 257.1 m/z, found 258.1 [M+1]+.

### Step 3: Synthesis of 2-bromo-6-methoxy-3-(4-methylthiazol-5-yl)-1H-inden-1-one

To a mixture of 6-methoxy-3-(4-methylthiazol-5-yl)-1H-inden-1-one (22-4) (600 mg, 2.33 mmol), NBS (498 mg, 2.80 mmol), and AIBN (76.6 mg, 0.466 mmol) was added CCl₄ (12 mL), and the resulting mixture was stirred at 70 °C for 3 hours. The mixture was concentrated under reduced pressure, and the residue was purified by silica gel column chromatography (15 % EtOAc/PE) to obtain 2-bromo-6-methoxy-3-(4-methylthiazol-5-yl)-1 H-inden-1-one (22-5) (400 mg, 80 % purity, 0.952 mmol, 40.8 % yield).

MS (ESI): C₁₄H₁₀BrNO₂S 335.0 m/z, found 336.1 [M+1]+.

### Step 4: Synthesis of 6-methoxy-2-(4-methylpyridin-3-yl)-3-(4-methylthiazol-5-yl)-1H-inden-1-one (Compound 22)

To a mixture of 2-bromo-6-methoxy-3-(4-methylthiazol-5-yl)-1H-inden-1-one (22-5) (400 mg, 1.19 mmol), (4-methylpyridin-3-yl)boranediol (196 mg, 1.43 mmol), Pd(dppf)Cl₂·CH₂Cl₂ (48.6 mg, 0.059 mmol), and K₂CO₃ (493 mg, 3.57 mmol) were added dioxane (8 mL) and water (2 mL), and the resulting mixture was stirred at 85 °C for 16 hours. The resulting mixture was treated with water (30 mL), and then extracted with EtOAc (3 x 30 mL). The combined organic phase was washed with brine, dried with Na₂SO₄, concentrated, and then purified by silica gel column chromatography (6 % MeOH/DCM) to obtain 6-methoxy-2-(4-methylpyridin-3-yl)-3-(4-methylthiazol-5-yl)-1H-inden-1-one (Compound 22) (300 mg, 90 % purity, 0.776 mmol, 65.1 %) as a yellow solid. Compound 22 (190 mg, 90 % purity) was purified by prep-HPLC (Gemini 5 µm C18 column, 150 x 21.2 mm, 25 - 70 % ACN/H₂O with 0.1 % FA) to obtain 91 mg (99 % purity) of the title compound.

The obtained Compound 22 (Formula 23 below) was confirmed by MS(ESI) and¹H NMR, which yielded the following data:

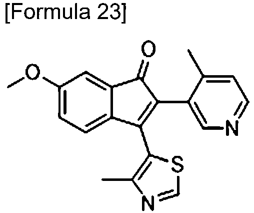

MS (ESI): C₂₀H₁₆N₂O₂S 348.1 m/z, found 349.1 [M+1]+.

¹H NMR (400 MHz, DMSO-d₆) δ 9.26 (s, 1H), 8.38 (d, *J* = 4.8Hz, 1H), 8.14 (s, 1H), 7.28 - 7.18 (m, 3H), 7.05 - 7.02 (m, 1H), 3.85 (s, 3H), 2.04 (s, 3H), 1.98 (s, 3H).

### Preparation Example 23. 3-(5-methylthiazol-4-yl)-2-(pyridin-3-yl)-6-(3-(pyridin-4-yl)propoxy)-1H-inden-1-one (Compound 23)

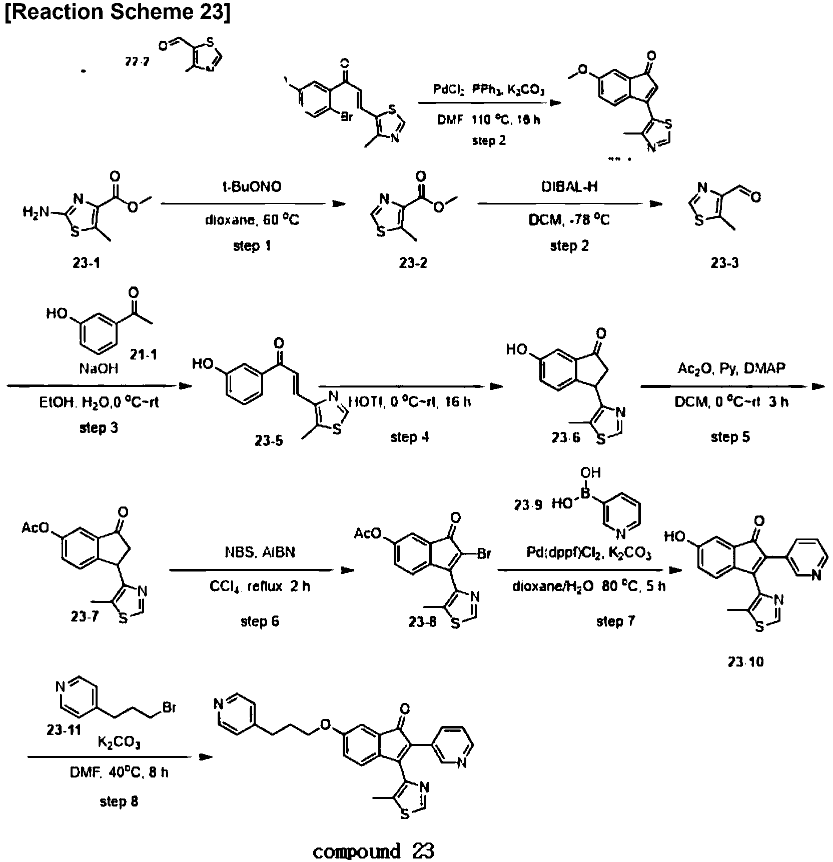

### Step 1: Synthesis of methyl 5-methylthiazole-4-carboxylate

To a solution of tert-butyl nitrite (31 mL, 290.7 mmol) in 1,4-dioxane (240 mL) was added a solution of methyl 2-amino-5-methylthiazole-4-carboxylate (10 g, 58.1 mmol) in 1,4-dioxane (90 mL). The resulting solution was stirred at 60 °C for 1 hour. After the solution was cooled to room temperature and the solvent was evaporated, the residue was purified by flash chromatography (0 - 20 % EtOAc/hexanes) to obtain methyl 5-methylthiazole-4-carboxylate (5.0 g, 31.8 mmol, 55 % yield).

### Step 2: Synthesis of 5-methylthiazole-4-carbaldehyde (23-3)

A solution of methyl 5-methylthiazole-4-carboxylate (5 g, 31.8 mmol) in DCM (100 mL) at -78 °C was added to a solution of diisobutylaluminium hydride (1 M in THF, 34 mL, 34 mmol). The resulting mixture was stirred at -78 °C for 2 hours. After diisobutylaluminium hydride (1 M in toluene, 10 mL, 10 mmol) was added, the mixture was stirred for 2 hours at -78 °C. The reaction was quenched by the addition of Rochelle's salt solution (500 mL), and DCM (200 mL) was additionally added to the mixture. The mixture was stirred very rapidly until the layers were well separated. After the organic layer was separated, it was dried with MgSO₄, filtered, evaporated, and then dried in vacuo to obtain 5-methylthiazole-4-carbaldehyde (2.5 g, 19.7 mmol, 61.95 % yield).

### Step 3: Synthesis of (E)-1-(3-hydroxyphenyl)-3-(5-methylthiazol-4-yl)prop-2-en-1-one

A solution of 5-methylthiazole-4-carbaldehyde (2.5 g, 19.69 mmol) and 1-(3-hydroxyphenyl)ethan-1-one (2.9 g, 21.65 mmol) in EtOH (30 mL) was stirred at 20-25 °C for 10 minutes. To the above solution was added a solution of NaOH (1.2 g, 29.52 mmol in 12.5 mL H₂O) at 0-5 °C. Then, the mixture was stirred at room temperature for 12 hours, and after adjusting the pH to 7 with 1 N HCl, the resulting solid was collected by filtration to obtain (E)-1-(3-hydroxyphenyl)-3-(5-methylthiazol-4-yl)prop-2-en-1-one (2.0 g, 8.16 mmol, 41.43 % yield).

MS (ESI): C₁₃H₁₁NO₂S 245.1 m/z, found 246.1 [M+1]+.

### Step 4: Synthesis of 6-hydroxy-3-(5-methylthiazol-4-yl)-2,3-dihydro-1H-inden-1-one

(E)-1-(3-hydroxyphenyl)-3-(5-methylthiazol-4-yl)prop-2-en-1-one (2 g, 8.2 mmol) was added to CF₃SO₃H (20 mL) at 0 °C. The mixture was then stirred at room temperature for 16 hours, and after adjusting the pH to 8 with an aqueous NaHCO₃ solution, the resulting solid was collected by filtration to obtain 6-hydroxy-3-(5-methylthiazol-4-yl)-2,3-dihydro-1H-inden-1-one (1.3 g, 5.3 mmol, 64.71 % yield).

### Step 5: Synthesis of 1-(5-methylthiazol-4-yl)-3-oxo-2,3-dihydro-1H-inden-5-yl acetate

To a mixture of 6-hydroxy-3-(5-methylthiazol-4-yl)-2,3-dihydro-1H-inden-1-one (1.3 g, 5.2988 mmol), pyridine (1.2 g, 15.8964 mmol), and 4-dimethylaminopyridine (129.5 mg, 1.0582 mmol) was added DCM (40 mL), and then Ac₂O (1.6 g, 15.8964 mmol) was added. The reaction mixture was stirred at 25 °C for 3 hours. The mixture was filtered through Celite, and the filtrate was concentrated under reduced pressure. Water was added to the residue, and the mixture was extracted with DCM (3 x 90 mL). The organic layer was washed with water and brine, dried with sodium sulfate, and concentrated in vacuo. The residue was then purified by silica gel column chromatography (50 % EtOAc/PE) to obtain 1-(5-methylthiazol-4-yl)-3-oxo-2,3-dihydro-1H-inden-5-yl acetate (1.2 g, 4.1811 mmol, 78.91 % yield) as a yellow solid.

MS (ESI): C₁₅H₁₃NO₃S 287.1 m/z, found 288.1 [M+1]+.

### Step 6: Synthesis of 2-bromo-3-(5-methylthiazol-4-yl)-1-oxo-1H-inden-6-yl acetate

To a mixture of 1-(5-methylthiazol-4-yl)-3-oxo-2,3-dihydro-1H-inden-5-yl acetate (1 g, 0.0035 mol), NBS (1.25 g, 0.007 mol), and AIBN (110 mg, 0.7 mmol) was added CCl₄ (20 mL). The reaction mixture was stirred at 80 °C for 2 hours. Water was added to the residue, and the mixture was extracted with DCM (3 x 40 mL). The organic layer was washed with water and brine, dried with sodium sulfate, and concentrated in vacuo. The residue was then purified by silica gel column chromatography (50 % EtOAc/PE) to obtain 2-bromo-3-(5-methylthiazol-4-yl)-1-oxo-1H-inden-6-yl acetate (500 mg, 0.0014 mol, 39.25 % yield) as a yellow solid.

### Step 7: Synthesis of 6-hydroxy-3-(5-methylthiazol-4-yl)-2-(pyridin-3-yl)-1H-inden-1-one

To a mixture of 2-bromo-3-(5-methylthiazol-4-yl)-1-oxo-1H-inden-6-yl acetate (500 mg, 1.3725 mmol), pyridin-3-ylboranediol (253 mg, 2.0575 mmol), and Pd(dppf)Cl₂·DCM (112 mg, 0.135 mmol) were added dioxane/water (20 mL; 4:1) and potassium carbonate (380 mg, 2.745 mmol). The reaction mixture was stirred at 80 °C for 5 hours under N₂. The residue was diluted with water, and then extracted with EtOAc (3 x 60 mL). The organic layer was washed with water and brine, dried with sodium sulfate, and concentrated in vacuo. The residue was then purified by silica gel column chromatography (10 % MeOH/DCM) to obtain 6-hydroxy-3-(5-methylthiazol-4-yl)-2-(pyridin-3-yl)-1H-inden-1-one (250 mg, 0.7813 mmol, 56.92 %) as a red solid.

### Step 8: Synthesis of 3-(5-methylthiazol-4-yl)-2-(pyridin-3-yl)-6-(3-(pyridin-4-yl)propoxy)-1H-inden-1-one (Compound 23)

To a mixture of 6-hydroxy-3-(5-methylthiazol-4-yl)-2-(pyridin-3-yl)-1H-inden-1-one (200 mg, 0.624 mmol), 4-(3-bromopropyl)pyridine (187.3 mg, 0.936 mmol), and potassium carbonate (172.5 mg, 1.248 mmol) was added DMF (8 mL). The reaction mixture was stirred at 40 °C for 8 hours. The mixture was extracted with EtOAc (3 x 40 mL), and the organic layer was washed with water and brine. After the organic layer was dried with sodium sulfate and concentrated in vacuo, the residue was purified by prep-HPLC (Gemini 5 µm C18 column, 150 x 21.2 mm, 30 % - 90 % ACN/H₂O with 0.1% FA) to obtain 3-(5-methylthiazol-4-yl)-2-(pyridin-3-yl)-6-(3-(pyridin-4-yl)propoxy)-1H-inden-1-one (25 mg, 0.0569 mmol, 9.12 % yield) as a red solid.

The obtained Compound 23 (Formula 24 below) was confirmed by MS (ESI) and 1H NMR, which yielded the following data:

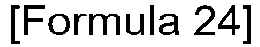

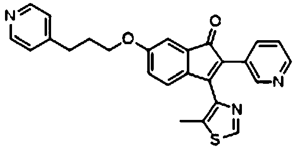

MS (ESI): C₂₆H₂₁N₃O₂S 439.1 m/z, found 440.1 [M+1]+.

¹H NMR (400 MHz, CDCl₃) δ 8.86 (s, 1H), 8.50 (m, 3H), 7.72 (d, *J* = 8.0 Hz, 1H), 7.33 - 7.25 (m, 2H), 7.23 - 7.12 (m, 4H), 6.82 (dd, *J* = 8.0, 2.4 Hz, 1H), 4.02 (t, *J* = 6.0 Hz, 2H), 2.86 (t, *J = 7.6* Hz, 2H), 2.38 - 2.07 (m, 2H), 1.93 (s, 3H).

### Preparation Example 24. 2-bromo-3-(1-methyl-1H-pyrazol-3-yl)-1-oxo-1H-inden-6-yl acetate (Compound 24)

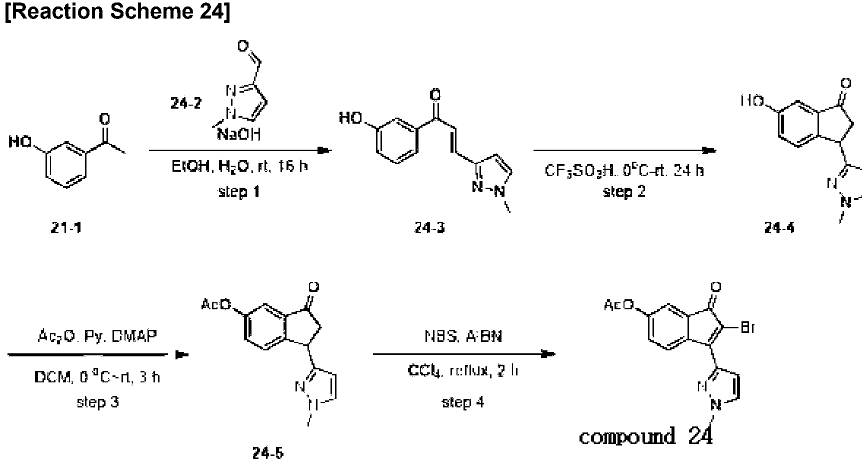

### Step 1: Synthesis of (E)-1-(3-hydroxyphenyl)-3-(1-methyl-1H-pyrazol-3-yl)prop-2-en-1-one

To a mixture of 1-(3-hydroxyphenyl)ethan-1-one (5 g, 36.76 mmol) and 1-methyl-1H-pyrazole-3-carbaldehyde (4.5 g, 40.44 mmol) was added EtOH (100 mL), and the mixture was stirred at 20-25 °C for 30 minutes. Then, a solution of NaOH (2.2 g, 55.14 mmol in 11 mL H₂O) was added at 0-5 °C, and after the mixture was stirred at room temperature for 16 hours and the pH was adjusted to 7 with 1N HCl, the resulting solid was collected by filtration to obtain (E)-1-(3-hydroxyphenyl)-3-(1-methyl-1H-pyrazol-3-yl)prop-2-en-1-one (7.2 g, 31.57 mmol, 85.91 % yield).

### Step 2: Synthesis of 6-hydroxy-3-(1-methyl-1H-pyrazol-3-yl)-2,3-dihydro-1H-inden-1-one

(E)-1-(3-hydroxyphenyl)-3-(1-methyl-1H-pyrazol-3-yl)prop-2-en-1-one (4 g, 17.54 mmol) was added to CF₃SO₃H (40 mL) at 0 °C. The solution was then stirred at room temperature for 12 hours, and after the pH was adjusted to 8 with an aqueous NaHCO₃ solution, the resulting solid was collected by filtration to obtain 6-hydroxy-3-(1-methyl-1H-pyrazol-3-yl)-2,3-dihydro-1H-inden-1-one (3.1 g, 13.60 mmol, 77.51 % yield).

MS (ESI): C₁₃H₁₂N₂O₂ 228.1 m/z, found 229.1 [M+1]+.

### Step 3: Synthesis of 1-(1-methyl-1H-pyrazol-3-yl)-3-oxo-2,3-dihydro-1H-inden-5-yl acetate

To a mixture of 6-hydroxy-3-(1-methyl-1H-pyrazol-3-yl)-2,3-dihydro-1H-inden-1-one (2.5 g, 0.0111 mol) and Ac₂O (3.4 g, 0.0333 mol) was added DCM (30 mL), and then 4-dimethylaminopyridine (0.27 g, 0.0022 mol) was added. The reaction mixture was stirred at room temperature for 4 hours. The residue was diluted with water and extracted with DCM (3 x 60 mL). The organic layer was washed with water and brine, dried with sodium sulfate, and concentrated in vacuo. The residue was then purified by silica gel column chromatography (50 % EtOAc/PE) to obtain 1-(1-methyl-1H-pyrazol-3-yl)-3-oxo-2,3-dihydro-1H-inden-5-yl acetate (2.0 g, 0.0074 mmol, 66.73 % yield).

MS (ESI): C₁₅H₁₄N₂O₃ 270.1 m/z, found 271.1 [M+1]+.

### Step 4: Synthesis of 2-bromo-3-(1-methyl-1H-pyrazol-3-yl)-1-oxo-1H-inden-6-yl acetate (Compound 24)

To a mixture of 1-(1-methyl-1H-pyrazol-3-yl)-3-oxo-2,3-dihydro-1H-inden-5-yl acetate (1 g, 3.7 mmol), AIBN (0.120 g, 0.7 mmol), and NBS (0.79 g, 4.4 mmol) was added CCl₄ (20 mL). The reaction mixture was stirred at 80 °C for 2 hours. The mixture was filtered through Celite, and the filtrate was concentrated in vacuo. Water was added to the residue, and the mixture was extracted with DCM (3 x 60 mL). The organic layer was washed with water and brine, dried with sodium sulfate, and concentrated in vacuo. The residue was then purified by silica gel column chromatography (5 % MeOH/DCM) to obtain 2-bromo-3-(1-methyl-1H-pyrazol-3-yl)-1-oxo-1H-inden-6-yl acetate (400 mg, 1.1527 mmol, 31.55 % yield) as a white solid.

The obtained Compound 24 (Formula 25 below) was confirmed by MS (ESI) and 1H NMR, which yielded the following data:

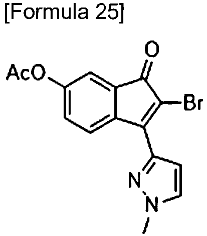

MS (ESI): C₁₅H₁₁BrN₂O₃ 346.0 m/z, found 347.0 [M+1]+.

¹H NMR (400 MHz, DMSO-d₆) δ 8.32 (d, *J* = 8.0 Hz, 1H), 8.04 (d, *J* = 2.4 Hz, 1H), 7.39 (d, *J = 2.4* Hz, 1H), 7.35 (d, *J* = 2.0 Hz, 1H), 7.28 (m, 1H), 4.06 (s, 3H), 2.29 (s, 3H).

### Preparation Example 25. 3-(4-methylthiazol-5-yl)-6-(3-phenylpropoxy)-2-(1H-pyrazol-3-yl)-1H-inden-1-one (Compound 25)

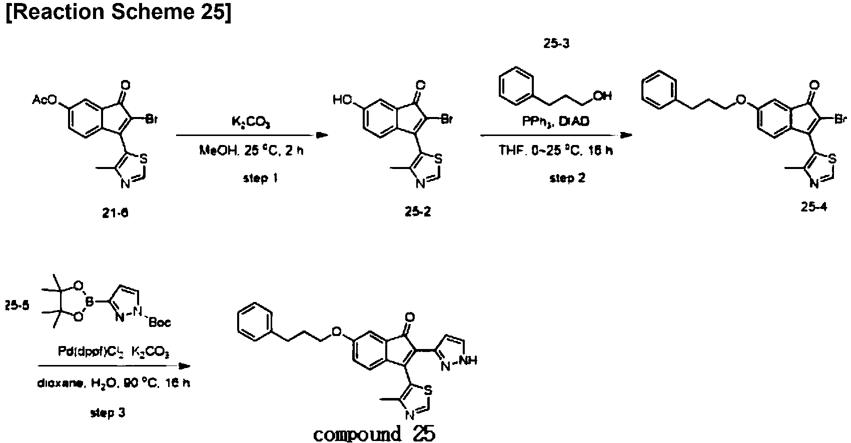

### Step 1: Synthesis of 2-bromo-6-hydroxy-3-(4-methylthiazol-5-yl)-1H-inden-1-one

To a solution of 2-bromo-3-(4-methylthiazol-5-yl)-1-oxo-1H-inden-6-yl acetate (21-6) (15 g, 0.04 mol), obtained in Step 4 of Preparation Example 21, in methanol (260 mL) was added K₂CO₃ (11 g, 0.08 mmol), and the mixture was stirred at room temperature for 2 hours. Water (100 mL) was added to the reaction mixture, and the mixture was extracted with DCM (3 x 200 mL). Using a conventional method, the organic layer was washed, dried, and concentrated under reduced pressure. The resulting residue was purified by silica gel column chromatography (3 % MeOH/DCM) to obtain the desired title compound (12 g, 93 %) as a white solid.

### Step 2: Synthesis of 2-bromo-3-(4-methylthiazol-5-yl)-6-(3-phenylpropoxy)-1H-inden-1-one

To a solution of 2-bromo-6-hydroxy-3-(4-methylthiazol-5-yl)-1H-inden-1-one (12 g, 0.04 mol), 3-phenylpropan-1-ol (11 g, 0.08 mol), and triphenylphosphine (21 g, 0.08 mol) in THF (500 mL) was added diisopropyl azodicarboxylate (16 g, 0.08 mol), and the mixture was stirred at room temperature for 16 hours. Water (200 mL) was added to the reaction mixture, and the mixture was extracted with DCM (3 x 300 mL). Using a conventional method, the organic layer was washed, dried, and concentrated under reduced pressure. The resulting residue was purified by silica gel column chromatography (1 % MeOH/DCM) to obtain the desired title compound (8 g, 45 %) as a red solid.

¹H NMR (400 MHz, DMSO-*d*₆) δ 9.37 (s, 1H), 7.41 - 7.11 (m, 6H), 7.04 (d, *J* = 8.0 Hz, 1H), 6.95 (dd, *J* = 8.0, 2.4 Hz, 1H), 4.09 - 3.94 (m, 2H), 2.79 - 2.67 (m, 2H), 2.44 (s, 3H), 2.14 - 1.83 (m, 3H).

### Step 3: Synthesis of 3-(4-methylthiazol-5-yl)-6-(3-phenylpropoxy)-2-(1H-pyrazol-3-yl)-1H-inden-1-one (Compound 25)

In the same manner as Step 5 of Preparation Example 21, the desired title compound was obtained as a yellow solid (170 mg, 57 % yield).

The obtained Compound 25 (Formula 26 below) was confirmed by LCMS(ESI-MS) and ¹H NMR, which yielded the following data:

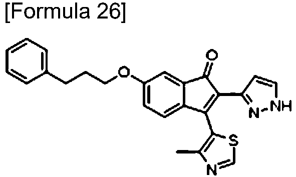

LCMS (ESI-MS): mass calcd. C₂₅H₂₁N₃O₂S 427.1 m/z, found 427.9 [M+H]⁺.

¹H NMR (400 MHz, DMSO-*d*₆) δ 12.99 - 12.83 (m, 1H), 9.28 - 9.23 (m, 1H), 7.74-7.48 (m, 1H), 7.37 - 6.90 (m, 8H), 6.61 - 6.05(m, 1H), 4.04 (t, *J* = 6.0 Hz, 2H), 2.81 - 2.71 (m, 2H), 2.18 - 1.94 (m, 5H).

### Preparation Example 26. 2-(1-methyl-1H-pyrazol-3-yl)-3-(4-methylthiazol-5-yl)-6-(3-phenylpropoxy)-1H-inden-1-one (Compound 26)

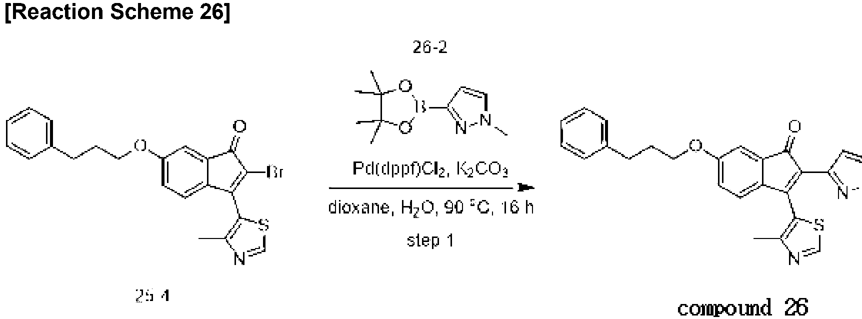

The desired title compound (23.0 mg, 23 % yield) was obtained as a yellow solid from 2-bromo-3-(4-methylthiazol-5-yl)-6-(3-phenylpropoxy)-1H-inden-1-one in the same manner as Step 5 of Preparation Example 21.

The obtained Compound 26 (Formula 27 below) was confirmed by LCMS(ESI-MS) and ¹H NMR, which yielded the following data:

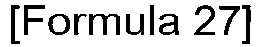

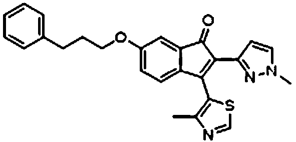

LCMS (ESI-MS): mass calcd. C₂₆H₂₃N₃O₂S 441.2 m/z, found 441.8 [M+H]⁺.

¹H NMR (400 MHz, DMSO-*d*₆) δ 9.25 (s, 1H), 7.67 (d, *J* = 2.0 Hz, 1H), 7.37 - 7.15 (m, 5H), 7.08 (d, *J* = 2.0 Hz, 1H), 7.02 - 6.89 (m, 2H), 6.48 (d, *J* = 2.0 Hz, 1H), 4.03 (t, *J =* 6.4 Hz, 2H), 3.76 (s, 3H), 2.79 - 2.67 (m, 2H), 2.12 (s, 3H), 2.10 - 1.94 (m, 2H).

### Preparation Example 27. 3-(4-methylthiazol-5-yl)-6-(3-phenylpropoxy)-2-(1H-pyrazol-4-yl)-1H-inden-1-one (Compound 27)

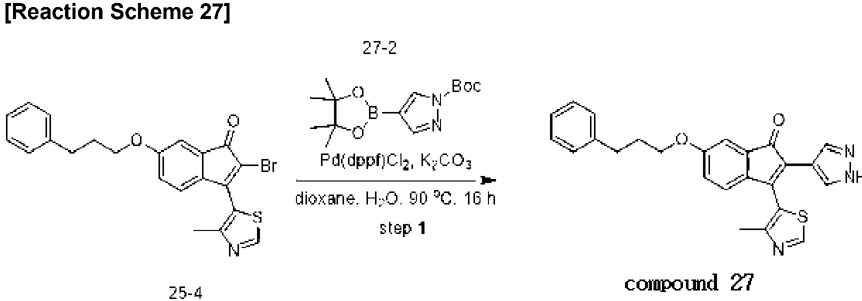

The desired title compound (60 mg, 35 % yield) was obtained as a red solid from 2-bromo-3-(4-methylthiazol-5-yl)-6-(3-phenylpropoxy)-1H-inden-1-one in the same manner as Step 5 of Preparation Example 21.

The obtained Compound 27 (Formula 28 below) was confirmed by LCMS(ESI-MS) and ¹H NMR, which yielded the following data:

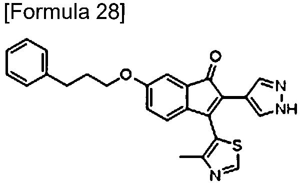

LCMS (ESI-MS): mass calcd. C₂₅H₂₁N₃O₂S 427.1 m/z, found 427.8 [M+H]⁺.

¹H NMR (400 MHz, DMSO-*d*₆) δ 13.08 (s, 1H), 9.34 (s, 1H), 7.87 (s, 1H), 7.34 - 7.14 (m, 6H), 7.07 (d, *J* = 2.4 Hz, 1H), 6.93 - 6.91 (m, 1H), 6.84 (d, *J* = 8.0 Hz, 1H), 4.02 (t, *J =* 6.2 Hz, 2H), 2.81 - 2.71 (m, 2H), 2.17 (s, 3H), 2.06 - 1.96 (m, 2H).

### Preparation Example 28. 2-(1-methyl-1H-pyrazol-4-yl)-3-(4-methylthiazol-5-yl)-6-(3-phenylpropoxy)-1H-inden-1-one (Compound 28)

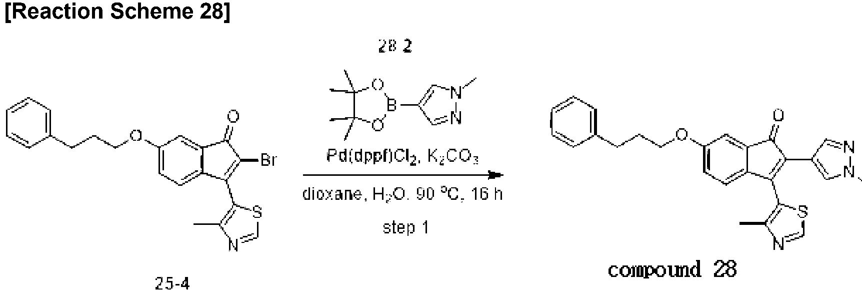

The desired title compound (60 mg, 53 % yield) was obtained as a red solid from 2-bromo-3-(4-methylthiazol-5-yl)-6-(3-phenylpropoxy)-1H-inden-1-one in the same manner as Step 5 of Preparation Example 21.

The obtained Compound 28 (Formula 29 below) was confirmed by LCMS(ESI-MS) and ¹H NMR, which yielded the following data:

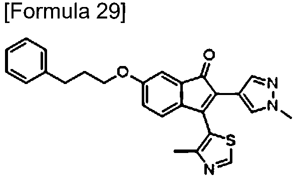

LCMS (ESI-MS): mass calcd. C₂₆H₂₃N₃O₂S 441.2 m/z, found 441.9 [M+H]⁺.

¹H NMR (400 MHz, DMSO-*d*₆) δ 9.34 (s, 1H), 7.91 (s, 1H), 7.33 - 7.16 (m, 6H), 7.07 (d, *J = 2.4* Hz, 1H), 6.91 (dd, *J* = 8.2, 2.4 Hz, 1H), 6.83 (d, *J* = 8.0 Hz, 1H), 4.01 (t, *J = 6.2* Hz, 2H), 3.83 (s, 3H), 2.78 - 2.69 (m, 2H), 2.19 (s, 3H), 2.09 - 1.96 (m, 2H).

### Preparation Example 29. 2-(1-(1-methylpiperidin-4-yl)-1H-pyrazol-3-yl)-3-(4-methylthiazol-5-yl)-6-(3-phenylpropoxy)-1H-inden-1-one (Compound 29)

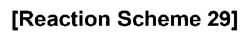

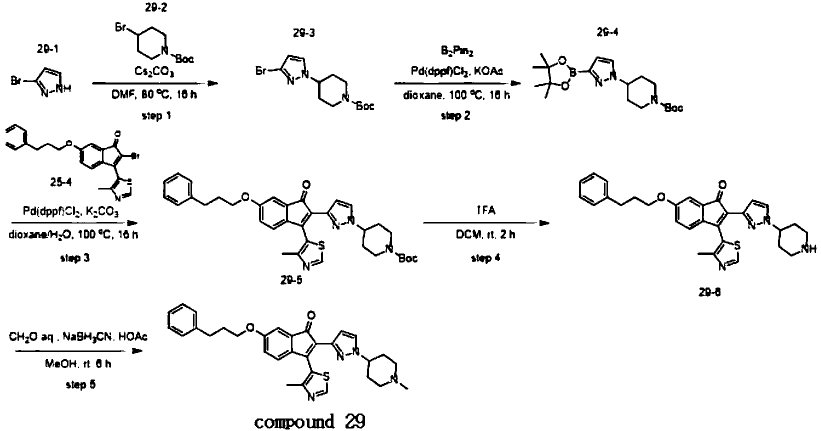

### Step 1: Synthesis of tert-butyl 4-(3-bromo-1H-pyrazol-1-yl)piperidine-1-carboxylate

A mixture of 3-bromo-1H-pyrazole (2 g, 13.61 mmol), tert-butyl 4-bromopiperidine-1-carboxylate (3.59 g, 13.61 mmol), and Cs₂CO₃ (8.85 g, 27.22 mmol) in DMF (100 mL) was stirred at 80 °C for 16 hours. The reaction mixture was cooled to room temperature, and water (50 mL) was added. The mixture was extracted with EA (3 x 100 mL). Using a conventional method, the organic layer was washed, dried, and concentrated under reduced pressure. The resulting residue was purified by silica gel column chromatography (20 % EtOAc in PE) to obtain the desired title compound (1 g, 22 %) as a white solid.

¹H NMR (400 MHz, DMSO-*d*₆) δ 7.83 (d, *J* = 2.4 Hz, 1H), 6.37 (d, *J* = 2.4 Hz, 1H), 4.37 - 4.31 (m, 1H), 4.04 - 4.01 (m, 2H), 2.87 (s, 2H), 1.97 (dd, *J* = 12.8, 2.4 Hz, 2H), 1.78-1.68 (m, 2H), 1.41 (s, 9H).

### Step 2: Synthesis of tert-butyl 4-(3-(4,4,5,5-tetramethyl-1,3,2-dioxaborolan-2-yl)-1H-pyrazol-1-yl)piperidine-1-carboxylate

A mixture of tert-butyl 4-(3-bromo-1H-pyrazol-1-yl)piperidine-1-carboxylate (500 mg, 1.52 mmol), bis(pinacolato)diboron (767 mg, 3.04 mmol), [1,1'-bis(diphenylphosphino)ferrocene]palladium(II) dichloride (110 mg, 0.15 mmol), and KOAc (298 mg, 3.04 mmol) in dioxane (15 mL) was stirred at 100 °C for 16 hours.

The reaction mixture was cooled to room temperature, and water (10 mL) was added. The mixture was then extracted with EA (3 x 20 mL). Using a conventional method, the organic layer was washed, dried, and concentrated under reduced pressure. The resulting residue was purified by silica gel column chromatography (30 % EtOAc/PE) to obtain the desired title compound (400 mg, 69.8 %).

### Step 3: Synthesis of tert-butyl 4-(3-(3-(4-methylthiazol-5-yl)-1-oxo-6-(3-phenylpropoxy)-1H-inden-2-yl)-1H-pyrazol-1-yl)piperidine-1-carboxylate

In the same manner as Step 5 of Preparation Example 21, using tert-butyl 4-(3-(4,4,5,5-tetramethyl-1,3,2-dioxaborolan-2-yl)-1H-pyrazol-1-yl)piperidine-1-carboxylate and 2-bromo-3-(4-methylthiazol-5-yl)-6-(3-phenylpropoxy)-1H-inden-1-one, the desired title compound (190 mg, 33 %) was obtained as a blue solid.

¹H NMR (400 MHz, DMSO-*d*₆) δ 9.23 (s, 1H), 7.80 (d, *J* = 2.4 Hz, 1H), 7.39 - 7.15 (m, 5H), 7.08 (d, *J* = 2.0 Hz, 1H), 7.02 - 6.89 (m, 2H), 6.58 (d, *J* = 2.4 Hz, 1H), 4.27 (s, 1H), 4.03 (t, *J = 6.4* Hz, 3H), 3.91 (d, *J* = 15.6 Hz, 3H), 2.87 (s, 2H), 2.82 - 2.66 (m, 2H), 2.09 (s, 3H), 2.04 - 1.98 (m, 2H), 1.86 (d, *J* = 10.4 Hz, 2H), 1.63 - 1.59 (m, 2H), 1.41 (s, 9H), 1.07 (s, 5H).

### Step 4: Synthesis of 3-(4-methylthiazol-5-yl)-6-(3-phenylpropoxy)-2-(1-(piperidin-4-yl)-1H-pyrazol-3-yl)-1H-inden-1-one

To tert-butyl 4-(3-(3-(4-methylthiazol-5-yl)-1-oxo-6-(3-phenylpropoxy)-1H-inden-2-yl)-1H-pyrazol-1-yl)piperidine-1-carboxylate (180 mg, 0.29 mmol) was added DCM / TFA (3 mL / 1 mL), and the mixture was stirred at room temperature for 2 hours. The reaction mixture was concentrated under reduced pressure to obtain the desired title compound (150 mg, crude) as a yellow solid.

### Step 5: Synthesis of 2-(1-(1-methylpiperidin-4-yl)-1H-pyrazol-3-yl)-3-(4-methylthiazol-5-yl)-6-(3-phenylpropoxy)-1H-inden-1-one (Compound 29)

A mixture of 3-(4-methylthiazol-5-yl)-6-(3-phenylpropoxy)-2-(1-(piperidin-4-yl)-1H-pyrazol-3-yl)-1H-inden-1-one (150 mg, 0.29 mmol), aqueous CH₂O (0.3 mL, 2.90 mmol), NaBH₃CN (37 mg, 0.58 mmol), and AcOH (34.8 mg, 0.58 mmol) in MeOH (3 mL) was stirred at room temperature for 6 hours. Water (5 mL) was added to the reaction mixture, and the mixture was extracted with EA (3 x 10 mL). Using a conventional method, the organic layer was washed, dried, and concentrated under reduced pressure. The resulting residue was purified by prep-HPLC (Gemini 5 µm C₁₈ column, 150 x 21.2 mm, 30 - 90 % ACN/H₂O with 0.1% FA) to obtain the desired title compound (5.5 mg, 4 %) as a red solid.

The obtained Compound 29 (Formula 30 below) was confirmed by LCMS(ESI-MS) and ¹H NMR, which yielded the following data:

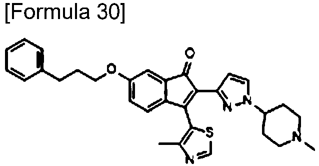

LCMS (ESI-MS): mass calcd. C₃₁H₃₂N₄O₂S 524.2 m/z, found 525.0 [M+H]⁺.

¹H NMR (400 MHz, DMSO-*d*₆) δ 9.26 (s, 1H), 7.77 (d, *J* = 2.4 Hz, 1H), 7.37 - 7.16 (m, 5H), 7.08 (d, *J* = 2.0 Hz, 1H), 6.96 (dt, *J* = 8.0, 5.2 Hz, 2H), 6.59 (d, *J* = 2.4 Hz, 1H), 4.05 - 3.99 (m, 3H), 2.75 (t, *J =* 7.6 Hz, 4H), 2.16 (s, 3H), 2.10 (s, 3H), 2.05 - 1.96 (m, 4H), 1.89 - 1.71 (m, 4H).

### Preparation Example 30. 2-(1-(1-methylpiperidin-4-yl)-1H-pyrazol-4-yl)-3-(4-methylthiazol-5-yl)-6-(3-phenylpropoxy)-1H-inden-1-one (Compound 30)

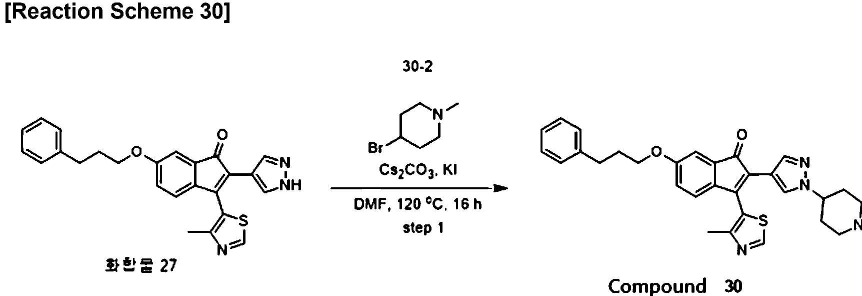

A mixture of 3-(4-methylthiazol-5-yl)-6-(3-phenylpropoxy)-2-(1H-pyrazol-4-yl)-1H-inden-1-one (170 mg, 0.3976 mmol), 4-bromo-1-methylpiperidine (106.2 mg, 0.5964 mmol), Cs₂CO₃ (388.64 mg, 1.1928 mmol), and KI (33 mg, 0.1988 mmol) in DMF (10 mL) was stirred at 120 °C for 16 hours under a nitrogen atmosphere. Water (30 mL) was added to the reaction mixture, and the mixture was extracted with EA (3 x 100 mL). Using a conventional method, the organic layer was washed, dried, and concentrated under reduced pressure. The resulting residue was purified by prep-HPLC (Gemini 5 µm C₁₈ column, 150 x 21.2 mm, 25 - 95 % ACN/H₂O with 0.1 % NH₃H₂O) to obtain the desired title compound (30 mg, 14 %) as a red solid.

The obtained Compound 30 (Formula 31 below) was confirmed by LCMS(ESI-MS) and ¹H NMR, which yielded the following data:

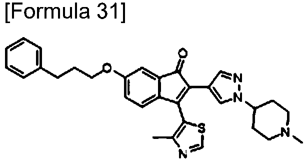

LCMS (ESI-MS): mass calcd. C₃₁H₃₂N₄O₂S 524.2 m/z, found 525.0 [M+H]⁺.

¹H NMR (400 MHz, DMSO-*d*₆) δ 9.34 (s, 1H), 7.89 (s, 1H), 7.31 - 7.17 (m, 6H), 7.07 (s, 1H), 6.92 (dd, *J* = 8.0, 2.2 Hz, 1H), 6.84 (d, *J* = 8.0 Hz, 1H), 4.22 - 4.11 (m, 1H), 4.01 (t, *J* = 6.2 Hz, 2H), 2.82 (d, *J =* 11.2 Hz, 2H), 2.74 (t, *J* = 7.6 Hz, 2H), 2.18 (s, 6H), 2.07 - 1.97 (m, 4H), 1.91 - 1.86 (m, 4H).

### Preparation Example 31. 3-(4-methylthiazol-5-yl)-6-(3-phenylpropoxy)-2-(1H-1,2,3-triazol-4-yl)-1H-inden-1-one (Compound 31)

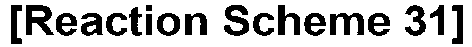

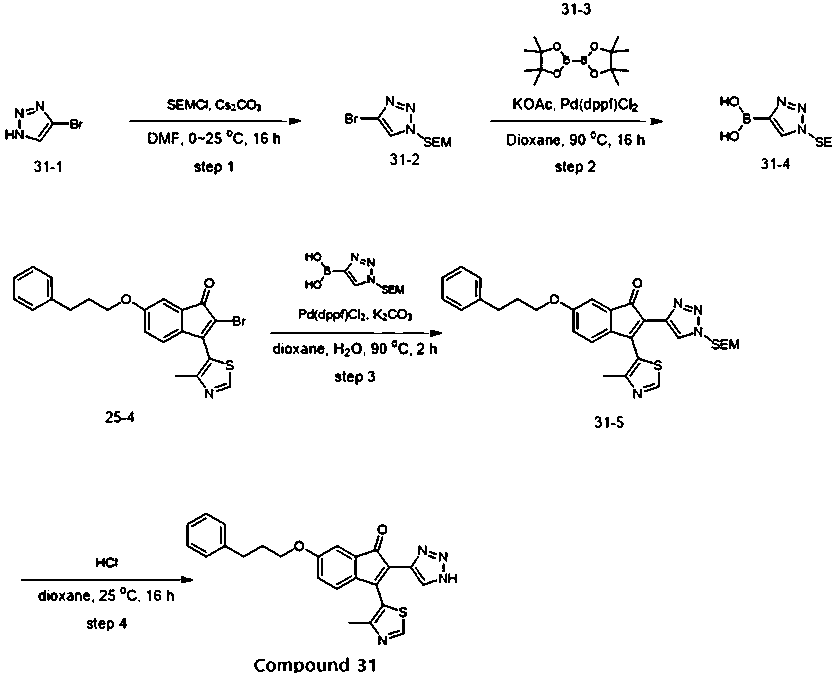

### Step 1: Synthesis of 4-bromo-1-((2-(trimethylsilyl)ethoxy)methyl)-1H-1,2,3-triazole

To a mixture of 4-bromo-1H-1,2,3-triazole (3 g, 0.0203 mol) and Cs₂CO₃ (7.94 g, 0.0243 mol) were added anhydrous DMF (100 mL) and 2-(trimethylsilyl)ethoxymethyl chloride (5.08 g, 0.0304 mol) at 0 °C. The reaction mixture was stirred at room temperature for 16 hours. Water (300 mL) was added to the reaction mixture, and the mixture was extracted with EA (3 x 400 mL). Using a conventional method, the organic layer was washed, dried, and concentrated under reduced pressure. The resulting residue was purified by flash chromatography (10 % EtOAc/Hexanes) to obtain the desired title compound (1.5 g, 23 %) as a white oil.

LCMS (ESI-MS): mass calcd. C₈H₁₆BrN₃OSi 277.0 m/z, found 278.0 [M+H]⁺.

### Step 2: Synthesis of 1-((2-(trimethylsilyl)ethoxy)methyl)-1H-1,2,3-triazol-4-yl)boronic acid

A mixture of 4-bromo-1-((2-(trimethylsilyl)ethoxy)methyl)-1H-1,2,3-triazole (1.5 g, 0.0054 mol), bis(pinacolato)diboron (1.65 g, 0.0065 mol), Pd(dppf)Cl₂ (0.4 g, 0.0005 mol), and KOAc (1.59 g, 0.0162 mol) in dioxane (20 mL) was stirred at 90 °C for 16 hours under a nitrogen atmosphere. Water (100 mL) was added to the reaction mixture, and the mixture was extracted with EA (3 x 100 mL). Using a conventional method, the organic layer was washed, dried, and concentrated under reduced pressure. The resulting residue was purified by flash chromatography (20 % EtOAc/Hexanes) to obtain the desired title compound (0.4 g, 20 %) as a white oil.

LCMS (ESI-MS): mass calcd. C₃H₁₃BN₃O₃Si 243.1 m/z, found 243.6 [M+H]⁺.

### Step 3: Synthesis of 3-(4-methylthiazol-5-yl)-6-(3-phenylpropoxy)-2-(1-((2-(trimethylsilyl)ethoxy)methyl)-1H-1,2,3-triazol-4-yl)-1H-inden-1-one

In the same manner as Step 5 of Preparation Example 21, using 2-bromo-3-(4-methylthiazol-5-yl)-6-(3-phenylpropoxy)-1H-inden-1-one and (1-((2-(trimethylsilyl)ethoxy)methyl)-1H-1 ,2,3-triazol-4-yl)boronic acid, the desired title compound (220 mg, 71 %) was obtained as a red solid.

LCMS (ESI-MS): mass calcd. C₃₀H₃₄N₄O₃SSi 558.2 m/z, found 559.0 [M+H]⁺.

### Step 4: Synthesis of 3-(4-methylthiazol-5-yl)-6-(3-phenylpropoxy)-2-(1H-1,2,3-triazol-4-yl)-1H-inden-1-one (Compound 31)

To 3-(4-methylthiazol-5-yl)-6-(3-phenylpropoxy)-2-(1-((2-(trimethylsilyl)ethoxy)methyl)-1H-1,2,3-triazol-4-yl)-1H-inden-1-one (260 mg, 0.4645 mmol) was added HCl/dioxane (15 mL), and the mixture was stirred at room temperature for 16 hours under a nitrogen atmosphere. Water (50 mL) was added to the reaction mixture, and the mixture was extracted with EA (3 x 50 mL). Using a conventional method, the organic layer was washed, dried, and concentrated under reduced pressure. The resulting residue was purified by silica gel column chromatography (5 % MeOH/DCM) to obtain the desired title compound (90 mg, 40 %) as a red solid.

The obtained Compound 31 (Formula 32 below) was confirmed by LCMS(ESI-MS) and ¹H NMR, which yielded the following data:

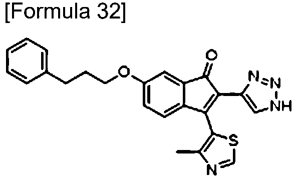

LCMS (ESI-MS): mass calcd. C₂₄H₂₀N₄O₂S 428.1 m/z, found 428.8 [M+H]⁺.

¹H NMR (400 MHz, DMSO-*d*₆) δ 9.30 (s, 1H), 8.43 - 7.95 (m, 1H), 7.34 - 7.12 (m, 6H), 7.06 - 6.98 (m, 2H), 4.06 - 4.03 (m, 2H), 2.77 - 2.75 (m, 2H), 2.11 (s, 3H), 2.11 - 1.98 (m, 2H).

### Preparation Example 32. 2-(1-methyl-1H-1,2,3-triazol-4-yl)-3-(4-methylthiazol-5-yl)-6-(3-phenylpropoxy)-1H-inden-1-one (Compound 32); and

### 2-(2-methyl-2H-1,2,3-triazol-4-yl)-3-(4-methylthiazol-5-yl)-6-(3-phenylpropoxy)-1H-inden-1-one (Compound 33)

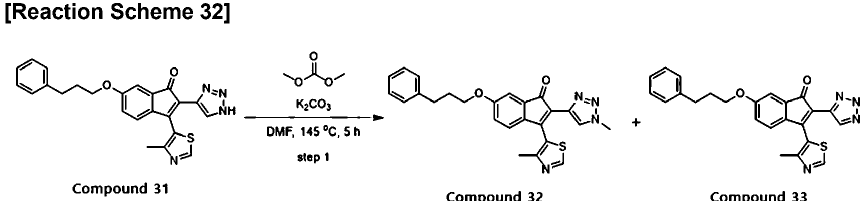

A mixture of 3-(4-methylthiazol-5-yl)-6-(3-phenylpropoxy)-2-(1H-1,2,3-triazol-4-yl)-1H-inden-1-one (200 mg, 0.4667 mmol), dimethyl carbonate (92.49 mg, 1.0267 mmol), and K₂CO₃ (161.26 mg, 1.1667 mmol) in DMF (10 mL) was stirred at 145 °C for 4 hours under a nitrogen atmosphere. Water (70 mL) was added to the reaction mixture, and the mixture was extracted with EA (3 x 100 mL). Using a conventional method, the organic layer was washed, dried, and concentrated under reduced pressure. The resulting residue was purified by prep-HPLC (Gemini 5 µm C18 column, 150 x 21.2 mm, 60 - 95 % ACN/H₂O with 0.1% FA) to obtain the desired title Compound 32 (3.7 mg, 2 %) and Compound 32-1 (6.4 mg, 3 %) as a red solid.

The obtained Compound 32 (Formula 33 below) was confirmed by LCMS(ESI-MS) and ¹H NMR, which yielded the following data:

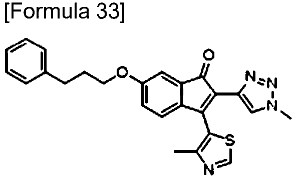

LCMS (ESI-MS): mass calcd. C₂₅H₂₂N₄O₂S 442.1 m/z, found 443.1 [M+H]⁺.

¹H NMR (400 MHz, DMSO-*d*₆) δ 9.28 (s, 1H), 8.42 (s, 1H), 7.31 - 7.18 (m, 5H), 7.13 (d, *J* = 2.2 Hz, 1H), 7.02 - 6.97 (m, 2H), 4.12 - 4.01 (m, 5H), 2.82 - 2.72 (m, 2H), 2.14 (s, 3H), 2.08 - 1.98 (m, 2H).

The obtained Compound 33 (Formula 34 below) was confirmed by LCMS(ESI-MS) and ¹H NMR, which yielded the following data:

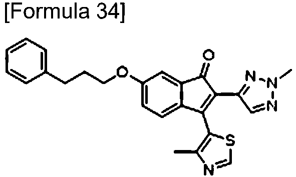

LCMS (ESI-MS): mass calcd. C₂₅H₂₂N₄O₂S 442.1 m/z, found 443.1 [M+H]⁺.

¹H NMR (400 MHz, DMSO-*d*₆) δ 9.30 (s, 1H), 7.95 (s, 1H), 7.32 - 7.16 (m, 5H), 7.13 (d, *J* = 2.4 Hz, 1H), 7.05 (d, *J* = 8.2 Hz, 1H), 6.98 (dd, *J* = 8.2, 2.4 Hz, 1H), 4.08 (s, 3H), 4.07 - 4.01 (m, 2H), 2.81 - 2.71 (m, 2H), 2.14 (s, 3H), 2.10 - 1.99 (m, 2H).

### Preparation Example 33. 3-(4-methylthiazol-5-yl)-1-oxo-6-(3-phenylpropoxy)-1H-indene-2-carbonitrile (Compound 34)

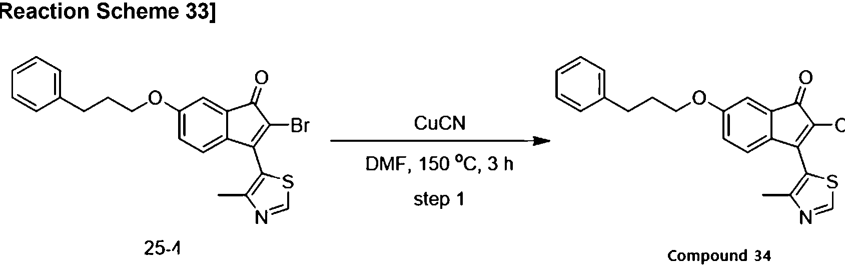

A mixture of 2-bromo-3-(4-methylthiazol-5-yl)-6-(3-phenylpropoxy)-1H-inden-1-one (500 mg, 1.14 mmol) and CuCN (303 mg, 3.41 mmol) in DMF (10 mL) was stirred at 150 °C for 3 hours. The resulting residue was purified by prep-HPLC (Gemini 5 µm C₁₈ column, 150 x 21.2 mm, 30 - 90 % ACN/H₂O with 0.1 % FA) to obtain the desired title compound (200 mg, 45 %) as a yellow solid.

The obtained Compound 34 (Formula 35 below) was confirmed by LCMS(ESI-MS) and ¹H NMR, which yielded the following data:

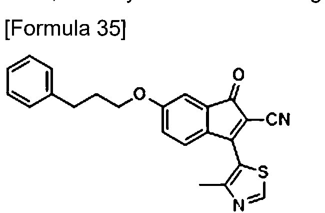

LCMS (ESI-MS): mass calcd. C₂₃H₁₈N₂O₂S 386.1 m/z, found 386.7 [M+H]⁺.

¹H NMR (400 MHz, DMSO-*d*₆) δ 9.52 (s, 1H), 7.45 (d, *J* = 8.0 Hz, 1H), 7.35 - 7.16 (m, 6H), 7.13 (dd, *J* = 8.0, 2.4 Hz, 1H), 4.10 (t, *J* = 6.4 Hz, 2H), 2.75 (t, *J* = 7.2 Hz, 2H), 2.58 (s, 3H), 2.14 - 1.96 (m, 2H).

### Preparation Example 34. 6-hydroxy-3-(4-methylthiazol-5-yl)-2-(pyridin-3-yl)-1H-inden-1-one (Compound 35)

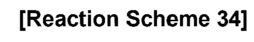

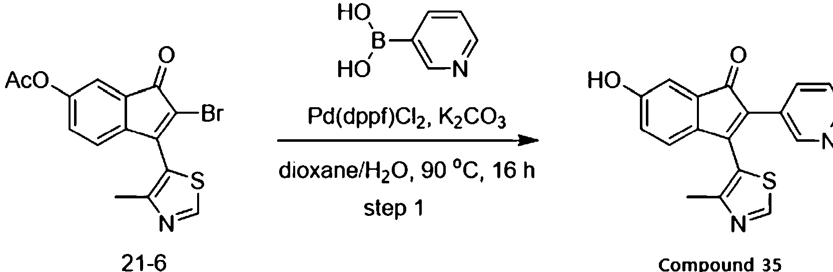

In the same manner as Step 5 of Preparation Example 21, using 2-bromo-3-(4-methylthiazol-5-yl)-1-oxo-1H-inden-6-yl acetate and pyridin-3-ylboronic acid, the desired title compound (17.3 mg, 16 % yield) was obtained as a red solid.

The obtained Compound 35 (Formula 36 below) was confirmed by MS(ESI) and¹H NMR, which yielded the following data:

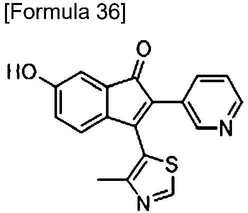

MS (ESI): mass calcd. C₁₈H₁₂N₂O₂S 320.1 m/z, found 321.0 [M+H]⁺. ¹H NMR (400 MHz, DMSO-*d*₆) δ 10.33 (s, 1H), 9.30 (s, 1H), 8.48 - 8.47 (m, 1H), 8.34 - 8.33 (m, 1H), 7.60 - 7.58 (m, 1H), 7.41 - 7.37 (m, 1H), 7.06 - 6.99 (m, 2H), 6.85 - 6.83 (m, 1H), 1.95 (s, 3H).

### Preparation Example 35. 6-(2-hydroxyethoxy)-3-(4-methylthiazol-5-yl)-2-(pyridin-3-yl)-1H-inden-1-one (Compound 36)

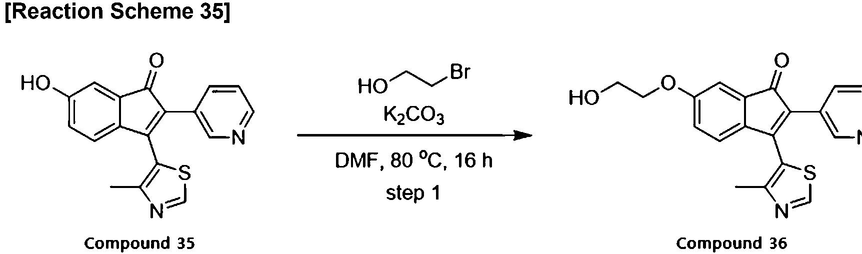

To a mixture of 6-hydroxy-3-(4-methylthiazol-5-yl)-2-(pyridin-3-yl)-1H-inden-1-one (100 mg, 0.31 mmol) and 2-bromoethan-1-ol (78 mg, 0.62 mmol) were added DMF (3 mL) and K₂CO₃ (86 mg, 0.62 mmol), and the resulting mixture was stirred at 80 °C for 16 hours. Water (5 mL) was added to the reaction mixture, and the mixture was extracted with EA (3 x 10 mL). Using a conventional method, the organic layer was washed, dried, and concentrated under reduced pressure. The resulting residue was purified by prep-HPLC (Gemini 5 µm C₁₈ column, 150 x 21.2 mm, 30 - 90 % ACN/H₂O with 0.1 % FA) to obtain the desired title compound (5.6 mg, 5 %) as a yellow solid.

The obtained Compound 36 (Formula 37 below) was confirmed by LCMS(ESI-MS) and ¹H NMR, which yielded the following data:

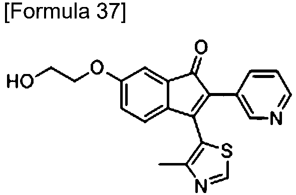

LCMS (ESI-MS): mass calcd. C₂₀H₁₆N₂O₃S 364.1 m/z, found 364.7 [M+H]⁺.

¹H NMR (400 MHz, DMSO-*d*₆) δ 9.31 (s, 1H), 8.49 (dd, *J* = 4.8, 1.2 Hz, 1H), 8.36 (d, *J* = 2.0 Hz, 1H), 7.61 (d, *J* = 7.6 Hz, 1H), 7.40 (dd, *J* = 8.0, 4.8 Hz, 1H), 7.18 (d, *J* = 2.4 Hz, 1H), 7.14 (d, *J* = 8.0 Hz, 1H), 7.04 (dd, *J* = 8.0, 2.4 Hz, 1H), 4.92 (t, *J* = 5.2 Hz, 1H), 4.09 (t, *J* = 4.8 Hz, 2H), 3.75 - 3.71 (m, 2H), 1.96 (s, 3H).

### Preparation Example 36. 6-(2-methoxyethoxy)-3-(4-methylthiazol-5-yl)-2-(pyridin-3-yl)-1H-inden-1-one (Compound 37)

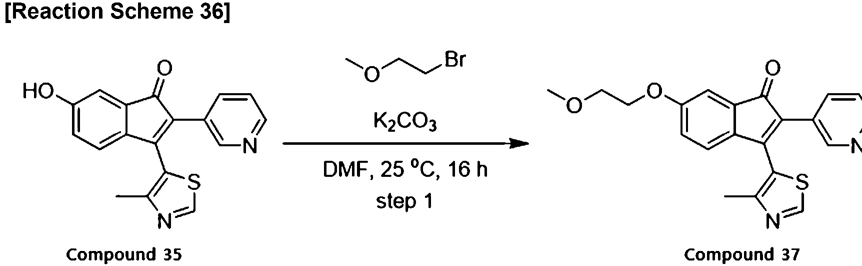

In the same manner as Preparation Example 35, using 1-bromo-2-methoxyethane, the desired title compound (20.1 mg, 28 %) was obtained as a yellow solid. The reaction was performed at room temperature.

The obtained Compound 37 (Formula 38 below) was confirmed by LCMS(ESI-MS) and ¹H NMR, which yielded the following data:

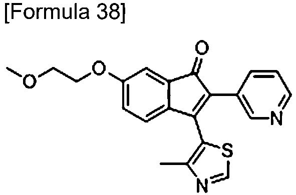

LCMS (ESI-MS): mass calcd. C₂₁H₁₈N₂O₃S 378.1 m/z, found 378.7 [M+H]⁺.

¹H NMR (400 MHz, DMSO-*d*₆) δ 9.31 (s, 1H), 8.49 (dd, *J* = 4.8, 1.6 Hz, 1H), 8.36 (d, *J* = 1.2Hz, 1H), 7.61 (dt, *J* = 8.0, 2.0 Hz, 1H), 7.40 (dd, *J* = 7.6, 4.8 Hz, 1H), 7.19 (d, *J* = 2.4 Hz, 1H), 7.14 (d, *J* = 8.0 Hz, 1H), 7.04 (dd, *J* = 8.4, 2.4 Hz, 1H), 4.21 - 4.19 (m, 2H), 3.80-3.57 (m, 2H), 3.31 (s, 3H), 1.96 (s, 3H).

### Preparation Example 37. 6-(methyl(3-phenylpropyl)amino)-3-(4-methylthiazol-5-yl)-2-(pyridin-3-yl)-1H-inden-1-one (Compound 38)

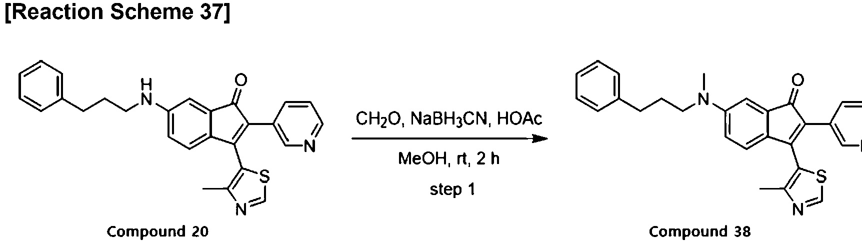

In the same manner as Step 5 of Preparation Example 29, using 3-(4-methylthiazol-5-yl)-6-((3-phenylpropyl)amino)-2-(pyridin-3-yl)-1 H-inden-1-one, the desired title compound (50 mg, 75 %) was obtained as a purple solid.

The obtained Compound 38 (Formula 39 below) was confirmed by LCMS(ESI-MS) and ¹H NMR, which yielded the following data:

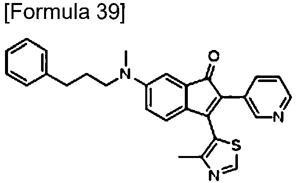

LCMS (ESI-MS): mass calcd. C₂₁H₁₇N₃OS 451.2 m/z, found 451.8 [M+H]⁺.

¹H NMR (400 MHz, DMSO-*d*₆) δ 9.29 (s, 1H), 8.44 (dd, *J* = 4.8, 1.6 Hz, 1H), 8.32 (d, *J = 1.6* Hz, 1H), 7.58 - 7.55 (m, 1H), 7.39 - 7.34 (m, 1H), 7.30 - 7.27 (m, 2H), 7.24 - 7.16 (m, 3H), 7.00 - 6.96 (m, 2H), 6.57 (dd, *J* = 8.4, 2.4 Hz, 1H), 3.52 - 3.41 (m, 2H), 3.00 (s, 3H), 2.65 - 2.58 (m, 2H), 1.95 (s, 3H), 1.89 - 1.77 (m, 2H).

### Preparation Example 38. 3-(4-methylthiazol-5-yl)-6-(3-phenylpropoxy)-2-(pyridin-4-yl)-1H-inden-1-one (Compound 39)

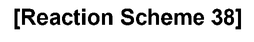

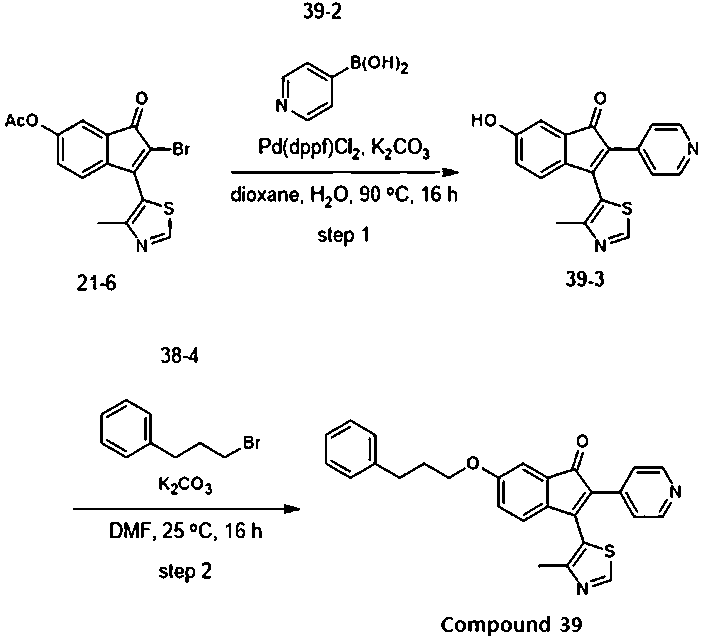

### Step 1: Synthesis of 6-hydroxy-3-(4-methylthiazol-5-yl)-2-(pyridin-4-yl)-1H-inden-1-one

In the same manner as Step 5 of Preparation Example 21, using 2-bromo-3-(4-methylthiazol-5-yl)-1-oxo-1H-inden-6-yl acetate (21-6) and pyridin-4-ylboronic acid, the desired title compound (87.2 mg, 39 % yield) was obtained as a red solid.

LCMS (ESI-MS): mass calcd. C₁₈H₁₂N₂O₂S 320.4 m/z, found 321.0 [M+H]⁺.

¹H NMR (400 MHz, DMSO-*d*₆) δ 10.38 (s, 1H), 9.32 (s, 1H), 8.53 - 8.52 (m, 2H), 7.15 - 7.14 (m, 2H), 7.07 - 7.05 (m, 1H), 7.00 - 6.99 (m, 1H), 6.86 - 6.83 (m, 1H), 2.00 (s, 3H).

### Step 2: Synthesis of 3-(4-methylthiazol-5-yl)-6-(3-phenylpropoxy)-2-(pyridin-4-yl)-1H-inden-1-one (Compound 39)

In the same manner as Preparation Example 36, using 6-hydroxy-3-(4-methylthiazol-5-yl)-2-(pyridin-4-yl)-1H-inden-1-one and (3-bromopropyl)benzene, the desired title compound (18.6 mg, 19 %) was obtained as a red solid.

The obtained Compound 39 (Formula 40 below) was confirmed by LCMS(ESI-MS) and ¹H NMR, which yielded the following data:

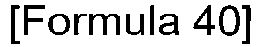

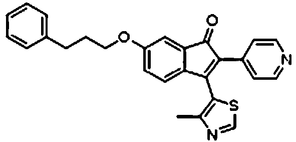

LCMS (ESI-MS): mass calcd. C₂₇H₂₂N₂O₂S 438.1 m/z, found 439.1 [M+H]⁺.

¹H NMR (400 MHz, DMSO-*d*₆) δ 9.33 (s, 1H), 8.55 - 8.53 (m, 2H), 7.31 - 7.23 (m, 4H), 7.21 - 7.14 (m, 5H), 7.04 - 7.02 (m, 1H), 4.08 - 4.05 (m, 2H), 2.77 - 2.73 (m, 2H), 2.07 - 2.00 (m, 2H), 1.97 (s, 3H).

### Preparation Example 39. 3-(4-methylthiazol-5-yl)-6-(3-phenylpropoxy)-2-(pyridin-2-yl)-1H-inden-1-one (Compound 40)

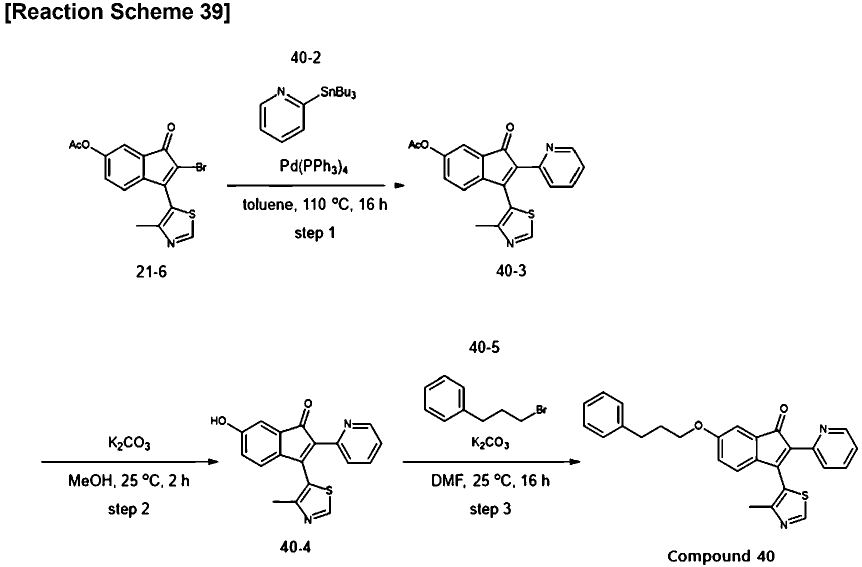

### Step 1: Synthesis of 3-(4-methylthiazol-5-yl)-1-oxo-2-(pyridin-2-yl)-1H-inden-6-yl acetate)

A mixture of 2-bromo-3-(4-methylthiazol-5-yl)-1-oxo-1H-inden-6-yl acetate (21-6) (278 mg, 0.7633 mmol), obtained from Step 4 of Preparation Example 21, 2-(tributylstannyl)pyridine (337.21 mg, 0.916 mmol), and tetrakis(triphenylphosphine)palladium(0) (88.2 mg, 0.07633 mmol) in toluene (10 mL) was stirred at 110 °C for 16 hours under a nitrogen atmosphere. Water (30 mL) was added to the reaction mixture, and the mixture was extracted with EA (3 x 25 mL). Using a conventional method, the organic layer was washed, dried, and concentrated under reduced pressure. The residue was purified by silica gel column chromatography (3 % MeOH/DCM) to obtain the desired title compound (100 mg, 36 %) as a blue solid.

LCMS (ESI-MS): mass calcd. C₂₀H₁₄N₂O₃S 362.1 m/z, found 363.0 [M+H]⁺.

### Step 2: Synthesis of 6-hydroxy-3-(4-methylthiazol-5-yl)-2-(pyridin-2-yl)-1H-inden-1-one)

The desired title compound (76 mg, 86 % yield) was obtained as a red solid from 3-(4-methylthiazol-5-yl)-1-oxo-2-(pyridin-2-yl)-1H-inden-6-yl acetate in the same manner as Step 1 of Preparation Example 25.

LCMS (ESI-MS): mass calcd. C₁₈H₁₂N₂O₂S 320.4 m/z, found 321.0 [M+H]⁺.

¹H NMR (400 MHz, DMSO-*d*₆) δ 10.35 (s, 1H), 9.49 (s, 1H), 8.70 (s, 1H), 7.84 - 7.80 (m, 1H), 7.52 - 7.50 (m, 1H), 7.27 - 7.26 (m, 1H), 7.07 - 6.97 (m, 2H), 6.85 - 6.82 (m, 1H), 1.92 (s, 3H).

### Step 3: Synthesis of 3-(4-methylthiazol-5-yl)-6-(3-phenylpropoxy)-2-(pyridin-2-yl)-1H-inden-1-one (Compound 40)

The desired title compound (20.9 mg, 22 %) was obtained as a red solid from 6-hydroxy-3-(4-methylthiazol-5-yl)-2-(pyridin-2-yl)-1 H-inden-1-one in the same manner as Step 2 of Preparation Example 38.

The obtained Compound 40 (Formula 41 below) was confirmed by LCMS(ESI-MS) and ¹H NMR, which yielded the following data:

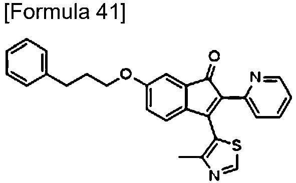

LCMS (ESI-MS): mass calcd. C₂₇H₂₂N₂O₂S 438.1 m/z, found 439.1 [M+H]⁺.

¹H NMR (400 MHz, DMSO-*d*₆) δ 9.24 (s, 1H), 8.48 - 8.46 (m, 1H), 7.86 - 7.82 (m, 1H), 7.54 - 7.52 (m, 1H), 7.31 - 7.19 (m, 6H), 7.16 - 7.14 (m, 2H), 7.03 - 7.00 (m, 1H), 4.08 - 4.05 (m, 2H), 2.77 - 2.74 (m, 2H), 2.06 - 2.00 (m, 2H), 1.93 (s, 3H).

### Preparation Example 40. 3-(4-methylthiazol-5-yl)-6-(3-phenylpropoxy)-2-(pyrazin-2-yl)-1H-inden-1-one (Compound 41)

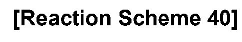

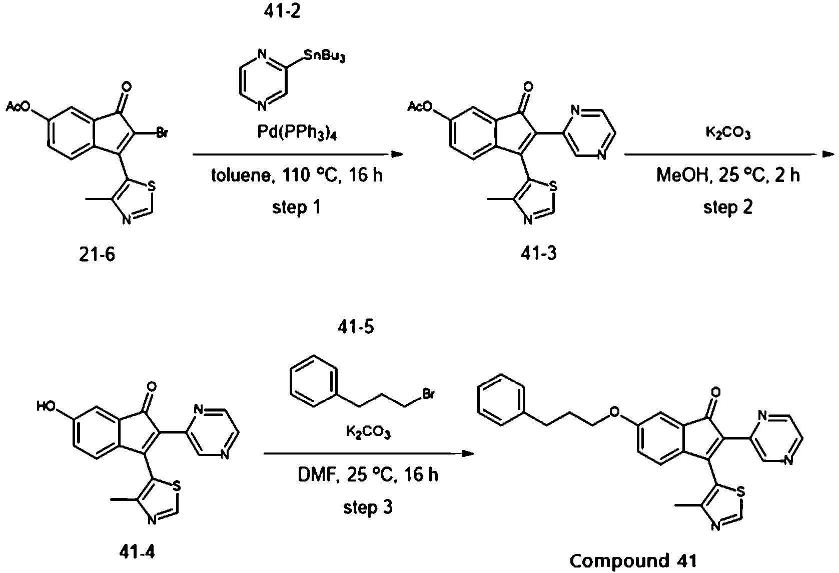

### Step 1: Synthesis of 3-(4-methylthiazol-5-yl)-1-oxo-2-(pyrazin-2-yl)-1H-inden-6-yl acetate

In the same manner as Step 1 of Preparation Example 39, using 2-bromo-3-(4-methylthiazol-5-yl)-1-oxo-1H-inden-6-yl acetate (21-6) and 2-(tributylstannyl)pyrazine, the desired title compound (100 mg, 40 %) was obtained as a red solid.

LCMS (ESI-MS): mass calcd. C₁₉H₁₃N₃O₃S 363.1 m/z, found 363.7 [M+H]⁺.

### Step 2: Synthesis of 6-hydroxy-3-(4-methylthiazol-5-yl)-2-(pyrazin-2-yl)-1H-inden-1-one

The desired title compound (84 mg, 94 %) was obtained as a red solid from 3-(4-methylthiazol-5-yl)-1-oxo-2-(pyrazin-2-yl)-1H-inden-6-yl acetate in the same manner as Step 1 of Preparation Example 25.

LCMS (ESI-MS): mass calcd. C₁₇H₁₁N₃O₂S 321.1 m/z, found 322.0 [M+H]⁺.

¹H NMR (400 MHz, DMSO-*d*₆) δ 10.52 (s, 1H), 9.27 (s, 1H), 8.77 - 8.76 (m, 1H), 8.55 - 8.51 (m, 2H), 7.12 - 7.01 (m, 2H), 6.86 - 6.84 (m, 1H), 1.96 (s, 3H).

### Step 3: Synthesis of 3-(4-methylthiazol-5-yl)-6-(3-phenylpropoxy)-2-(pyrazin-2-yl)-1H-inden-1-one (Compound 41)

The desired title compound (20.8 mg, 19 %) was obtained as a red solid from 6-hydroxy-3-(4-methylthiazol-5-yl)-2-(pyrazin-2-yl)-1H-inden-1-one in the same manner as Step 2 of Preparation Example 38.

The obtained Compound 41 (Formula 42 below) was confirmed by LCMS(ESI-MS) and ¹H NMR, which yielded the following data:

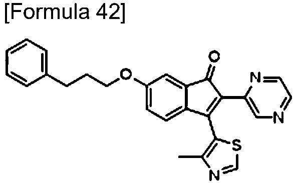

LCMS (ESI-MS): mass calcd. C₂₅H₂₁N₃O₂S 439.1 m/z, found 440.1 [M+H]⁺.

¹H NMR (400 MHz, DMSO-*d*₆) δ 9.28 (s, 1H), 8.78 - 8.77 (m, 1H), 7.57 - 7.53 (m, 2H), 7.31 - 7.23 (m, 4H), 7.20 - 7.18 (m, 3H), 7.06 - 7.03 (m, 1H), 4.09 - 4.06 (m, 2H), 2.78 - 2.74 (m, 2H), 2.08 - 2.01 (m, 2H), 1.97 (s, 3H).

### Preparation Example 41. 3-(1-methyl-1H-pyrazol-4-yl)-6-(3-phenylpropoxy)-2-(pyridin-3-yl)-1H-inden-1-one (Compound 42)

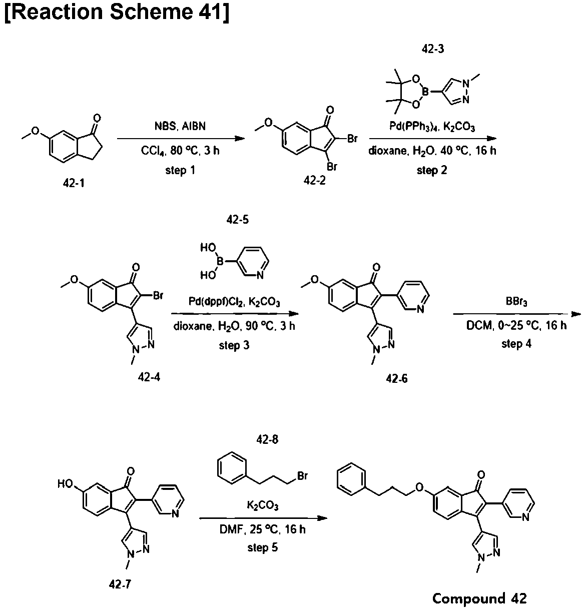

### Step 1: Synthesis of 2,3-dibromo-6-methoxy-1H-inden-1-one

A mixture of 6-methoxy-2,3-dihydroinden-1-one (1 g, 0.0062 mol), NBS (3.31 g, 0.0186 mol), and AIBN (0.1 g, 0.00062 mol) in CCl₄ was stirred at 80 °C for 3 hours. The reaction mixture was concentrated under reduced pressure, and the residue was purified by silica gel column chromatography (2 % EtOAc/Hexane) to obtain the desired title compound (1.8 g, 92 %) as a red solid.

¹H NMR (400 MHz, CDCl₃) δ 7.09 - 7.06 (m, 2H), 6.85 - 6.83 (m, 1H), 3.84 (s, 3H).

### Step 2: Synthesis of 2-bromo-6-methoxy-3-(1-methyl-1H-pyrazol-4-yl)-1H-inden-1-one

A mixture of 2,3-dibromo-6-methoxy-1H-inden-1-one (800 mg, 2.5160 mmol), 1-methyl-4-(4,4,5,5-tetramethyl-1,3,2-dioxaborolan-2-yl)pyrazole (523.49 mg, 2.516 mmol), Pd(PPh₃)₄ (145.37 mg, 0.1258 mmol), and K₂CO₃ (1041.62 mg, 7.548 mmol) in dioxane (24 mL) / H₂O (8 mL) was stirred at 40 °C for 16 hours under a nitrogen atmosphere. Water (50 mL) was added to the reaction mixture, and the mixture was extracted with EA (3 x 30 mL). Using a conventional method, the organic layer was washed, dried, and concentrated under reduced pressure. The residue was purified by silica gel column chromatography (MeOH/DCM) to obtain the desired title compound (500 mg, 62 %) as a red solid.

LCMS (ESI-MS): mass calcd. C₁₄H₁₁BrN₂O₂ 318.0 m/z, found 318.7 [M+H]⁺.

### Step 3: Synthesis of 6-methoxy-3-(1-methyl-1H-pyrazol-4-yl)-2-(pyridin-3-yl)-1H-inden-1-one

In the same manner as Step 5 of Preparation Example 21, using 2-bromo-6-methoxy-3-(1-methyl-1H-pyrazol-4-yl)-1H-inden-1-one and pyridin-3-ylboronic acid, the desired title compound (390 mg, 89 %) was obtained as a red solid.

LCMS (ESI-MS): mass calcd. C₁₉H₁₅N₃O₂ 317.1 m/z, found 318.0 [M+H]⁺.

¹H NMR (400 MHz, DMSO-*d*₆) δ 8.56 - 8.54 (m, 1H), 8.47 - 8.46 (m, 1H), 8.31 (s, 1H), 7.72 - 7.69 (m, 1H), 7.57 - 7.55 (m, 1H), 7.46 - 7.43 (m, 2H), 7.12 - 7.11 (m, 1H), 7.03 - 7.01 (m, 1H), 3.90 (s, 3H), 3.86 (s, 3H).

### Step 4: Synthesis of 6-hydroxy-3-(1-methyl-1H-pyrazol-4-yl)-2-(pyridin-3-yl)-1H-inden-1-one

To a solution of 6-methoxy-3-(1-methyl-1H-pyrazol-4-yl)-2-(pyridin-3-yl)-1H-inden-1-one (370 mg, 1.1659 mmol) in DCM (8 mL) was added BBr₃ (4 mL), and the mixture was stirred at 25 °C for 16 hours. The reaction was quenched by the addition of water (5 mL), and the pH was adjusted to 7 with an aqueous NaHCO₃ solution. The mixture was then extracted with DCM (3 x 40 mL). Using a conventional method, the organic layer was washed, dried, and concentrated under reduced pressure. The residue was purified by silica gel column chromatography (3 % MeOH/DCM) to obtain the desired title compound (300 mg, 85 %) as a red solid.

LCMS (ESI-MS): mass calcd. C₁₃H₁₃N₃O₂ 303.1 m/z, found 301.8 [M-H]⁻.

¹H NMR (400 MHz, DMSO-*d*₆) δ 10.17 (s, 1H), 8.54 - 8.52 (m, 1H), 8.45 - 8.44 (m, 1H), 8.27 (s, 1H), 7.70 - 7.67 (m, 1H), 7.46 - 7.40 (m, 3H), 6.94 - 6.93 (m, 1H), 6.85 - 6.82 (m, 1H), 3.89 (s, 3H).

### Step 5: Synthesis of 3-(1-methyl-1H-pyrazol-4-yl)-6-(3-phenylpropoxy)-2-(pyridin-3-yl)-1H-inden-1-one (Compound 42)

The desired title compound (20.9 mg, 14 %) was obtained as a red solid from 6-hydroxy-3-(1-methyl-1 H-pyrazol-4-yl)-2-(pyridin-3-yl)-1 H-inden-1-one in the same manner as Step 2 of Preparation Example 38.

The obtained Compound 42 (Formula 43 below) was confirmed by LCMS(ESI-MS) and ¹H NMR, which yielded the following data:

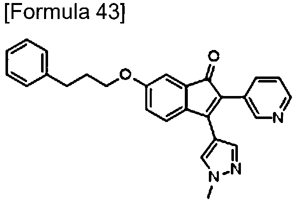

LCMS (ESI-MS): mass calcd. C₂₇H₂₃N₃O₂ 421.2 m/z, found 422.2 [M+H]⁺.

¹H NMR (400 MHz, DMSO-*d*₆) δ 8.56 - 8.54 (m, 1H), 8.47 - 8.46 (m, 1H), 8.31 (s, 1H), 7.72 - 7.70 (m, 1H), 7.56 - 7.54 (m, 1H), 7.46 - 7.42 (m, 2H), 7.32 - 7.17 (m, 5H), 7.11 - 7.10 (m, 1H), 7.03 - 7.00 (m, 1H), 4.09 - 4.06 (m, 2H), 3.90 (s, 3H), 2.78 - 2.74 (m, 2H), 2.08 - 2.02 (m, 2H).

### Preparation Example 42. 6-(3-phenylpropoxy)-3-(1H-pyrazol-4-yl)-2-(pyridin-3-yl)-1H-inden-1-one (Compound 43)

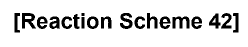

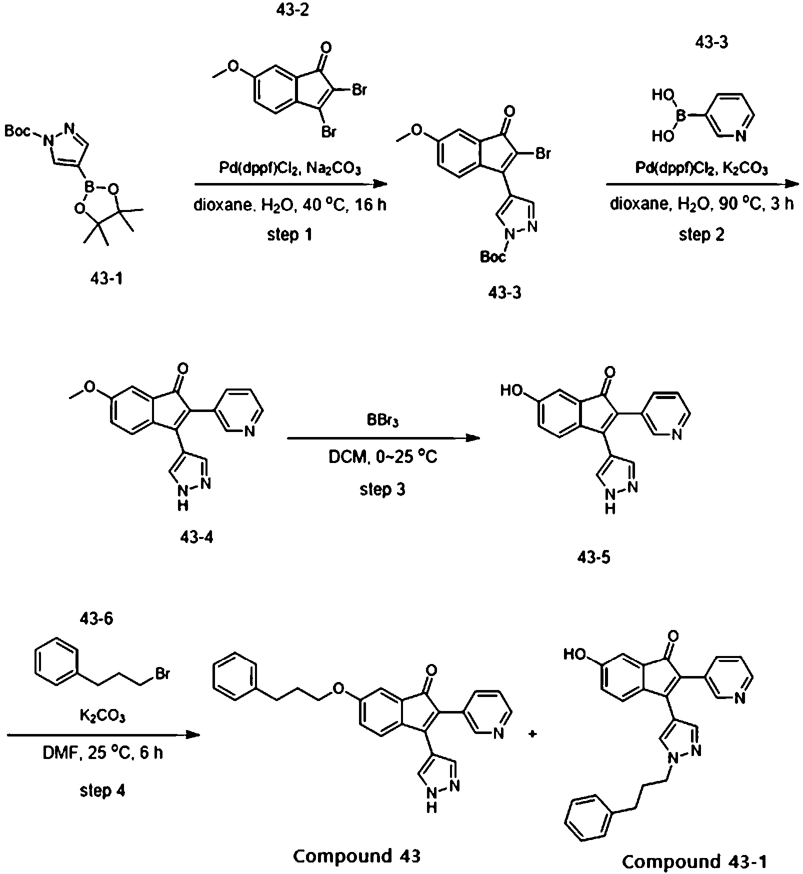

### Step 1: Synthesis of tert-butyl 4-(2-bromo-6-methoxy-1-oxo-1H-inden-3-yl)-1H-pyrazole-1-carboxylate

In the same manner as Step 2 of Preparation Example 41, the desired title compound (300 mg, 22 %) was obtained as a yellow solid.

LCMS (ESI-MS): mass calcd. C₁₈H₁₇BrN₂O₄ 404.0 m/z, found 404.6 [M+H]⁺.

### Step 2: Synthesis of 6-methoxy-3-(1H-pyrazol-4-yl)-2-(pyridin-3-yl)-1H-inden-1-one

In the same manner as Step 5 of Preparation Example 21, the desired title compound (170 mg, 76 %) was obtained as a white solid.

¹H NMR (400 MHz, DMSO-*d*₆) δ 13.51 (s, 1H), 8.54 (dd, *J* = 4.8, 1.6 Hz, 1H), 8.46 (d, *J* = 1.6 Hz, 1H), 8.29 (s, 1H), 7.71 (dt, *J* = 8.0, 2.0 Hz, 1H), 7.64 - 7.37 (m, 3H), 7.12 (d, *J* = 2.4 Hz, 1H), 7.02 (dd, *J* = 8.0, 2.4 Hz, 1H), 3.85 (s, 3H).

### Step 3: Synthesis of 6-hydroxy-3-(1H-pyrazol-4-yl)-2-(pyridin-3-yl)-1H-inden-1-one

In the same manner as Step 4 of Preparation Example 41, the desired title compound (60 mg, 37 %) was obtained as a blue solid.

LCMS (ESI-MS): mass calcd. C₁₇H₁₁N₃O₂ 289.1 m/z, found 289.7 [M+H]⁺.

### Step 4: Synthesis of 6-(3-phenylpropoxy)-3-(1H-pyrazol-4-yl)-2-(pyridin-3-yl)-1H-inden-1-one (Compound 43)

In the same manner as Step 5 of Preparation Example 41, the desired title compound (2.9 mg, 4 %) and 6-hydroxy-3-(1-(3-phenylpropyl)-1H-pyrazol-4-yl)-2-(pyridin-3-yl)-1H-inden-1-one (Compound 42-1, 5.7 mg, 7 %) as a reaction byproduct were obtained as a red solid.

The obtained Compound 43 (Formula 44 below) was confirmed by LCMS(ESI-MS) and ¹H NMR, which yielded the following data:

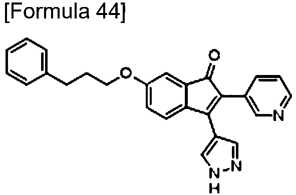

LCMS (ESI-MS): mass calcd. C₂₈H₂₁N₃O₂ 407.2 m/z, found 407.8 [M+H]⁺.

¹H NMR (400 MHz, DMSO-*d*₆) δ 13.51 (s, 1H), 8.54 (dd, *J* = 4.8, 1.6 Hz, 1H), 8.45 (d, *J* = 2.0 Hz, 1H), 8.31 - 8.23 (m, 1H), 7.71 (dt, *J* = 8.0, 1.6 Hz, 1H), 7.64 - 7.40 (m, 3H), 7.36 - 7.17 (m, 5H), 7.11 (d, *J* = 2.4 Hz, 1H), 7.01 (dd, *J* = 8.0, 2.4 Hz, 1H), 4.07 (t, *J* = 6.4 Hz, 2H), 2.92 - 2.72 (m, 2H), 2.15 - 1.93 (m, 2H).

The obtained Compound 43-1 was confirmed by LCMS(ESI-MS) and ¹H NMR, which yielded the following data:
LCMS (ESI-MS): mass calcd. C₂₈H₂₁N₃O₂ 407.2 m/z, found 407.9 [M+H]⁺.
¹H NMR (400 MHz, DMSO-*d*₆) δ 10.19 (s, 1H), 8.53 (dd, *J* = 4.8, 1.6 Hz, 1H), 8.46 (d, *J = 1.6* Hz, 1H), 8.28 (s, 1H), 7.70 (dt, *J* = 8.0, 2.0 Hz, 1H), 7.56 - 7.40 (m, 3H), 7.36 - 7.24 (m, 2H), 7.19 (dd, *J* = 7.2, 3.2 Hz, 3H), 6.94 (d, *J* = 2.4 Hz, 1H), 6.84 (dd, *J =* 8.0, 2.4 Hz, 1H), 4.16 (t, *J* = 6.8 Hz, 2H), 2.55 (s, 2H), 2.17 - 1.94 (m, 2H).

### Preparation Example 43. 3-(2-methoxyphenyl)-6-(3-phenylpropoxy)-2-(pyridin-3-yl)-1H-inden-1-one (Compound 44)

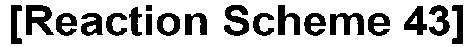

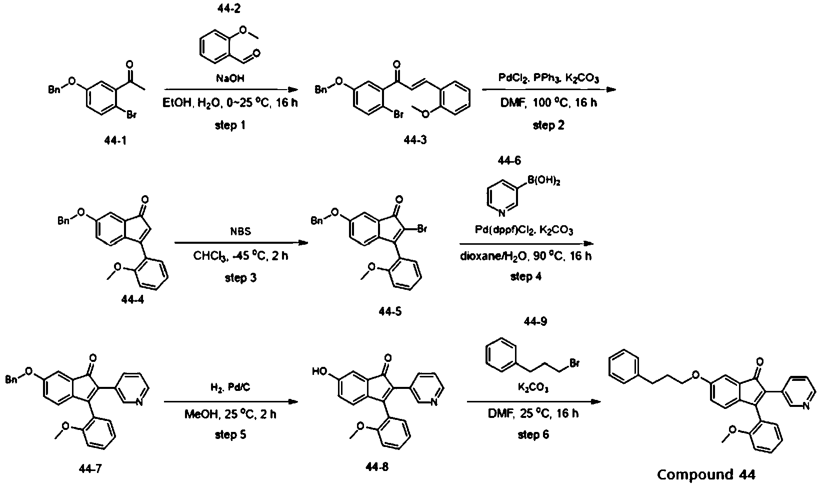

### Step 1: Synthesis of (E)-1-(5-(benzyloxy)-2-bromophenyl)-3-(2-methoxyphenyl)prop-2-en-1-one

In the same manner as Step 1 of Preparation Example 21, the desired title compound (1.3 mg, 84 %) was obtained as a yellow solid.

LCMS (ESI-MS): mass calcd. C₂₃H₁₉BrO₃ 422.1 m/z, found 422.7 [M+H]⁺.

### Step 2: Synthesis of 6-(benzyloxy)-3-(2-methoxyphenyl)-1H-inden-1-one)

To a mixture of (E)-1-(5-(benzyloxy)-2-bromophenyl)-3-(2-methoxyphenyl)prop-2-en-1-one (1.3 g, 0.0031 mol), PdCl₂ (0.0274 g, 0.000155 mol), PPh₃ (0.16 g, 0.0006 mol), and K₂CO₃ (1.29 g, 0.0093 mol) was added DMF (28 mL), and the resulting mixture was stirred at 100 °C for 16 hours under a nitrogen atmosphere. Water (50 mL) was added to the reaction mixture, and the mixture was extracted with EA (3 x 80 mL). Using a conventional method, the organic layer was washed, dried, and concentrated under reduced pressure. The residue was purified by silica gel column chromatography (10 % EtOAc/hexane) to obtain the desired title compound (900 mg, 77 %) as a yellow solid.

LCMS (ESI-MS): mass calcd. C₂₃H₁₈O₃ 342.1 m/z, found 342.8 [M+H]⁺.

### Step 3: Synthesis of 6-(benzyloxy)-2-bromo-3-(2-methoxyphenyl)-1H-inden-1-one

To a solution of 6-(benzyloxy)-3-(2-methoxyphenyl)-1H-inden-1-one (880 mg, 2.56 mmol) in CHCl₃ (35 mL) was added NBS (478.9 mg, 2.69 mmol) at -45 °C, and the mixture was stirred at the same temperature for 2 hours. Water (50 mL) was added to the reaction mixture, and the mixture was extracted with EA (3 x 80 mL). Using a conventional method, the organic layer was washed, dried, and concentrated under reduced pressure. The residue was purified by silica gel column chromatography (2 % EtOAc/hexane) to obtain the desired title compound (350 mg, 26 %) as a yellow solid.

LCMS (ESI-MS): mass calcd. C₂₃H₁₇BrO₃ 420.0 m/z, found 420.7 [M+H]⁺.

¹H NMR (400 MHz, DMSO-*d*₆) δ 7.60 - 7.51 (m, 1H), 7.45 (d, *J* = 6.8 Hz, 2H), 7.40 (t, *J =* 7.2Hz, 3H), 7.35 (d, *J =* 7.2 Hz, 1H), 7.24 (d, *J* = 8.4 Hz, 1H), 7.19 (d, *J =* 2.4 Hz, 1H), 7.13 (t, *J* = 7.4 Hz, 1H), 6.99 (dd, *J* = 8.0, 2.4 Hz, 1H), 6.81 (d, *J* = 8.0 Hz, 1H), 5.17 (s, 2H), 3.80 (s, 3H).

### Step 4: Synthesis of 6-(benzyloxy)-3-(2-methoxyphenyl)-2-(pyridin-3-yl)-1H-inden-1-one

In the same manner as Step 5 of Preparation Example 21, the desired title compound (270 mg, 71 %) was obtained as a yellow solid.

LCMS (ESI-MS): mass calcd. C₂₈H₂₁NO₃ 419.2m/z, found 419.9 [M+H]⁺.

### Step 5: Synthesis of 6-hydroxy-3-(2-methoxyphenyl)-2-(pyridin-3-yl)-1H-inden-1-one

To a mixture of 6-(benzyloxy)-3-(2-methoxyphenyl)-2-(pyridin-3-yl)-1H-inden-1-one (270 mg, 0.6421 mmol) and Pd/C (10 mg) was added MeOH (13 mL), and the mixture was stirred at room temperature for 2 hours under a hydrogen atmosphere. The reaction mixture was filtered through Celite, and the resulting filtrate was concentrated. The residue was purified by silica gel column chromatography (50 % EtOAc/hexane) to obtain the desired title compound (70 mg, 31 %) as an orange solid.

LCMS (ESI-MS): mass calcd. C₂₁H₁₅NO₃ 329.1 m/z, found 329.7 [M+H]⁺.

¹H NMR (400 MHz, DMSO-*d*₆) δ 10.26 (s, 1H), 8.39 (d, *J* = 3.2 Hz, 1H), 8.27 (d, *J =* 1.6 Hz, 1H), 7.54 (d, *J* = 8.0 Hz, 1H), 7.51 - 7.44 (m, 1H), 7.32 - 7.29 (m, 1H), 7.24 (dd, *J =* 7.6, 1.6 Hz, 1H), 7.16 (d, *J =* 8.4 Hz, 1H), 7.03 (t, *J* = 7.6 Hz, 1H), 6.95 (s, 1H), 6.76 (s, 2H), 3.56 (s, 3H).

### Step 6: Synthesis of 3-(2-methoxyphenyl)-6-(3-phenylpropoxy)-2-(pyridin-3-yl)-1H-inden-1-one (Compound 44)

In the same manner as Step 2 of Preparation Example 38, the desired title compound (5.0 mg, 6 %) was obtained as a red solid.

The obtained Compound 44 (Formula 45 below) was confirmed by LCMS(ESI-MS) and ¹H NMR, which yielded the following data:

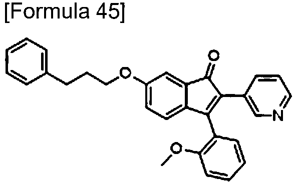

LCMS (ESI-MS): mass calcd. C₃₀H₂₅NO₃ 447.2 m/z, found 447.9 [M+H]⁺.

¹H NMR (400 MHz, DMSO-*d*₆) δ 8.41 - 8.40 (m, 1H), 8.29 (s, 1H), 7.55 (dd, J = 6.0, 4.0 Hz, 1H), 7.50 - 7.46 (m, 1H), 7.34 - 7.31 (m, 1H), 7.31 - 7.27 (m, 2H), 7.24 (d, *J =* 7.2 Hz, 3H), 7.19 (t, *J =* 8.4 Hz, 2H), 7.13 - 7.12 (m, 1H), 7.04 (t, *J* = 7.4 Hz, 1H), 6.95 (dd, *J =* 8.0, 2.4 Hz, 1H), 6.85 (d, *J* = 8.0 Hz, 1H), 4.04 (t, *J* = 6.4 Hz, 2H), 3.58 (s, 3H), 2.78 - 2.73 (m, 2H), 2.08 - 2.00 (m, 2H).

### Preparation Example 44. 3-(2,6-dimethoxyphenyl)-6-(3-phenylpropoxy)-2-(pyridin-3-yl)-1H-inden-1-one (Compound 45)

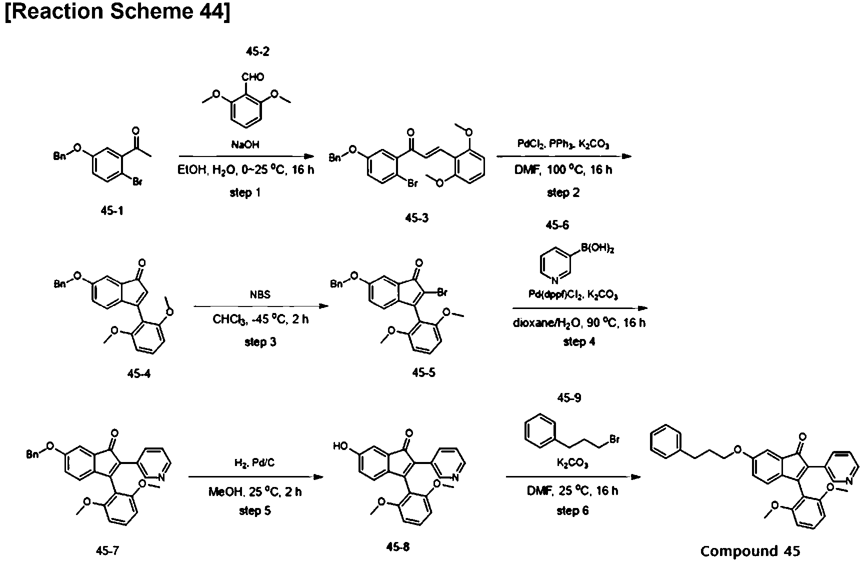

### Step 1: Synthesis of (E)-1-(5-(benzyloxy)-2-bromophenyl)-3-(2,6-dimethoxyphenyl)prop-2-en-1-one

In the same manner as Step 1 of Preparation Example 21, the desired title compound (1.81 g, 95 %) was obtained as a yellow solid.

LCMS (ESI-MS): mass calcd. C₂₄H₂₁BrO₄ 452.1 m/z, found 453.1 [M+H]⁺.

### Step 2: Synthesis of 6-(benzyloxy)-3-(2,6-dimethoxyphenyl)-1H-inden-1-one

In the same manner as Step 2 of Preparation Example 43, the desired title compound (1.04 g, 70 %) was obtained as a yellow solid.

LCMS (ESI-MS): mass calcd. C₂₄H₂₀O₄ 372.1 m/z, found 372.7 [M+H]⁺.

### Step 3: Synthesis of 6-(benzyloxy)-2-bromo-3-(2,6-dimethoxyphenyl)-1H-inden-1-one

In the same manner as Step 5 of Preparation Example 46, the desired title compound (100 mg, 71 %) was obtained as a yellow solid.

LCMS (ESI-MS): mass calcd. C₂₄H₁₉BrO₄ 450.0 m/z, found 451.0 [M+H]⁺.

### Step 4: Synthesis of 6-(benzyloxy)-3-(2,6-dimethoxyphenyl)-2-(pyridin-3-yl)-1H-inden-1-one

In the same manner as Step 5 of Preparation Example 21, the desired title compound (200 mg, 81 %) was obtained as a red solid.

LCMS (ESI-MS): mass calcd. C₂₉H₂₃NO₄ 449.2m/z, found 450.1 [M+H]⁺.

### Step 5: Synthesis of 3-(2,6-dimethoxyphenyl)-6-hydroxy-2-(pyridin-3-yl)-1H-inden-1-one

In the same manner as Step 5 of Preparation Example 43, the desired title compound (120 mg, 75 %) was obtained as a red solid.

LCMS (ESI-MS): mass calcd. C₂₂H₁₇NO₄ 359.1 m/z, found 360.0 [M+H]⁺.

¹H NMR (400 MHz, DMSO-*d*₆) δ 10.07 (s, 1H), 8.38 - 8.30 (m, 2H), 7.57 - 7.54 (m, 1H), 7.45 - 7.41 (m, 1H), 7.31 - 7.28 (m, 1H), 6.92 - 6.91 (m, 1H), 6.78 - 6.72 (m, 3H), 6.58 - 6.56 (m, 1H), 3.58 (s, 6H).

### Step 6: Synthesis of 3-(2,6-dimethoxyphenyl)-6-(3-phenylpropoxy)-2-(pyridin-3-yl)-1H-inden-1-one (Compound 45)

In the same manner as Step 2 of Preparation Example 38, the desired title compound (20 mg, 16 %) was obtained as a red solid.

The obtained Compound 45 (Formula 46 below) was confirmed by LCMS(ESI-MS) and ¹H NMR, which yielded the following data:

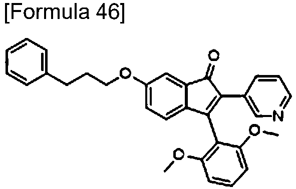

LCMS (ESI-MS): mass calcd. C₃₁H₂₇NO₄ 477.2 m/z, found 478.3 [M+H]⁺.

¹H NMR (400 MHz, DMSO-*d*₆) δ 8.41 - 8.31 (m, 2H), 7.59 - 7.56 (m, 1H), 7.46 - 7.42 (m, 1H), 7.33 - 7.17 (m, 6H), 7.10 - 7.09 (m, 1H), 6.93 - 6.90 (m, 1H), 6.79 - 6.77 (m, 2H), 6.68 - 6.66 (m, 1H), 4.05 - 4.02 (m, 2H), 3.58 (s, 6H), 2.77 - 2.73 (m, 2H), 2.06 - 2.01 (m, 2H).

### Preparation Example 45. 4-methyl-3-(4-methylthiazol-5-yl)-6-(3-phenylpropoxy)-2-(pyridin-3-yl)-1H-inden-1-one (Compound 46)

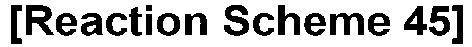

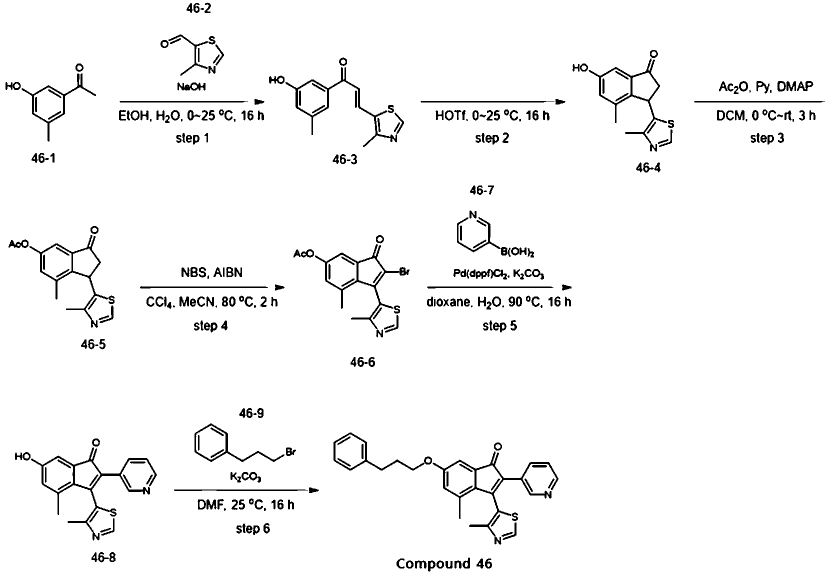

### Step 1: Synthesis of (E)-1-(3-hydroxy-5-methylphenyl)-3-(4-methylthiazol-5-yl)prop-2-en-1-one

In the same manner as Step 4 of Preparation Example 21, the desired title compound (180 mg, 38 %) was obtained as a yellow solid.

LCMS (ESI-MS): mass calcd. C₁₄H₁₃NO₂S 259.1 m/z, found 260.0 [M+H]⁺.

### Step 2: Synthesis of 6-hydroxy-4-methyl-3-(4-methylthiazol-5-yl)-2,3-dihydro-1H-inden-1-one

In the same manner as Step 3 of Preparation Example 78, the desired title compound (0.4 g, 7 %) was obtained as a yellow solid.

LCMS (ESI-MS): mass calcd. C₁₄H₁₃NO₂S 259.1 m/z, found 259.7[M+H]⁺.

### Step 3: Synthesis of 7-methyl-1-(4-methylthiazol-5-yl)-3-oxo-2,3-dihydro-1H-inden-5-yl acetate

In the same manner as Step 3 of Preparation Example 21, the desired title compound (0.9 g, 69 %) was obtained as a yellow oil.

LCMS (ESI-MS): mass calcd. C₁₆H₁₅NO₃S 301.1 m/z, found 301.7 [M+H]⁺.

### Step 4: Synthesis of 2-bromo-4-methyl-3-(4-methylthiazol-5-yl)-1-oxo-1H-inden-6-yl acetate

In the same manner as Step 4 of Preparation Example 21, the desired title compound (180 mg, 38 %) was obtained as a yellow solid.

LCMS (ESI-MS): mass calcd. C₁₆H₁₂BrNO₃S 377.0 m/z, found 377.6 [M+H]⁺.

### Step 5: Synthesis of 6-hydroxy-4-methyl-3-(4-methylthiazol-5-yl)-2-(pyridin-3-yl)-1H-inden-1-one

In the same manner as Step 5 of Preparation Example 21, the desired title compound (50 mg, 72 %) was obtained as a red solid.

LCMS (ESI-MS): mass calcd. C₁₉H₁₄N₂O₂S 334.1 m/z, found 335.0 [M+H]⁺.

### Step 6: Synthesis of 4-methyl-3-(4-methylthiazol-5-yl)-6-(3-phenylpropoxy)-2-(pyridin-3-yl)-1H-inden-1-one (Compound 46)

In the same manner as Step 2 of Preparation Example 38, the desired title compound (40 mg, 53 %) was obtained as a red solid.

The obtained Compound 46 (Formula 47 below) was confirmed by LCMS(ESI-MS) and ¹H NMR, which yielded the following data:

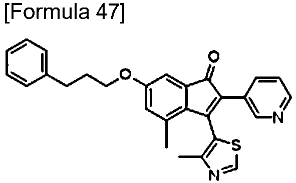

LCMS (ESI-MS): mass calcd. C₂₈H₂₄N₂O₂S 452.2 m/z, found 452.9 [M+H]⁺.

¹H NMR (400 MHz, DMSO-*d*₆) δ 9.23 (s, 1H), 8.44 - 8.43 (m, 1H), 8.30 (s, 1H), 7.57 - 7.52 (m, 1H), 7.41 - 7.16 (m, 6H), 7.02 - 7.01 (m, 1H), 6.78 (s, 1H), 4.04 (t, *J* = 6.4 Hz, 2H), 2.79 - 2.70 (m, 2H), 2.24 (s, 3H), 2.07 - 1.98 (m, 2H), 1.84 (s, 3H).

### Preparation Example 46. 4-methoxy-3-(4-methylthiazol-5-yl)-6-(3-phenylpropoxy)-2-(pyridin-3-yl)-1H-inden-1-one (Compound 47)

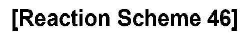

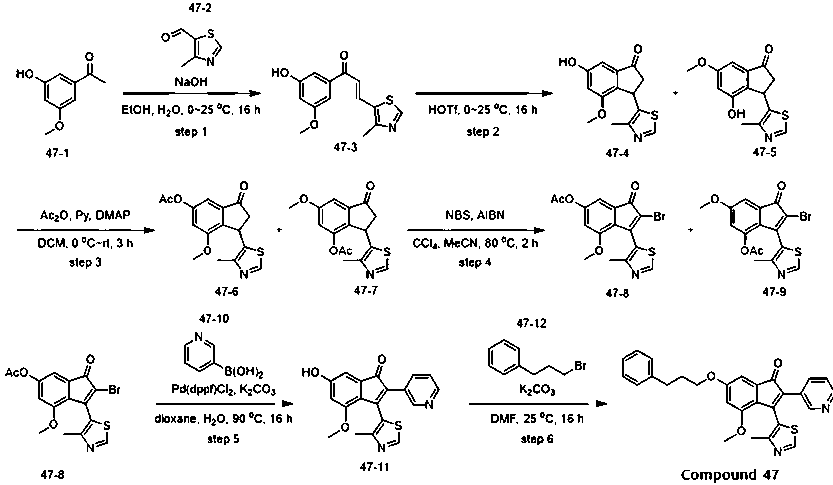

### Step 1: Synthesis of (E)-1-(3-hydroxy-5-methoxyphenyl)-3-(4-methylthiazol-5-yl)prop-2-en-1-one

In the same manner as Step 1 of Preparation Example 21, the desired title compound (0.95 g, 57 %) was obtained as a yellow solid.

¹H NMR (400 MHz, DMSO-*d*₆) δ 9.85 (s, 1H), 9.14 (s, 1H), 7.88 (d, *J* = 15.2 Hz, 1H), 7.37 (d, *J =* 15.2 Hz, 1H), 7.06 (d, *J* = 1.2 Hz, 2H), 6.62 (t, *J* = 2.4 Hz, 1H), 3.79 (s, 3H), 2.55 (s, 3H).

### Step 2: Synthesis of 6-hydroxy-4-methoxy-3-(4-methylthiazol-5-yl)-2,3-dihydro-1H-inden-1-one (47-4) and 4-hydroxy-6-methoxy-3-(4-methylthiazol-5-yl)-2,3-dihydro-1H-inden-1-one

In the same manner as Step 2 of Preparation Example 21, two structural isomers of the desired title compound (650 mg, 68 %) were obtained as a yellow solid and used in the next reaction without purification.

LCMS (ESI-MS): mass calcd. C₁₄H₁₃NO₃S 275.1 m/z, found 275.6 [M+H]⁺.

### Step 3: Synthesis of 7-methoxy-1-(4-methylthiazol-5-yl)-3-oxo-2,3-dihydro-1H-inden-5-yl acetate and 6-methoxy-3-(4-methylthiazol-5-yl)-1-oxo-2,3-dihydro-1H-inden-4-yl acetate

In the same manner as Step 3 of Preparation Example 21, two structural isomers of the desired title compound (260 mg, 35 %) were obtained as a yellow solid and used in the next reaction without purification.

LCMS (ESI-MS): mass calcd. C₁₆H₁₅NO₄S 317.1 m/z, found 317.6 [M+H]⁺.

### Step 4: Synthesis of 2-bromo-4-methoxy-3-(4-methylthiazol-5-yl)-1-oxo-1H-inden-6-yl acetate and 2-bromo-6-methoxy-3-(4-methylthiazol-5-yl)-1-oxo-1H-inden-4-yl acetate

In the same manner as Step 4 of Preparation Example 21, the reaction and purification were performed to obtain title compounds A (130 mg, 42 %) and B (150 mg, 50 %) as a yellow solid.

The results of ¹H NMR measurement for Compound 46-8 are as follows:

¹H NMR (400 MHz, DMSO-*d*₆) δ 9.24 (s, 1H), 7.08 (s, 1H), 7.04 (s, 1H), 3.64 (s, 3H), 2.35 (s, 3H), 2.28 (s,3H).

### Step 5: Synthesis of 6-hydroxy-4-methoxy-3-(4-methylthiazol-5-yl)-2-(pyridin-3-yl)-1H-inden-1-one

In the same manner as Step 5 of Preparation Example 21, the desired title compound (70 mg, 67 %) was obtained as a yellow solid.

### Step 6: Synthesis of 4-methoxy-3-(4-methylthiazol-5-yl)-6-(3-phenylpropoxy)-2-(pyridin-3-yl)-1H-inden-1-one (Compound 47)

In the same manner as Step 2 of Preparation Example 38, the desired title compound (30 mg, 38 %) was obtained as a purple solid.

The obtained Compound 47 (Formula 48 below) was confirmed by LCMS(ESI-MS) and ¹H NMR, which yielded the following data:

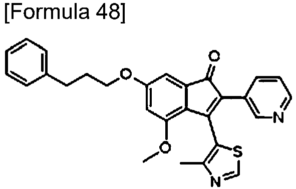

LCMS (ESI-MS): mass calcd. C₂₈H₂₄N₂O₃S 468.2 m/z, found 468.9 [M+H]⁺.

¹H NMR (400 MHz, DMSO-*d*₆) δ 9.16 (s, 1H), 8.43 (dd, *J* = 4.8, 1.6 Hz, 1H), 8.22 (d, *J = 1.6* Hz, 1H), 7.48 (d, *J* = 8.0 Hz, 1H), 7.39 - 7.14 (m, 6H), 6.84 (d, *J* = 2.0 Hz, 1H), 6.71 (d, *J =* 1.6 Hz, 1H), 4.11 (t, *J* = 6.4 Hz, 2H), 3.67 (s, 3H), 2.82 - 2.72 (m, 2H), 2.14 - 1.96 (m, 5H).

### Preparation Example 47. 4-hydroxy-3-(4-methylthiazol-5-yl)-6-(3-phenylpropoxy)-2-(pyridin-3-yl)-1H-inden-1-one (Compound 48)

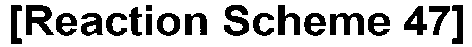

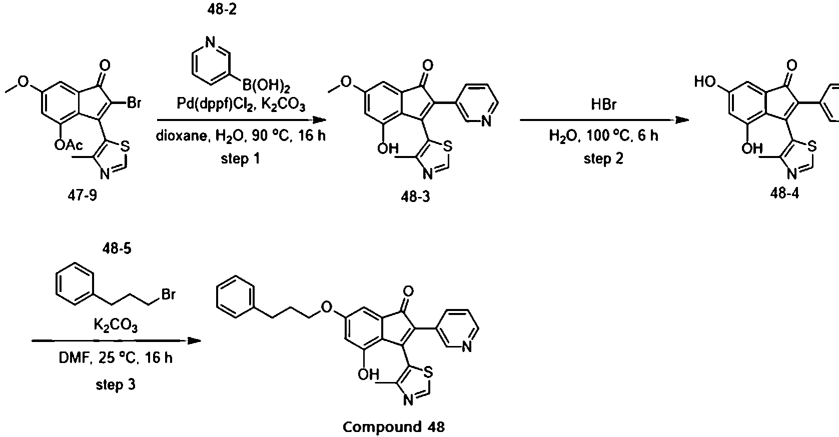

### Step 1: Synthesis of 4-hydroxy-6-methoxy-3-(4-methylthiazol-5-yl)-2-(pyridin-3-yl)-1H-inden-1-one

In the same manner as Step 5 of Preparation Example 46, the desired title compound (100 mg, 71 %) was obtained as a yellow solid.

### Step 2: Synthesis of 4,6-dihydroxy-3-(4-methylthiazol-5-yl)-2-(pyridin-3-yl)-1H-inden-1-one

To 4-hydroxy-6-methoxy-3-(4-methylthiazol-5-yl)-2-(pyridin-3-yl)-1H-inden-1-one (95 mg, 0.27 mmol) was added an aqueous HBr solution (1 mL), and the mixture was stirred at 100 °C for 6 hours.

The reaction mixture was concentrated to obtain the desired title compound (100 mg, crude) as a yellow solid.

### Step 3: Synthesis of 4-hydroxy-3-(4-methylthiazol-5-yl)-6-(3-phenylpropoxy)-2-(pyridin-3-yl)-1H-inden-1-one (Compound 48)

In the same manner as Step 2 of Preparation Example 38, the desired title compound (3 mg, 3 %) was obtained as a yellow solid.

The obtained Compound 48 (Formula 49 below) was confirmed by LCMS(ESI-MS) and ¹H NMR, which yielded the following data:

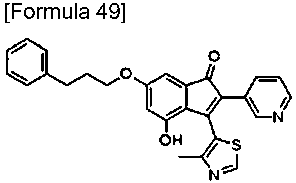

LCMS (ESI-MS): mass calcd. C₂₇H₂₂N₂O₃S 454.1 m/z, found 454.9 [M+H]⁺.

¹H NMR (400 MHz, DMSO-*d*₆) δ 9.25 (s, 1H), 7.78 (d, *J* = 2.0 Hz, 1H), 7.35 - 7.16 (m, 5H), 7.09 (s, 1H), 6.97 (d, *J* = 4.4 Hz, 2H), 6.60 (d, *J* = 2.0 Hz, 1H), 4.03 (s, 3H), 3.74 (s, 2H), 2.87 - 2.70 (m, 4H), 2.35 - 2.20 (m, 2H), 2.10 (s, 3H), 2.07 - 1.97 (m, 2H), 1.97 - 1.72 (m, 4H).

### Preparation Example 48. 6-(cyclopentylamino)-3-(4-methylthiazol-5-yl)-2-(pyridin-3-yl)-1H-inden-1-one (Compound 49)

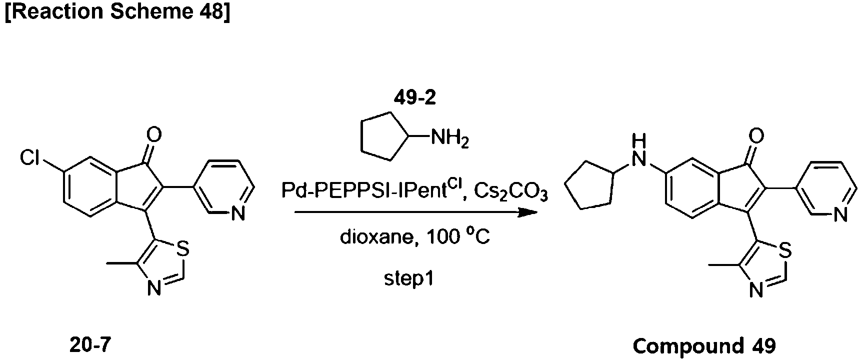

A mixture of 6-chloro-3-(4-methylthiazol-5-yl)-2-(pyridin-3-yl)-1H-inden-1-one (100 mg, 0.30 mmol), cyclopentylamine (51 mg, 0.60 mmol), PD-PEPPSI-IPent^{CI} (28 mg, 0.03 mmol), and Cs₂CO₃ (195 mg, 0.60 mmol) in dioxane/H₂O (5 mL/1 mL) was stirred at 100 °C for 16 hours under a nitrogen atmosphere. The resulting reaction mixture was cooled to room temperature, and then treated with water (5 mL) and extracted with EA (3 x 10 mL). Using a conventional method, the organic layer was washed, dried, and concentrated under reduced pressure. The residue was purified by prep-HPLC (Gemini 5 µm C₁₈ column, 150 x 21.2 mm, 30 - 90 % ACN/H₂O with 0.1% FA) to obtain the desired title compound (38 mg, 33 %) as a blue solid.

The obtained Compound 49 (Formula 50 below) was confirmed by LCMS (ESI-MS) and 1H NMR, which yielded the following data:

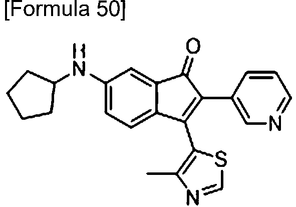

LCMS (ESI-MS): mass calcd. C₂₃H₂₁N₃OS 387.1 m/z, found 387.7 [M+H]⁺.

¹H NMR (400 MHz, DMSO-*d*₆) δ 9.28 (s, 1H), 8.43 (dd, *J* = 4.8, 1.6 Hz, 1H), 8.30 (d, *J* = 2.0 Hz, 1H), 7.55 (dt, J = 8.0, 2.0 Hz, 1H), 7.36 (dd, *J =* 8.0, 4.8 Hz, 1H), 6.91 (dd, *J =* 5.2, 3.6 Hz, 2H), 6.56 (d, *J* = 6.8 Hz, 1H), 6.46 (dd, *J* = 8.0, 2.0 Hz, 1H), 3.93 - 3.66 (m, 1H), 2.02 - 1.84 (m, 5H), 1.79 - 1.31 (m, 6H).

### Preparation Example 49. 6-(methylamino)-3-(4-methylthiazol-5-yl)-2-(pyridin-3-yl)-1H-inden-1-one (Compound 50)

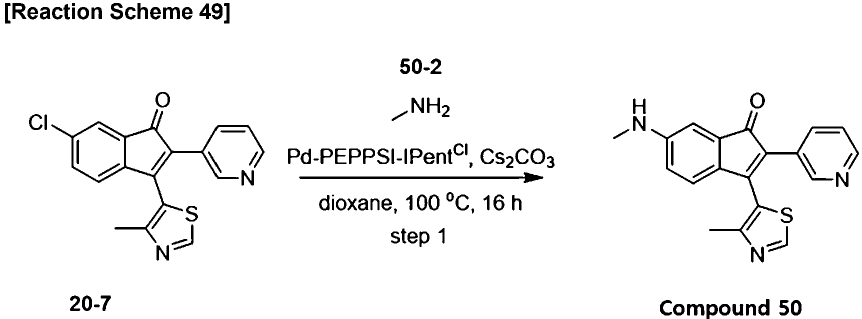

In the same manner as Preparation Example 48, using methanamine, the desired title compound (11.9 mg, 12 %) was obtained as a blue solid.

The obtained Compound 50 (Formula 51 below) was confirmed by LCMS(ESI-MS) and ¹H NMR, which yielded the following data:

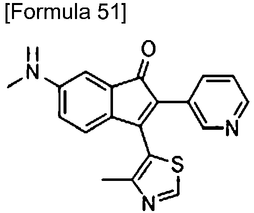

LCMS (ESI-MS): mass calcd. C₁₉H₁₅N₃OS 333.1 m/z, found 333.7 [M+H]⁺.

¹H NMR (400 MHz, DMSO-*d*₆) δ 9.28 (s, 1H), 8.44 (dd, J = 4.8, 1.6 Hz, 1H), 8.31 (d, *J* = 1.6 Hz, 1H), 7.56 (dt, J = 8.0, 2.0 Hz, 1H), 7.36 (dd, *J* = 8.0, 4.8 Hz, 1H), 6.94 (d, *J =* 8.0 Hz, 1H), 6.89 (d, *J = 2.4* Hz, 1H), 6.60 (d, *J =* 5.2 Hz, 1H), 6.42 (dd, *J* = 8.0, 2.0 Hz, 1H), 2.76 (d, *J = 5.2* Hz, 3H), 1.95 (s, 3H).

### Preparation Example 50. 6-((1-methylpiperidin-4-yl)amino)-3-(4-methylthiazol-5-yl)-2-(pyridin-3-yl)-1H-inden-1-one (Compound 51)

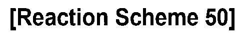

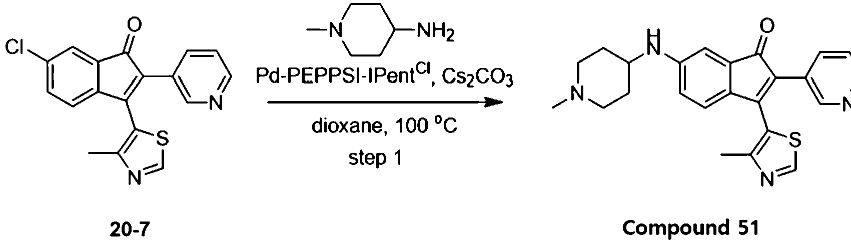

In the same manner as Preparation Example 48, using 1-methylpiperidin-4-amine, the desired title compound (37 mg, 35 %) was obtained as a blue solid.

The obtained Compound 51 (Formula 52 below) was confirmed by LCMS(ESI-MS) and ¹H NMR, which yielded the following data:

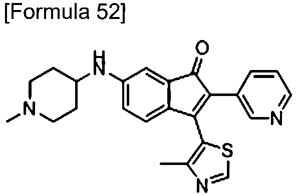

LCMS (ESI-MS): mass calcd. C₂₄H₂₄N₄OS 416.2 m/z, found 416.8 [M+H]⁺.

¹H NMR (400 MHz, DMSO-*d*₆) δ 9.28 (s, 1H), 8.44 (dd, *J* = 4.8, 1.6 Hz, 1H), 8.30 (d, *J* = 2.0 Hz, 1H), 8.20 (s, 1H), 7.56 - 7.53 (m, 1H), 7.37 - 7.34 (m, 1H), 6.93 - 6.89 (m, 2H), 6.54 - 6.41 (m, 2H), 3.35 (s, 1H), 2.81 (d, *J* = 11.6 Hz, 2H), 2.25 (s, 3H), 2.17 (t, *J =* 10.6 Hz, 2H), 1.94 - 1.89 (m, 5H), 1.44 (q, *J* = 13.4 Hz, 2H).

### Preparation Example 51. 3-(4-methylthiazol-5-yl)-2-(pyridin-3-yl)-6-(((tetrahydro-2H-pyran-4-yl)methyl)amino)-1H-inden-1-one (Compound 52)

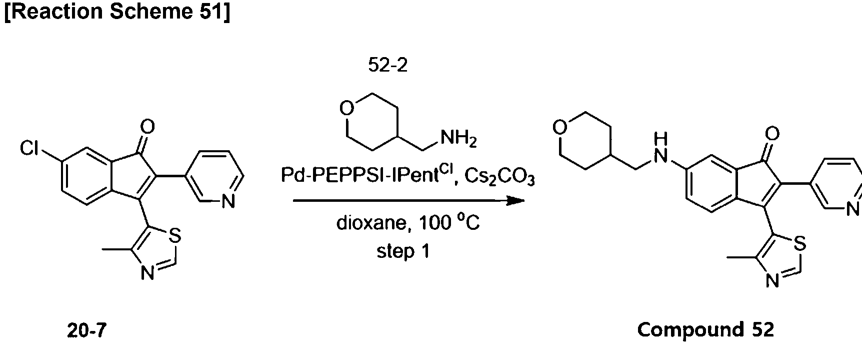

In the same manner as Preparation Example 48, using (tetrahydro-2H-pyran-4-yl)methanamine, the desired title compound (35 mg, 33 %) was obtained as a blue solid.

The obtained Compound 52 (Formula 53 below) was confirmed by LCMS(ESI-MS) and ¹H NMR, which yielded the following data:

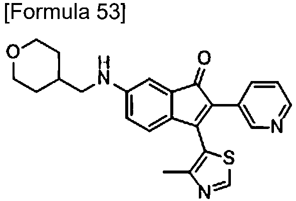

LCMS (ESI-MS): mass calcd. C₂₄H₂₃N₃O₂S 417.2 m/z, found 417.9 [M+H]⁺.

¹H NMR (400 MHz, DMSO-*d*₆) δ 9.28 (s, 1H), 8.43 (dd, J = 4.8, 1.6 Hz, 1H), 8.30 (d, *J* = 1.6 Hz, 1H), 7.56 - 7.54 (m, 1H), 7.36 (dd, *J = 7.4,* 4.8 Hz, 1H), 6.94 - 6.90 (m, 2H), 6.64 (t, *J* = 5.6 Hz, 1H), 6.53 - 6.47 (m, 1H), 3.86 (dd, *J* = 11.2, 3.2 Hz, 2H), 3.28 (t, *J* = 10.8 Hz, 2H), 3.01 (t, *J* = 6.2 Hz, 2H), 1.94 (s, 3H), 1.89 - 1.74 (m, 1H), 1.66 (d, *J* = 12.8 Hz, 2H), 1.31 - 1.15 (m, 2H).

### Preparation Example 52. 6-((2-hydroxyethyl)amino)-3-(4-methylthiazol-5-yl)-2-(pyridin-3-yl)-1H-inden-1-one (Compound 53)

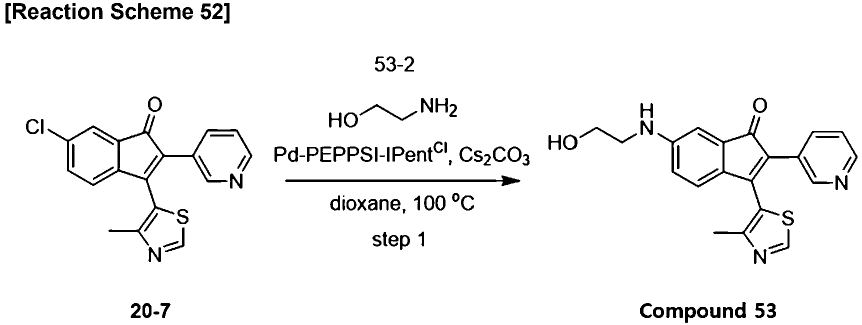

In the same manner as Preparation Example 48, using 2-aminoethan-1-ol, the desired title compound (20.0 mg, 22 %) was obtained as a blue solid.

The obtained Compound 53 (Formula 54 below) was confirmed by LCMS(ESI-MS) and ¹H NMR, which yielded the following data:

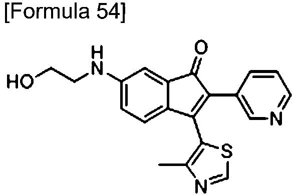

LCMS (ESI-MS): mass calcd. C₂₀H₁₇N₃O₂S 363.1 m/z, found 363.7 [M+H]⁺.

¹H NMR (400 MHz, DMSO-*d*₆) δ 9.28 (s, 1H), 8.43 (dd, *J* = 4.8, 1.7 Hz, 1H), 8.31 (d, *J* = 1.6 Hz, 1H), 7.55 (dt, J = 8.0, 2.0 Hz, 1H), 7.36 (dd, J = 8.0, 4.8 Hz, 1H), 6.93 (m, 2H), 6.59 - 6.46 (m, 2H), 4.78 (s, 1H), 3.56 (d, *J* = 5.2 Hz, 2H), 3.18 (q, *J* = 6.0 Hz, 2H), 1.95 (s, 3H).

### Preparation Example 53. 6-((2-methoxyethyl)amino)-3-(4-methylthiazol-5-yl)-2-(pyridin-3-yl)-1H-inden-1-one (Compound 54)

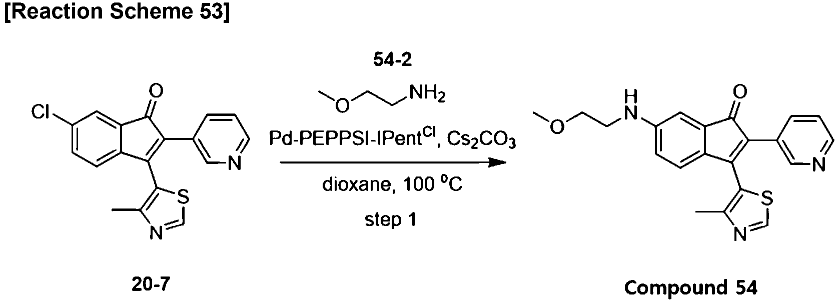

In the same manner as Preparation Example 48, using 2-methoxyethan-1-amine, the desired title compound (5.3 mg, 5 %) was obtained as a blue solid.

The obtained Compound 54 (Formula 55 below) was confirmed by LCMS(ESI-MS) and ¹H NMR, which yielded the following data:

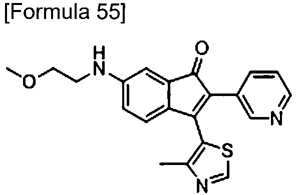

LCMS (ESI-MS): mass calcd. C₂₁H₁₉N₃O₂S 377.1 m/z, found 377.7 [M+H]⁺.

¹H NMR (400 MHz, CDCl₃) δ 8.92 (s, 1H), 8.48 (d, J = 6.4 Hz, 2H), 7.69 (d, *J = 7.6* Hz, 1H), 7.34 - 7.28 (m, 1H), 6.94 (dd, J = 6.8, 5.2 Hz, 2H), 6.48 (dd, J = 8.0, 2.3 Hz, 1H), 3.63 (t, *J* = 5.2 Hz, 2H), 3.41 (s, 3H), 3.36 (t, *J* = 5.2 Hz, 2H), 2.10 (s, 3H).

### Preparation Example 54. 3-(4-methylthiazol-5-yl)-6-((2-phenoxyethyl)amino)-2-(pyridin-3-yl)-1H-inden-1-one (Compound 55)

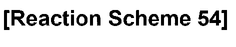

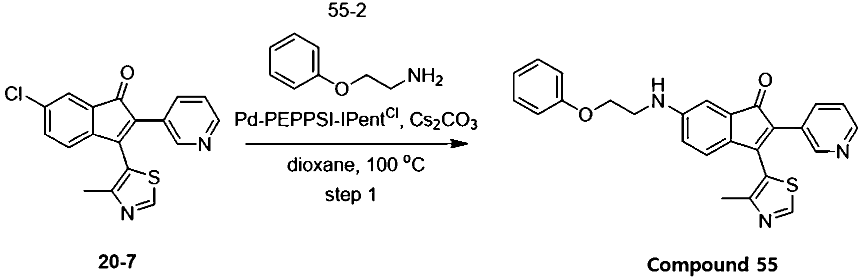

In the same manner as Preparation Example 48, using 2-phenoxyethan-1-amine, the desired title compound (28.0 mg, 25 %) was obtained as a blue solid.

The obtained Compound 55 (Formula 56 below) was confirmed by LCMS(ESI-MS) and ¹H NMR, which yielded the following data:

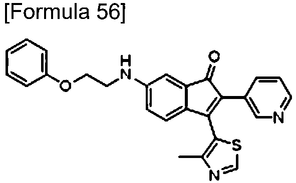

LCMS (ESI-MS): mass calcd. C₂₆H₂₁N₃O₂S 439.1 m/z, found 439.8 [M+H]⁺.

¹H NMR (400 MHz, DMSO-*d*₆) δ 9.29 (s, 1H), 8.44 (dd, J = 4.8, 1.6 Hz, 1H), 8.31 (d, *J = 1.6* Hz, 1H), 7.56 (dt, J = 8.0, 1.9 Hz, 1H), 7.35 - 7.28 (m, 3H), 7.04 - 6.92 (m, 5H), 6.79 (t, *J* = 5.6 Hz, 1H), 6.58 (dd, *J* = 8.0, 2.4 Hz, 1H), 4.13 (t, *J* = 5.2 Hz, 2H), 3.53 (q, *J* = 5.2 Hz, 2H), 1.95 (s, 3H).

### Preparation Example 55. 3-(4-methylthiazol-5-yl)-2-(pyridin-3-yl)-6-((tetrahydro-2H-pyran-4-yl)amino)-1H-inden-1-one (Compound 56)

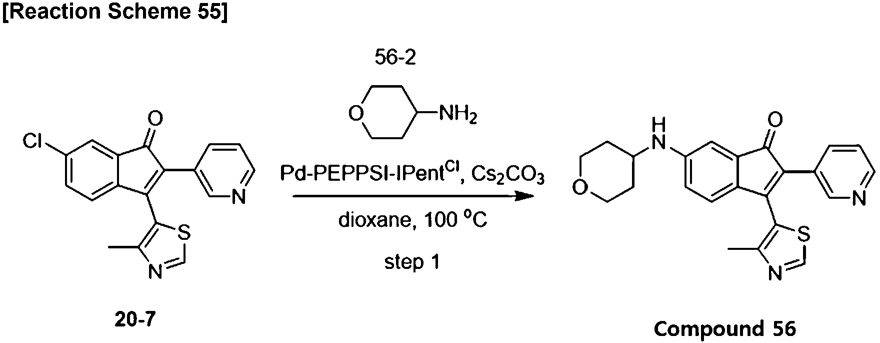

In the same manner as Preparation Example 48, using tetrahydro-2H-pyran-4-amine, the desired title compound (22.6 mg, 19 %) was obtained as a blue solid.

The obtained Compound 56 (Formula 57 below) was confirmed by LCMS(ESI-MS) and ¹H NMR, which yielded the following data:

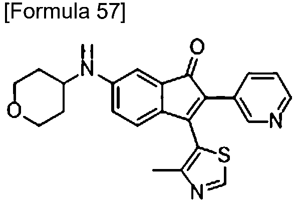

LCMS (ESI-MS): mass calcd. C₂₃H₂₁N₃O₂S 403.1 m/z, found 403.8 [M+H]⁺.

¹H NMR (400 MHz, DMSO-*d*₆) δ 9.28 (s, 1H), 8.44 (d, J = 4.8 Hz, 1H), 8.31 (s, 1H), 7.60 - 7.51 (m, 1H), 7.36 (dd, J = 8.0, 4.8 Hz, 1H), 7.03 - 6.88 (m, 2H), 6.56 - 6.47 (m, 2H), 3.88 - 3.86 (m, 2H), 3.59 (s, 1H), 3.44 (t, *J* = 11.2 Hz, 2H), 2.03 - 1.78 (m, 5H), 1.43 - 1.36 (m, 2H).

### Preparation Example 56. 3-(4-methylthiazol-5-yl)-6-((3-phenylpropyl)thio)-2-(pyridin-3-yl)-1H-inden-1-one (Compound 57)

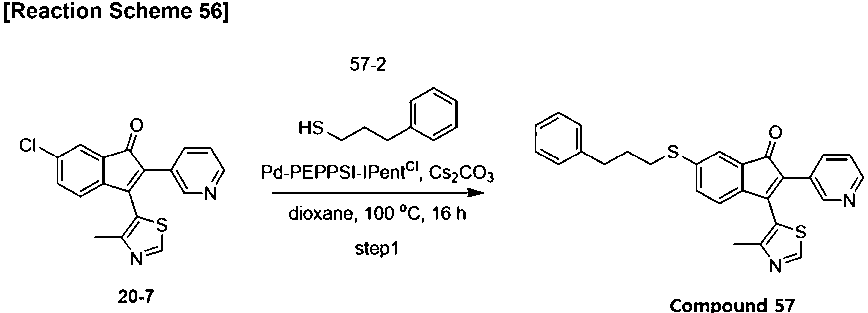

In the same manner as Preparation Example 48, using 3-phenylpropane-1-thiol, the desired title compound (16 mg, 30 %) was obtained as a red solid.

The obtained Compound 57 (Formula 58 below) was confirmed by LCMS(ESI-MS) and 1H NMR, which yielded the following data:

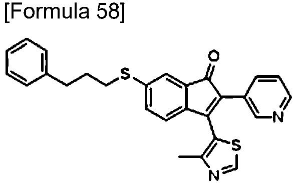

LCMS (ESI-MS): mass calcd. C₂₇H₂₂N₂OS₂ 454.1 m/z, found 455.1 [M+H]⁺.

¹H NMR (400 MHz, DMSO-*d*₆) δ 8.95 (s, 1H), 8.51 - 8.50 (m, 2H), 7.72 - 7.70 (m, 1H), 7.53 - 7.52 (m, 1H), 7.35 - 7.28 (m, 4H), 7.23 - 7.18 (m, 3H), 7.06 - 7.04 (m, 1H), 2.99 (t, *J =* 7.2 Hz, 2H), 2.78 (t, *J* = 7.2 Hz, 2H), 2.10 (s, 3H), 2.05 - 1.97 (m, 2H).

### Preparation Example 57. 3-(4-methylthiazol-5-yl)-6-((1-phenylpiperidin-3-yl)amino)-2-(pyridin-3-yl)-1H-inden-1-one (Compound 58)

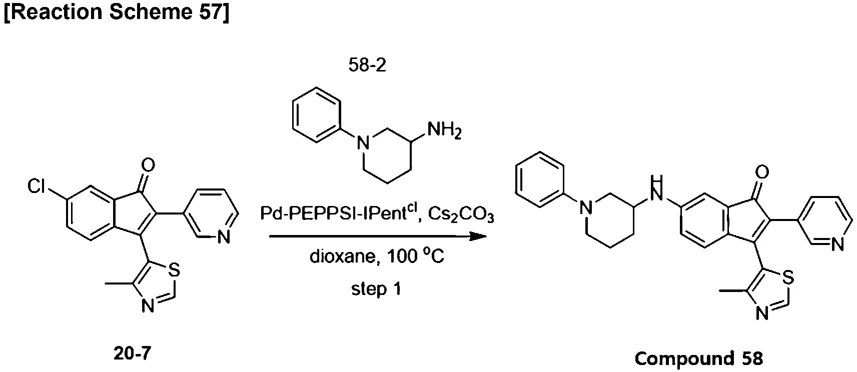

In the same manner as Preparation Example 48, using 1-phenylpiperidin-3-amine, the desired title compound (20.0 mg, 22 %) was obtained as a blue solid.

The obtained Compound 58 (Formula 59 below) was confirmed by LCMS(ESI-MS) and ¹H NMR, which yielded the following data:

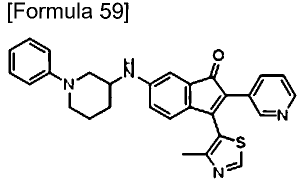

LCMS (ESI-MS): mass calcd. C₂₉H₂₆N₄OS 478.2 m/z, found 479.0 [M+H]⁺.

¹H NMR (400 MHz, DMSO-*d*₆) δ 9.29 (s, 1H), 8.44 - 8.43 (m, 1H), 8.31 - 8.30 (m, 1H), 7.56 (d, *J* = 8.0 Hz, 1H), 7.36 (dd, *J* = 8.0, 4.8 Hz, 1H), 7.22 - 7.20 (m, 2H), 7.00 (s 1H), 6.96 - 6.94 (m, 3H), 6.77 (t, *J* = 7.2 Hz, 1H), 6.60 - 6.52 (m, 2H), 3.68 - 3.65 (m, 1H), 3.60 (s, 1H), 3.51 - 3.48 (m, 1H), 2.80 (t, *J* = 10.0 Hz, 1H), 2.66 - 2.63 (m, 1H), 2.02 - 1.99 (m, 1H), 1.95 (s, 3H), 1.82 - 1.81 (m, 1H), 1.68 - 1.67 (m, 1H), 1.43 - 1.41 (m, 1H).

### Preparation Example 58. 6-amino-3-(4-methylthiazol-5-yl)-2-(pyridin-3-yl)-1H-inden-1-one (Compound 59)

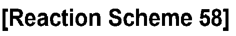

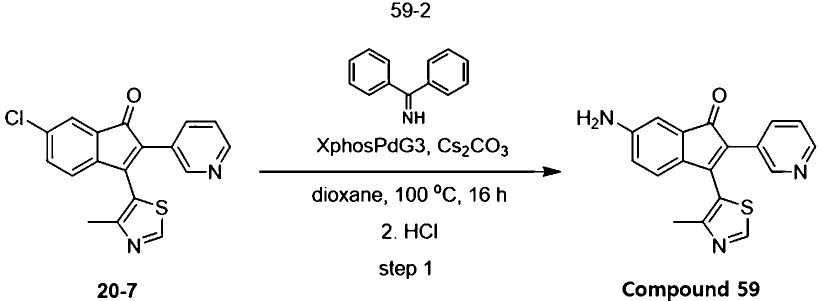

A mixture of 6-chloro-3-(4-methylthiazol-5-yl)-2-(pyridin-3-yl)-1H-inden-1-one (600 mg, 1.78 mmol), diphenylamine (642 mg, 3.55 mmol), Cs₂CO₃ (1153 mg, 3.55 mmol), and XphosPdG3 (152 mg, 0.18 mmol) in dioxane (30 mL) was stirred at 100 °C for 16 hours under a nitrogen atmosphere. HCl/dioxane (3 ml) was added to the reaction mixture, and the mixture was stirred at room temperature for 2 hours.

Water (20 mL) was added to the reaction mixture, and the mixture was extracted with EA (3 x 30 mL). Using a conventional method, the organic layer was washed, dried, and concentrated under reduced pressure. The residue was purified by silica gel column chromatography (5 % MeOH/DCM) to obtain the desired title compound (180 mg, 32 %) as a blue solid.

The obtained Compound 59 (Formula 60 below) was confirmed by LCMS (ESI-MS) and ¹H NMR, which yielded the following data:

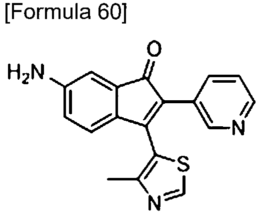

LCMS (ESI-MS): mass calcd. C₁₈H₁₃N₃OS 319.1 m/z, found 319.6 [M+H]⁺.

¹H NMR (400 MHz, DMSO-*d*₆) δ 9.28 (s, 1H), 8.43 - 8.42 (m, 1H), 8.30 - 8.29 (m, 1H), 7.55 - 7.54 (m, 1H), 7.36 - 7.34 (m, 1H), 6.86 - 6.84 (m, 2H), 6.52 - 6.50 (m, 1H), 5.97 (s, 2H), 1.94 (s, 3H).

### Preparation Example 59. N-(3-(4-methylthiazol-5-yl)-1-oxo-2-(pyridin-3-yl)-1H-inden-6-yl)-3-phenylpropanamide (Compound 60)

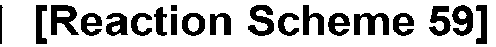

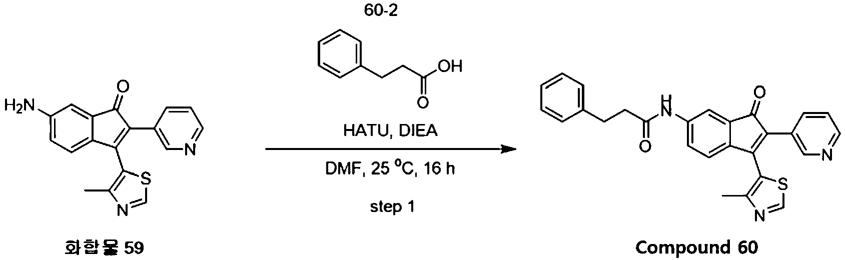

To a solution of 3-phenylpropanoic acid (86 mg, 0.57 mmol), 2-(7-azabenzotriazol-1-yl)-N,N,N',N'-tetramethyluronium hexafluorophosphate (217 mg, 0.57 mmol), and DIPEA (98 mg, 0.76 mmol) in DMF (6 mL) was added 6-amino-3-(4-methylthiazol-5-yl)-2-(pyridin-3-yl)-1H-inden-1-one (120 mg, 0.38 mmol), and the mixture was stirred at room temperature for 16 hours. Water (6 mL) was added to the reaction mixture, and the mixture was extracted with EA (3 x 15 mL). Using a conventional method, the organic layer was washed, dried, and concentrated under reduced pressure. The residue was purified by prep-HPLC (Gemini 5 µm C₁₈ column, 150 x 21.2 mm, 30 - 90 % ACN/H₂O with 0.1 % FA) to obtain the desired title compound (23.1 mg, 13 %) as a yellow solid.

The obtained Compound 60 (Formula 61 below) was confirmed by LCMS(ESI-MS) and ¹H NMR, which yielded the following data:

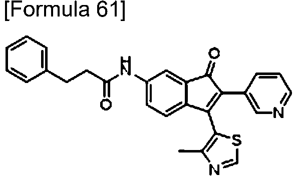

LCMS (ESI-MS): mass calcd. C₂₇H₂₁N₃O₂S 451.1 m/z, found 452.2 [M+H]⁺.

¹H NMR (400 MHz, DMSO-*d*₆) δ 10.28 (s, 1H), 9.32 (s, 1H), 8.49 - 8.48 (m, 1H), 8.36 (s, 1H), 7.92 (s, 1H), 7.70 - 7.65 (m, 2H), 7.42 - 7.39 (m, 1H), 9.31 - 7.17 (m, 6H), 2.93 (t, *J* = 7.2 Hz, 2H), 2.67 (t, *J* = 7.2 Hz, 2H), 1.97 (s, 3H).

### Preparation Example 60. N-(3-(4-methylthiazol-5-yl)-1-oxo-2-(pyridin-3-yl)-1H-inden-6-yl)tetrahydro-2H-pyran-4-carboxamide (Compound 61)

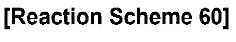

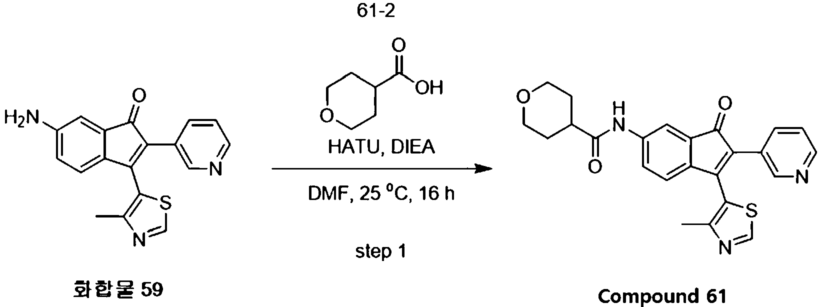

In the same manner as Preparation Example 59, using tetrahydro-2H-pyran-4-carboxylic acid, the desired title compound (18.7 mg, 29 %) was obtained as a white solid.

The obtained Compound 61 (Formula 62 below) was confirmed by LCMS(ESI-MS) and ¹H NMR, which yielded the following data:

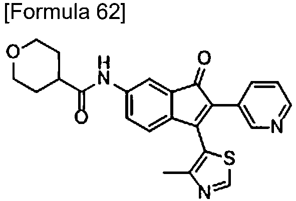

LCMS (ESI-MS): mass calcd. C₂₄H₂₁N₃O₃S 431.1 m/z, found 431.8 [M+H]⁺.

¹H NMR (400 MHz, DMSO-*d*₆) δ 10.26 (s, 1H), 9.32 (s, 1H), 8.50 - 8.49 (m, 1H), 8.36 (s, 1H), 7.92 (s, 1H), 7.77 - 7.56 (m, 2H), 7.41 - 7.39 (m, 1H), 7.19 (d, *J* = 8.0 Hz, 1H), 3.98 - 3.85 (m, 2H), 3.38 - 3.36 (m, 2H), 2.66 - 2.56 (m, 1H), 1.99 (s, 3H), 1.77 - 1.61 (m, 4H).

### Preparation Example 61. 6-(dimethylamino)-3-(4-methylthiazol-5-yl)-2-(pyridin-3-yl)-1H-inden-1-one (Compound 62)

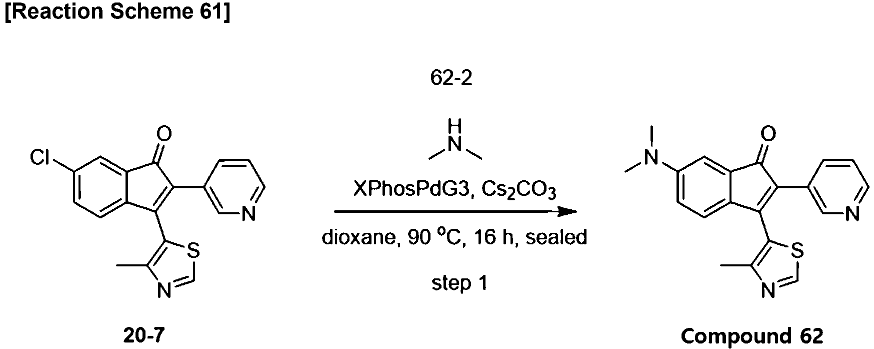

In the same manner as Preparation Example 58, using dimethylamine, the desired title compound (60 mg, 65 %) was obtained as a purple solid.

The obtained Compound 62 (Formula 63 below) was confirmed by LCMS(ESI-MS) and ¹H NMR, which yielded the following data:

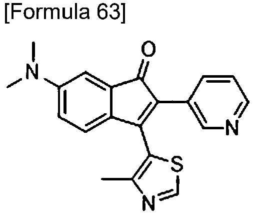

LCMS (ESI-MS): mass calcd. C₂₀H₁₇N₃OS 347.1 m/z, found 347.7 [M+H]⁺.

¹H NMR (400 MHz, DMSO-*d*₆) δ 9.29 (s, 1H), 8.45 (dd, *J* = 4.8, 1.6 Hz, 1H), 8.33 (d, *J* = 1.8 Hz, 1H), 7.59 - 7.56 (m, 1H), 7.39 - 7.35 (m, 1H), 7.04 - 7.00 (m, 2H), 6.63 - 6.61 (m, 1H), 3.03 (s, 6H), 1.96 (s, 3H).

### Preparation Example 62. 6-(cyclopropylamino)-3-(4-methylthiazol-5-yl)-2-(pyridin-3-yl)-1H-inden-1-one (Compound 63)

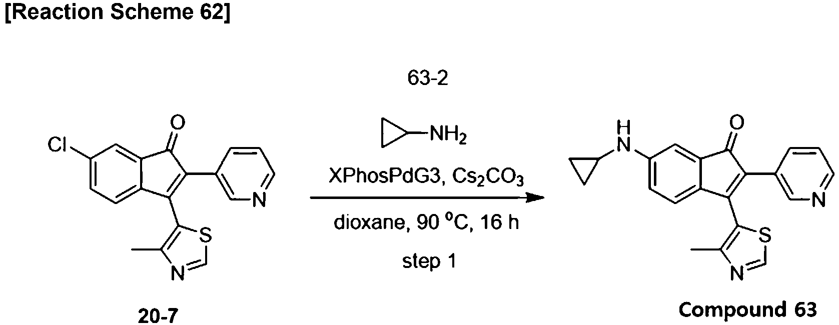

In the same manner as Preparation Example 58, using cyclopropanamine, the desired title compound (80 mg, 67 %) was obtained as a purple solid.

The obtained Compound 63 (Formula 64 below) was confirmed by LCMS(ESI-MS) and ¹H NMR, which yielded the following data:

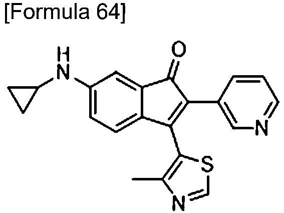

LCMS (ESI-MS): mass calcd. C₂₁H₁₇N₃OS 359.1 m/z, found 359.7 [M+H]⁺.

¹H NMR (400 MHz, DMSO-*d*₆) δ 9.28 (s, 1H), 8.44 (dd, *J* = 4.8, 1.6 Hz, 1H), 8.31 (d, *J* = 1.6 Hz, 1H), 7.61 - 7.52 (m, 1H), 7.36 (dd, *J =* 7.6, 5.2 Hz, 1H), 7.02 - 6.90 (m, 3H), 6.67 (dd, J = 8.2, 2.2 Hz, 1H), 2.47 - 2.41 (m, 1H), 0.80 - 0.69 (m, 2H), 0.44 - 0.41 (m, 2H).

### Preparation Example 63. 6-(4-methylpiperazin-1-yl)-3-(4-methylthiazol-5-yl)-2-(pyridin-3-yl)-1H-inden-1-one (Compound 64)

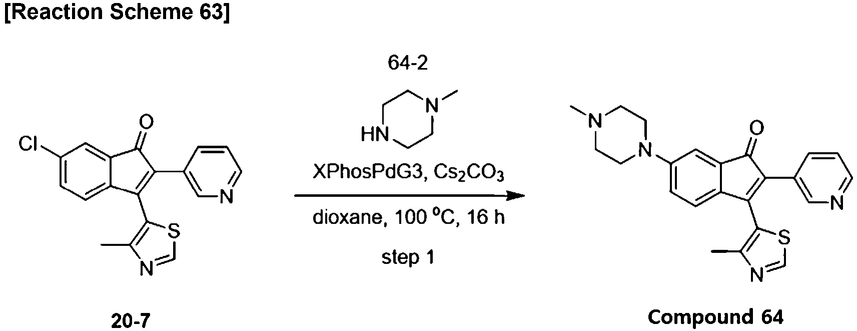

In the same manner as Preparation Example 58, using 1-methylpiperazine, the desired title compound (15.1 mg, 16 %) was obtained as a yellow solid.

The obtained Compound 64 (Formula 65 below) was confirmed by LCMS(ESI-MS) and ¹H NMR, which yielded the following data:

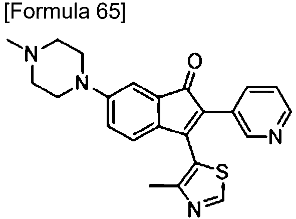

LCMS (ESI-MS): mass calcd. C₂₃H₂₂N₄OS 402.2 m/z, found 403.1 [M+H]⁺.

¹H NMR (400 MHz, DMSO-*d*₆) δ 9.30 (s, 1H), 8.46 (dd, *J* = 4.8, 1.6 Hz, 1H), 8.34 (d, *J* = 2.0 Hz, 1H), 7.59 (dt, *J* = 8.0, 2.0 Hz, 1H), 7.38 (dd, *J* = 8.0, 4.8 Hz, 1H), 7.27 (d, *J =* 2.4 Hz, 1H), 7.03 (d, *J* = 8.4 Hz, 1H), 6.90 (dd, *J* = 8.4, 2.4 Hz, 1H), 3.31 - 3.24 (m, 4H), 2.47 - 2.41 (m, 4H), 2.22 (s, 3H), 1.95 (s, 3H).

### Preparation Example 64. 6-(methyl(3-phenylpropyl)amino)-3-(4-methylthiazol-5-yl)-2-(pyridin-3-yl)-1H-inden-1-one (Compound 65)

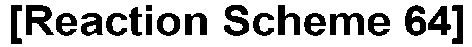

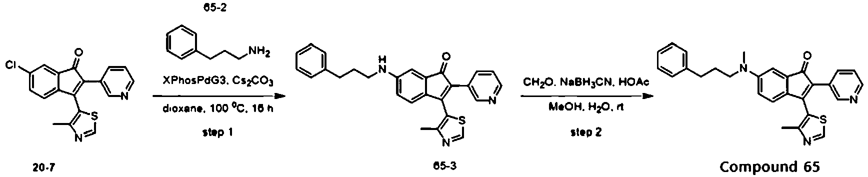

### Step 1: Synthesis of 3-(4-methylthiazol-5-yl)-6-((3-phenylpropyl)amino)-2-(pyridin-3-yl)-1H-inden-1-one

In the same manner as Preparation Example 58, using 3-phenylpropan-1-amine, the desired title compound (220 mg, 34 %) was obtained as a yellow solid.

LCMS (ESI-MS): mass calcd. C₂₇H₂₃N₃OS 437.2 m/z, found 437.8 [M+H]⁺.

### Step 2: Synthesis of 6-(methyl(3-phenylpropyl)amino)-3-(4-methylthiazol-5-yl)-2-(pyridin-3-yl)-1H-inden-1-one (Compound 65)

In the same manner as Step 5 of Preparation Example 29, the desired title compound (12 mg, 12 %) was obtained as a yellow solid.

The obtained Compound 65 (Formula 66 below) was confirmed by LCMS(ESI-MS) and ¹H NMR, which yielded the following data:

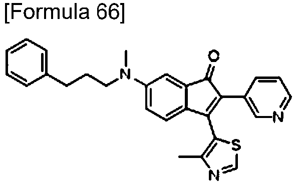

LCMS (ESI-MS): mass calcd. C₂₈H₂₅N₃OS 451.2 m/z, found 452.0 [M+H]⁺.

¹H NMR (400 MHz, DMSO-*d*₆) δ 9.29 (s, 1H), 8.44 (dd, *J* = 4.8, 1.2 Hz, 1H), 8.32 (d, *J* = 2.0 Hz, 1H), 7.62 - 7.51 (m, 1H), 7.39 - 7.36 (m, 1H), 7.31 - 7.17 (m, 5H), 7.04 - 6.91 (m, 2H), 6.57 (dd, *J* = 8.4, 2.4 Hz, 1H), 3.51 - 3.42 (m, 2H), 3.01 (s, 3H), 2.67 - 2.59 (m, 2H), 1.95 (s, 3H), 1.90 - 1.77 (m, 2H).

### Preparation Example 65. 3-(4-methylthiazol-5-yl)-6-phenoxy-2-(pyridin-3-yl)-1H-inden-1-one (Compound 66)

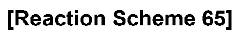

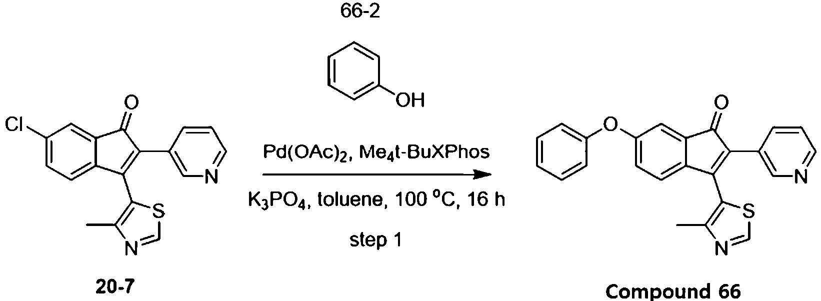

A mixture of 6-chloro-3-(4-methylthiazol-5-yl)-2-(pyridin-3-yl)-1H-inden-1-one (40 mg, 0.1181 mmol), phenol (16.67 mg, 0.17715 mmol), Pd(OAc)₂ (2.65 mg, 0.01181 mmol), Me₄t-BuXPhos (11.36 mg, 0.02362 mmol), and K₃PO₄ (75.21 mg, 0.3543 mmol) in toluene (5 mL) was stirred at 100 °C for 16 hours under a nitrogen atmosphere. The reaction mixture was cooled to room temperature, and water (20 mL) was added to the reaction mixture. The mixture was extracted with EA (3 x 15 mL). Using a conventional method, the organic layer was washed, dried, and concentrated under reduced pressure. The resulting residue was purified by silica gel column chromatography (40 % EA/Hexane) to obtain the desired title compound (5.62 mg, 12 % yield) as a red solid.

The obtained Compound 66 (Formula 67 below) was confirmed by LCMS(ESI-MS) and ¹H NMR, which yielded the following data:

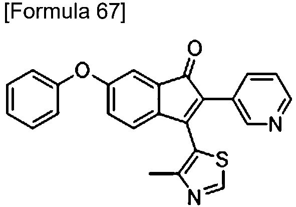

LCMS (ESI-MS): mass calcd. C₂₄H₁₆N₂O₂S 396.1 m/z, found 397.1 [M+H]⁺.

¹H NMR (400 MHz, DMSO-*d*₆) δ 8.96 (s, 1H), 8.54 - 8.52 (m, 2H), 7.74 - 7.72 (m, 1H), 7.42 - 7.36 (m, 4H), 7.22 - 7.18 (m, 1H), 7.13 - 7.01 (m, 4H), 2.12 (s, 3H).

### Preparation Example 66. 6-(methylamino)-2,3-diphenyl-1H-inden-1-one (Compound 67)

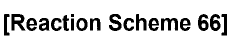

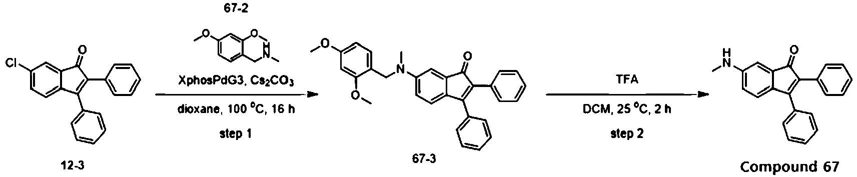

### Step 1: Synthesis of 6-((2,4-dimethoxybenzyl)(methyl)amino)-2,3-diphenyl-1H-inden-1-one

In the same manner as Preparation Example 58, using 1-(2,4-dimethoxyphenyl)-N-methylmethanamine, the desired title compound (70 mg, 38 %) was obtained as a yellow solid.

LCMS (ESI-MS): mass calcd. C₃₁H₂₇NO₃ 461.2 m/z, found 461.8 [M+H]⁺.

### Step 2: Synthesis of 6-(methylamino)-2,3-diphenyl-1H-inden-1-one (Compound 67)

To 6-((2,4-dimethoxybenzyl)(methyl)amino)-2,3-diphenyl-1H-inden-1-one (70 mg, 0.15 mmol) was added DCM/TFA (1 mL/0.3 mL), and the mixture was stirred at room temperature. Water (3 mL) was added to the reaction mixture, and the mixture was extracted with DCM (3 x 80 mL). Using a conventional method, the organic layer was washed, dried, and concentrated under reduced pressure. The residue was purified by prep-HPLC (Gemini 5 µm C₁₈ column, 150 x 21.2 mm, 30 - 90 % ACN/H₂O with 0.1 % NH₃H₂O) to obtain the desired title compound (20.2 mg, 43 %) as a blue solid.

The obtained Compound 67 (Formula 68 below) was confirmed by LCMS(ESI-MS) and 1H NMR, which yielded the following data:

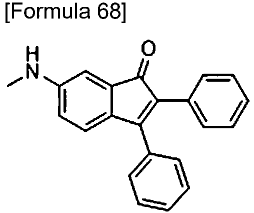

LCMS (ESI-MS): mass calcd. C₂₂H₁₇NO 311.1 m/z, found 311.8 [M+H]⁺.

¹H NMR (400 MHz, DMSO-*d*₆) δ 7.47 - 7.40 (m, 3H), 7.37 - 7.31 (m, 2H), 7.28 - 7.16 (m, 3H), 7.12 (d, *J* = 6.8 Hz, 2H), 6.89 - 6.80 (m, 2H), 6.48 - 6.33 (m, 2H), 2.75 (d, *J =* 5.2 Hz, 3H).

### Preparation Example 67. 6-amino-2,3-diphenyl-1H-inden-1-one (Compound 68)

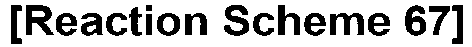

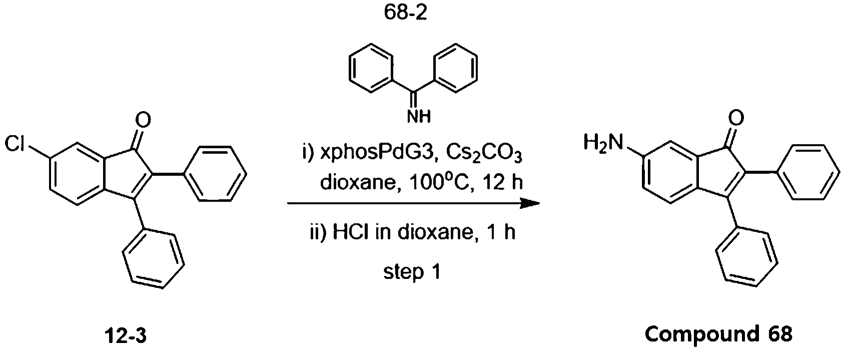

In the same manner as Preparation Example 58, using diphenylmethanimine, the desired title compound (200 mg, 27 %) was obtained as a white solid.

The obtained Compound 68 (Formula 69 below) was confirmed by LCMS(ESI-MS) and ¹H NMR, which yielded the following data:

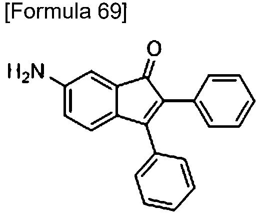

LCMS (ESI-MS): mass calcd. C₂₁H₁₅NO 297.1 m/z, found 298.1 [M+H]⁺.

¹H NMR (400 MHz, DMSO-*d*₆) δ 7.46 - 7.41 (m, 3H), 7.35 - 7.33 (m, 2H), 7.30 - 7.17 (m, 3H), 7.11 (d, *J =* 6.4 Hz, 2H), 6.83 (d, *J =* 2.0 Hz, 1H), 6.78 (d, *J =* 8.0 Hz, 1H), 6.47 (dd, *J =* 7.6, 2.0 Hz, 1H), 5.82 (s, 2H).

### Preparation Example 68. 6-(dimethylamino)-2,3-diphenyl-1H-inden-1-one (Compound 69)

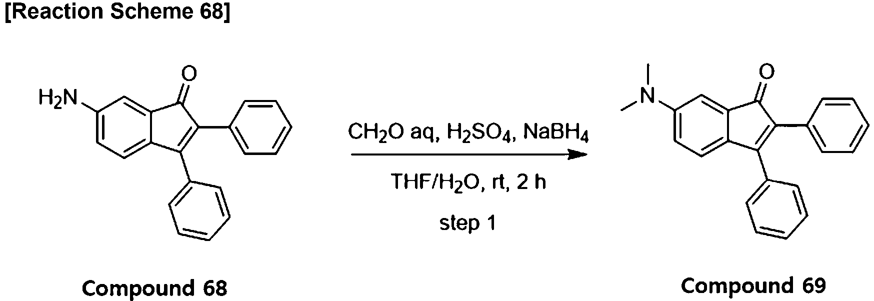

In the same manner as Step 5 of Preparation Example 29, using NaBH₄ and an aqueous H₂SO₄ solution, the desired title compound (20 mg, 20 %) was obtained as a white solid.

The obtained Compound 69 (Formula 70 below) was confirmed by LCMS(ESI-MS) and ¹H NMR, which yielded the following data:

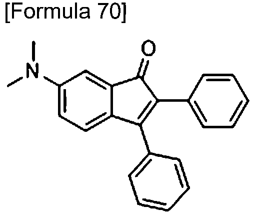

LCMS (ESI-MS): mass calcd. C₂₃H₁₉NO 325.1 m/z, found 325.7 [M+H]+.

¹H NMR (400 MHz, DMSO-*d*₆) δ 7.48 - 7.41 (m, 3H), 7.37 - 7.35 (m, 2H), 7.30 - 7.19 (m, 3H), 7.16 - 7.10 (m, 2H), 7.00 (d, *J* = 2.4 Hz, 1H), 6.93 (d, *J* = 8.0 Hz, 1H), 6.58 (dd, J *=* 8.4, 2.4 Hz, 1H), 3.01 (s, 6H).

### Preparation Example 69. 6-(4-(2-hydroxyethyl)piperazin-1-yl)-2,3-diphenyl-1H-inden-1-one (Compound 70)

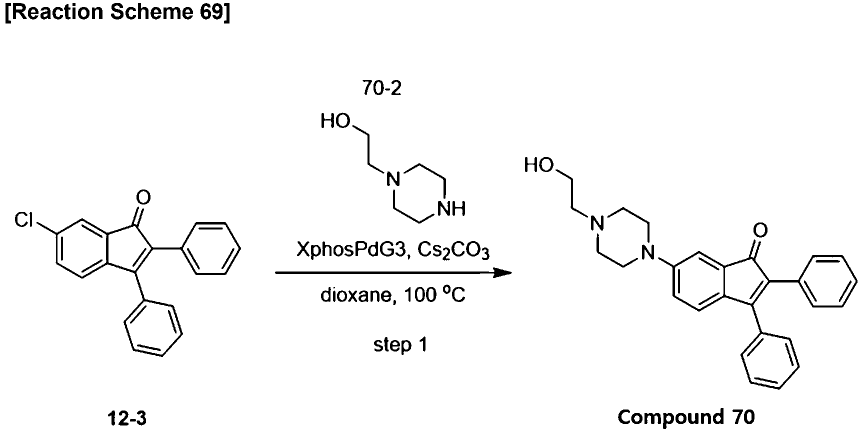

In the same manner as Preparation Example 58, using 2-(piperazin-1-yl)ethan-1-ol, the desired title compound (8.9 mg, 4 %) was obtained as a yellow solid.

The obtained Compound 70 (Formula 71 below) was confirmed by LCMS(ESI-MS) and ¹H NMR, which yielded the following data:

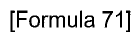

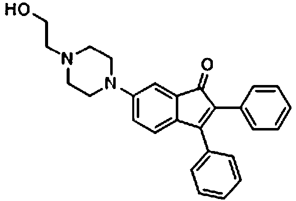

LCMS (ESI-MS): mass calcd. C₂₇H₂₆N₂O₂ 410.2 m/z, found 411.0 [M+H]⁺.

¹H NMR (400 MHz, DMSO-*d*₆) δ 7.50 - 7.39 (m, 3H), 7.39 - 7.34 (m, 2H), 7.31 - 7.19 (m, 4H), 7.17 - 7.11 (m, 2H), 6.96 (d, *J* = 8.0 Hz, 1H), 6.85 (dd, J = 8.0, 2.0 Hz, 1H), 4.44 (t, *J* = 5.6 Hz, 1H), 3.54 (q, *J* = 6.0 Hz, 2H), 3.27 - 3.20 (m, 4H), 2.58 - 2.52 (m, 4H), 2.44 (t, *J* = 6.0 Hz, 2H).

### Preparation Example 70. 4-methyl-6-(4-methylpiperazin-1-yl)-2,3-diphenyl-1H-inden-1-one (Compound 71)

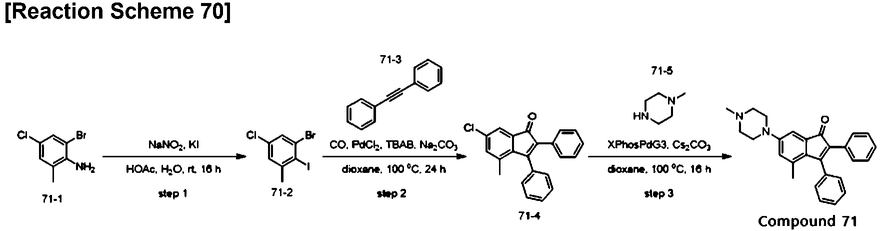

### Step 1: Synthesis of 1-bromo-5-chloro-2-iodo-3-methylbenzene

To a solution of 2-bromo-4-chloro-6-methylaniline (3 g, 0.0136 mol) in HCl (18 mL) and H₂O (24 mL) was added an aqueous solution of NaNO₂ (1.03 g in 5 mL H₂O) at -5 °C, and the mixture was stirred at the same temperature for 30 minutes. KI (2.71 g, 0.0163 mol) was added to the reaction mixture, and the mixture was warmed to room temperature and stirred for 16 hours. Water (50 mL) was added to the reaction mixture, and the mixture was extracted with ethyl acetate (50 mL x 3). Using a conventional method, the organic layer was washed, dried, and concentrated under reduced pressure. The residue was purified by silica gel column chromatography (5 % EtOAc/hexane) to obtain the desired title compound (1.6 g, 31 %) as a yellow solid.

### Step 2: Synthesis of 6-chloro-4-methyl-2,3-diphenyl-1H-inden-1-one

A mixture of 1-bromo-5-chloro-2-iodo-3-methylbenzene (300 mg, 0.9258 mmol), 1,2-diphenylethyne (110 mg, 0.6172 mmol), PdCl₂ (10.94 mg, 0.06172 mmol), tetrabutylammonium bromide (198.97 mg, 0.6172 mmol), and Na₂CO₃ (196.25 mg, 1.8516 mmol) in dioxane (10 mL) was stirred at 100 °C for 24 hours under a CO atmosphere. The reaction mixture was filtered and washed with EtOAc (10 mL), and the combined filtrate was concentrated. The residue was purified by silica gel column chromatography (5 % EtOAc/hexane) to obtain the desired title compound (130 mg, 39 %) as a yellow solid.

LCMS (ESI-MS): mass calcd. C₂₂H₁₅ClO 330.1 m/z, found 330.6 [M+H]⁺.

### Step 3: Synthesis of 4-methyl-6-(4-methylpiperazin-1-yl)-2,3-diphenyl-1H-inden-1-one (Compound 71)

In the same manner as Preparation Example 58, the desired title compound (50 mg, 37 %) was obtained as a white solid.

The obtained Compound 71 (Formula 72 below) was confirmed by LCMS(ESI-MS) and ¹H NMR, which yielded the following data:

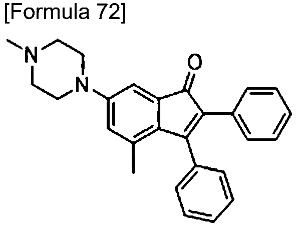

LCMS (ESI-MS): mass calcd. C₂₇H₂₆N₂O 394.2 m/z, found 395.1 [M+H]⁺.

¹H NMR (400 MHz, DMSO-*d*₆) δ 7.48 - 7.32 (m, 5H), 7.24 - 7.03 (m, 6H), 6.56 (d, *J* = 2.2 Hz, 1H), 3.27 - 3.19 (m, 4H), 2.45 - 2.39 (m, 4H), 2.21 (s, 3H), 1.66 (s, 3H).

### Preparation Example 71. 3-(2,6-dimethylphenyl)-6-(4-methylpiperazin-1-yl)-2-phenyl-1H-inden-1-one (Compound 72)

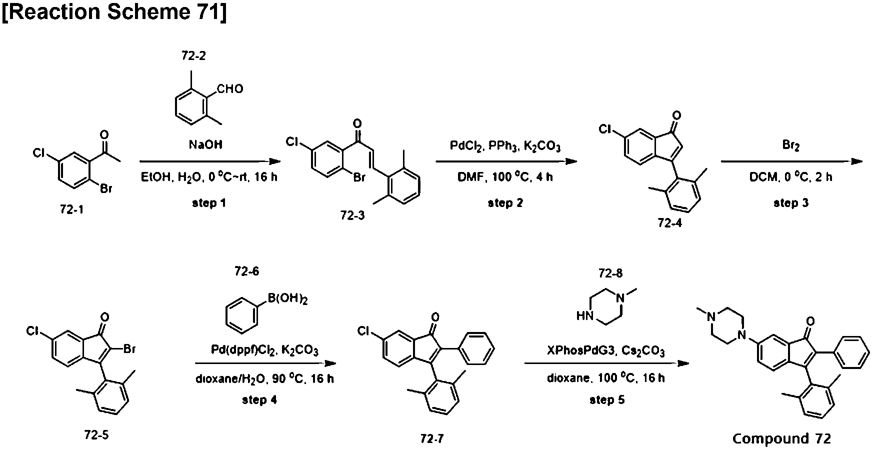

### Step 1: Synthesis of (E)-1-(2-bromo-5-chlorophenyl)-3-(2,6-dimethylphenyl)prop-2-en-1-one

In the same manner as Step 1 of Preparation Example 21, the desired title compound (2.8 g, 83 %) was obtained as a yellow oil.

LCMS (ESI-MS): mass calcd. C₁₇H₁₄BrClO 350.0 m/z, found 350.6 [M+H]⁺.

### Step 2: Synthesis of 6-chloro-3-(2,6-dimethylphenyl)-1H-inden-1-one

A mixture of (E)-1-(2-bromo-5-chlorophenyl)-3-(2,6-dimethylphenyl)prop-2-en-1-one (2.5 g, 7.1 mmol), PdCl₂ (0.08 g, 0.355 mmol), PPh₃ (0.19 g, 0.72 mmol), and K₂CO₃ (2.96 g, 21.4 mmol) in DMF (20 mL) was stirred at 100 °C for 4 hours under a nitrogen atmosphere. Water (80 mL) was added to the reaction mixture, and the mixture was extracted with ethyl acetate (3 x 100 mL). Using a conventional method, the organic layer was washed, dried, and concentrated under reduced pressure. The residue was purified by silica gel column chromatography (10 % EtOAc/hexane) to obtain the desired title compound (1 g, 26 %) as a yellow solid.

LCMS (ESI-MS): mass calcd. C₁₇H₁₃ClO 268.1 m/z, found 268.7 [M+H]⁺.

### Step 3: Synthesis of 2-bromo-6-chloro-3-(2,6-dimethylphenyl)-1H-inden-1-one

To a solution of 6-chloro-3-(2,6-dimethylphenyl)-1H-inden-1-one (1 g, 0.0037 mol) in DCM (30 mL) was added Br₂ (0.89 g, 0.0055 mol) at 0 °C, and the mixture was stirred at the same temperature for 2 hours. Water (80 mL) was added to the reaction mixture, and the mixture was extracted with ethyl acetate (3 x 100 mL). Using a conventional method, the organic layer was washed, dried, and concentrated under reduced pressure. The residue was purified by silica gel column chromatography (5 % EtOAc/hexane) to obtain the desired title compound (0.8 g, 56 %) as a yellow oil.

LCMS (ESI-MS): mass calcd. C₁₇H₁₂BrClO 348.0 m/z, found 348.6 [M+H]⁺.

### Step 4: Synthesis of 6-chloro-3-(2,6-dimethylphenyl)-2-phenyl-1H-inden-1-one

In the same manner as Step 5 of Preparation Example 21, the desired title compound (80 mg, 40 %) was obtained as a white solid.

LCMS (ESI-MS): mass calcd. C₂₃H₁₇ClO 344.1 m/z, found 344.7 [M+H]⁺.

¹H NMR (400 MHz, DMSO-*d*₆) δ 7.57- 7.44 (m, 1H), 7.42 - 7.39 (m, 2H), 7.26 - 7.20 (m, 4H), 7.16 - 7.10 (m, 4H), 2.11 (s, 6H).

### Step 5: Synthesis of 3-(2,6-dimethylphenyl)-6-(4-methylpiperazin-1-yl)-2-phenyl-1H-inden-1-one (Compound 72)

In the same manner as Preparation Example 58, the desired title compound (20.2 mg, 27 %) was obtained as a purple solid.

The obtained Compound 72 (Formula 73 below) was confirmed by LCMS(ESI-MS) and ¹H NMR, which yielded the following data:

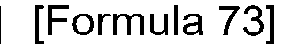

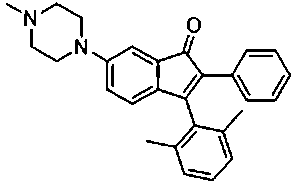

LCMS (ESI-MS): mass calcd. C₂₈H₂₈N₂O 408.2 m/z, found 409.1 [M+H]⁺.

¹H NMR (400 MHz, DMSO-*d*₆) δ 7.21 - 7.11 (m, 9H), 6.82 - 6.80 (m, 1H), 6.61 - 6.59 (m, 1H), 3.32 - 3.24 (m, 4H), 2.48 - 2.47 (m, 4H), 2.33 (s, 3H), 2.24 (s, 3H), 1.96 (s, 3H).

### Preparation Example 72. 6-(4-methylpiperazin-1-yl)-2-(3-morpholinophenyl)-3-phenyl-1H-inden-1-one (Compound 73)

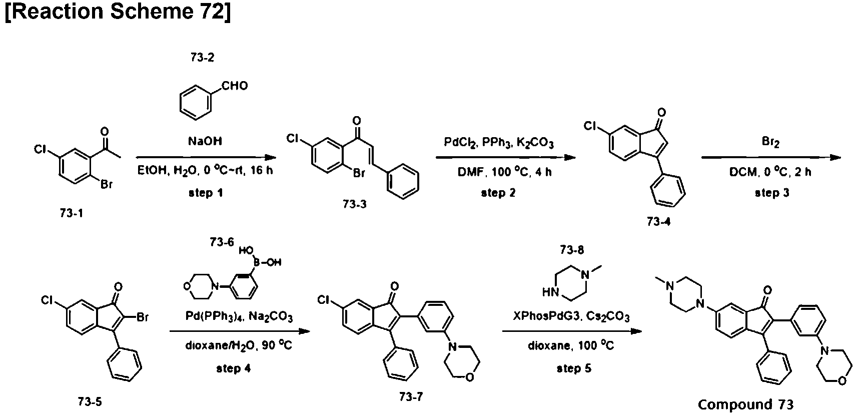

### Step 1: Synthesis of (E)-1-(2-bromo-5-chlorophenyl)-3-phenylprop-2-en-1-one

In the same manner as Step 1 of Preparation Example 21, the desired title compound (4 g, 65 %) was obtained as a yellow solid.

LCMS (ESI-MS): mass calcd. C₁₅H₁₀BrClO 322.0 m/z, found 322.6 [M+H]⁺.

### Step 2: Synthesis of 6-chloro-3-phenyl-1H-inden-1-one

In the same manner as Step 2 of Preparation Example 22, the desired title compound (2.6 g, 74 %) was obtained as a yellow solid.

LCMS (ESI-MS): mass calcd. C₁₅H₉ClO 240.0 m/z, found 240.6 [M+H]⁺.

### Step 3: Synthesis of 2-bromo-6-chloro-3-phenyl-1H-inden-1-one

In the same manner as Step 3 of Preparation Example 22, the desired title compound (0.8 g, 38 %) was obtained as a yellow oil.

LCMS (ESI-MS): mass calcd. C₁₅H₈BrClO 319.9 m/z, found 320.5 [M+H]⁺.

### Step 4: Synthesis of 6-chloro-2-(3-morpholinophenyl)-3-phenyl-1H-inden-1-one

In the same manner as Step 5 of Preparation Example 21, the desired title compound (220 mg, 52 %) was obtained as a red solid.

LCMS (ESI-MS): mass calcd. C₂₅H₂₀ClNO₂ 401.1 m/z, found 402.0 [M+H]⁺.

### Step 5: Synthesis of 6-(4-methylpiperazin-1-yl)-2-(3-morpholinophenyl)-3-phenyl-1H-inden-1-one (Compound 73)

In the same manner as Preparation Example 58, the desired title compound (70 mg, 45 %) was obtained as a red solid.

The obtained Compound 73 (Formula 74 below) was confirmed by LCMS(ESI-MS) and ¹H NMR, which yielded the following data:

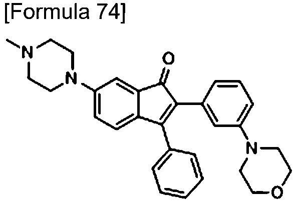

LCMS (ESI-MS): mass calcd. C₃₀H₃₁N₃O₂ 465.2 m/z, found 466.2 [M+H]⁺.

¹H NMR (400 MHz, DMSO-*d*₆) δ 7.47 - 7.45 (m, 3H), 7.38 - 7.36 (m, 2H), 7.20 (d, *J* = 2.2 Hz, 1H), 7.12 (t, *J* = 7.8 Hz, 1H), 6.93 (d, *J* = 8.2 Hz, 1H), 6.85 - 6.81 (m, 2H), 6.68 - 6.62 (m, 2H), 3.69 - 3.61 (m, 4H), 3.27 - 3.21 (m, 4H), 2.92 - 2.84 (m, 4H), 2.49 - 2.44 (m, 4H), 2.22 (s, 3H).

### Preparation Example 73. 6-(4-(2-hydroxyethyl)piperazin-1-yl)-2-(4-(4-methylpiperazin-1-yl)phenyl)-3-(4-methylthiazol-5-yl)-1H-inden-1-one (Compound 74)

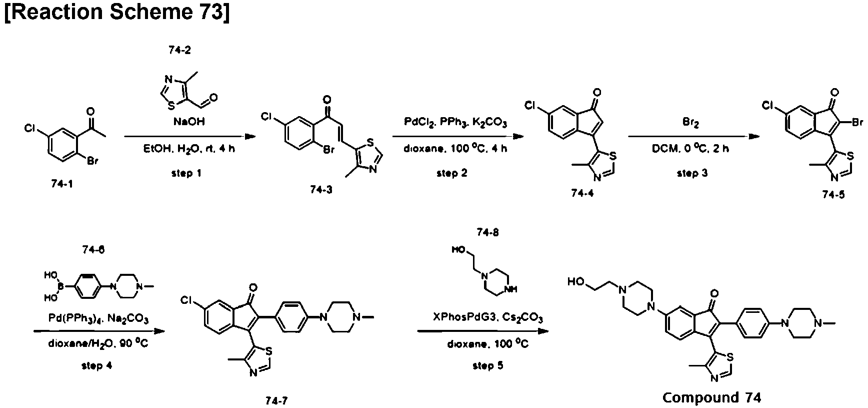

### Step 1: Synthesis of (E)-1-(2-bromo-5-chlorophenyl)-3-(4-methylthiazol-5-yl)prop-2-en-1-one

In the same manner as Step 1 of Preparation Example 21, the desired title compound (6 g, 73 %) was obtained as a yellow solid.

LCMS (ESI-MS): mass calcd. C₁₃H₉BrClNOS 342.9 m/z, found 343.6 [M+H]⁺.

### Step 2: Synthesis of 6-chloro-3-(4-methylthiazol-5-yl)-1H-inden-1-one

In the same manner as Step 2 of Preparation Example 22, the desired title compound (2.7 g, 63 %) was obtained as a yellow solid.

LCMS (ESI-MS): mass calcd. C₁₃H₈ClNOS 261.0 m/z, found 261.6 [M+H]⁺.

### Step 3: Synthesis of 2-bromo-6-chloro-3-(4-methylthiazol-5-yl)-1H-inden-1-one

In the same manner as Step 3 of Preparation Example 22, the desired title compound (2.7 g, 58 %) was obtained as a yellow solid.

LCMS (ESI-MS): mass calcd. C₁₃H₇BrClNOS 340.9 m/z, found 341.5 [M+H]⁺.

### Step 4: Synthesis of 6-chloro-2-(4-(4-methylpiperazin-1-yl)phenyl)-3-(4-methylthiazol-5-yl)-1H-inden-1-one

In the same manner as Step 5 of Preparation Example 21, the desired title compound (200 mg, 81 %) was obtained as a red solid.

LCMS (ESI-MS): mass calcd. C₂₄H₂₂ClN₃OS 435.1 m/z, found 435.8 [M+H]⁺.

### Step 5: Synthesis of 6-(4-(2-hydroxyethyl)piperazin-1-yl)-2-(4-(4-methylpiperazin-1-yl)phenyl)-3-(4-methylthiazol-5-yl)-1H-inden-1-one (Compound 74)

In the same manner as Preparation Example 58, the desired title compound (60 mg, 55 %) was obtained as a purple solid.

The obtained Compound 74 (Formula 75 below) was confirmed by LCMS(ESI-MS) and ¹H NMR, which yielded the following data:

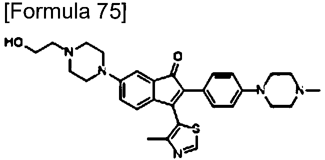

LCMS (ESI-MS): mass calcd. C₃₀H₃₅N₅O₂S 529.3 m/z, found 530.1 [M+H]⁺.

¹H NMR (400 MHz, DMSO-*d*₆) δ 9.24 (s, 1H), 7.18 (d, *J* = 2.2 Hz, 1H), 7.07 (d, *J =* 8.8 Hz, 2H), 6.93 - 6.78 (m, 4H), 4.43 (s, 1H), 3.53 (d, *J* = 5.6 Hz, 2H), 3.27 - 3.11 (m, 8H), 2.59 - 2.53 (m, 4H), 2.45 - 2.39 (m, 6H), 2.20 (s, 3H), 1.99 (s, 3H).

### Preparation Example 74. 6-(4-methylpiperazin-1-yl)-2-(4-(4-methylpiperazin-1-yl)phenyl)-3-(4-methylthiazol-5-yl)-1H-inden-1-one (Compound 75)

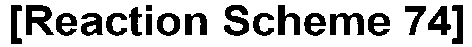

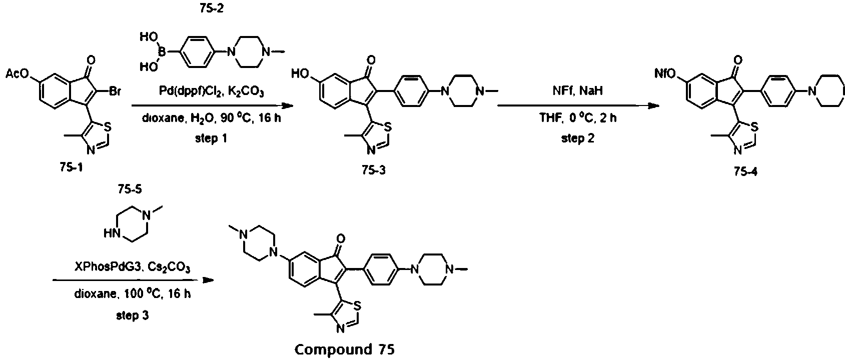

### Step 1: Synthesis of 6-hydroxy-2-(4-(4-methylpiperazin-1-yl)phenyl)-3-(4-methylthiazol-5-yl)-1H-inden-1-one

In the same manner as Step 5 of Preparation Example 21, the desired title compound (324 mg, 45 %) was obtained as a purple solid.

LCMS (ESI-MS): mass calcd. C₂₄H₂₃N₃O₂S 417.2 m/z, found 418.0 [M+H]⁺.

### Step 2: Synthesis of 2-(4-(4-methylpiperazin-1-yl)phenyl)-3-(4-methylthiazol-5-yl)-1-oxo-1H-inden-6-yl 1,1,2,2,3,3,4,4,4-nonafluorobutane-1-sulfonate

To a solution of 6-hydroxy-2-(4-(4-methylpiperazin-1-yl)phenyl)-3-(4-methylthiazol-5-yl)-1H-inden-1-one (240 mg, 0.5748 mmol) in THF (10 mL) were added NaH (57.48 mg, 1.437 mmol, 60 % in mineral oil) and 1,1,2,2,3,3,4,4,4-nonafluorobutane-1-sulfonyl fluoride (434.1 mg, 1.437 mmol) at 0 °C, and the mixture was stirred at the same temperature for 2 hours.

Water (20 mL) was added to the reaction mixture, and the mixture was extracted with ethyl acetate (3 x 20 mL). Using a conventional method, the organic layer was washed, dried, and concentrated under reduced pressure. The residue was purified by silica gel column chromatography (3 % MeOH/DCM) to obtain the desired title compound (324 mg, 45 %) as a purple solid.

LCMS (ESI-MS): mass calcd. C₂₈H₂₂F₉N₃O₄S₂ 699.1 m/z, found 700.1 [M+H]⁺.

### Step 3: Synthesis of 6-(4-methylpiperazin-1-yl)-2-(4-(4-methylpiperazin-1-yl)phenyl)-3-(4-methylthiazol-5-yl)-1H-inden-1-one (Compound 75)

In the same manner as Preparation Example 58, the desired title compound (36 mg, 18 %) was obtained as a purple solid.

The obtained Compound 75 (Formula 76 below) was confirmed by LCMS(ESI-MS) and ¹H NMR, which yielded the following data:

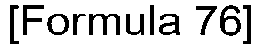

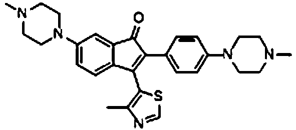

LCMS (ESI-MS): mass calcd. C₂₉H₃₃N₅OS 499.2 m/z, found 500.3 [M+H]⁺.

¹H NMR (400 MHz, DMSO-*d*₆) δ 9.24 (s, 1H), 7.18 - 7.17 (m, 1H), 7.08 - 7.16 (m, 2H), 6.92 - 6.83 (m, 4H), 3.24 - 3.22 (m, 4H), 3.17 - 3.15 (m, 4H), 2.45 - 2.40 (m, 8H), 2.22 - 2.20 (m, 6H), 1.99 (s, 3H).

### Preparation Example 75. 2-(4-(4-methylpiperazin-1-yl)phenyl)-3-(4-methylthiazol-5-yl)-6-(3-phenylpropoxy)-1H-inden-1-one (Compound 76)

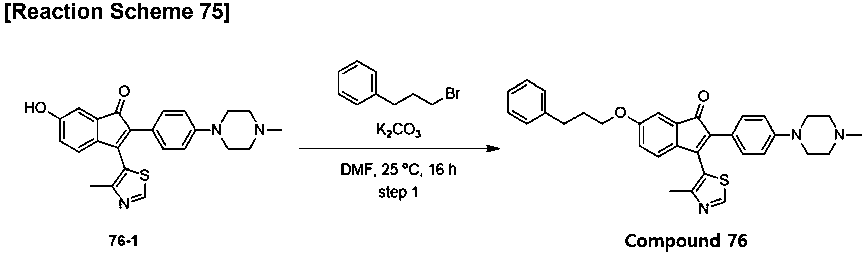

In the same manner as Preparation Example 35, the desired title compound (11.1 mg, 5 %) was obtained as a purple solid.

The obtained Compound 76 (Formula 77 below) was confirmed by LCMS(ESI-MS) and ¹H NMR, which yielded the following data:

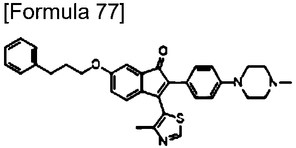

LCMS (ESI-MS): mass calcd. C₃₃H₃₃N₃O₂S 535.2 m/z, found 536.2 [M+H]⁺.

¹H NMR (400 MHz, CDCl₃) δ 8.89 (s, 1H), 7.31 - 7.29 (m, 2H), 7.22 - 7.18 (m, 5H), 7.16 - 7.15 (m, 1H), 6.96 - 6.94 (m, 1H), 6.83 - 6.78 (m, 3H), 4.01 - 3.98 (m, 2H), 3.37 (s, 4H), 2.83 - 2.77 (m, 6H), 2.49 (s, 3H), 2.13 - 2.10 (m, 5H).

### Preparation Example 76. 2,3-bis(4-methylthiazol-5-yl)-6-(3-phenylpropoxy)-1H-inden-1-one (Compound 77)

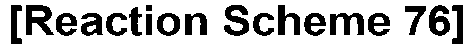

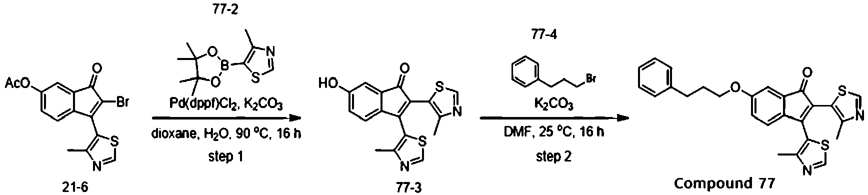

### Step 1: Synthesis of 6-hydroxy-2,3-bis(4-methylthiazol-5-yl)-1H-inden-1-one

In the same manner as Step 5 of Preparation Example 21, the desired title compound (149 mg, 69 %) was obtained as a yellow solid.

LCMS (ESI-MS): mass calcd. C₁₇H₁₂N₂O₂S₂ 340.0 m/z, found 341.0 [M+H]⁺.

### Step 2: Synthesis of 2,3-bis(4-methylthiazol-5-yl)-6-(3-phenylpropoxy)-1H-inden-1-one (Compound 77)

In the same manner as Preparation Example 35, the desired title compound (19 mg, 9 %) was obtained as a yellow solid.

The obtained Compound 77 (Formula 78 below) was confirmed by LCMS(ESI-MS) and ¹H NMR, which yielded the following data:

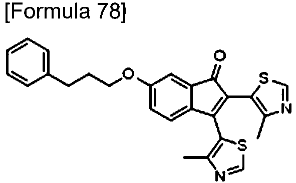

LCMS (ESI-MS): mass calcd. C₂₆H₂₂N₂O₂S₂ 458.1 m/z, found 459.1 [M+H]⁺.

¹H NMR (400 MHz, DMSO-*d*₆) δ 9.32 (s, 1H), 9.08 (s, 1H), 7.31 - 7.16 (m, 7H), 7.04 - 7.01 (m, 1H), 4.06 (t, *J* = 6 Hz, 2H), 2.75 (t, *J* = 8 Hz, 2H), 2.07 - 2.00 (m, 2H), 1.98 (s, 3H), 1.91 (s, 3H).

### Preparation Example 77. 2,3-diphenyl-6-(3-phenylpropoxy)-1H-inden-1-one (Compound 78)

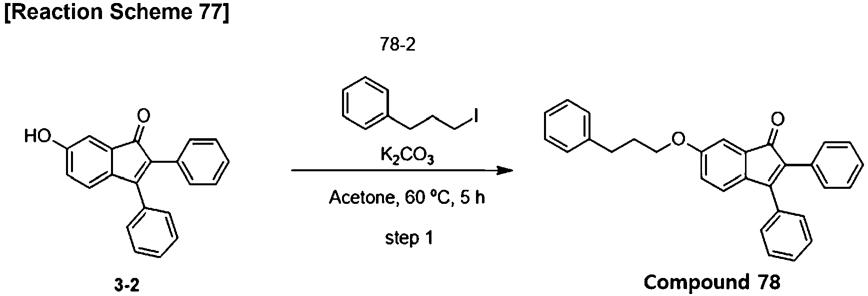

In the same manner as Preparation Example 35, the desired title compound (70 mg, 47 %) was obtained as a white solid.

The obtained Compound 78 (Formula 79 below) was confirmed by LCMS(ESI-MS) and ¹H NMR, which yielded the following data:

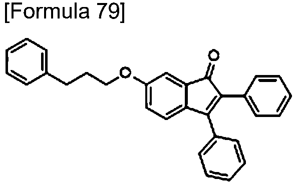

LCMS (ESI-MS): mass calcd. C₃₀H₂₄O₂ 416.2 m/z, found 417.2 [M+H]⁺.

¹H NMR (400 MHz, DMSO-*d*₆) δ 7.47 - 7.44 (m, 3H), 7.40 - 7.37 (m, 2H), 7.32 - 7.24 (m, 7H), 7.18 - 7.13 (m, 4H), 7.06 - 7.04 (m, 1H), 6.98 - 6.95 (m, 1H), 4.05 (t, *J* = 6.4 Hz, 2H), 2.80 - 2.71 (m, 2H), 2.09 - 2.00 (m, 2H).

### Preparation Example 78. 6-(4-methylpiperazin-1-yl)-2,3-bis(4-methylthiazol-5-yl)-1H-inden-1-one (Compound 79)

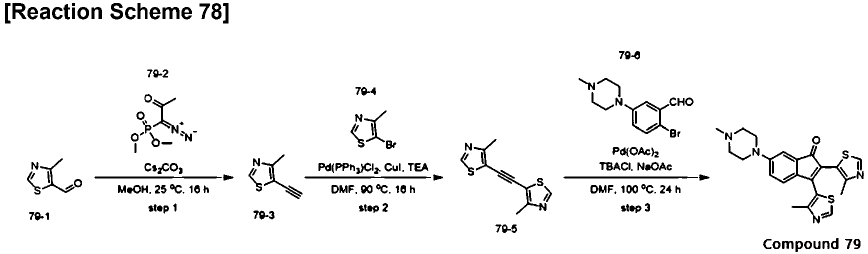

### Step 1: Synthesis of 5-ethynyl-4-methylthiazole

A mixture of 4-methylthiazole-5-carbaldehyde (5.0 g, 0.0393 mol), dimethyl (1-diazo-2-oxopropyl)phosphonate (8.3 g, 0.04323 mol), and Cs₂CO₃ (14.09 g, 0.04323 mol) in MeOH (50 mL) was stirred at room temperature for 16 hours. Water (100 mL) was added to the reaction mixture, and the mixture was extracted with EA (3 x 100 mL). Using a conventional method, the organic layer was washed, dried, and concentrated under reduced pressure. The residue was purified by silica gel column chromatography (10 % EtOAc/hexane) to obtain the desired title compound (5 g, 50 %) as a yellow oil.

LCMS (ESI-MS): mass calcd. C₆H₅NS 123.0 m/z, found 124.0 [M+H]⁺.

### Step 2: Synthesis of 1,2-bis(4-methylthiazol-5-yl)ethyne

A mixture of 5-ethynyl-4-methylthiazole (1.2 g, 0.0097 mol), 5-bromo-4-methylthiazole (1.73 g, 0.0097 mol), Pd(PPh₃)₂Cl₂ (0.34 g, 0.0005 mol), Cul (0.09 g, 0.0005 mol), and TEA (2.94 g, 0.0291 mol) in DMF (15 mL) was stirred at 90 °C for 16 hours under a nitrogen atmosphere. Water (100 mL) was added to the reaction mixture, and the mixture was extracted with EA (3 x 100 mL). Using a conventional method, the organic layer was washed, dried, and concentrated under reduced pressure. The residue was purified by silica gel column chromatography (20 % EtOAc/hexane) to obtain the desired title compound (0.8 g, 34 %) as a yellow oil.

LCMS (ESI-MS): mass calcd. C₁₀H₈N₂S₂ 220.0 m/z, found 220.6 [M+H]⁺.

### Step 3: Synthesis of 6-(4-methylpiperazin-1-yl)-2,3-bis(4-methylthiazol-5-yl)-1H-inden-1-one (Compound 79)

A mixture of 1,2-bis(4-methylthiazol-5-yl)ethyne (77.8 mg, 0.3532 mmol), 2-bromo-5-(4-methylpiperazin-1-yl)benzaldehyde (100 mg, 0.3532 mmol), Pd(OAc)₂ (3.96 mg, 0.0177 mmol), TBACI (98.16 mg, 0.3532 mmol), and NaOAc (115.8 mg, 1.4128 mmol) in DMF (4 mL) was stirred at 100 °C for 24 hours under a nitrogen atmosphere. Water (30 mL) was added to the reaction mixture, and the mixture was extracted with EA (3 x 40 mL). Using a conventional method, the organic layer was washed, dried, and concentrated under reduced pressure. The residue was purified by prep-HPLC (Gemini 5 µm C₁₈ column, 150 x 21.2 mm, 30 - 95 % ACN/H₂O with 0.1 % NH₃H₂O) to obtain the desired title compound (50 mg, 30 %) as a purple solid.

The obtained Compound 79 (Formula 80 below) was confirmed by LCMS(ESI-MS) and ¹H NMR, which yielded the following data:

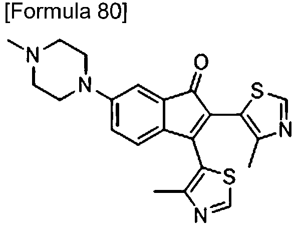

LCMS (ESI-MS): mass calcd. C₂₂H₂₂N₄OS₂ 422.1 m/z, found 423.1 [M+H]⁺.

¹H NMR (400 MHz, DMSO-*d*₆) δ 9.30 (s, 1H), 9.05 (s, 1H), 7.25 (d, *J* = 2.4 Hz, 1H), 7.10 (d, *J =* 8.2 Hz, 1H), 6.89 (dd, *J* = 8.2, 2.4 Hz, 1H), 3.30 - 3.25 (m, 4H), 2.46 - 2.41 (m, 4H), 2.22 (s, 3H), 1.98 (s, 3H), 1.90 (s, 3H).

### Preparation Example 79. 6-(4-methylpiperazin-1-yl)-2,3-bis(3-morpholinophenyl)-1H-inden-1-one (Compound 80)

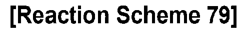

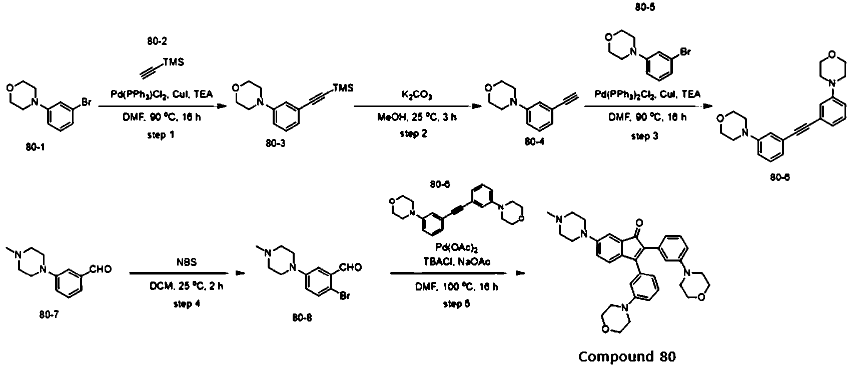

### Step 1: Synthesis of 4-(3-((trimethylsilyl)ethynyl)phenyl)morpholine

In the same manner as Step 2 of Preparation Example 78, the desired title compound (4.2 g, 75 % purity, 97 % yield) was obtained as a yellow oil.

LCMS (ESI-MS): mass calcd. C₁₅H₂₁NOSi 259.1 m/z, found 259.7 [M+H]⁺.

### Step 2: Synthesis of 4-(3-ethynylphenyl)morpholine

A mixture of 4-(3-((trimethylsilyl)ethynyl)phenyl)morpholine (4.12 g, 0.0159 mol) and K₂CO₃ (3.29 g, 0.02385 mol) in MeOH (100 mL) was stirred at room temperature for 3 hours.

Water (200 mL) was added to the reaction mixture, and the mixture was extracted with DCM (3 x 100 mL). Using a conventional method, the organic layer was washed, dried, and concentrated under reduced pressure. The residue was purified by silica gel column chromatography (5 % EtOAc/hexane) to obtain the desired title compound (2.87 g, 90 % purity, 87 % yield) as a yellow oil.

LCMS (ESI-MS): mass calcd. C₁₂H₁₃NO 187.1 m/z, found 187.7 [M+H]⁺.

### Step 3: Synthesis of 1,2-bis(3-morpholinophenyl)ethyne

In the same manner as Step 2 of Preparation Example 78, the desired title compound (340 mg, 45 %) was obtained as a yellow oil.

LCMS (ESI-MS): mass calcd. C₂₂H₂₄N₂O₂ 348.2 m/z, found 349.2 [M+H]⁺.

### Step 4: Synthesis of 2-bromo-5-(4-methylpiperazin-1-yl)benzaldehyde

A mixture of 3-(4-methylpiperazin-1-yl)benzaldehyde (408 mg, 1.9974 mmol) and NBS (710.99 mg, 3.9948 mmol) in DCM (10 mL) was stirred at room temperature for 2 hours. The reaction mixture was concentrated, and the residue was purified by silica gel column chromatography (15 % EtOAc/hexane) to obtain the desired title compound (400 mg, 71 %) as a yellow oil.

LCMS (ESI-MS): mass calcd. C₁₂H₁₅BrN₂O 282.0 m/z, found 282.9 [M+H]⁺.

### Step 5: Synthesis of 6-(4-methylpiperazin-1-yl)-2,3-bis(3-morpholinophenyl)-1H-inden-1-one (Compound 80)

In the same manner as Step 3 of Preparation Example 78, the desired title compound (55.4 g, 22 %) was obtained as a yellow oil.

The obtained Compound 80 (Formula 81 below) was confirmed by LCMS(ESI-MS) and ¹H NMR, which yielded the following data:

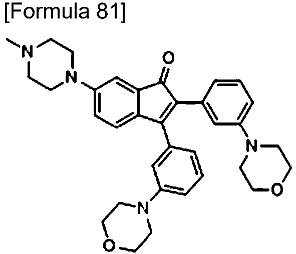

LCMS (ESI-MS): mass calcd. C₃₄H₃₈N₄O₃ 550.3 m/z, found 551.73 [M+H]⁺.

¹H NMR (400 MHz, DMSO-*d₆*) δ 7.33 - 7.29 (m, 1H), 7.19 - 7.12 (m, 2H), 7.04 - 6.95 (m, 2H), 6.87 - 6.79 (m, 4H), 6.71 - 6.69 (m, 2H), 3.69 - 3.65 (m, 8H), 3.24 (t, *J* = 4.8 Hz, 4H), 3.01 (t, *J* = 4.8 Hz, 4H), 2.90 (t, *J* = 4.8 Hz, 4H), 2.44 (d, *J* = 4.8 Hz, 4H), 2.22 (s, 3H).

### Preparation Example 80. 6-(4-(2-hydroxyethyl)piperazin-1-yl)-2,3-bis(4-methylthiazol-5-yl)-1H-inden-1-one (Compound 81)

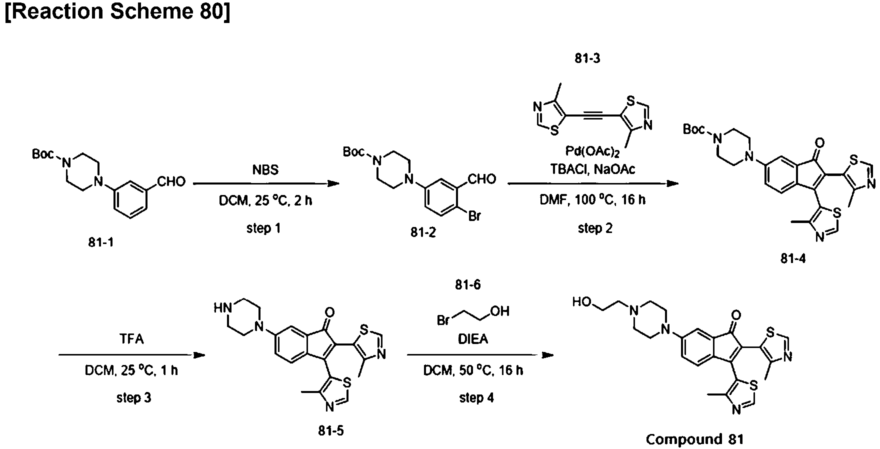

### Step 1: Synthesis of tert-butyl 4-(4-bromo-3-formylphenyl)piperazine-1-carboxylate

In the same manner as Step 4 of Preparation Example 79, the desired title compound (500 mg, 70 %) was obtained as a yellow solid.

LCMS (ESI-MS): mass calcd. C₁₆H₂₁BrN₂O₃ 368.1 m/z, found 368.7 [M+H]⁺.

### Step 2: Synthesis of tert-butyl 4-(2,3-bis(4-methylthiazol-5-yl)-1-oxo-1H-inden-6-yl)piperazine-1-carboxylate

In the same manner as Step 3 of Preparation Example 78, the desired title compound (0.6 g, 40 %) was obtained as a yellow solid.

LCMS (ESI-MS): mass calcd. C₂₆H₂₈N₄O₃S₂ 508.2 m/z, found 509.1 [M+H]⁺.

### Step 3: Synthesis of 2,3-bis(4-methylthiazol-5-yl)-6-(piperazin-1-yl)-1H-inden-1-one

In the same manner as Step 4 of Preparation Example 29, the desired title compound (180 mg, 67 %) was obtained as a purple solid.

LCMS (ESI-MS): mass calcd. C₂₁H₂₀N₄OS₂ 408.1 m/z, found 409.0 [M+H]⁺.

### Step 4: Synthesis of 6-(4-(2-hydroxyethyl)piperazin-1-yl)-2,3-bis(4-methylthiazol-5-yl)-1H-inden-1-one (Compound 81)

A mixture of 2,3-bis(4-methylthiazol-5-yl)-6-(piperazin-1-yl)-1H-inden-1-one (100 mg, 0.2448 mmol), 2-bromoethan-1-ol (458.8 mg, 3.67 mmol), and DIEA (632.8 mg, 4.89 mmol) in DCM (10 mL) was stirred at 50 °C for 16 hours under a nitrogen atmosphere. The residue, obtained by concentrating the reaction mixture under reduced pressure, was purified by prep-HPLC (Gemini 5 µm C₁₈ column, 150 x 21.2 mm, 5 - 95 % ACN/H₂O with 0.1% FA) to obtain the desired title compound (20 mg, 18 %) as a purple solid.

The obtained Compound 81 (Formula 82 below) was confirmed by LCMS(ESI-MS) and ¹H NMR, which yielded the following data:

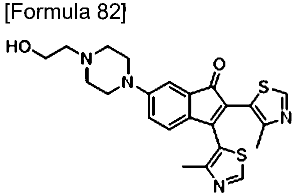

LCMS (ESI-MS): mass calcd. C₂₃H₂₄N₄O₂S₂ 452.1 m/z, found 452.8 [M+H]⁺.

¹H NMR (400 MHz, DMSO-*d*₆) δ 9.30 (s, 1H), 9.04 (s, 1H), 8.17 (s, 1H), 7.24 (d, *J =* 2.4 Hz, 1H), 7.10 (d, *J* = 8.2 Hz, 1H), 6.89 (dd, *J* = 8.2, 2.4 Hz, 1H), 3.54 (t, *J* = 6.2 Hz, 2H), 3.31 - 3.25 (m, 4H), 2.59 - 2.53 (m, 4H), 2.44 (t, *J* = 6.2 Hz, 2H), 1.98 (s, 3H), 1.91 (s, 3H).

### Preparation Example 81. 6-hydroxy-2,3-di-o-tolyl-1H-inden-1-one (Compound 82)

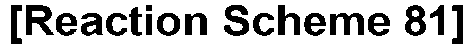

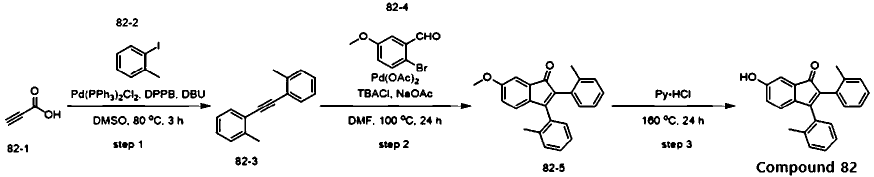

### Step 1: Synthesis of 1,2-di-o-tolylethyne

A mixture of propiolic acid (3 g, 0.0428 mol), 1-iodo-2-methylbenzene (18.6 g, 0.0856 mol), Pd(PPh₃)₂Cl₂ (1.5 g, 0.0021 mol), DPPB (1.83 g, 0.0042 mol), and DBU (13.0 g, 0.0856 mol) in DMSO (100 mL) was stirred at 80 °C for 3 hours under a nitrogen atmosphere. Water (300 mL) was added to the reaction mixture, and the mixture was extracted with EA (3 x 400 mL). Using a conventional method, the organic layer was washed, dried, and concentrated under reduced pressure. The residue was purified by silica gel column chromatography (5 % EtOAc/hexane) to obtain the desired title compound (5 g, 50 %) as a yellow solid.

¹H NMR (400 MHz, CDCl₃) δ 7.52- 7.50 (m, 2H), 7.26 - 7.23 (m, 6H), 2.53 (s, 6H).

### Step 2: Synthesis of 6-methoxy-2,3-di-o-tolyl-1H-inden-1-one

In the same manner as Step 3 of Preparation Example 78, the desired title compound (0.4 g, 7 %) was obtained as a yellow solid.

LCMS (ESI-MS): mass calcd. C₂₄H₂₀O₂ 340.1 m/z, found 340.8 [M+H]⁺.

### Step 3: Synthesis of 6-hydroxy-2,3-di-o-tolyl-1H-inden-1-one (Compound 82)

A mixture of 6-methoxy-2,3-di-o-tolyl-1H-inden-1-one (200 mg, 0.5875 mmol) and Py·HCl (271.57 mg, 2.35 mmol) was stirred at 160 °C for 24 hours under a nitrogen atmosphere. Water (20 mL) was added to the reaction mixture, and the mixture was extracted with EA (3 x 30 mL). Using a conventional method, the organic layer was washed, dried, and concentrated under reduced pressure. The residue was purified by prep-HPLC (Gemini 5 µm C₁₈ column, 150 x 21.2 mm, 55 - 95 % ACN/H₂O with 0.1 % FA) to obtain the desired title compound (80 mg, 37 %) as a white solid.

The obtained Compound 82 (Formula 83 below) was confirmed by LCMS(ESI-MS) and ¹H NMR, which yielded the following data:

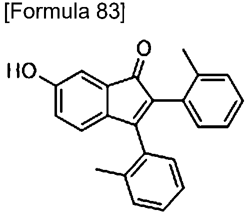

LCMS (ESI-MS): mass calcd. C₂₃H₁₈O₂ 326.1 m/z, found 327.1 [M+H]⁺.

¹H NMR (400 MHz, DMSO-*d*₆) δ 10.12 (s, 1H), 7.28- 7.14 (m, 6H), 7.02 - 6.94 (m, 1H), 6.93 - 6.89 (m, 2H), 6.75 - 6.73 (m, 1H), 6.64 - 6.62 (m, 1H), 2.07 (s, 6H).

### Preparation Example 82. 6-(3-phenylpropoxy)-2,3-di(thiazol-5-yl)-1H-inden-1-one (Compound 83)

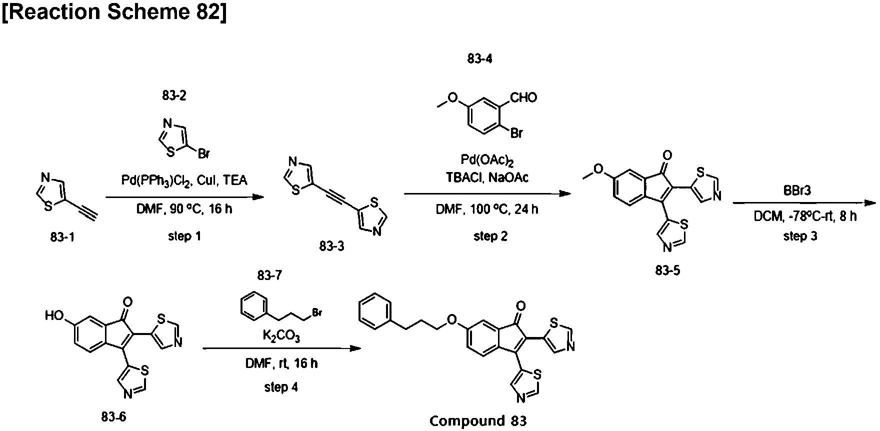

### Step 1: Synthesis of 1,2-di(thiazol-5-yl)ethyne

In the same manner as Step 2 of Preparation Example 78, the desired title compound (0.9 g, 53 %) was obtained as a white solid.

LCMS (ESI-MS): mass calcd. C₈H₄N₂S₂ 192.0 m/z, found 192.6 [M+H]⁺.

### Step 2: Synthesis of 6-methoxy-2,3-di(thiazol-5-yl)-1H-inden-1-one

In the same manner as Step 3 of Preparation Example 78, the desired title compound (0.35 g, 39 %) was obtained as a purple solid.

LCMS (ESI-MS): mass calcd. C₁₆H₁₀N₂O₂S₂ 326.0 m/z, found 326.6 [M+H]⁺.

### Step 3: Synthesis of 6-hydroxy-2,3-di(thiazol-5-yl)-1H-inden-1-one

In the same manner as Step 4 of Preparation Example 41, the desired title compound (180 mg, 74 %) was obtained as a red solid.

LCMS (ESI-MS): mass calcd. C₁₅H₈N₂O₂S₂ 312.0 m/z, found 312.6 [M+H]⁺.

### Step 4: Synthesis of 6-(3-phenylpropoxy)-2,3-di(thiazol-5-yl)-1H-inden-1-one (Compound 83)

In the same manner as Step 2 of Preparation Example 38, the desired title compound (60 mg, 39 %) was obtained as a purple solid.

The obtained Compound 83 (Formula 84 below) was confirmed by LCMS(ESI-MS) and ¹H NMR, which yielded the following data:

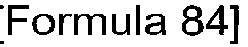

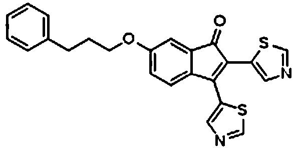

LCMS (ESI-MS): mass calcd. C₂₄H₁₈N₂O₂S₂ 430.1 m/z, found 430.7 [M+H]⁺.

¹H NMR (400 MHz, DMSO-*d*₆) δ 9.44 (s, 1H), 9.15 (s, 1H), 8.42 (s, 1H), 8.10 (s, 1H), 7.34 - 7.14 (m, 7H), 7.02 (dd, J = 8.2, 2.4 Hz, 1H), 4.06 (t, *J = 6.4* Hz, 2H), 2.75 (t, *J = 7.6* Hz, 2H), 2.11 - 1.98 (m, 2H).

### Preparation Example 83. 3-(2,6-dimethylphenyl)-6-hydroxy-2-(o-tolyl)-1H-inden-1-one (Compound 84)

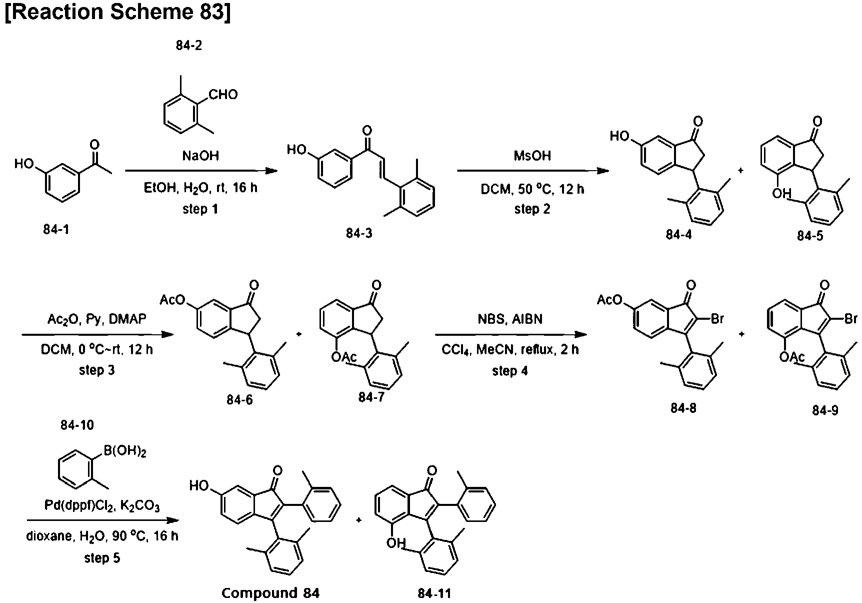

### Step 1: Synthesis of (E)-3-(2,6-dimethylphenyl)-1-(3-hydroxyphenyl)prop-2-en-1-one

In the same manner as Step 1 of Preparation Example 21, the desired title compound (10.8 g, 53 %) was obtained as a yellow solid.

¹H NMR (400 MHz, DMSO-*d*₆) *δ* 9.84 (s, 1H), 7.82 (d, *J* = 16.0 Hz, 1H), 7.53 (d, *J* = 8.0 Hz, 1H), 7.43 - 7.40 (m, 1H), 7.40 - 7.29 (m, 2H), 7.20 - 7.13 (m, 3H), 7.07 (dd, J = 8.0, 2.4 Hz, 1H), 2.38 (s, 6H).

### Step 2: Synthesis of 3-(2,6-dimethylphenyl)-6-hydroxy-2,3-dihydro-1H-inden-1-one

To a solution of (E)-3-(2,6-dimethylphenyl)-1-(3-hydroxyphenyl)prop-2-en-1-one (2 g, 7.94 mmol) in DCM (15 mL) was added MsOH (3 mL), and the mixture was stirred at 50 °C for 12 hours.

The reaction mixture was adjusted to pH 6 with an aqueous NaOH solution (1 N), and the resulting solid was collected by filtration to obtain a mixture of the title compound and a structural isomer, 3-(2,6-dimethylphenyl)-4-hydroxy-2,3-dihydro-1H-inden-1-one, (1.2 g, 60 %) as a yellow solid. The mixture was used in the next reaction without purification.

LCMS (ESI-MS): mass calcd. C₁₇H₁₆O₂ 252.1 m/z, found 252.7 [M+H]⁺.

### Step 3: Synthesis of 1-(2,6-dimethylphenyl)-3-oxo-2,3-dihydro-1H-inden-5-yl acetate

In the same manner as Step 3 of Preparation Example 21, a mixture of the desired title compound and 3-(2,6-dimethylphenyl)-1-oxo-2,3-dihydro-1H-inden-4-yl acetate (500 mg, 36 %) was obtained as a white solid.

LCMS (ESI-MS): mass calcd. C₁₉H₁₈O₃ 294.1 m/z, found 294.8 [M+H]⁺.

### Step 4: Synthesis of 2-bromo-3-(2,6-dimethylphenyl)-1-oxo-1H-inden-6-yl acetate

In the same manner as Step 4 of Preparation Example 21, the desired title compound (70 mg, 11 %) was obtained as a red solid.

LCMS (ESI-MS): mass calcd. C₁₉H₁₅BrO₃ 370.0 m/z, found 370.8 [M+H]⁺.

### Step 5: Synthesis of 3-(2,6-dimethylphenyl)-4-hydroxy-2-(o-tolyl)-1H-inden-1-one (Compound 84-11)

In the same manner as Step 5 of Preparation Example 21, the desired title Compound 84 (15.4, 15 %) and Compound 84-11 (5.4 mg, 5 %) were obtained as a yellow solid.

The obtained Compound 84-11 was confirmed by LCMS(ESI-MS) and ¹H NMR, which yielded the following data:
LCMS (ESI-MS): mass calcd. C₂₄H₂₀O₂ 340.1 m/z, found 340.8 [M+H]⁺.

¹H NMR (400 MHz, DMSO-*d*₆) *δ* 10.09 (s, 1H), 7.25 - 7.06 (m, 4H), 7.04 - 6.97 (m, 2H), 6.93 (d, *J* = 2.0 Hz, 1H), 6.89 (s, 2H), 6.79 (dd, *J* = 8.0, 2.0 Hz, 1H), 2.17 (s, 6H), 1.97 (s, 3H).

The obtained Compound 84 (Formula 85 below) was confirmed by LCMS(ESI-MS) and ¹H NMR, which yielded the following data:

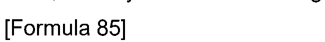

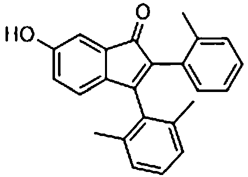

LCMS (ESI-MS): mass calcd. C₂₄H₂₀O₂ 340.1 m/z, found 341.1 [M+H]⁺.

¹H NMR (400 MHz, DMSO-*d*₆) *δ* 10.04 (s, 1H), 7.15 - 7.03 (m, 6H), 6.92 - 6.89 (m, 2H), 6.74 - 6.72 (m, 1H), 6.64 (d, *J* = 8.0 Hz, 1H), 2.26 (s, 3H), 2.05 - 1.99 (m, 6H).

### Preparation Example 84. 2-(4-(4-methylpiperazin-1-yl)phenyl)-3-(4-methylthiazol-5-yl)-1H-inden-1-one (Compound 85)

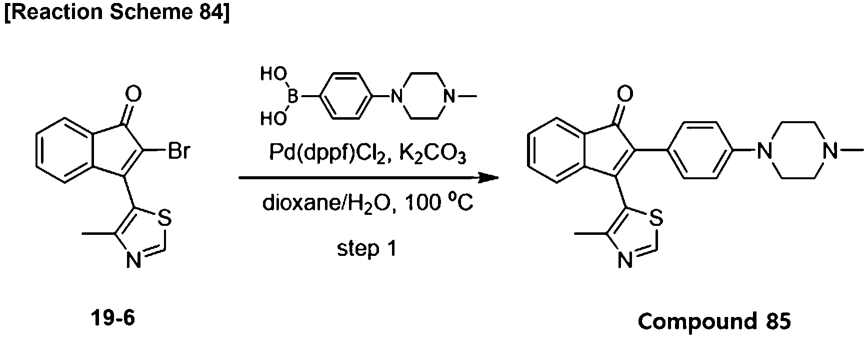

In the same manner as Step 5 of Preparation Example 21, the desired title compound (23.6 mg, 18 %) was obtained as a red solid.

The obtained Compound 85 (Formula 86 below) was confirmed by LCMS(ESI-MS) and ¹H NMR, which yielded the following data:

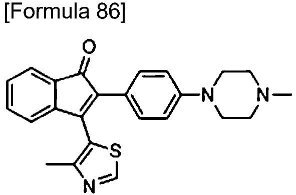

LCMS (ESI-MS): mass calcd. C₂₄H₂₃N₃OS 401.2 m/z, found 401.8 [M+H]⁺.

¹H NMR (400 MHz, DMSO-*d*₆) *δ* 9.27 (s, 1H), 7.58 - 7.43 (m, 2H), 7.34 (t, *J* = 7.6 Hz, 1H), 7.14 - 7.09 (m, 3H), 6.89 (d, *J* = 8.8 Hz, 2H), 3.23 - 3.11 (m, 4H), 2.45 - 2.39 (m, 4H), 2.21 (s, 3H), 2.01 (s, 3H).

### Preparation Example 85. 2-(4-((4-methylpiperazin-1-yl)methyl)phenyl)-3-(4-methylthiazol-5-yl)-1H-inden-1-one (Compound 86)

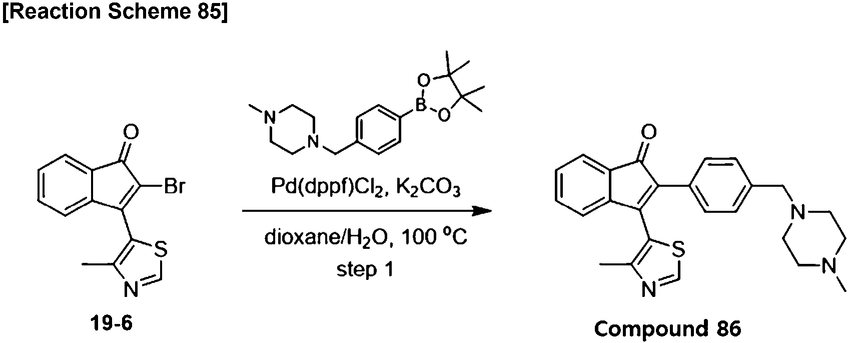

In the same manner as Step 5 of Preparation Example 21, the desired title compound (24.2 mg, 17 %) was obtained as a yellow solid.

The obtained Compound 86 (Formula 87 below) was confirmed by LCMS(ESI-MS) and ¹H NMR, which yielded the following data:

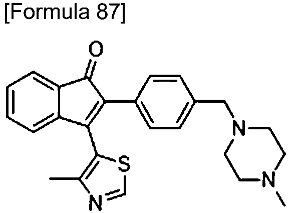

LCMS (ESI-MS): mass calcd. C₂₅H₂₅N₃OS 415.2 m/z, found 416.1 [M+H]⁺.

¹H NMR (400 MHz, DMSO-*d*₆) *δ* 9.29 (s, 1H), 7.58 - 7.53 (m, 2H), 7.43 - 7.38 (m, 1H), 7.27 (d, *J* = 8.0 Hz, 2H), 7.21 - 7.16 (m, 3H), 3.45 (s, 2H), 2.36 (s, 8H), 2.18 (s, 3H), 1.92 (s, 3H).

### Preparation Example 86. 3-(4-methylthiazol-5-yl)-2-(4-(morpholinomethyl)phenyl)-1H-inden-1-one (Compound 87)

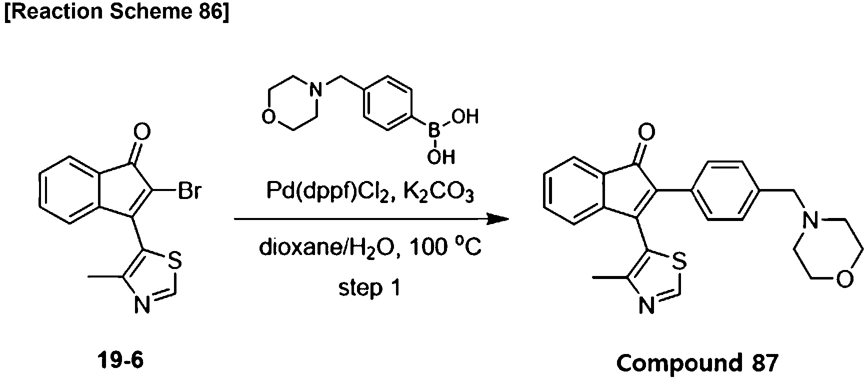

In the same manner as Step 5 of Preparation Example 21, the desired title compound (23.8 mg, 18 %) was obtained as a yellow solid.

The obtained Compound 87 (Formula 88 below) was confirmed by LCMS(ESI-MS) and ¹H NMR, which yielded the following data:

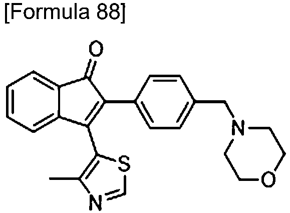

LCMS (ESI-MS): mass calcd. C₂₄H₂₂N₂O₂S 402.1 m/z, found 403.1 [M+H]⁺.

¹H NMR (400 MHz, DMSO-*d*₆) *δ* 9.30 (s, 1H), 7.63 - 7.50 (m, 2H), 7.41 (t, *J =* 7.2 Hz, 1H), 7.29 (d, *J* = 8.0 Hz, 2H), 7.19 (t, *J* = 8.0 Hz, 3H), 3.63 - 3.53 (m, 4H), 3.45 (s, 2H), 2.34 (d, *J* = 4.4 Hz, 4H), 1.91 (s, 3H).

### Preparation Example 87. 2-(3-((4-methylpiperazin-1-yl)methyl)phenyl)-3-(4-methylthiazol-5-yl)-1H-inden-1-one (Compound 88)

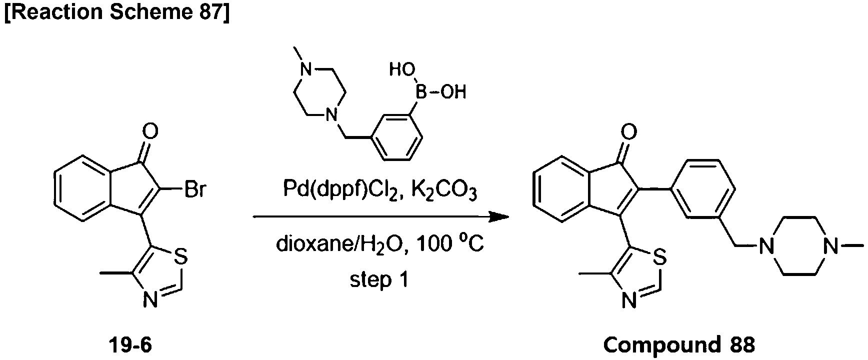

In the same manner as Step 5 of Preparation Example 21, the desired title compound (24.6 mg, 18 %) was obtained as a yellow solid.

The obtained Compound 88 (Formula 89 below) was confirmed by LCMS(ESI-MS) and ¹H NMR, which yielded the following data:

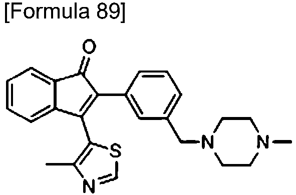

LCMS (ESI-MS): mass calcd. C₂₅H₂₅N₃OS 415.2 m/z, found 416.2 [M+H]⁺.

¹H NMR (400 MHz, DMSO-*d*₆) δ 9.29 (s, 1H), 7.64 - 7.50 (m, 2H), 7.41 (t, *J =* 7.2 Hz, 1H), 7.33 (t, *J =* 7.6 Hz, 1H), 7.27 - 7.12 (m, 3H), 7.08 (s, 1H), 3.36 (s, 2H), 2.25 (s, 8H), 2.18 (s, 3H), 1.91 (s, 3H).

### Preparation Example 88. 3-(4-methylthiazol-5-yl)-2-(3-(morpholinomethyl)phenyl)-1H-inden-1-one (Compound 89)

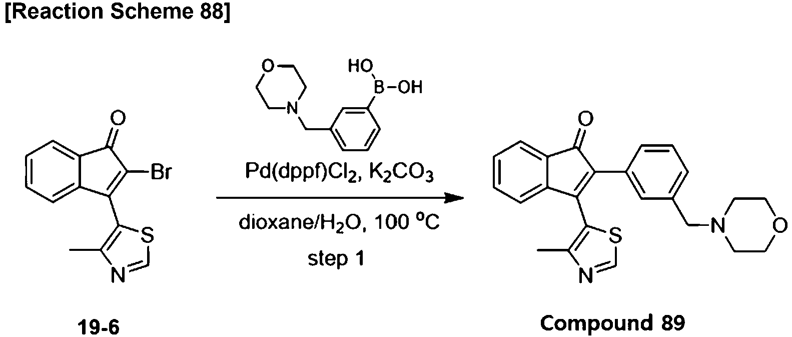

In the same manner as Step 5 of Preparation Example 21, the desired title compound (24.7 mg, 19 %) was obtained as a yellow solid.

The obtained Compound 89 (Formula 90 below) was confirmed by LCMS(ESI-MS) and ¹H NMR, which yielded the following data:

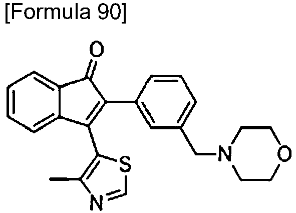

LCMS (ESI-MS): mass calcd. C₂₄H₂₂N₂O₂S 402.1 m/z, found 403.1 [M+H]⁺.

¹H NMR (400 MHz, DMSO-*d*₆) *δ* 9.30 (s, 1H), 7.65 - 7.50 (m, 2H), 7.46 - 7.39 (m, 1H), 7.35 (t, *J* = 7.6 Hz, 1H), 7.27 - 7.15 (m, 3H), 7.08 (s, 1H), 3.62 - 3.44 (m, 4H), 3.37 (s, 2H), 2.21 (d, *J* = 4.4 Hz, 4H), 1.91 (s, 3H).

### Preparation Example 89. 2-(4-(4-methylpiperazin-1-yl)phenyl)-3-phenyl-1H-inden-1-one (Compound 90)

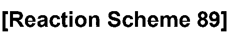

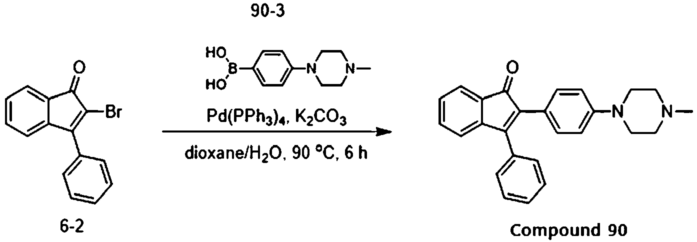

In the same manner as Preparation Example 14, the desired title compound (60 mg, 46 %) was obtained as a red solid.

The obtained Compound 90 (Formula 91 below) was confirmed by LCMS(ESI-MS) and ¹H NMR, which yielded the following data:

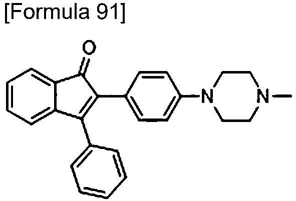

LCMS (ESI-MS): mass calcd. C₂₆H₂₄N₂O 380.2 m/z, found 380.9 [M+H]⁺.

¹H NMR (400 MHz, DMSO-*d*₆) δ 7.51 - 7.45 (m, 5H), 7.42 - 7.38 (m, 2H), 7.33 (t, *J* = 7.4 Hz, 1H), 7.09 - 7.06 (m, 3H), 6.83 (d, *J* = 8.8 Hz, 2H), 3.17 - 3.12 (m, 4H), 2.43 - 2.38 (m, 4H), 2.20 (s, 3H).

### Preparation Example 90. 2-(3-(4-methylpiperazin-1-yl)phenyl)-3-phenyl-1H-inden-1-one (Compound 91)

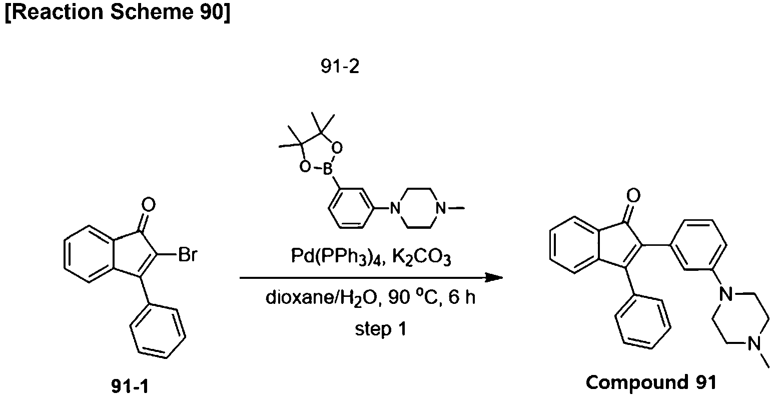

In the same manner as Preparation Example 14, the desired title compound (60 mg, 40 %) was obtained as a yellow solid.

The obtained Compound 91 (Formula 92 below) was confirmed by LCMS(ESI-MS) and ¹H NMR, which yielded the following data:

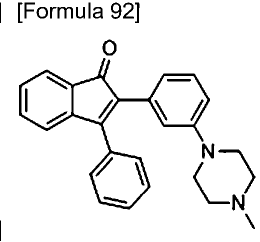

LCMS (ESI-MS): mass calcd. C₂₆H₂₄N₂O 380.2 m/z, found 380.9 [M+H]⁺.

¹H NMR (400 MHz, DMSO-*d*₆) δ 7.56 - 7.45 (m, 5H), 7.42 - 7.36 (m, 3H), 7.16 - 7.09 (m, 2H), 6.85 (dd, *J* = 8.4, 2.2 Hz, 1H), 6.70 (s, 1H), 6.65 (d, *J =* 7.6 Hz, 1H), 2.97 - 2.90 (m, 4H), 2.40 - 2.32 (m, 4H), 2.18 (s, 3H).

### Preparation Example 91. 3-(4-((4-methylpiperazin-1-yl)methyl)phenyl)-2-(4-methylthiazol-5-yl)-1H-inden-1-one (Compound 92)

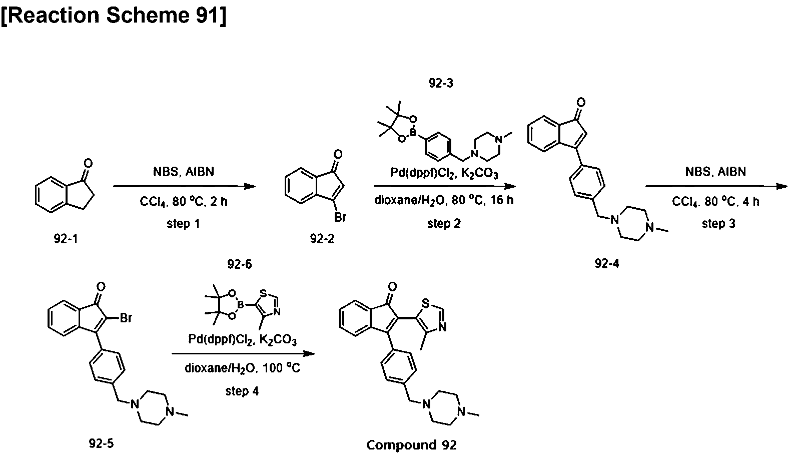

### Step 1: Synthesis of 3-bromo-1H-inden-1-one)

In the same manner as Step 4 of Preparation Example 21, the desired title compound (1.6 mg, 17 %) was obtained as a yellow solid.

LCMS (ESI-MS): mass calcd. C₉H₅BrO 208.0 m/z, found 208.5 [M+H]⁺.

### Step 2: Synthesis of 3-(4-((4-methylpiperazin-1-yl)methyl)phenyl)-1H-inden-1-one

In the same manner as Step 5 of Preparation Example 21, the desired title compound (510 mg, 22 %) was obtained as a yellow solid.

¹H NMR (400 MHz, DMSO-*d*₆) *δ* 7.77 (d, *J* = 8.0 Hz, 2H), 7.65 - 7.47 (m, 6H), 7.45 - 7.38 (m, 1H), 3.54 (s, 2H), 2.40 - 2.34 (m, 8H), 2.16 (s, 3H).

### Step 3: Synthesis of 2-bromo-3-(4-((4-methylpiperazin-1-yl)methyl)phenyl)-1H-inden-1-one

A mixture of 3-(4-((4-methylpiperazin-1-yl)methyl)phenyl)-1H-inden-1-one (510 mg, 1.6 mmol), NBS (570 mg, 3.2 mmol), and AIBN (262 mg, 1.6 mmol) in CCl₄ was stirred at 80 °C for 4 hours. Water (5 mL) was added to the reaction mixture, and the mixture was extracted with EA (3 x 10 mL). Using a conventional method, the organic layer was washed, dried, and concentrated under reduced pressure. The reaction mixture was concentrated under reduced pressure, and the residue was purified by silica gel column chromatography (35 % EtOAc/PE) to obtain the desired title compound (210 mg, 33 %) as a red solid.

LCMS (ESI-MS): mass calcd. C₂₁H₂₁BrN₂O 396.1 m/z, found 396.2 [M+H]⁺.

### Step 4: Synthesis of 3-(4-((4-methylpiperazin-1-yl)methyl)phenyl)-2-(4-methylthiazol-5-yl)-1H-inden-1-one (Compound 92)

A mixture of 2-bromo-3-(4-((4-methylpiperazin-1-yl)methyl)phenyl)-1H-inden-1-one (162 g, 0.0004 mol), 4-methyl-5-(4,4,5,5-tetramethyl-1,3,2-dioxaborolan-2-yl)thiazole (0.1 g, 0.0005 mol), Pd(dppf)Cl₂ (30 mg, 0.00004 mol), and K₂CO₃ (118 mg, 0.0012 mol) in dioxane (1.5 mL) was stirred at 100 °C for 3 hours under a nitrogen atmosphere. Water (10 mL) was added to the reaction mixture, and the mixture was extracted with EA (3 x 10 mL). Using a conventional method, the organic layer was washed, dried, and concentrated under reduced pressure. The resulting residue was purified by flash chromatography (50 % EtOAc/PE) to obtain the desired title compound (5.1 mg, 3 %) as a red solid.

The obtained Compound 92 (Formula 93 below) was confirmed by LCMS(ESI-MS) and ¹H NMR, which yielded the following data:

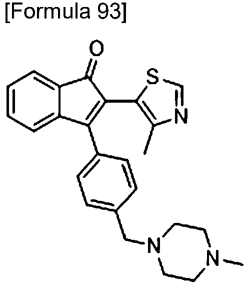

LCMS (ESI-MS): mass calcd. C₂₅H₂₅N₃OS 415.2 m/z, found 415.9 [M+H]⁺.

¹H NMR (400 MHz, DMSO-*d*₆) *δ* 9.06 (s, 1H), 7.64 - 7.50 (m, 2H), 7.48 - 7.26 (m, 6H), 3.52 (s, 2H), 2.38 (s, 8H), 2.17 (s, 3H), 1.80 (s, 3H).

### Preparation Example 92. 3-(3-((4-methylpiperazin-1-yl)methyl)phenyl)-2-(4-methylthiazol-5-yl)-1H-inden-1-one (Compound 93)

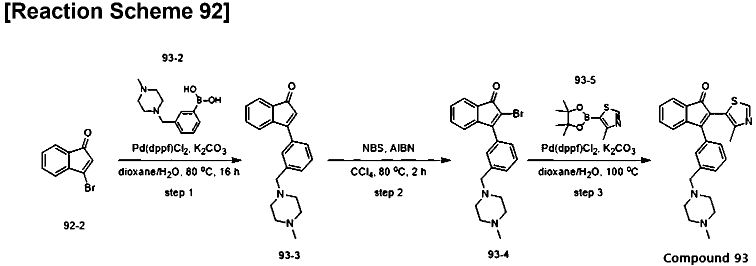

### Step 1: Synthesis of -(4-(4-methylpiperazin-1-yl)phenyl)-1H-inden-1-one

In the same manner as Step 5 of Preparation Example 21, the desired title compound (190 mg, 31 %) was obtained as a yellow solid.

¹H NMR (400 MHz, DMSO-*d*₆) *δ* 7.68 (d, *J* = 8.8 Hz, 2H), 7.57 - 7.46 (m, 5H), 7.42 (d, *J* = 7.2 Hz, 1H), 6.24 (s, 1H), 3.56 (s, 2H), 2.41 - 2.33 (m, 8H), 2.15 (s, 3H).

### Step 2: Synthesis of bromo-3-(3-((4-methylpiperazin-1-yl)methyl)phenyl)-1H-inden-1-one

In the same manner as Step 3 of Preparation Example 22, the desired title compound (100 mg, crude) was obtained as a yellow solid.

### Step 3: Synthesis of 3-(3-((4-methylpiperazin-1-yl)methyl)phenyl)-2-(4-methylthiazol-5-yl)-1H-inden-1-one (Compound 93)

In the same manner as Step 5 of Preparation Example 21, the desired title compound (8.2 mg, 13 %) was obtained as a red solid.

The obtained Compound 93 (Formula 94 below) was confirmed by LCMS(ESI-MS) and ¹H NMR, which yielded the following data:

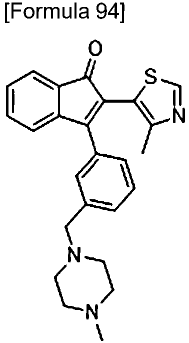

LCMS (ESI-MS): mass calcd. C₂₅H₂₅N₃OS 415.2 m/z, found 416.0 [M+H]⁺.

¹H NMR (400 MHz, DMSO-*d*₆) *δ* 9.05 (s, 1H), 7.63 - 7.35 (m, 6H), 7.33 - 7.23 (m, 2H), 3.44 (s, 2H), 2.24 (s, 8H), 2.13 (s, 3H), 1.81 (s, 3H).

### Preparation Example 93. 3-(4-(4-methylpiperazin-1-yl)phenyl)-2-phenyl-1H-inden-1-one (Compound 94)

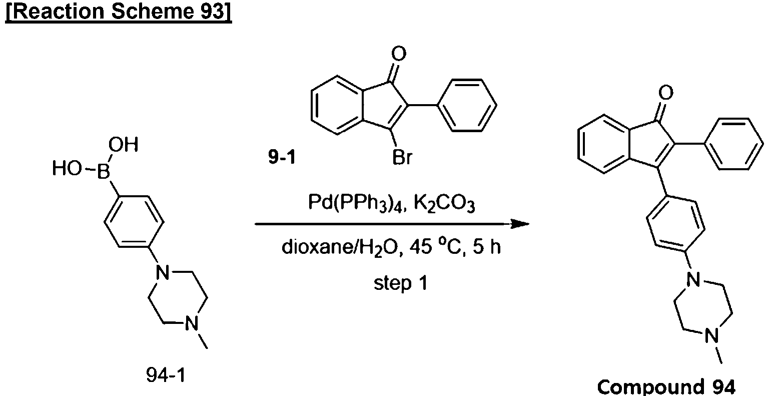

In the same manner as Step 5 of Preparation Example 21, the desired title compound (80 mg, 35 %) was obtained as a white solid.

The obtained Compound 94 (Formula 95 below) was confirmed by LCMS(ESI-MS) and ¹H NMR, which yielded the following data:

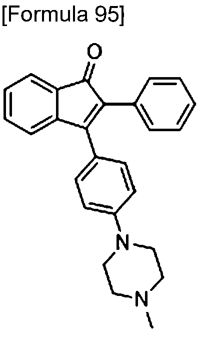

LCMS (ESI-MS): mass calcd. C₂₆H₂₄N₂O 380.2 m/z, found 381.1 [M+H]⁺.

¹H NMR (400 MHz, DMSO-*d*₆) δ 7.51 (t, *J* = 7.2 Hz, 2H), 7.40 - 7.36 (m, 1H), 7.34 - 7.30 (m, 3H), 7.27 - 7.26 (m, 3H), 7.22 - 7.19 (m, 2H), 6.98 - 6.96 (m, 2H), 3.33 - 3.24 (m, 4H), 2.51 - 2.44 (m, 4H), 2.22 (s, 3H).

### Preparation Example 94. 3-(3-(4-methylpiperazin-1-yl)phenyl)-2-phenyl-1H-inden-1-one (Compound 95)

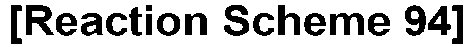

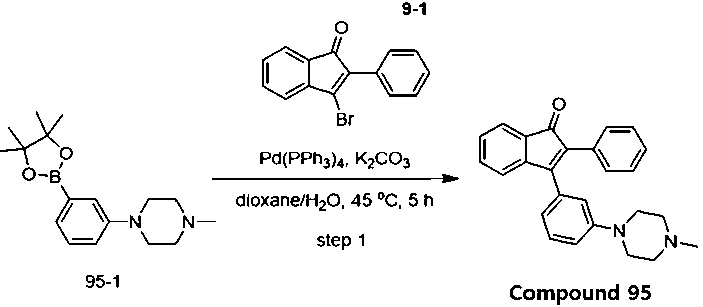

In the same manner as Step 5 of Preparation Example 21, the desired title compound (60 mg, 46 %) was obtained as a white solid.

The obtained Compound 95 (Formula 96 below) was confirmed by LCMS(ESI-MS) and ¹H NMR, which yielded the following data:

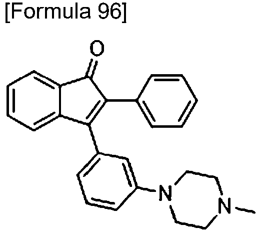

LCMS (ESI-MS): mass calcd. C₂₆H₂₄N₂O 380.2 m/z, found 381.1 [M+H]⁺.

¹H NMR (400 MHz, DMSO-*d*₆) δ 7.55 - 7.49 (m, 2H), 7.38 (t, *J* = 7.4 Hz, 1H), 7.33 - 7.27 (m, 4H), 7.22 - 7.20 (m, 3H), 7.04 - 7.01 (m, 1H), 6.87 (s, 1H), 6.79 (d, *J =* 7.4 Hz, 1H), 3.07 - 3.00 (m, 4H), 2.40 - 2.35 (m, 4H), 2.19 (s, 3H).

### Preparation Example 95. 3-(3,5-di-tert-butylphenyl)-2-phenyl-1H-inden-1-one (Compound 96)

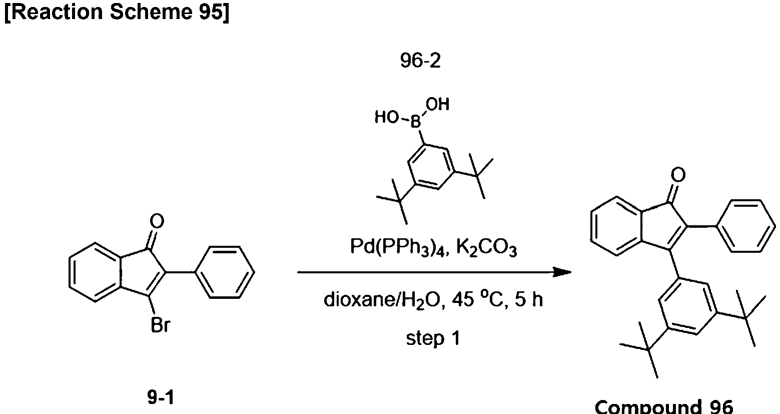

In the same manner as Step 5 of Preparation Example 21, the desired title compound (40 mg, 17 %) was obtained as a white solid.

The obtained Compound 96 (Formula 97 below) was confirmed by LCMS(ESI-MS) and ¹H NMR, which yielded the following data:

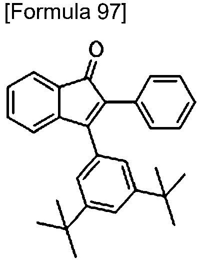

LCMS (ESI-MS): mass calcd. C₂₉H₃₀O 394.2 m/z, found 395.2 [M+H]⁺.

¹H NMR (400 MHz, CDCl₃) δ 7.59 (d, *J* = 7.0 Hz, 1H), 7.43 - 7.37 (m, 2H), 7.31 - 7.28 (m, 1H), 7.26 - 7.22 (m, 6H), 7.21 (d, *J* = 1.6 Hz, 2H), 1.24 (s, 18H).

### Preparation Example 96. 4,6-dimethoxy-2,3-diphenyl-1H-inden-1-one (Compound 97)

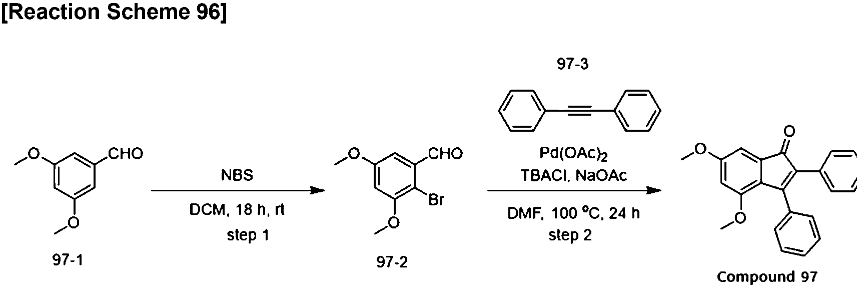

### Step 1: Synthesis of 2-bromo-3,5-dimethoxybenzaldehyde

In the same manner as Step 4 of Preparation Example 79, the desired title compound (400 mg, 48 %) was obtained as a yellow solid.

LCMS (ESI-MS): mass calcd. C₉H₉BrO₃ 244.0 m/z, found 244.9 [M+H]⁺.

### Step 2: Synthesis of 6-dimethoxy-2,3-diphenyl-1H-inden-1-one (Compound 97)

In the same manner as Step 3 of Preparation Example 78, the desired title compound (80 mg, 25 %) was obtained as a yellow solid.

The obtained Compound 97 (Formula 98 below) was confirmed by LCMS(ESI-MS) and ¹H NMR, which yielded the following data:

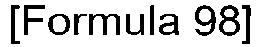

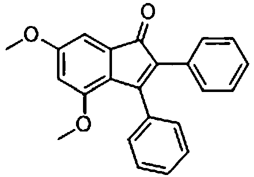

LCMS (ESI-MS): mass calcd. C₂₃H₁₈O₃ 342.1 m/z, found 343.1 [M+H]⁺.

¹H NMR (400 MHz, DMSO-*d*₆) δ 7.37 - 7.27 (m, 5H), 7.21 - 7.18 (m, 3H), 7.04 - 7.01 (m,2H), 6.80 (d, *J* = 2.2 Hz, 1H), 6.67 (d, *J* = 2.2 Hz, 1H), 3.86 (s, 3H), 3.57 (s, 3H).

### Preparation Example 97. 6,7-diphenyl-5H-cyclopenta[b]pyridin-5-one (Compound 98)

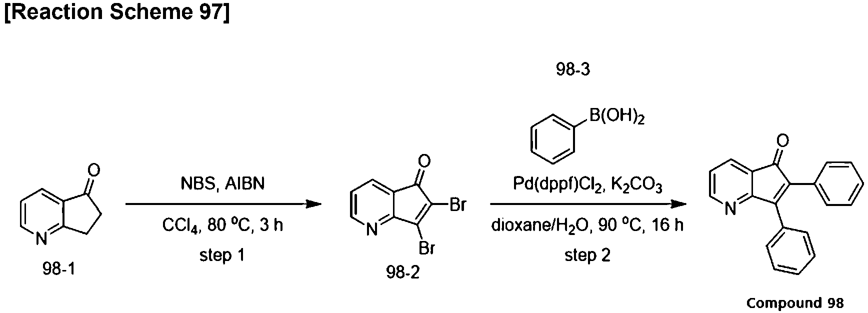

### Step 1: Synthesis of 6,7-dibromo-5H-cyclopenta[b]pyridin-5-one

In the same manner as Step 1 of Preparation Example 41, the desired title compound (350 mg, 11 %) was obtained as a yellow solid.

MS (ESI): mass calcd. C₈H₃Br₂NO 288.9 m/z, found 289.8 [M+H]⁺.

### Step 2: Synthesis of 6,7-diphenyl-5H-cyclopenta[b]pyridin-5-one (Compound 98)

In the same manner as Step 5 of Preparation Example 21, the desired title compound (60 mg, 16 %) was obtained as a yellow solid.

The obtained Compound 98 (Formula 99 below) was confirmed by LCMS(ESI-MS) and ¹H NMR, which yielded the following data:

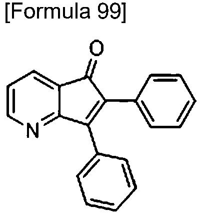

MS (ESI): mass calcd. C₁₂H₁₃NO 283.1 m/z, found 284.0 [M+H]⁺.

¹H NMR (400 MHz, CDCl₃) δ 8.58 - 8.56 (m, 1H), 7.84 - 7.82 (m, 1H), 7.64 - 7.61 (m, 2H), 7.42 - 7.40 (m, 3H), 7.33 - 7.31 (m, 5H), 7.23 - 7.20 (m, 1H).

### Preparation Example 98. N-methyl-2-(1-oxo-3-(o-tolyl)-1H-inden-2-yl)benzamide (Compound 99)

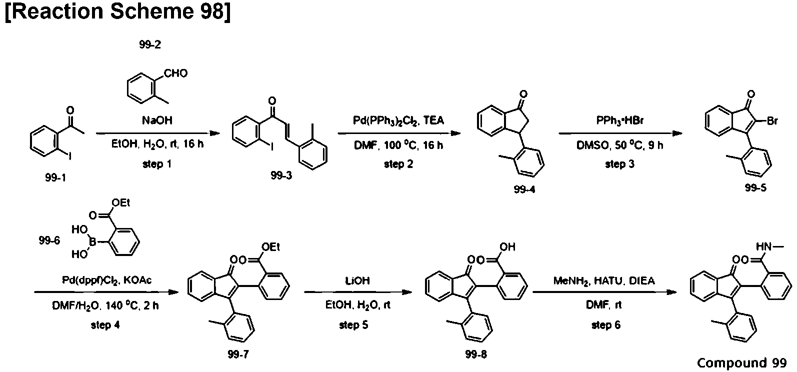

### Step 1: Synthesis of (E)-1-(2-iodophenyl)-3-(o-tolyl)prop-2-en-1-one

In the same manner as Step 1 of Preparation Example 21, the desired title compound (6 g, 76 %) was obtained as a white solid. LCMS (ESI-MS): mass calcd. C₁₆H₁₃IO 348.0 m/z, found 348.6 [M+H]⁺.

### Step 2: Synthesis of 3-(o-tolyl)-2,3-dihydro-1H-inden-1-one

A mixture of (E)-1-(2-iodophenyl)-3-(o-tolyl)prop-2-en-1-one (6.7 g, 0.0192 mol), Pd(PPh₃)₂Cl₂ (0.67 g, 0.00096 mol), TEA (5.83 g, 0.0576 mol), and DMF (100 mL) was stirred at 100 °C for 16 hours. Water (200 mL) was added to the reaction mixture, and the mixture was extracted with EA (3 x 300 mL). Using a conventional method, the organic layer was washed, dried, and concentrated under reduced pressure. The residue was purified by silica gel column chromatography (10 % EtOAc/hexane) to obtain the desired title compound (2.9 g, 60 %) as a yellow solid.

LCMS (ESI-MS): mass calcd. C₁₆H₁₄O 222.1 m/z, found 223.1 [M+H]⁺.

### Step 3: Synthesis of 2-bromo-3-(2-methylphenyl)inden-1-one

In the same manner as Step 1 of Preparation Example 6, the desired title compound (1.8 g, 60 %) was obtained as a yellow solid.

LCMS (ESI-MS): mass calcd. C₁₆H₁₁BrO 298.0 m/z, found 298.6 [M+H]⁺.

### Step 4: Synthesis of ethyl 2-(1-oxo-3-(o-tolyl)-1H-inden-2-yl)benzoate

A mixture of 2-bromo-3-(2-methylphenyl)inden-1-one (2.6 g, 0.0087 mmol), ethyl 2-(dihydroxyboranyl)benzoate (3.38 g, 0.0174 mol), Pd(dppf)Cl₂ (0.32 g, 0.0004 mol), potassium acetate (2.56 g, 0.0261 mol), and DMF/H₂O (20 mL / 4 mL) was stirred at 140 °C for 2 hours. Water (60 mL) was added to the reaction mixture, and the mixture was extracted with EA (3 x 100 mL). Using a conventional method, the organic layer was washed, dried, and concentrated under reduced pressure. The residue was purified by silica gel column chromatography (5 % MeOH/DCM) to obtain the desired title compound (0.5 g, 13 %) as a yellow solid.

LCMS (ESI-MS): mass calcd. C₂₅H₂₀O₃ 368.1 m/z, found 368.8 [M+H]⁺.

### Step 5: Synthesis of 2-(1-oxo-3-(o-tolyl)-1H-inden-2-yl)benzoic acid

A mixture of ethyl 2-(1-oxo-3-(o-tolyl)-1H-inden-2-yl)benzoate (400 mg, 1.0857 mmol), LiOH (234 mg, 9.7713 mmol), and EtOH/H₂O (20 mL / 2 mL) was stirred at room temperature for 3 hours. The pH of the mixture was adjusted to 6 with 1M HCl and then filtered. The resulting filter cake was washed with 50 mL of DCM. In a conventional manner, the organic layer was washed, dried, and concentrated under reduced pressure. The resulting residue was purified by prep-HPLC (Gemini 5 µm C₁₈ column, 150 x 21.2 mm, 30 - 90 % ACN/H₂O with 0.1 % NH₃H₂O) to obtain the desired title compound (140 mg, 34 %) as a yellow solid.

LCMS (ESI-MS): mass calcd. C₂₃H₁₆O₃ 340.1 m/z, found 340.8 [M+H]⁺.

### Step 6: Synthesis of N-methyl-2-(1-oxo-3-(o-tolyl)-1H-inden-2-yl)benzamide (Compound 99)

A mixture of 2-(1-oxo-3-(o-tolyl)-1H-inden-2-yl)benzoic acid (90 mg, 0.2644 mmol), MeNH₂ (8.21 mg, 0.2644 mmol), HATU (130.69 mg, 0.3437 mmol), DIEA (102.51 mg, 0.7932 mmol), and DMF (6 mL) was stirred at room temperature for 16 hours under a nitrogen atmosphere. A small amount of water was added to the reaction mixture, and the mixture was extracted with EA (3 x 40 mL). Using a conventional method, the organic layer was washed, dried, and concentrated under reduced pressure. The residue was purified by silica gel column chromatography (5 % MeOH/DCM) and then by prep-HPLC (Gemini 5 µm C18 column, 150 x 21.2 mm, 50 - 95 % ACN/H₂O with 0.05% FA) to obtain the desired title compound (10 mg, 9 %) as a white solid.

The obtained Compound 99 (Formula 100 below) was confirmed by LCMS (ESI-MS) and ¹H NMR, which yielded the following data:

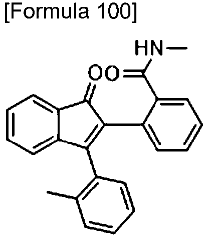

LCMS (ESI-MS): mass calcd. C₂₄H₁₉NO₂ 353.1 m/z, found 354.0 [M+H]⁺.

¹H NMR (400 MHz, CDCl₃) δ 8.57 (s, 1H), 8.22- 8.20 (m, 1H), 7.72 - 7.70 (m, 1H), 7.35 - 7.22 (m, 6H), 7.21 - 7.13 (m, 2H), 6.89 (s, 1H), 3.88 (s, 3H), 1.95 - 1.36 (m, 4H).

### Preparation Example 99. 6-(4-benzylpiperazin-1-yl)-3-(4-methylthiazol-5-yl)-2-(pyridin-3-yl)-1H-inden-1-one (Compound 100)

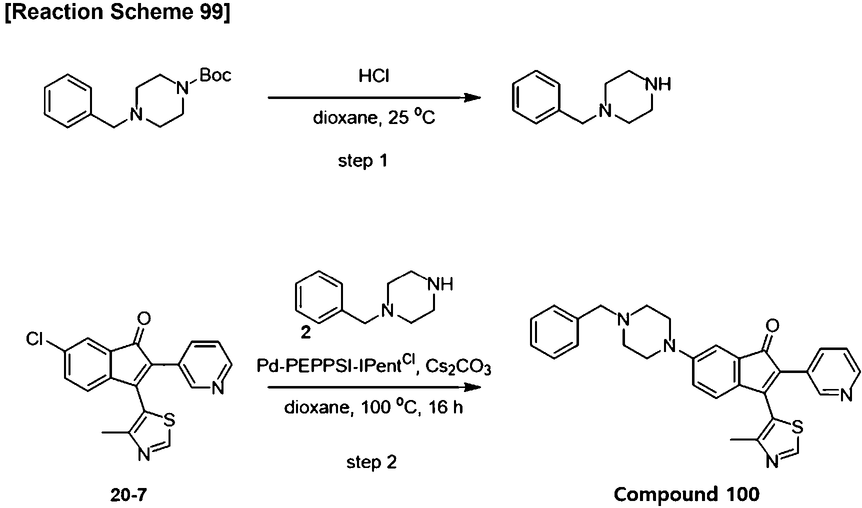

### Step 1: Synthesis of 1-benzylpiperazine

To tert-butyl 4-benzylpiperazine-1-carboxylate (200 mg, 0.721 mmol) was added HCl-1,4-dioxane (5 mL), and the mixture was stirred at 25 °C for 1 hour under a nitrogen atmosphere. The reaction was quenched by the addition of NH₃-MeOH (5 mL), water (10 mL) was added, and the mixture was extracted with EA (3 x 10 mL). The organic layer was washed, dried, and concentrated under reduced pressure in a conventional manner to obtain the title compound (900 mg, 77 %) as a white solid.

LCMS (ESI-MS): mass calcd. C₁₁H₁₆N₂ 176.1 m/z, found 177.1 [M+H]⁺.

### Step 2: Synthesis of 6-(4-benzylpiperazin-1-yl)-3-(4-methylthiazol-5-yl)-2-(pyridin-3-yl)-1H-inden-1-one (Compound 100)

A mixture of 6-chloro-3-(4-methylthiazol-5-yl)-2-(pyridin-3-yl)-1H-inden-1-one (80 mg, 0.236 mmol), 1-benzylpiperazine (62.42 mg, 0.354 mmol), Pd-PEPPSI-IPentCI (22.97 mg, 0.023 mmol), and Cs₂CO₃ (153.85 mg, 0.472 mmol) in 1,4-dioxane (4 mL) was stirred at 100 °C for 16 hours under a nitrogen atmosphere. Water (10 mL) was added to the reaction mixture, and the mixture was extracted with EA (3 x 10 mL). Using a conventional method, the organic layer was washed, dried, and concentrated under reduced pressure. The residue was purified by prep-HPLC (Gemini 5 µm C₁₈ column, 150 x 21.2 mm, 70 - 95 % MeCN/H₂O with 0.1 % FA) to obtain the desired title compound (35.2 mg, 29 %) as a blue solid.

The obtained Compound 100 (Formula 101 below) was confirmed by LCMS(ESI-MS) and ¹H NMR, which yielded the following data:

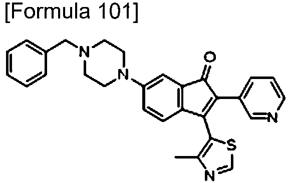

LCMS (ESI-MS): mass calcd. C₂₉H₂₆N₄OS 478.2 m/z, found 478.9 [M+H]⁺.

¹H NMR (400 MHz, CDCl₃) δ 8.92 (s, 1H), 8.48 - 8.46 (m, 2H), 7.65 - 7.63 (m, 1H), 7.41 - 7.38 (m, 5H), 7.25 (s, 2H), 7.01 - 6.99 (m, 1H), 6.76 - 6.74 (m, 1H), 3.68 (s, 2H), 3.39 (s, 4H), 2.70 (s, 4H), 1.71 (s, 3H).

### Preparation Example 100. 3-(4-methylthiazol-5-yl)-6-((3-phenylpiperidin-1-yl)methyl)-2-(pyridin-3-yl)-1H-inden-1-one (Compound 101)

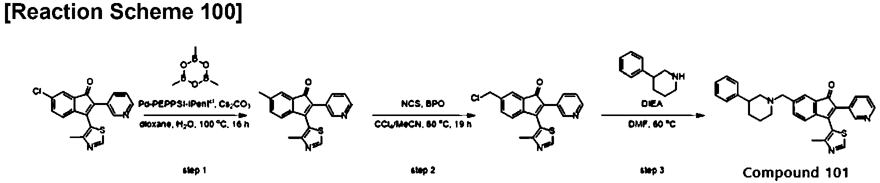

### Step 1: Synthesis of 6-methyl-3-(4-methylthiazol-5-yl)-2-(pyridin-3-yl)-1H-inden-1-one

A mixture of 6-chloro-3-(4-methylthiazol-5-yl)-2-(pyridin-3-yl)-1H-inden-1-one (1.5 g, 0.004 mol), 2,4,6-trimethyl-1,3,5,2,4,6-trioxatriborinane (1.66 g, 0.013 mol), Pd-PEPPSI-IPentCl (0.43 g, 0.0004 mol), and Cs₂CO₃ (2.87 g, 0.008 mol) in dioxane (70 mL) / H₂O (20 mL) was stirred at 100 °C for 16 hours under a nitrogen atmosphere. Water (50 mL) was added to the reaction mixture, and the mixture was extracted with EA (3 x 80 mL). Using a conventional method, the organic layer was washed, dried, and concentrated under reduced pressure. The residue was purified by silica gel column chromatography (20 % EtOAc/hexane) to obtain the desired title compound (1.1 g, 70 %) as a yellow solid.

LCMS (ESI-MS): mass calcd. C₁₉H₁₄N₂OS 318.1 m/z, found 318.7 [M+H]⁺.

### Step 2: Synthesis of 6-(chloromethyl)-3-(4-methylthiazol-5-yl)-2-(pyridin-3-yl)-1H-inden-1-one

To a solution of 6-methyl-3-(4-methylthiazol-5-yl)-2-(pyridin-3-yl)-1H-inden-1-one (1 g, 0.003 mol) in CCl₄ (21 mL) / MeCN (7 mL) were added NCS (0.79 g, 0.003 mol) and benzoyl peroxide (0.15 g, 0.0006 mol). The reaction mixture was stirred at 80 °C for 16 hours under a nitrogten atmosphere.

Water (30 mL) was added to the reaction mixture, and the mixture was extracted with EA (3 x 50 mL). Using a conventional method, the organic layer was washed, dried, and concentrated under reduced pressure. The residue was purified by silica gel column chromatography (50 % EtOAc/hexane) to obtain the desired title compound (200 mg, 12.9 %) as a yellow solid.

LCMS (ESI-MS): mass calcd. C₁₉H₁₃ClN₂OS 352.0 m/z, found 352.7 [M+H]⁺.

### Step 3: Synthesis of 3-(4-methylthiazol-5-yl)-6-((3-phenylpiperidin-1-yl)methyl)-2-(pyridin-3-yl)-1H-inden-1-one (Compound 101)

To a solution of 6-(chloromethyl)-3-(4-methylthiazol-5-yl)-2-(pyridin-3-yl)-1H-inden-1-one (50 mg, 0.141 mmol) in DMF (3 mL) were added 3-phenylpiperidine (27.42 mg, 0.170 mol) and DIEA (54.94 mg, 0.425 mol), and the mixture was stirred at 60 °C for 1 hour under a nitrogen atmosphere. Water (5 mL) was added to the reaction mixture, and the mixture was extracted with EA (3 x 8 mL). Using a conventional method, the organic layer was washed, dried, and concentrated under reduced pressure. The residue was purified by prep-HPLC (Gemini 5 µm C₁₈ column, 150 x 21.2 mm, 70 - 95 % MeCN/H₂O with 0.1% FA) to obtain the desired title compound (2.7 mg, 4 %) as a red solid.

The obtained Compound 101 (Formula 102 below) was confirmed by LCMS(ESI-MS) and ¹H NMR, which yielded the following data:

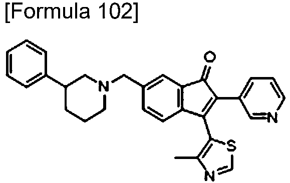

LCMS (ESI-MS): mass calcd. C₃₀H₂₇N₃OS 477.2 m/z, found 478.0 [M+H]⁺.

¹H NMR (400 MHz, DMSO-*d6*) δ 9.27 (s, 1H), 8.46 - 8.48 (m, 1H), 8.39 - 8.40 (m, 1H), 7.64 - 7.62 (m, 1H), 7.47 (s, 1H), 7.39 - 7.31 (m, 2H), 7.21 - 7.13 (m, 3H), 7.06 - 7.04 (m, 1H), 6.92 (s, 2H), 3.64 (s, 2H), 2.58 (s, 3H), 2.39 (s, 3H), 1.90 (s, 2H), 1.57 - 1.50 (m, 2H), 1.27 - 1.24 (m, 2H).

### Example 1: Amyloid-Beta and Tau Disaggregation Test

### 1.1. Preparation of Samples

Each compound was dissolved in DMSO to a concentration of 10 mM. Then, the compounds were diluted with 6 % DMSO (in DW) for use.

Amyloid-beta and tau solutions were prepared by dissolving Aβ₁₋₄₂ or Tau-RD3 (Tau repeat domain 3) monomers in DMSO to a concentration of 10 mM. Then, the solutions were diluted to 100 µM with DW and stored on ice.

Meanwhile, Thioflavin-T was dissolved in 50 mM glycine buffer (pH 8.5) to a concentration of 5 mM. Then, the solution was diluted to 5 µM with 50 mM glycine buffer (pH 8.5) and stored in the dark, protected from light.

### 1.2. Amyloid-Beta and Tau Disaggregation Test by Thioflavin-T Analysis

The amyloid-beta and tau solutions prepared in Example 1.1 were each placed in 1.5 mL microcentrifuge tubes to a concentration of 50 µM or 35 µM, respectively, and then reacted at 37 °C for 24 hours.

To each 1.5 mL microcentrifuge tube in which the amyloid-beta and tau aggregation was complete, the compounds were added at concentrations ranging from 500 µM down to 0 µM by serial dilution, and then further reacted at 37 °C for 48 hours.

25 µL of each completed reaction solution was added to each well of a 96-well fluorescence analysis plate, and 75 µL of the Thioflavin-T solution was added to each well. After reacting for 5 minutes at room temperature in the dark, the fluorescence value was measured at 450 nm (excitation) and 485 nm (emission) using a multi-mode microplate reader.

The fluorescence value of the group (control group) treated with only amyloid-beta or tau and aggregated for 72 hours was designated as 100 %. The fluorescence values measured after treating the amyloid-beta and tau aggregates (formed by reacting for 24 hours) with each compound by concentration and reacting for an additional 48 hours were converted to relative values. The results are shown in Table 1, respectively.

Table 1 shows the amyloid-beta disaggregation effect (Control group: Aβ1-42 50 µM treated group, aggregated for 72 hours; Treated group: Aβ1-42 50 µM aggregated for 24 hours + treated with each compound by concentration and reacted for 48 hours) and the tau disaggregation effect (Control group: Tau-RD3 35 µM treated group, aggregated for 72 hours; Treated group: Tau-RD3 35 µM aggregated for 24 hours + treated with each compound by concentration and reacted for 48 hours) according to the treatment concentration (0 - 500 µM) of each compound and each compound intermediate. The concentration capable of disaggregating 50 % of the amyloid-beta and tau aggregates compared to the control group (EC₅₀) upon treatment with each compound and each compound intermediate was determined and indicated by the following symbols according to its potency, which means that each compound exhibits a concentration-dependent disaggregation effect:
+++ : Strong effect (EC₅₀ ≤ 20 µM);
++ : Moderate effect (20 < EC₅₀ ≤ 100 µM);
+: Weak effect (100 < EC₅₀ ≤ 250 µM); and
-: No effect (250 µM < EC₅₀).

As shown in Table 1 below, it was confirmed that when Compounds 1 to 99 and their intermediates (Compounds 5-4, 6-2, 12-3, 23-10, 39-3, 40-4, 41-4, 42-6, 42-7, 44-8, 45-8, 72-7, 73-7, 74-7, 76-1, 83-5, 83-6, 99-7, and 99-8) were administered, an amyloid-beta disaggregation and/or tau disaggregation effect was observed.

In particular, when treated with Compounds 13, 20, 21, 23, 27, 28, 30-33, 46, 47, 57, 63, 65, 66, 70, 72, 74-77, 83, 84, 90; and the compound intermediates, Compounds 5-4, 74-7, and 76-1, a remarkably excellent disaggregation effect was exhibited for both amyloid-beta protein aggregates and tau protein aggregates.

That is, according to the above results, it was confirmed that the compounds of Formula 1 exhibit a remarkably excellent effect on amyloid-beta disaggregation and/or tau disaggregation, and thus can be utilized for the prevention, improvement, or treatment of neurodegenerative diseases.

**[Table 1]**

| **Compound No.** | **EC₅₀ (µM)** | | **Compound No.** | **EC₅₀ (µM)** | |
|---|---|---|---|---|---|
| | **Aβ** | **Tau** | | **Aβ** | **Tau** |
| **1** | +++ | - | **61** | - | - |
| **2** | +++ | - | **62** | - | - |
| **3** | +++ | - | **63** | +++ | +++ |
| **4** | ++ | - | **64** | ++ | + |
| **5** | ++ | - | **65** | +++ | +++ |
| **6** | ++ | - | **66** | +++ | +++ |
| **7** | +++ | - | **67** | - | - |
| **8** | + | ++ | **68** | - | - |
| **9** | ++ | ++ | **69** | +++ | - |
| **10** | - | - | **70** | +++ | +++ |
| **11** | + | - | **71** | ++ | + |
| **12** | +++ | - | **72** | +++ | +++ |
| **13** | +++ | +++ | **73** | + | + |
| **14** | +++ | - | **74** | +++ | +++ |
| **15** | ++ | - | **75** | +++ | +++ |
| **16** | ++ | - | **76** | +++ | +++ |
| **17** | +++ | - | **77** | +++ | +++ |
| **18** | +++ | - | **78** | + | - |
| **19** | - | + | **79** | ++ | ++ |
| **20** | +++ | +++ | **80** | ++ | + |
| **21** | +++ | +++ | **81** | ++ | ++ |
| **22** | - | ++ | **82** | +++ | ++ |
| **23** | +++ | +++ | **83** | +++ | +++ |
| **24** | - | +++ | **84** | +++ | +++ |
| **25** | +++ | - | **85** | - | - |
| **26** | +++ | - | **86** | ++ | +++ |
| **27** | +++ | +++ | **87** | +++ | ++ |
| **28** | +++ | +++ | **88** | +++ | ++ |
| **29** | - | - | **89** | ++ | + |
| **30** | +++ | +++ | **90** | +++ | +++ |
| **31** | +++ | +++ | **91** | +++ | - |
| **32** | +++ | +++ | **92** | ++ | ++ |
| **33** | +++ | +++ | **93** | ++ | ++ |
| **34** | - | + | **94** | +++ | ++ |
| **35** | ++ | ++ | **95** | ++ | - |
| **36** | + | ++ | **96** | - | - |
| **37** | ++ | ++ | **97** | +++ | - |
| **38** | +++ | - | **98** | - | - |
| **39** | +++ | - | **99** | - | - |
| **40** | +++ | - | **5-4** | +++ | +++ |
| **41** | +++ | - | **6-2** | ++ | +++ |
| **42** | - | - | **12-3** | + | - |
| **43** | - | - | **23-10** | - | ++ |
| **44** | +++ | - | **39-3** | - | - |
| **45** | +++ | + | **40-4** | - | - |
| **46** | +++ | +++ | **41-4** | - | - |
| **47** | +++ | +++ | **42-6** | ++ | ++ |
| **48** | +++ | - | **42-7** | - | + |
| **49** | +++ | - | **44-8** | + | ++ |
| **50** | - | - | **45-8** | + | - |
| **51** | +++ | ++ | **72-7** | +++ | - |
| **52** | - | - | **73-7** | +++ | - |
| **53** | - | - | **74-7** | +++ | +++ |
| **54** | - | - | **76-1** | +++ | +++ |
| **55** | +++ | - | **83-5** | ++ | ++ |
| **56** | ++ | ++ | **83-6** | - | + |
| **57** | +++ | +++ | **99-7** | + | - |
| **58** | +++ | - | **99-8** | - | ++ |
| **59** | ++ | ++ | | | |
| **60** | ++ | +++ | | | |

### Example 2: Confirmation of Aggregate Reduction Effect by Compound Treatment in an Amyloid-Beta Oligomer-Expressing Cell Line

### 2.1. Preparation of an Amyloid-Beta Oligomer-Overexpressing Cell Line

To observe the change in intracellular amyloid-beta oligomers, a plasmid with the APP E693Δ (Osaka mutation) mutant, which is associated with early-onset Alzheimer's disease and is known to induce intracellular amyloid-beta oligomer formation, was obtained from GenScript. This plasmid was transfected into HEK293 cells using a liposome-mediated method (Lipofectamine) to prepare a cell line that overexpresses intracellular amyloid-beta oligomers.

### 2.2. Compound Treatment

HEK293 cells transfected with the APP E693Δ mutant plasmid were incubated for 48 hours in a 37 °C, 5 % CO₂ incubator, and then treated with the compounds prepared in 1.1, which were diluted to a concentration of 10 µM in the cell culture medium. The untransfected control group was treated with 0.1 % DMSO, the same as the compound-treated group.

The compound-treated cell lines were again incubated for 48 hours in a 37 °C, 5 % CO₂ incubator and then used in the experiment.

### 2.3. Confirmation of Oligomer Change via Dot Blot

After incubating the control and compound-treated cells from Example 2.2, a dot blot was performed using the cell lysate. A 3.5 µg sample was spotted onto a nitrocellulose membrane and air-dried, and then the membrane to which the sample was adhered was blocked with 5 % skim milk. After incubating with the amyloid oligomer antibody A11 to allow binding, the membrane was washed 4 times with TBST, and the secondary antibody was bound. The image signal was measured using a chemiluminescent substrate.

As a result, FIG. 1 shows the change in amyloid-beta oligomers by each compound (dot blot image) and a graph representing the relative change with the untransfected HEK293 cells not treated with any compound as the control group. It was confirmed that the amount of amyloid-beta oligomers was reduced by each compound treatment compared to the control group.

That is, the results of FIG. 1 mean that each compound exhibits an amyloid-beta oligomer reduction effect through the disaggregation of amyloid-beta oligomer aggregates in amyloid-beta oligomer-expressing cells.

According to the above results, it was confirmed that the compounds of Formula 1 exhibit a remarkably excellent effect on amyloid-beta disaggregation and/or tau disaggregation, and thus can be utilized for the prevention, improvement, or treatment of neurodegenerative diseases.

## Claims

1. A compound of Formula 1 or a salt thereof: 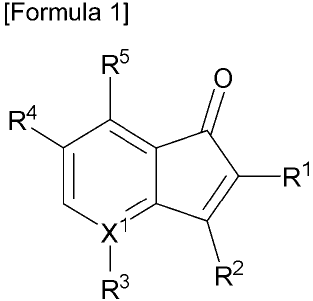 wherein in the Formula 1,
R¹ and R² are each independently hydrogen, halogen, CN, OR^{a}, SR^{a}, CO₂R^{a}, CH₂CO₂R^{a}, CONR^{b}R^{c}, NR^{b}R^{c}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X¹ is carbon or nitrogen;
wherein when the X¹ is carbon, R³ is hydrogen, halogen, CN, OR^{a}, SR^{a}, CO₂R^{a}, CH₂CO₂R^{a}, CONR^{b}R^{c}, NRbRc, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl, and when the X¹ is nitrogen, R³ is absent;
R⁴ and R⁵ are each independently hydrogen, halogen, CN, OR^{a}, SR^{a}, O(CO)R^{a}, CO₂R^{a}, NHR^{a}, NH(CO)R^{a}, CH₂CO₂R^{a}, CONR^{b}R^{c}, NR^{b}R^{c}, X^{a}(CH₂)ₚX^{b}(CH₂)_{q}R^{d}, OCH₂CH=CHR^{d}, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
R^{a}, R^{b}, R^{c}, and R^{d} are each independently hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X^{a} and X^{b} are each independently a direct bond, oxygen, nitrogen, or sulfur; and
p and q are each independently an integer selected from 0 to 2.

2. The compound of claim 1 or a salt thereof, wherein
R¹ and R² are each independently hydrogen, halogen, CN, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl;
X¹ is carbon or nitrogen;
when the X¹ is carbon, R³ is hydrogen, halogen, OR^{a}, or substituted or unsubstituted C₁₋₆ alkyl, and when the X¹ is nitrogen, R³ is absent; R⁴ and R⁵ are each independently hydrogen, halogen, OR^{a}, SR^{a}, O(CO)R^{a}, NHR^{a}, NH(CO)R^{a}, NR^{b}R^{c}, X^{a}(CH₂)ₚX^{b}(CH₂)_{q}R^{d}, substituted or unsubstituted C₁₋₆ alkyl, substituted or unsubstituted 3- to 7-membered cycloalkyl, or substituted or unsubstituted 3- to 7-membered heterocycloalkyl; and
R^{a}, R^{b}, R^{c}, and R^{d} are each independently hydrogen, halogen, substituted or unsubstituted C₁₋₆ alkyl, substituted or unsubstituted 3- to 7-membered cycloalkyl, substituted or unsubstituted 3- to 7-membered heterocycloalkyl, substituted or unsubstituted 5- to 10-membered aryl, or substituted or unsubstituted 5- to 10-membered heteroaryl.

3. The compound of claim 1 or a salt thereof, wherein
R¹ and R² are each independently hydrogen, halogen, CN, substituted or unsubstituted 5- to 7-membered cycloalkyl, substituted or unsubstituted 5- to 7-membered heterocycloalkyl,
substituted or unsubstituted 5- to 6-membered aryl, or substituted or unsubstituted 5- to 6-membered heteroaryl;
X¹ is carbon or nitrogen;
when the X¹ is carbon, R³ is hydrogen, halogen, OR^{a}, or substituted or unsubstituted C₁₋₃ alkyl, and when the X¹ is nitrogen, R³ is absent; R⁴ is hydrogen, halogen, OR^{a}, SR^{a}, O(CO)R^{a}, NHR^{a}, NH(CO)R^{a}, NR^{b}R^{c}, X^{a}(CH₂)ₚX^{b}(CH₂)_{q}R^{d}, substituted or unsubstituted C₁₋₄ alkyl, substituted or unsubstituted 5- to 7-membered cycloalkyl, or substituted or unsubstituted 5- to 7-membered heterocycloalkyl;
R^{a}, R^{b}, R^{c}, and R^{d} are each independently hydrogen, halogen, substituted or unsubstituted C₁₋₄ alkyl, substituted or unsubstituted 3- to 7-membered cycloalkyl, substituted or unsubstituted 3- to 7-membered heterocycloalkyl, substituted or unsubstituted 5- to 6-membered aryl, or substituted or unsubstituted 5- to 6-membered heteroaryl; and
R⁵ is hydrogen or halogen.

4. The compound of claim 1 or a salt thereof, wherein
R¹ and R² are each independently hydrogen, halogen, CN, substituted or unsubstituted 5- to 7-membered cycloalkyl, substituted or unsubstituted 5- to 7-membered heterocycloalkyl, substituted or unsubstituted 5- to 6-membered aryl, or substituted or unsubstituted 5- to 6-membered heteroaryl;
wherein the substituted 5- to 7-membered cycloalkyl, substituted 5-to 7-membered heterocycloalkyl, substituted 5- to 6-membered aryl, and substituted 5- to 6-membered heteroaryl in R¹ and R² are each independently substituted with one or more selected from the group consisting of halogen, C₁₋₄ alkyl, 5- to 6-membered heterocycloalkyl, (C₁₋₆ alkyl)-(5- to 6-membered heterocycloalkyl), (5- to 6-membered heterocycloalkyl)-(C₁₋₆ alkyl), -O-(C₁₋₆ alkyl), (C₁₋₆ alkyl)-(5- to 6-membered heterocycloalkyl)-(C₁₋₆ alkyl), and -(CO)HN(C₁₋₆ alkyl); X¹ is carbon or nitrogen;
when the X¹ is carbon, R³ is hydrogen, halogen, OH, -O-(C₁₋₃ alkyl), or unsubstituted C₁₋₃ alkyl, and when the X¹ is nitrogen, R³ is absent; R⁴ is hydrogen, halogen, OR^{a}, SR^{a}, O(CO)R^{a}, NHR^{a}, NH(CO)R^{a}, NR^{b}R^{c}, X^{a}(CH₂)ₚX^{b}(CH₂)_{q}R^{d}, substituted or unsubstituted C₁₋₄ alkyl, substituted or unsubstituted 5- to 7-membered cycloalkyl, or substituted or unsubstituted 5- to 7-membered heterocycloalkyl;
wherein the substituted C₁₋₄ alkyl, substituted 5- to 7-membered cycloalkyl, and substituted 5- to 7-membered heterocycloalkyl in R⁴ are each independently substituted with one or more selected from the group consisting of halogen, C₁₋₄ alkyl, C₁₋₄ alcohol, (C₁₋₄ alkyl)-(5- to 6-membered aryl), (5- to 6-membered heterocycloalkyl)-(5- to 6-membered aryl), and (5- to 6-membered heteroaryl)-(5- to 6-membered aryl);
R^{a}, R^{b}, R^{c}, and R^{d} are each independently hydrogen, halogen, substituted or unsubstituted C₁₋₄ alkyl, substituted or unsubstituted 3- to 7-membered cycloalkyl, substituted or unsubstituted 3- to 7-membered heterocycloalkyl, substituted or unsubstituted 5- to 6-membered aryl, or substituted or unsubstituted 5- to 6-membered heteroaryl; and
wherein the substituted C₁₋₄ alkyl, substituted 3- to 7-membered cycloalkyl, substituted 3- to 7-membered heterocycloalkyl, substituted 5- to 6-membered aryl, and substituted 5- to 6-membered heteroaryl in R^{a}, R^{b}, R^{c}, and R^{d} are each independently substituted with one or more selected from the group consisting of halogen, OH, 5- to 6-membered heterocycloalkyl, 5- to 6-membered aryl, and 5- to 6-membered heteroaryl.

5. The compound of claim 1 or a salt thereof, wherein
R¹ and R² are each independently hydrogen, halogen, CN, 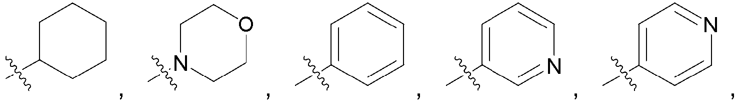 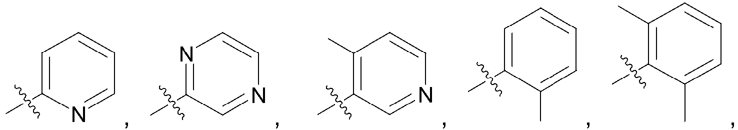 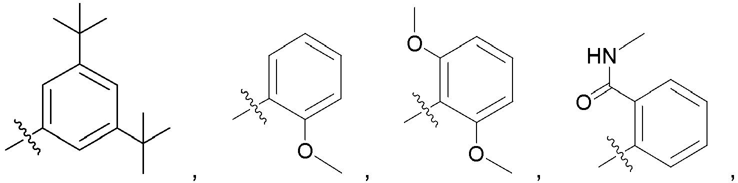 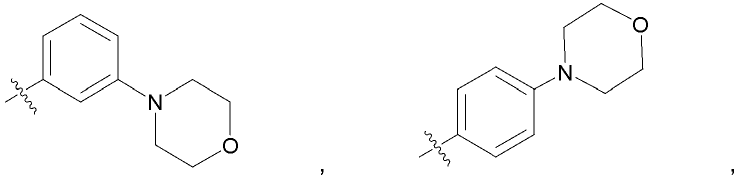 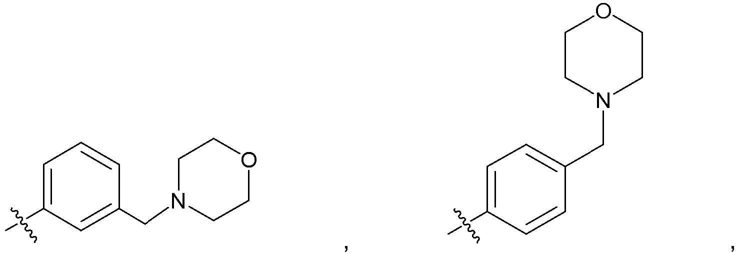 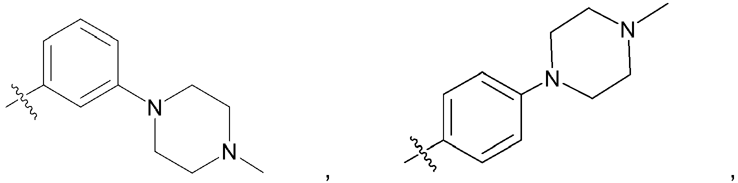 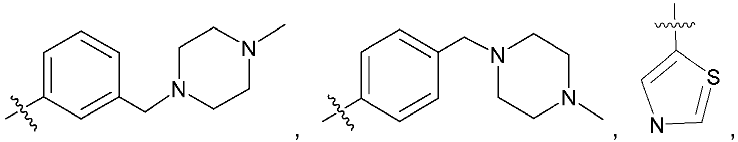 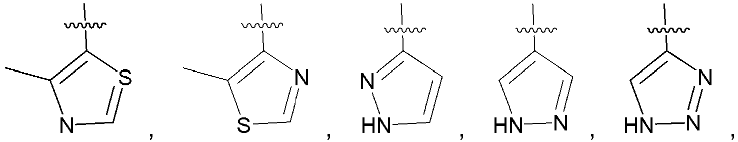 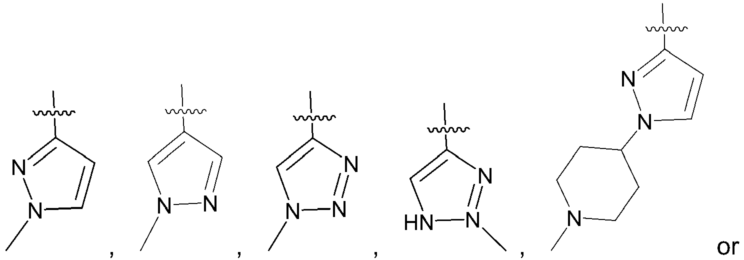 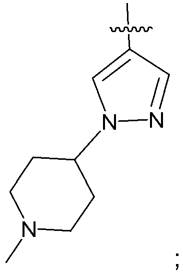
X¹ is carbon or nitrogen;
when the X¹ is carbon, R³ is hydrogen, halogen, -CH₃, -OH, or 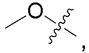 and when the X¹ is nitrogen, R³ is absent; and
R⁴ is hydrogen, halogen, -OH, -NH₂,

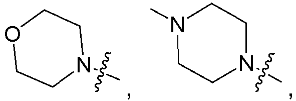

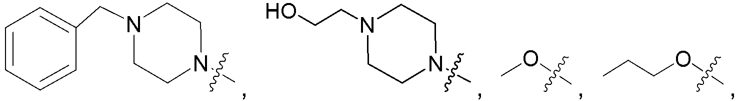

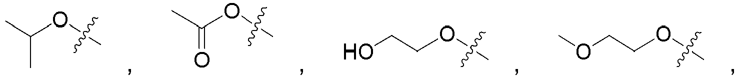

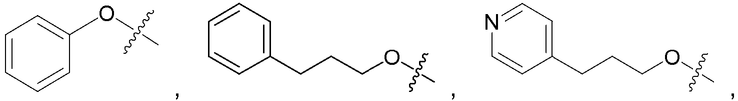

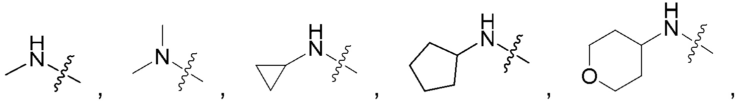

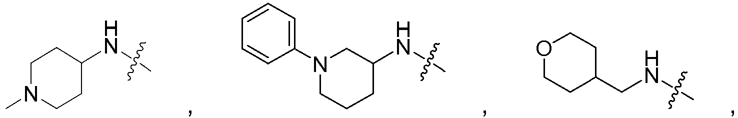

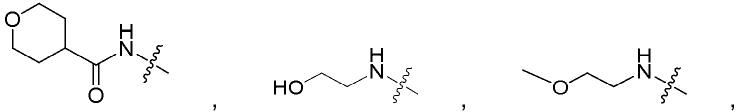

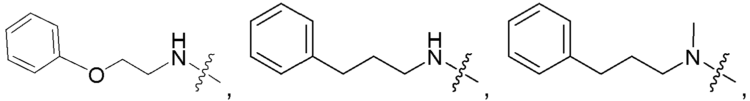

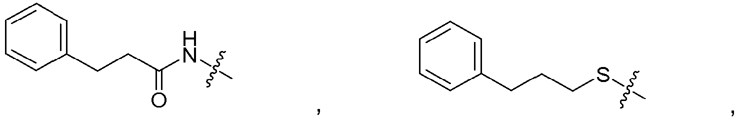

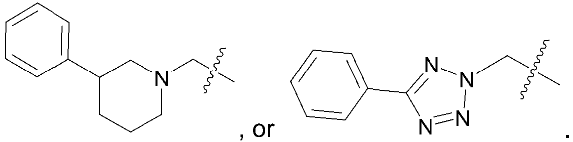

6. The compound of claim 1 or a salt thereof,
wherein the compound or a salt thereof is one selected from the group consisting of the following (1) to (101):
(1) 2,3-diphenylinden-1-one,
(2) 6-methoxy-2,3-diphenyl-1H-inden-1-one,
(3) 2,3-diphenyl-6-propoxy-1H-inden-1-one,
(4) isopropoxy-2,3-diphenyl-1H-inden-1-one,
(5) 2-phenyl-3-(o-tolyl)-1H-inden-1-one,
(6) 3-phenyl-2-(o-tolyl)-1H-inden-1-one,
(7) 3-phenyl-2-(pyridin-3-yl)-1H-inden-1-one,
(8) 2-morpholino-3-phenyl-1H-inden-1-one,
(9) 2-phenyl-3-(pyridin-3-yl)-1H-inden-1-one,
(10) 3-cyclohexyl-2-phenyl-1H-inden-1-one,
(11) 2-cyclohexyl-3-phenyl-1H-inden-1-one,
(12) 6-morpholino-2,3-diphenyl-1H-inden-1-one,
(13) 6-(4-methylpiperazin-1-yl)-2,3-diphenyl-1H-inden-1-one,
(14) 3-(4-morpholinophenyl)-2-phenyl-1H-inden-1-one,
(15) 3-(3-morpholinophenyl)-2-phenyl-1H-inden-1-one,
(16) 3-(4-(morpholinomethyl)phenyl)-2-phenyl-1H-inden-1-one,
(17) 2-(4-morpholinophenyl)-3-phenyl-1H-inden-1-one,
(18) 2-(3-morpholinophenyl)-3-phenyl-1H-inden-1-one,
(19) 3-(4-methylthiazol-5-yl)-2-(pyridin-3-yl)-1H-inden-1-one,
(20) 3-(4-methylthiazol-5-yl)-6-((3-phenylpropyl)amino)-2-(pyridin-3-yl)-1H-inden-1-one,
(21) 2-(4-methylpyridin-3-yl)-3-(4-methylthiazol-5-yl)-6-(3-phenylpropoxy)-1H-inden-1-one,
(22) 6-methoxy-2-(4-methylpyridin-3-yl)-3-(4-methylthiazol-5-yl)-1H-inden-1-one,
(23) 3-(5-methylthiazol-4-yl)-2-(pyridin-3-yl)-6-(3-(pyridin-4-yl)propoxy)-1H-inden-1-one,
(24) 2-bromo-3-(1-methyl-1H-pyrazol-3-yl)-1-oxo-1H-inden-6-yl acetate,
(25) 3-(4-methylthiazol-5-yl)-6-(3-phenylpropoxy)-2-(1H-pyrazol-3-yl)-1H-inden-1-one,
(26) 2-(1-methyl-1H-pyrazol-3-yl)-3-(4-methylthiazol-5-yl)-6-(3-phenylpropoxy)-1H-inden-1-one,
(27) 3-(4-methylthiazol-5-yl)-6-(3-phenylpropoxy)-2-(1H-pyrazol-4-yl)-1H-inden-1-one,
(28) 2-(1-methyl-1H-pyrazol-4-yl)-3-(4-methylthiazol-5-yl)-6-(3-phenylpropoxy)-1H-inden-1-one,
(29) 2-(1-(1-methylpiperidin-4-yl)-1H-pyrazol-3-yl)-3-(4-methylthiazol-5-yl)-6-(3-phenylpropoxy)-1H-inden-1-one,
(30) 2-(1-(1-methylpiperidin-4-yl)-1H-pyrazol-4-yl)-3-(4-methylthiazol-5-yl)-6-(3-phenylpropoxy)-1H-inden-1-one,
(31) 3-(4-methylthiazol-5-yl)-6-(3-phenylpropoxy)-2-(1H-1,2,3-triazol-4-yl)-1H-inden-1-one,
(32) 2-(1-methyl-1H-1,2,3-triazol-4-yl)-3-(4-methylthiazol-5-yl)-6-(3-phenylpropoxy)-1H-inden-1-one,
(33) 2-(2-methyl-2H-1,2,3-triazol-4-yl)-3-(4-methylthiazol-5-yl)-6-(3-phenylpropoxy)-1H-inden-1-one,
(34) 3-(4-methylthiazol-5-yl)-1-oxo-6-(3-phenylpropoxy)-1H-indene-2-carbonitrile,
(35) 6-hydroxy-3-(4-methylthiazol-5-yl)-2-(pyridin-3-yl)-1H-inden-1-one,
(36) 6-(2-hydroxyethoxy)-3-(4-methylthiazol-5-yl)-2-(pyridin-3-yl)-1H-inden-1-one,
(37) 6-(2-methoxyethoxy)-3-(4-methylthiazol-5-yl)-2-(pyridin-3-yl)-1H-inden-1-one,
(38) 6-(methyl(3-phenylpropyl)amino)-3-(4-methylthiazol-5-yl)-2-(pyridin-3-yl)-1H-inden-1-one,
(39) 3-(4-methylthiazol-5-yl)-6-(3-phenylpropoxy)-2-(pyridin-4-yl)-1H-inden-1-one,
(40) 3-(4-methylthiazol-5-yl)-6-(3-phenylpropoxy)-2-(pyridin-2-yl)-1H-inden-1-one,
(41) 3-(4-methylthiazol-5-yl)-6-(3-phenylpropoxy)-2-(pyrazin-2-yl)-1H-inden-1-one,
(42) 3-(1-methyl-1H-pyrazol-4-yl)-6-(3-phenylpropoxy)-2-(pyridin-3-yl)-1H-inden-1-one,
(43) 6-(3-phenylpropoxy)-3-(1H-pyrazol-4-yl)-2-(pyridin-3-yl)-1H-inden-1-one,
(44) 3-(2-methoxyphenyl)-6-(3-phenylpropoxy)-2-(pyridin-3-yl)-1H-inden-1-one,
(45) 3-(2,6-dimethoxyphenyl)-6-(3-phenylpropoxy)-2-(pyridin-3-yl)-1H-inden-1-one,
(46) 4-methyl-3-(4-methylthiazol-5-yl)-6-(3-phenylpropoxy)-2-(pyridin-3-yl)-1H-inden-1-one,
(47) 4-methoxy-3-(4-methylthiazol-5-yl)-6-(3-phenylpropoxy)-2-(pyridin-3-yl)-1H-inden-1-one,
(48) 4-hydroxy-3-(4-methylthiazol-5-yl)-6-(3-phenylpropoxy)-2-(pyridin-3-yl)-1H-inden-1-one,
(49) 6-(cyclopentylamino)-3-(4-methylthiazol-5-yl)-2-(pyridin-3-yl)-1H-inden-1-one,
(50) 6-(methylamino)-3-(4-methylthiazol-5-yl)-2-(pyridin-3-yl)-1H-inden-1-one,
(51) 6-((1-methylpiperidin-4-yl)amino)-3-(4-methylthiazol-5-yl)-2-(pyridin-3-yl)-1H-inden-1-one,
(52) 3-(4-methylthiazol-5-yl)-2-(pyridin-3-yl)-6-(((tetrahydro-2H-pyran-4-yl)methyl)amino)-1H-inden-1-one,
(53) 6-((2-hydroxyethyl)amino)-3-(4-methylthiazol-5-yl)-2-(pyridin-3-yl)-1H-inden-1-one,
(54) 6-((2-methoxyethyl)amino)-3-(4-methylthiazol-5-yl)-2-(pyridin-3-yl)-1H-inden-1-one,
(55) 3-(4-methylthiazol-5-yl)-6-((2-phenoxyethyl)amino)-2-(pyridin-3-yl)-1H-inden-1-one,
(56) 3-(4-methylthiazol-5-yl)-2-(pyridin-3-yl)-6-((tetrahydro-2H-pyran-4-yl)amino)-1H-inden-1-one,
(57) 3-(4-methylthiazol-5-yl)-6-((3-phenylpropyl)thio)-2-(pyridin-3-yl)-1H-inden-1-one,
(58) 3-(4-methylthiazol-5-yl)-6-((1-phenylpiperidin-3-yl)amino)-2-(pyridin-3-yl)-1H-inden-1-one,
(59) 6-amino-3-(4-methylthiazol-5-yl)-2-(pyridin-3-yl)-1H-inden-1-one,
(60) N-(3-(4-methylthiazol-5-yl)-1-oxo-2-(pyridin-3-yl)-1H-inden-6-yl)-3-phenylpropanamide,
(61) N-(3-(4-methylthiazol-5-yl)-1-oxo-2-(pyridin-3-yl)-1H-inden-6-yl)tetrahydro-2H-pyran-4-carboxamide,
(62) 6-(dimethylamino)-3-(4-methylthiazol-5-yl)-2-(pyridin-3-yl)-1H-inden-1-one,
(63) 6-(cyclopropylamino)-3-(4-methylthiazol-5-yl)-2-(pyridin-3-yl)-1H-inden-1-one,
(64) 6-(4-methylpiperazin-1-yl)-3-(4-methylthiazol-5-yl)-2-(pyridin-3-yl)-1H-inden-1-one,
(65) 6-(methyl(3-phenylpropyl)amino)-3-(4-methylthiazol-5-yl)-2-(pyridin-3-yl)-1H-inden-1-one,
(66) 3-(4-methylthiazol-5-yl)-6-phenoxy-2-(pyridin-3-yl)-1H-inden-1-one,
(67) 6-(methylamino)-2,3-diphenyl-1H-inden-1-one,
(68) 6-amino-2,3-diphenyl-1H-inden-1-one,
(69) 6-(dimethylamino)-2,3-diphenyl-1H-inden-1-one,
(70) 6-(4-(2-hydroxyethyl)piperazin-1-yl)-2,3-diphenyl-1H-inden-1-one,
(71) 4-methyl-6-(4-methylpiperazin-1-yl)-2,3-diphenyl-1H-inden-1-one,
(72) 3-(2,6-dimethylphenyl)-6-(4-methylpiperazin-1-yl)-2-phenyl-1H-inden-1-one,
(73) 6-(4-methylpiperazin-1-yl)-2-(3-morpholinophenyl)-3-phenyl-1H-inden-1-one,
(74) 6-(4-(2-hydroxyethyl)piperazin-1-yl)-2-(4-(4-methylpiperazin-1-yl)phenyl)-3-(4-methylthiazol-5-yl)-1H-inden-1-one,
(75) 6-(4-methylpiperazin-1-yl)-2-(4-(4-methylpiperazin-1-yl)phenyl)-3-(4-methylthiazol-5-yl)-1H-inden-1-one,
(76) 2-(4-(4-methylpiperazin-1-yl)phenyl)-3-(4-methylthiazol-5-yl)-6-(3-phenylpropoxy)-1H-inden-1-one,
(77) 2,3-bis(4-methylthiazol-5-yl)-6-(3-phenylpropoxy)-1H-inden-1-one,
(78) 2,3-diphenyl-6-(3-phenylpropoxy)-1H-inden-1-one,
(79) 6-(4-methylpiperazin-1-yl)-2,3-bis(4-methylthiazol-5-yl)-1H-inden-1-one,
(80) 6-(4-methylpiperazin-1-yl)-2,3-bis(3-morpholinophenyl)-1H-inden-1-one,
(81) 6-(4-(2-hydroxyethyl)piperazin-1-yl)-2,3-bis(4-methylthiazol-5-yl)-1H-inden-1-one,
(82) 6-hydroxy-2,3-di-o-tolyl-1H-inden-1-one,
(83) 6-(3-phenylpropoxy)-2,3-di(thiazol-5-yl)-1H-inden-1-one,
(84) 3-(2,6-dimethylphenyl)-6-hydroxy-2-(o-tolyl)-1H-inden-1-one,
(85) 2-(4-(4-methylpiperazin-1-yl)phenyl)-3-(4-methylthiazol-5-yl)-1H-inden-1-one,
(86) 2-(4-((4-methylpiperazin-1-yl)methyl)phenyl)-3-(4-methylthiazol-5-yl)-1H-inden-1-one,
(87) 3-(4-methylthiazol-5-yl)-2-(4-(morpholinomethyl)phenyl)-1H-inden-1-one,
(88) 2-(3-((4-methylpiperazin-1-yl)methyl)phenyl)-3-(4-methylthiazol-5-yl)-1H-inden-1-one,
(89) 3-(4-methylthiazol-5-yl)-2-(3-(morpholinomethyl)phenyl)-1H-inden-1-one,
(90) 2-(4-(4-methylpiperazin-1-yl)phenyl)-3-phenyl-1H-inden-1-one,
(91) 2-(3-(4-methylpiperazin-1-yl)phenyl)-3-phenyl-1H-inden-1-one,
(92) 3-(4-((4-methylpiperazin-1-yl)methyl)phenyl)-2-(4-methylthiazol-5-yl)-1H-inden-1-one,
(93) 3-(3-((4-methylpiperazin-1-yl)methyl)phenyl)-2-(4-methylthiazol-5-yl)-1H-inden-1-one,
(94) 3-(4-(4-methylpiperazin-1-yl)phenyl)-2-phenyl-1H-inden-1-one,
(95) 3-(3-(4-methylpiperazin-1-yl)phenyl)-2-phenyl-1H-inden-1-one,
(96) 3-(3,5-di-tert-butylphenyl)-2-phenyl-1H-inden-1-one,
(97) 4,6-dimethoxy-2,3-diphenyl-1H-inden-1-one,
(98) 6,7-diphenyl-5H-cyclopenta[b]pyridin-5-one,
(99) N-methyl-2-(1-oxo-3-(o-tolyl)-1H-inden-2-yl)benzamide,
(100) 6-(4-benzylpiperazin-1-yl)-3-(4-methylthiazol-5-yl)-2-(pyridin-3-yl)-1H-inden-1-one, and
(101) 3-(4-methylthiazol-5-yl)-6-((3-phenylpiperidin-1-yl)methyl)-2-(pyridin-3-yl)-1H-inden-1-one.

7. A composition comprising the compound of claim 1, or a salt thereof.

8. A pharmaceutical composition comprising the compound of claim 1, or a pharmaceutically acceptable salt thereof.

9. A pharmaceutical composition for preventing or treating a neurodegenerative disease, comprising the compound of claim 1, or a pharmaceutically acceptable salt thereof.

10. The pharmaceutical composition for preventing or treating a neurodegenerative brain disease of claim 9, for administration to a subject selected from:
(1) a subject in whom the level of amyloid-beta aggregation is higher than that of a normal individual without a neurodegenerative brain disease, or who is at risk thereof;
(2) a subject in whom the level of tau protein aggregation is higher than that of a normal individual without a neurodegenerative brain disease, or who is at risk thereof;
(3) a subject in whom the level of tau protein phosphorylation is higher than that of a normal individual without a neurodegenerative brain disease, or who is at risk thereof; and
(4) a subject corresponding to one or more of the (1) to (3) above.

11. The pharmaceutical composition for preventing or treating a neurodegenerative brain disease of claim 9,
wherein the neurodegenerative brain disease is one or more selected from the group consisting of dementia, Alzheimer's disease, preclinical Alzheimer's disease, Parkinson's disease, Huntington's disease, mild cognitive impairment, cerebral amyloid angiopathy, Down syndrome, amyloid-related stroke, systemic amyloidosis, Dutch-type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

12. A pharmaceutical composition for inhibiting aggregation of an amyloid-beta protein or disaggregating an aggregate of an amyloid-beta protein, comprising the compound of claim 1, or a pharmaceutically acceptable salt thereof.

13. A pharmaceutical composition for inhibiting aggregation of a tau protein, disaggregating an aggregate of a tau protein, or inhibiting phosphorylation of a tau protein, comprising the compound of claim 1, or a pharmaceutically acceptable salt thereof.

14. A health functional food comprising the compound of claim 1, or a food acceptable salt thereof.

15. A health functional food for preventing or ameliorating a neurodegenerative disease, comprising the compound of claim 1, or a food acceptable salt thereof.

16. A health functional food for inhibiting aggregation of an amyloid-beta protein or disaggregating an aggregate of an amyloid-beta protein, comprising the compound of claim 1, or a food acceptable salt thereof.

17. A health functional food for inhibiting aggregation of a tau protein, disaggregating an aggregate of a tau protein, or inhibiting phosphorylation of a tau protein, comprising the compound of claim 1, or a food acceptable salt thereof.
